# EUROPEAN PATENT APPLICATION

(11) **EP 4 011 874 A1**
(43) Date of publication of application: **15.06.2022**
(21) Application number: 20213018.3
(22) Date of filing: 10.12.2020
(51) Int. Cl.: C07D 401/04, C07D 401/14, A61P 9/10, A61K 31/496

(54) **SUBSTITUTED PYRAZOLO PIPERIDINE CARBOXYLIC ACIDS**

(71) Applicant: Bayer Aktiengesellschaft, 51373 Leverkusen (DE)
(72) Inventor: The designation of the inventor has not yet been filed
(74) Representative: BIP Patents

(57) **Abstract**

The invention relates to substituted pyrazolo piperidine carboxylic acids, their salts and to processes for their preparation, and also to their use for preparing medicaments for the treatment and/or prophylaxis of diseases, in particular cardiovascular and cardiac diseases, preferably heart failure with reduced and preserved ejection fraction (HFrEF and HFpEF), hypertension (HTN), peripheral arterial diseases (PAD, PAOD), cardio-renal and kidney diseases, preferably chronic and diabetic kidney disease (CKD and DKD), cardiopulmonary and lung diseases, preferable pulmonary hypertension (PH), and other diseases, preferably neurodegenerative diseases and different forms of dementias, fibrotic diseases, systemic sclerosis (SSc), sickle cell disease (SCD), wound healing disorders such as diabetic foot ulcer (DFU).

## Description

The invention relates to substituted pyrazolo piperidine carboxylic acids, their salts and to processes for their preparation, and also to their use for preparing medicaments for the treatment and/or prophylaxis of diseases, in particular cardiovascular and cardiac diseases, preferably heart failure with reduced and preserved ejection fraction (HFrEF and HFpEF), hypertension (HTN), peripheral arterial diseases (PAD, PAOD), cardio-renal and kidney diseases, preferably chronic and diabetic kidney disease (CKD and DKD), cardiopulmonary and lung diseases, preferable pulmonary hypertension (PH), and other diseases, preferably neurodegenerative diseases and different forms of dementias, fibrotic diseases, systemic sclerosis (SSc), sickle cell disease (SCD), wound healing disorders such as diabetic foot ulcer (DFU).

In addition, the same above-mentioned pathophysiological mechanisms are effective when blood transfusions (for example by storage etc. with an elevated concentration of free Hb) are administered to patients having a transfusion indication.

Furthermore, in the future the combination of an sGC activator with a synthetic Hb-based oxygen carrier may mitigate the side effects hitherto observed [Weiskopf, Anaesthesia & Analgesia, 110:3; 659-661,2010] which are caused by reduced availability of NO, thus allowing clinical application.

One of the most important cellular transmission systems in mammalian cells is cyclic guanosine monophosphate (cGMP). Together with nitric oxide (NO), which is released from the endothelium and transmits hormonal and mechanical signals, it forms the NO/cGMP system. Guanylate cyclases catalyse the biosynthesis of cGMP from guanosine triphosphate (GTP). The representatives of this family disclosed to date can be divided both according to structural features and according to the type of ligands into two groups: the particulate guanylate cyclases which can be stimulated by natriuretic peptides, and the soluble guanylate cyclases which can be stimulated by NO. The soluble guanylate cyclases consist of two subunits and very probably contain one haem per heterodimer, which is part of the regulatory site. The latter is of central importance for the mechanism of activation. NO is able to bind to the iron atom of haem and thus markedly increase the activity of the enzyme. Haem-free preparations cannot, by contrast, be stimulated by NO. Carbon monoxide (CO) is also able to attach to the central iron atom of haem, but the stimulation by CO is distinctly less than that by NO.

Through the production of cGMP and the regulation, resulting therefrom, of phosphodiesterases, ion channels and protein kinases, guanylate cyclase plays a crucial part in various physiological processes, in particular in the relaxation and proliferation of smooth muscle cells, in platelet aggregation and adhesion and in neuronal signal transmission, and in disorders caused by an impairment of the aforementioned processes. Under pathophysiological conditions, the NO/cGMP system may be suppressed, which may lead for example to high blood pressure, platelet activation, increased cellular proliferation and fibrosis, endothelial dysfunction, atherosclerosis, angina pectoris, heart failure, thromboses, stroke and myocardial infarction.

A possible way of treating such disorders which is independent of NO and aims at influencing the cGMP signaling pathway in organisms is a promising approach because of the high efficiency and few side effects which are to be expected.

Compounds, such as organic nitrates, whose effect is based on NO have to date been exclusively used for the therapeutic stimulation of soluble guanylate cyclase. NO is produced by bioconversion and activates soluble guanylate cyclase by attaching to the central iron atom of haem. Besides the side effects, the development of tolerance is one of the crucial disadvantages of this mode of treatment [O.V. Evgenov et al., Nature Rev. Drug Disc. 5 (2006), 755].

Substances which directly stimulate soluble guanylate cyclase, i.e. without previous release of NO, have been identified in recent years. The indazole derivative YC-1 was the first NO-independent but haem-dependent sGC stimulator described [Evgenov et al., ibid.]. Based on YC-1, further substances were discovered which are more potent than YC-1 and show no relevant inhibition of phosphodiesterases (PDE). This led to the identification of the pyrazolopyridine derivatives BAY 41-2272, BAY 41-8543,BAY 63-2521 and BAY 102-1189. Together with the recently published structurally different substances CMF-1571 and A-350619, these compounds form the new class of the sGC stimulators [Evgenov et al., ibid.]. A common characteristic of this substance class is a NO-independent and selective activation of the haem-containing sGC. In addition, the sGC stimulators in combination with NO have a synergistic effect on sGC activation based on a stabilization of the nitrosyl-haem complex. The exact binding site of the sGC stimulators at the sGC is still being debated. If the haem group is removed from the soluble guanylate cyclase, the enzyme still has a detectable catalytic basal activity, i.e. cGMP is still being formed. The remaining catalytic basal activity of the haem-free enzyme cannot be stimulated by any of the stimulators mentioned above [Evgenov et al., ibid.].

In addition, NO- and haem-independent sGC activators, with BAY 58-2667 as prototype of this class, have been identified. Common characteristics of these substances are that in combination with NO they only have an additive effect on enzyme activation, and that the activation of the oxidized or haem-free enzyme is markedly higher than that of the haem-containing enzyme [Evgenov et al., ibid.; J.P. Stasch et al., Br. J. Pharmacol. 136 (2002), 773; J.P. Stasch et al., J. Clin. Invest. 116 (2006), 2552]. Spectroscopic studies show that BAY 58-2667 displaces the oxidized haem group which, as a result of the weakening of the iron-histidine bond, is attached only weakly to the sGC. It has also been shown that the characteristic sGC haem binding motif Tyr-x-Ser-x-Arg is absolutely essential both for the interaction of the negatively charged propionic acids of the haem group and for the action of BAY 58-2667. Against this background, it is assumed that the binding site of BAY 58-2667 at the sGC is identical to the binding site of the haem group [J.P. Stasch et al., J. Clin. Invest. 116 (2006), 2552].

The sGC activator Runcaciguat (Hahn et al., Drugs Future 43 (2018), 738, WO 2012/139888) is in clinical development by BAYER (https://www.clinicaltrials.gov/NCT04507061). our understanding of the redox equilibrium of the sGC in health and diseases is limited. Therefore, the treatment potential of sGC activators is not fully clear yet. However, since oxidative stress could render the sGC enzyme heme-free the sGC activators, sGC activators might have an even broader treatment potential which still needs to be identified and proved in the future.

The compounds described in the present invention are now likewise capable of activating the haem-free form of soluble guanylate cyclase. This is also confirmed by the fact that these novel activators firstly have no synergistic action with NO at the haem-containing enzyme and that secondly their action cannot be blocked by the haem-dependent inhibitor of soluble guanylate cyclase, 1H-1,2,4-oxadiazolo[4,3-a]-quinoxalin-1-one (ODQ), but is even potentiated by this inhibitor [cf. O.V. Evgenov et al., Nature Rev. Drug Disc. 5 (2006), 755; J.P. Stasch et al., J. Clin. Invest. 116 (2006), 2552].

In WO 2012/058132 substituted pyrazolo pyridine carboxylic acids are diclosed as sGC activators. In contrast to the compounds according to the present invention these compounds do have a heteroaromatic pyridine moiety linking the pyrazole carboxylic acid to the rest of the molecule. Furthermore the pyridine nitrogen has another position than the piperidine nitrogen of the compounds according to the present invention. However these compounds do only show mediocre pharmacokinetic properties, like e.g. moderate clearance (CL) and intermediate half-life and mean residence time (MRT) after intraveneous (iv) administration in preclinical pharmacokinetic models.

It is therefore an object of the present invention to provide novel sGC activator compounds for the treatment ad / or prophylaxis of of diseases, in particular cardiovascular and cardiac diseases, preferably heart failure with reduced and preserved ejection fraction (HFrEF and HFpEF), hypertension (HTN), peripheral arterial diseases (PAD, PAOD), cardio-renal and kidney diseases, preferably chronic and diabetic kidney disease (CKD and DKD), cardiopulmonary and lung diseases, preferable pulmonary hypertension (PH), and other diseases, preferably neurodegenerative diseases and different forms of dementias, fibrotic diseases, systemic sclerosis (SSc), sickle cell disease (SCD), wound healing disorders such as diabetic foot ulcer (DFU), in humans and animals, which compounds show a good pharmacokinetic behavior with a good pharmacological activity profile as well as beneficial physico chemical properties (e.g. solubility).

Surprisingly, it has now been found that certain substituted pyrazolo piperidine carboxylic acids as well as their corresponding salts represent highly potent sGC activators with good pharmacokinetic behavior as well as beneficial physico chemical properties (e.g. solubility).

The invention provides compounds of the formula (I) 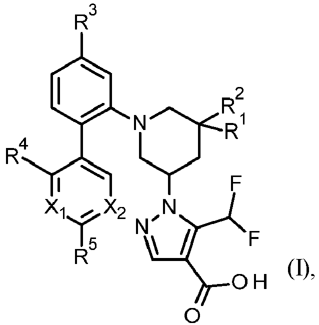 in which
- R¹: represents hydrogen or halogen,
- R²: represents hydrogen or halogen,
- R³: represents chloro or trifluoromethyl,
- R⁴: represents hydrogen, C₁-C₄-alkyl,
- R⁵: represents a group of the formula 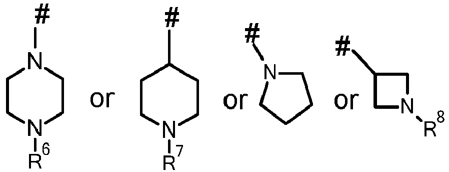
where # is the point of attachment to the aromatic or heteroaromatic 6 ring system
- R⁶: represents
C₁-C₆-alkyl, optionally substituted by one or more substituent independently selected from the group consisting of methyl, trifluoromethoxy, nitril, amido;
C₂-C₆-halogenoalkyl, substituted by 1 to 5 fluoro substituents,
C₃-C₆-cycloalkyl,
C₃-C₆-cycloalkyl-methyl, optionally substituted by 1 to 5 fluoro substituents or a trifluoromethyl group,
C₁-C₆-alkylcarbonyl, optionally substituted by 1 to 3 fluoro substituents,
C₃-C₆-cycloalkyl-carbonyl, optionally substituted by 1 to 3 fluoro substituents, oxetanyl,
spiro[2.2]pentan-2-ylmethyl or [(3-fluoro-1-bicyclo[1.1.1]pentanyl)methyl,
- R⁷: represents C₁-C₄-alkylcarbonyl, optionally substituted by a C₃-C₆-cycloalkyl group,
- R⁸: represents C₂-C₄- halogenoalkyl substituted by 1 to 6 fluoro substituents,
- X₁: represents nitrogen or carbon,
- X₂: represents nitrogen or carbon
and the salts thereof, the solvates thereof and the solvates of the salts thereof.

The term "substituted" means that one or more hydrogen atoms on the designated atom or group are replaced with a selection from the indicated group, provided that the designated atom's normal valence under the existing circumstances is not exceeded. Combinations of substituents and/or variables are permissible.

As used herein, the term "one or more", *e.g.* in the definition of the substituents of the compounds of general formula (I) of the present invention, means "1, 2, 3, 4 or 5, particularly 1, 2, 3 or 4, more particularly 1, 2 or 3, even more particularly 1 or 2".

In the context of the present invention, unless specified otherwise, the substituents are defined as follows:
The term "halogen" or "halogeno" like in combinations e.g. in halogenoalkyl means a fluorine, chlorine, bromine or iodine atom, particularly a fluorine, chlorine or bromine atom, even more particularly fluorine or chlorine.

The term "C₁-C₄-alkyl", "C₁-C₅-alkyl" and "C₁-C₆-alkyl" means a linear or branched, saturated, monovalent hydrocarbon group having 1, 2, 3, or 4 carbon atoms, 1, 2, 3, 4 or 5 carbon atoms, and 1, 2, 3, 4, 5 or 6 carbon atoms, *e.g.* a methyl, ethyl, propyl, isopropyl, *n*-butyl, *sec*-butyl, isobutyl, *tert-butyl,* pentyl, isopentyl, 2-methylbutyl, 1-methylbutyl, 1-ethylpropyl, 1,2-dimethylpropyl, *neo*-pentyl, 1,1-dimethylpropyl, hexyl, 1-methylpentyl, 2-methylpentyl, 3-methylpentyl, 4-methylpentyl, 1-ethylbutyl, 2-ethylbutyl, 1,1-dimethylbutyl, 2,2-dimethylbutyl, 3,3-dimethylbutyl, 2,3-dimethylbutyl, 1,2-dimethylbutyl or 1,3-dimethylbutyl group, or an isomer thereof. Particularly, said group has 1, 2, 3 or 4 carbon atoms ("C₁-C₄-alkyl"), *e.g.* a methyl, ethyl, propyl, isopropyl, butyl, sec-butyl isobutyl, or *tert-butyl* group, more particularly 1, 2 or 3 carbon atoms ("C₁-C₃-alkyl"), e.g. a methyl, ethyl, *n*-propyl or isopropyl group.

The term "C₁-C₆-halogenoalkyl", "C₂-C₆-halogenoalkyl", "C₁-C₄-halogenoalkyl", "C₂-C₄-halogenoalkyl", "C₁-C₃-halogenoalkyl" and "C₁-C₂-halogenoalkyl" represents a linear or branched, saturated, monovalent hydrocarbon group in which the term "alkyl" is as defined *supra,* and in which one or more of the hydrogen atoms are replaced, identically or differently, with a halogen atom. Particularly, said halogen atom is a fluorine atom. Said C₁-C₆-halogenoalkyl group is, for example fluoromethyl, difluoromethyl, trifluoromethyl, 2-fluoroethyl, 2,2-difluoroethyl, 2,2,2-trifluoroethyl, pentafluoroethyl, 3,3,3-tri-fluoropropan-1-yl, 1,1,1-trifluoropropan-2-yl, 1,3-difluoropropan-2-yl, 3-fluoropropan-1-yl, 1,1,1-trifluoro-butan-2-yl, and 3,3,3-trifluoro-1-methyl-propan-1-yl.

The term "C₁-C₄-halogenoalkoxy" and "C₁-C₃-halogenoalkoxy" represents a linear or branched, saturated, monovalent C₁-C₄-alkoxy or C₁-C₃-alkoxy group (where alkoxy represents a straight-chain or branched, saturated, monovalent alkoxy radical having 1 to 4 or 1 to 3 carbon atoms, by way of example and with preference methoxy, ethoxy, n-propoxy, isopropoxy), in which one or more of the hydrogen atoms is replaced, identically or differently, with a halogen atom. Particularly, said halogen atom is a fluorine atom. Said C₁-C₃-halogenoalkoxy group is, for example, fluoromethoxy, difluoromethoxy, trifluoromethoxy, 2,2,2-trifluoroethoxy or pentafluoroethoxy.

The term "(C₁-C₄)-alkylcarbonyl" represents a straight-chain or branched alkyl radical having 1 to 4 carbon atoms which is attached via a carbonyl group [-C(=O)-] to the remainder of the molecule. The following may be mentioned by way of example and by way of preference: acetyl, propionyl, n-butyryl, isobutyryl, t-butyryl, n-pentanoyl and pivaloyl.

The term "C₃-C₆-cycloalkyl" means a saturated, monovalent, monocyclic hydrocarbon ring which contains 3, 4, 5 or 6 carbon atoms. Said C₃-C₆-cycloalkyl group is for example a cyclopropyl, cyclobutyl, cyclopentyl or cyclohexyl group.

Compounds according to the invention are the compounds of the formula (I) and the salts, solvates and solvates of the salts thereof, and also the compounds encompassed by formula (I) and specified hereinafter as working example(s), and the salts, solvates and solvates of the salts thereof, to the extent that the compounds encompassed by formula (I) and specified hereinafter are not already salts, solvates and solvates of the salts.

The inventive compounds may, depending on their structure, exist in different stereoisomeric forms, i.e. in the form of configurational isomers or else, if appropriate, of conformational isomers (enantiomers and/or diastereomers, including those in the case of rotamers and atropisomers). The present invention therefore encompasses the enantiomers and diastereomers, and the respective mixtures thereof. The stereoisomerically uniform constituents can be isolated from such mixtures of enantiomers and/or diastereomers in a known manner; chromatography processes are preferably used for this, especially HPLC chromatography on an achiral or chiral phase.

The present invention includes all possible tautomers of the compounds of the present invention as single tautomers, or as any mixture of said tautomers, in any ratio.

In the context of the present invention, the term "enantiomerically pure" is understood to mean that the compound in question with respect to the absolute configuration of the chiral centre is present in an enantiomeric excess of more than 95%, preferably more than 97%. The enantiomeric excess (ee value) is calculated in this case by evaluation of the corresponding HPLC chromatogram on a chiral phase with the aid of the formula below: $ee = \left[{E}^{A}\left(area%\right)-{E}^{B}\left(area%\right)\right] \times 100 % / \left[{E}^{A}\left(area%\right)+{E}^{B}\left(area%\right)\right]$ (E^{A}: enantiomer in excess, E^{B}: enantiomer in deficiency)

The present invention also encompasses all suitable isotopic variants of the compounds according to the invention. An isotopic variant of an inventive compound is understood here as meaning a compound in which at least one atom within the inventive compound has been exchanged for another atom of the same atomic number, but with a different atomic mass than the atomic mass which usually or predominantly occurs in nature. Examples of isotopes which can be incorporated into a compound according to the invention are those of hydrogen, carbon, nitrogen, oxygen, phosphorus, sulfur, fluorine, chlorine, bromine and iodine, such as ²H (deuterium), ³H (tritium), ¹³C, ¹⁴C, ¹⁵N, ¹⁷O, ¹⁸O, ³²P, ³³P, ³³S, ³⁴S, ³⁵S, ³⁶S, ¹⁸F, ³⁶Cl, ⁸²Br, ¹²³I, ¹²⁴I, ¹²⁹I and ¹³¹I. Particular isotopic variants of a compound according to the invention, especially those in which one or more radioactive isotopes have been incorporated, may be beneficial, for example, for the examination of the mechanism of action or of the active ingredient distribution in the body; due to comparatively easy preparability and detectability, especially compounds labelled with ³H or ¹⁴C isotopes are suitable for this purpose. In addition, the incorporation of isotopes, for example of deuterium, may lead to particular therapeutic benefits as a consequence of greater metabolic stability of the compound, for example an extension of the half-life in the body or a reduction in the active dose required; such modifications of the inventive compounds may therefore in some cases also constitute a preferred embodiment of the present invention. Isotopic variants of the compounds according to the invention can be prepared by the processes known to those skilled in the art, for example by the methods described further below and the procedures described in the working examples, by using corresponding isotopic modifications of the respective reagents and/or starting compounds.

Preferred salts in the context of the present invention are physiologically acceptable salts of the compounds according to the invention. However, the invention also encompasses salts which themselves are unsuitable for pharmaceutical applications but which can be used, for example, for the isolation or purification of the compounds according to the invention.

Physiologically acceptable salts of the compounds according to the invention include acid addition salts of mineral acids, carboxylic acids and sulfonic acids, for example salts of hydrochloric acid, hydrobromic acid, sulfuric acid, phosphoric acid, methanesulfonic acid, ethanesulfonic acid, toluenesulfonic acid, benzenesulfonic acid, naphthalenedisulfonic acid, acetic acid, trifluoroacetic acid, propionic acid, lactic acid, tartaric acid, malic acid, citric acid, fumaric acid, maleic acid and benzoic acid.

Physiologically acceptable salts of the compounds according to the invention also include salts of conventional bases, by way of example and with preference alkali metal salts (e.g. sodium and potassium salts), alkaline earth metal salts (e.g. calcium and magnesium salts) and ammonium salts derived from ammonia or organic amines having 1 to 16 carbon atoms, by way of example and with preference ethylamine, diethylamine, triethylamine, ethyldiisopropylamine, monoethanolamine, diethanolamine, triethanolamine, dicyclohexylamine, dimethylaminoethanol, procaine, dibenzylamine, *N*-methylmorpholine, arginine, lysine, ethylenediamine, *N*-methylpiperidine and choline.

The present invention includes all possible salts of the compounds according to the invention as single salts, or as any mixture of said salts, in any ratio.

Solvates in the context of the invention are described as those forms of the inventive compounds which form a complex in the solid or liquid state by coordination with solvent molecules. The compounds according to the invention may contain polar solvents, in particular water, methanol or ethanol for example, as structural element of the crystal lattice of the compounds. Hydrates are a specific form of the solvates in which the coordination is with water. It is possible for the amount of polar solvents, in particular water, to exist in a stoichiometric or non-stoichiometric ratio. In the case of stoichiometric solvates, e.g. a hydrate, hemi-, (semi-), mono-, sesqui-, di-, tri-, tetra-, penta- *etc.* solvates or hydrates, respectively, are possible. The present invention includes all such hydrates or solvates.

Further, the compounds according to the invention can exist as N-oxides, which are defined in that at least one nitrogen of the compounds of the present invention is oxidised in a known manner. The present invention includes all such possible N-oxides.

The present invention additionally also encompasses prodrugs of the inventive compounds. The term "prodrugs" encompasses compounds which for their part may be biologically active or inactive but are converted during their residence time in the body into compounds according to the invention (for example by metabolism or hydrolysis).

In the formulae of the group which may represent R⁵, the end point of the line marked by # in each case does not represent a carbon atom or a CH₂ group, but is part of the bond to the atom to which R⁵ is attached.

Preference is given to compound of formula (I)
in which
- R¹: represents hydrogen or halogen,
- R²: represents hydrogen or halogen,
- R³: represents chloro or trifluoromethyl,
- R⁴: represents hydrogen, C₁-C₄-alkyl,
- R⁵: represents a group of the formula 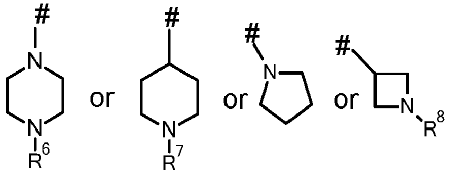
where # is the point of attachment to the aromatic or heteroaromatic 6 ring system
- R⁶: represents
C₁-C₆-alkyl, substituted by one or more substituent independently selected from the group consisting of trifluoromethoxy, nitril, amido;
C₂-C₆-halogenoalkyl, substituted by 1 to 5 fluoro substituents,
C₃-C₆-cycloalkyl,
C₃-C₆-cycloalkyl-methyl, optionally substituted by 1 to 5 fluoro substituents or a trifluoromethyl group,
C₁-C₆-alkylcarbonyl, optionally substituted by 1 to 3 fluoro substituents,
C₃-C₆-cycloalkyl-carbonyl, optionally substituted by 1 to 3 fluoro substituents, oxetanyl,
spiro[2.2]pentan-2-ylmethyl or [(3-fluoro-1-bicyclo[1.1.1]pentanyl)methyl,
- R⁷: represents C₁-C₄-alkylcarbonyl, optionally substituted by a C₃-C₆-cycloalkyl group,
- R⁸: represents C₂-C₄- halogenoalkyl substituted by 1 to 6 fluoro substituents,
- X₁: represents nitrogen or carbon,
- X₂: represents nitrogen or carbon
and the salts thereof, the solvates thereof and the solvates of the salts thereof.

Preference is given to compound of formula (I) in which
- R¹: represents hydrogen, fluorine
- R²: represents hydrogen, fluorine
- R³: represents chloro or trifluoromethyl,
- R⁴: represents hydrogen or methyl
- R⁵: represents a group of the formula 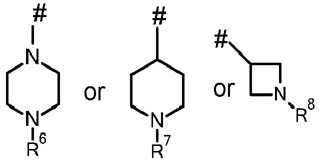
where # is the point of attachment to the aromatic or heteroaromatic 6 ring system
- R⁶: represents
C₁-C₄-alkyl, optionally substituted by one or more substituent independently selected from the group consisting of methyl, trifluoromethoxy, nitril,
C₂-C₆-halogenoalkyl, substituted by 1 to 5 fluoro substituents,
C₃-C₆-cycloalkyl-methyl, optionally substituted by 1 to 2 fluoro substituents or a trifluoromethyl group,
C₁-C₃-alkylcarbonyl, optionally substituted by 1 to 3 fluoro substituents,
C₃-C₆-cycloalkyl-carbonyl,
- R⁷: represents C₁-C₃-alkylcarbonyl, optionally substituted by cyclopropyl
- R⁸: represents C₂-C₄-halogenoalkyl, optionally substituted by 1 to 3 fluoro substituents,
- X₁: represents nitrogen or carbon
- X₂: represents nitrogen or carbon
or one of the salts thereof, solvates thereof or solvates of the salts thereof.

Preference is given to compound of formula (I) in which
- R¹: represents hydrogen, fluorine
- R²: represents hydrogen, fluorine
- R³: represents chloro or trifluoromethyl,
- R⁴: represents hydrogen or methyl
- R⁵: represents a group of the formula 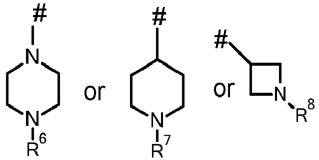
where # is the point of attachment to the aromatic or heteroaromatic 6 ring system
- R⁶: represents
C₁-C₄-alkyl, substituted by one or more substituent independently selected from the group consisting of methyl, trifluoromethoxy, nitril,
C₂-C₆-halogenoalkyl, substituted by 1 to 5 fluoro substituents,
C₃-C₆-cycloalkyl-methyl, optionally substituted by 1 to 2 fluoro substituents or a trifluoromethyl group,
C₁-C₃-alkylcarbonyl, optionally substituted by 1 to 3 fluoro substituents,
C₃-C₆-cycloalkyl-carbonyl,
- R⁷: represents C₁-C₃-alkylcarbonyl, optionally substituted by cyclopropyl
- R⁸: represents C₂-C₄-halogenoalkyl, optionally substituted by 1 to 3 fluoro substituents,
- X₁: represents nitrogen or carbon
- X₂: represents nitrogen or carbon
or one of the salts thereof, solvates thereof or solvates of the salts thereof.

Preference is given to compound of formula (I) in which
- R¹: represents hydrogen
- R²: represents hydrogen
- R³: represents chloro
- R⁴: represents hydrogen
- R⁵: represents a group of the formula

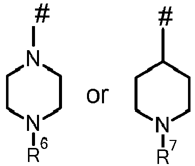

where # is the point of attachment to the aromatic or heteroaromatic 6 ring system
R⁶ represents C₁-C₄-alkyl, optionally substituted by trifluoromethoxy or nitril, C₂-C₃-halogenoalkyl, substituted by 1 to 5 fluoro substituents, C₃-C₄-cycloalkyl-methyl, optionally substituted by 1 to 2 fluoro substituents or a trifluoromethyl group, C₁-C₃-alkylcarbonyl, optionally substituted by 1 to 3 fluoro substituents, cyclopropyl-carbonyl,
R⁷ represents C₁-C₃-alkylcarbonyl, optionally substituted by cyclopropyl
X₁ represents carbon
X₂ represents carbon
or one of the salts thereof, solvates thereof or solvates of the salts thereof.

Preference is given to compound of formula (I) in which
- R¹: represents hydrogen
- R²: represents hydrogen
- R³: represents chloro
- R⁴: represents hydrogen
- R⁵: represents a group of the formula 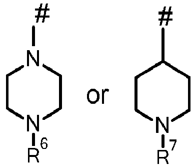
where # is the point of attachment to the aromatic or heteroaromatic 6 ring system
- R⁶: represents C₁-C₄-alkyl, substituted by trifluoromethoxy or nitril, C₂-C₃-halogenoalkyl, substituted by 1 to 5 fluoro substituents, C₃-C₄-cycloalkyl-methyl, optionally substituted by 1 to 2 fluoro substituents or a trifluoromethyl group, C₁-C₃-alkylcarbonyl, optionally substituted by 1 to 3 fluoro substituents, cyclopropyl-carbonyl,
- R⁷: represents C₁-C₃-alkylcarbonyl, optionally substituted by cyclopropyl
- X₁: represents carbon
- X₂: represents carbon
or one of the salts thereof, solvates thereof or solvates of the salts thereof.

Preference is given to compound of formula (I) in which
- R¹: represents hydrogen
- R²: represents hydrogen
- R³: represents chloro or trifluoromethyl,
- R⁴: represents hydrogen
- R⁵: represents a group of the formula 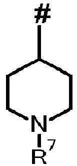
where # is the point of attachment to the aromatic or heteroaromatic 6 ring system
- R⁷: represents C₁-C₃-alkylcarbonyl, optionally substituted by cyclopropyl
- X₁: represents carbon or nitrogen
- X₂: represents carbon
or one of the salts thereof, solvates thereof or solvates of the salts thereof.

Preference is also given to compounds of the formula (I) in which
- R¹: represents hydrogen
- R²: represents hydrogen
- R³: represents chloro
- R⁴: represents hydrogen
- R⁵: represents a group of the formula

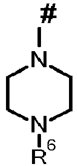

where # is the point of attachment to the aromatic or heteroaromatic 6 ring system
R⁶ represents C₁-C₄-alkyl, optionally substituted by trifluoromethoxy or nitril, C₂-C₃-halogenoalkyl, substituted by 1 to 5 fluoro substituents, C₃-C₄-cycloalkyl-methyl, optionally substituted by 1 to 2 fluoro substituents or atrifluoromethyl group, C₁-C₃-alkylcarbonyl, optionally substituted by 1 to 3 fluoro substituents,
X₁ represents carbon or nitrogen
X₂ represents carbon
and the salts thereof, the solvates thereof and the solvates of the salts thereof.

Preference is also given to compounds of the formula (I) in which
- R¹: represents hydrogen
- R²: represents hydrogen
- R³: represents chloro
- R⁴: represents hydrogen
- R⁵: represents a group of the formula 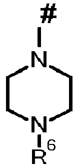
where # is the point of attachment to the aromatic or heteroaromatic 6 ring system
- R⁶: represents C₁-C₄-alkyl, substituted by trifluoromethoxy or nitril, C₂-C₃-halogenoalkyl, substituted by 1 to 5 fluoro substituents, C₃-C₄-cycloalkyl-methyl, optionally substituted by 1 to 2 fluoro substituents or a trifluoromethyl group, C₁-C₃-alkylcarbonyl, optionally substituted by 1 to 3 fluoro substituents,
- X₁: represents carbon or nitrogen
- X₂: represents carbon
and the salts thereof, the solvates thereof and the solvates of the salts thereof.

Preference is also given to compounds of the formula (I) in which
- R¹: represents hydrogen
- R²: represents hydrogen
- R³: represents chloro
- R⁴: represents hydrogen
- R⁵: represents a group of the formula

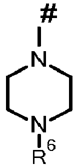

where # is the point of attachment to the aromatic or heteroaromatic 6 ring system
R⁶ represents C₁-C₄-alkyl, optionally substituted by trifluoromethoxy or nitril, trifluoroethyl ,C₃-C₄-cycloalkyl-methyl, optionally substituted by 1 to 2 fluoro substituents or a trifluoromethyl group, C₁-C₃-alkylcarbonyl, optionally substituted by 1 to 3 fluoro substituents,
X₁ represents carbon or nitrogen
X₂ represents carbon
and the salts thereof, the solvates thereof and the solvates of the salts thereof.

Preference is also given to compounds of the formula (I) in which
- R¹: represents hydrogen
- R²: represents hydrogen
- R³: represents chloro
- R⁴: represents hydrogen
- R⁵: represents a group of the formula 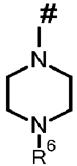
where # is the point of attachment to the aromatic or heteroaromatic 6 ring system
- R⁶: represents C₁-C₄-alkyl, substituted by trifluoromethoxy or nitril, trifluoroethyl ,C₃-C₄-cycloalkyl-methyl, optionally substituted by 1 to 2 fluoro substituents or a trifluoromethyl group, C₁-C₃-alkylcarbonyl, optionally substituted by 1 to 3 fluoro substituents,
- X₁: represents carbon or nitrogen
- X₂: represents carbon
and the salts thereof, the solvates thereof and the solvates of the salts thereof.

Preference is also given to compounds of the formula (I) in which
- R¹: represents hydrogen
- R²: represents hydrogen
- R³: represents chloro or trifluoromethyl
- R⁴: represents hydrogen
- R⁵: represents a group of the formula 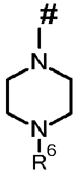
where # is the point of attachment to the aromatic or heteroaromatic 6 ring system
- R⁶: represents n-propyl, trifluoroethyl, (cyclopropyl)-methyl, acetyl, 1-propionyl
- X₁: represents carbon or nitrogen
- X₂: represents carbon
and the salts thereof, the solvates thereof and the solvates of the salts thereof.

Preference is also given to compounds of the formula (I) in which
- R¹: represents hydrogen
- R²: represents hydrogen
- R³: represents chloro or trifluoromethyl
- R⁴: represents hydrogen
- R⁵: represents a group of the formula 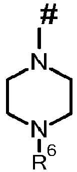
where # is the point of attachment to the aromatic or heteroaromatic 6 ring system
- R⁶: represents trifluoroethyl, (cyclopropyl)-methyl, acetyl, 1-propionyl
- X₁: represents carbon or nitrogen
- X₂: represents carbon
and the salts thereof, the solvates thereof and the solvates of the salts thereof.

Preference is also given to compounds of the formula (I) in which
- R¹: represents hydrogen
- R²: represents hydrogen
- R³: represents chloro
- R⁴: represents hydrogen
- R⁵: represents a group of the formula 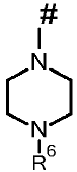
where # is the point of attachment to the aromatic or heteroaromatic 6 ring system
- R⁶: represents n-propyl, trifluoroethyl, (cyclopropyl)-methyl, acetyl, 1-propionyl
- X₁: represents carbon or nitrogen
- X₂: represents carbon
and the salts thereof, the solvates thereof and the solvates of the salts thereof.

Preference is also given to compounds of the formula (I) in which
- R¹: represents hydrogen
- R²: represents hydrogen
- R³: represents chloro
- R⁴: represents hydrogen
- R⁵: represents a group of the formula 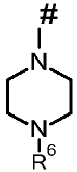
where # is the point of attachment to the aromatic or heteroaromatic 6 ring system
- R⁶: represents trifluoroethyl, (cyclopropyl)-methyl, acetyl, 1-propionyl
- X₁: represents carbon or nitrogen
- X₂: represents carbon
and the salts thereof, the solvates thereof and the solvates of the salts thereof.

Preference is also given to compounds of the formula (I) in which
- R¹: represents hydrogen
- R²: represents hydrogen
- R³: represents chloro
- R⁴: represents hydrogen
- R⁵: represents a group of the formula 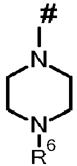
where # is the point of attachment to the aromatic or heteroaromatic 6 ring system
- R⁶: represents n-propyl, trifluoroethyl, (cyclopropyl)-methyl, acetyl, 1-propionyl
- X₁: represents carbon
- X₂: represents carbon
and the salts thereof, the solvates thereof and the solvates of the salts thereof.

Preference is also given to compounds of the formula (I) in which
- R¹: represents hydrogen
- R²: represents hydrogen
- R³: represents chloro
- R⁴: represents hydrogen
- R⁵: represents a group of the formula 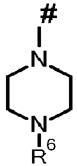
where # is the point of attachment to the aromatic or heteroaromatic 6 ring system
- R⁶: represents trifluoroethyl, (cyclopropyl)-methyl, acetyl, 1-propionyl
- X₁: represents carbon
- X₂: represents carbon
and the salts thereof, the solvates thereof and the solvates of the salts thereof.

Preference is also given to compounds selected from the group consisting of 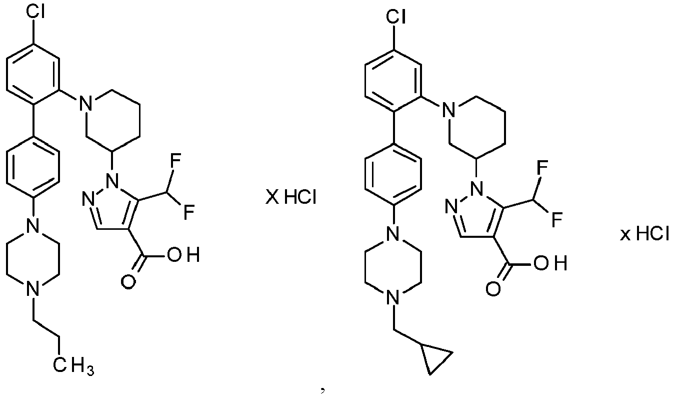 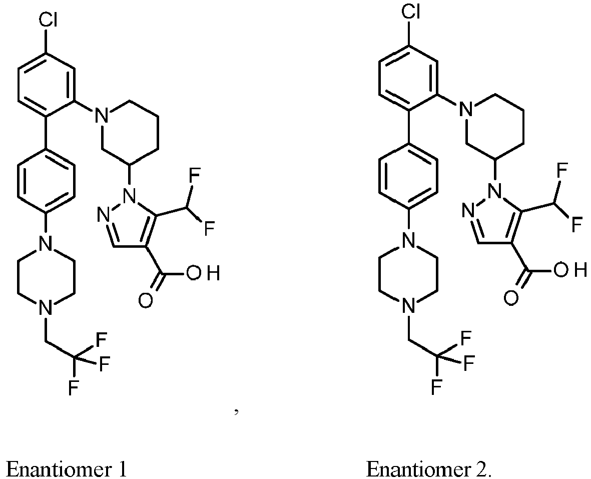 and the salts thereof, the solvates thereof and the solvates of the salts thereof.

Preference is also given to compounds selected from the group consisting of 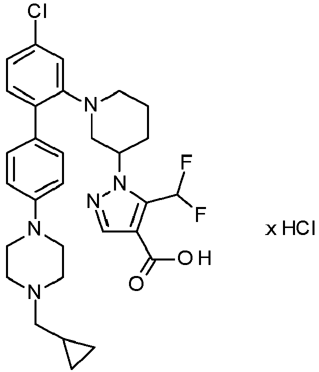 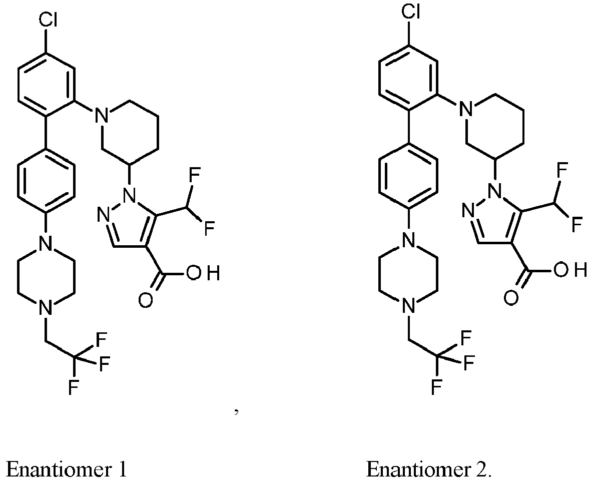 and the salts thereof, the solvates thereof and the solvates of the salts thereof.

The invention further provides a process for preparing compounds of the formula (I), or salts thereof, solvates thereof or solvates of the salts thereof, wherein
in a first step [B] the compounds of the formula (III) 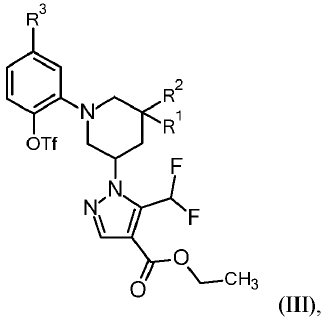 in which R¹, R² and R³ are defined as above,
are reacted with compounds of the formula (IV) 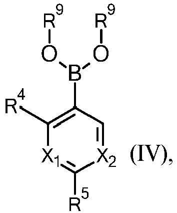 in which R⁴, R⁵, and X₁ and X₂ are defined as above,
and
in which R⁹ represents hydrogen, methyl, or both R⁹ form via the adjacent oxygen atoms a 4,4,5,5-tetramethyl-1,3,2-dioxaborolane
in the presence of a palladium source, a suitable ligand and a base to provide compounds of the formula (II) 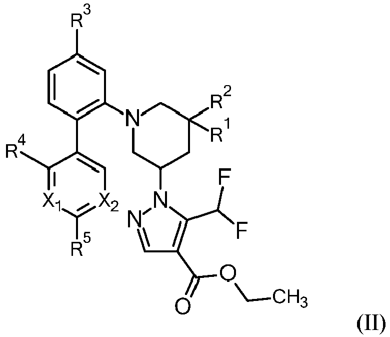 in which R¹, R², R³, R⁴, R⁵ and X₁ and X₂ are defined as above
and
in a second step [A]
compounds of formula (II) are reacted with a base to provide compounds of the formula (I), 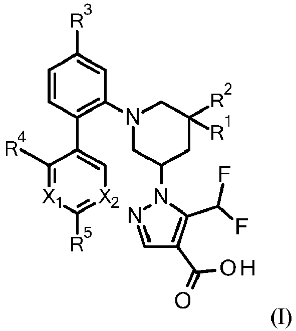 in which R¹, R², R³, R⁴, R⁵ and X₁ and X₂ are defined as above,
optionally compounds of formula (I) are transferred into the corresponding salts of formula (Ia) 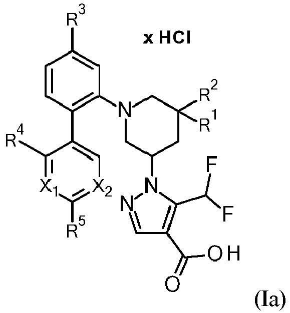 in the presence of a suitable acid in a suitable solvent
in which R¹, R², R³, R⁴, R⁵ and X₁ and X₂ are defined as above.

### Reaction [A]* (salt formation)

The reaction [A]* is generally carried out in inert solvents in the presence of an acid preferably in a temperature range from 0°C to 60°C at atmospheric pressure.

Suitable acids for the salt formation are generally sulfuric acid, hydrogen chloride/hydrochloric acid, hydrogen bromide/hydrobromic acid, phosphoric acid, acetic acid, trifluoroacetic acid, toluenesulfonic acid, methanesulfonic acid or trifluoromethanesulfonic acid, or mixtures thereof, optionally with addition of water. Preference is given to hydrogen chloride hydrogen bromide, toluenesulfonic acid, methanesulfonic acid or sulfuric acid.

Suitable inert solvents for the salt formation are, for example, ethers such as diethyl ether, dioxane, tetrahydrofuran, glycol dimethyl ether or diethylene glycol dimethyl ether, or other solvents such as acetone, ethyl acetate, ethanol, n-propanol, isopropanol, acetonitrile, dimethyl sulphoxide, N,N-dimethylformamide, N,N-dimethylacetamide, N,N'-dimethylpropyleneurea (DMPU) or N-methylpyrrolidone (NMP). It is also possible to use mixtures of the solvents mentioned. Preference is given to diethyl ether, dioxane, tetrahydrofuran or mixtures of these solvents.

### Reaction [A] (ester hydrolyses)

The hydrolysis of the ester group in compounds of formula II is carried out by customary methods, by treating the esters in inert solvents with acids or bases, where in the latter variant the salts initially formed are converted into the free carboxylic acids by treatment with acid. In the case of the *tert-butyl* esters, the ester hydrolysis is preferably effected with acids.

Suitable inert solvents for these reactions are water or the organic solvents customary for ester cleavage. These preferably include alcohols such as methanol, ethanol, *n*-propanol, isopropanol, *n*-butanol or *tert-*butanol, ethers such as diethyl ether, tetrahydrofuran, 1,4-dioxane or 1,2-dimethoxyethane, or other solvents such as dichloromethane, acetone, methyl ethyl ketone, *N,N*-dimethylformamide or dimethyl sulphoxide. It is equally possible to use mixtures of these solvents. In the case of a basic ester hydrolysis, preference is given to using mixtures of water with dioxane, tetrahydrofuran, methanol, ethanol and/or dimethylformamide or mixtures of tetrahydrofuran and methanol or ethanol. In the case of the reaction with trifluoroacetic acid, preference is given to using dichloromethane, and in the case of the reaction with hydrogen chloride preference is given to using tetrahydrofuran, diethyl ether, dioxane or water.

Suitable bases are the customary inorganic bases. These especially include alkali metal or alkaline earth metal hydroxides, for example lithium hydroxide, sodium hydroxide, potassium hydroxide or barium hydroxide, or alkali metal or alkaline earth metal carbonates, such as sodium carbonate, potassium carbonate or calcium carbonate. Preference is given to lithium hydroxide, sodium hydroxide or potassium hydroxide.
Suitable acids for the ester hydrolysis are generally sulfuric acid, hydrogen chloride/
hydrochloric acid, hydrogen bromide/hydrobromic acid, phosphoric acid, acetic acid, trifluoroacetic acid, toluenesulfonic acid, methanesulfonic acid or trifluoromethanesulfonic acid, or mixtures thereof, optionally with addition of water. Preference is given to hydrogen chloride or trifluoroacetic acid in the case of the *tert-*butyl esters and to hydrochloric acid in the case of the methyl esters.

The ester hydrolysis is generally carried out within a temperature range from -20°C to +120°C, preferably at 0°C to +80°C.

The compounds of the formula (II) 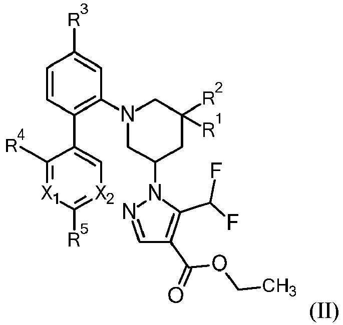 in which R¹, R², R³, R⁴, R⁵ and X₁ and X₂ are defined as above are novel.

The compounds of the formula (II) can be synthesized from the corresponding starting compounds of formula (III) by

### [B] reacting the compounds of the formula (III)

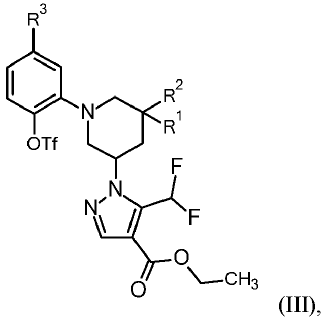

in which R¹, R² and R³ are defined as above,
in the presence of a suitable palladium catalyst, base and a suitable solvent
with compounds of the formula (IV) 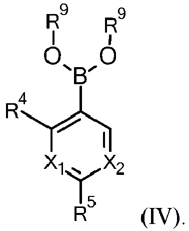 in which R⁴, R⁵, R⁹ and X₁ and X₂ are defined as above,
in the presence of a palladium source, a suitable ligand and a base to provide compounds of the formula (II).

### Reaction [B] (Suzuki coupling)

The reaction [B] is generally carried out in the presence of a suitable palladium catalyst and a suitable base in inert solvents, preferably at temperature range from room temperature up to reflux of the solvents at atmospheric pressure.

Inert solvents for reaction step [B] are for example alcohols like methanol, ethanol, n-propanol, isopropanol, n-butanol or tert.-butanol, ether like diethylether, dioxane, tetrahydrofuran, glycoldimethylether or di-ethylenglycoldimethylether, hydrocarbons like benzene, xylol, toluene, hexane, cyclohexane or petroleum oil, or other solvents like dimethylformamide (DMF), dimethylsulfoxide (DMSO), *N,N'*-dimethylpropylene urea (DMPU), *N*-methylpyrrolidone (NMP), pyridine, acetonitrile or also water. It is also possible to utilize mixtures of the aforementioned solvents. Preferred is a mixture of dimethylformamide / water and toluene / ethanol.

Suitable bases for reaction steps are the customary inorganic bases. These especially include alkali metal or alkaline earth metal hydroxides, for example lithium hydroxide, sodium hydroxide, potassium hydroxide or barium hydroxide alkali metal hydrogencarbonates like sodium or potassium hydrogencarbonate, or alkali metal or alkaline earth metal carbonates such as lithium, sodium, potassium, calcium or cesium carbonate, or alkali hydrogenphosphates like disodium or dipotassium hydrogenphosphate. Preferably used bases are sodium or potassium carbonate.

Examples of suitable palladium catalysts for reaction steps ["Suzuki-coupling"] are e.g. palladium on charcoal, palladium(II)-acetate, tetrakis-(triphenylphosphine)-palladium(0), bis-(triphenylphosphine)-palladium(II)-chloride, bis-(acetonitrile)-palladium(II)-chloride and [1,1'-bis(diphenylphosphino)ferro-cene]dichloropalladium(II)-dichlormethane-complex [cf. e.g. Hassan J. et al., Chem. Rev. 102, 1359-1469 (2002)].

The reaction steps are generally carried out within a temperature range from +20°C to +150°C, preferably at +50°C to +100°C.

The compounds of the formula (IV) 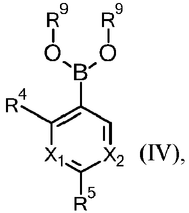 in which R⁴, R⁵, R⁹ and X₁ and X₂ are defined as above
and
in which
- R⁵: represents a group of the formula 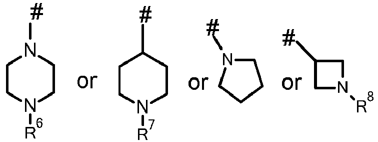
where # is the point of attachment to the aromatic or heteroaromatic 6 ring system
- R⁶: represents C₁-C₆-alkyl, optionally substituted by one or more substituent independently selected from the group consisting of methyl, trifluoromethoxy, nitril, amido,
C₂-C₆-halogenoalkyl, substituted by 1 to 5 fluoro substituents,
C₃-C₆-cycloalkyl,
C₃-C₆-cycloalkyl-methyl, optionally substituted by 1 to 5 fluoro substituents or a trifluoromethyl group,
C₁-C₆-alkylcarbonyl, optionally substituted by 1 to 3 fluoro substituents,
C₃-C₆-cycloalkyl-carbonyl, optionally substituted by 1 to 3 fluoro substituents, oxetanyl,
spiro[2.2]pentan-2-ylmethyl or [(3-fluoro-1-bicyclo[1.1.1]pentanyl)methyl,
- R⁷: represents C₁-C₄-alkylcarbonyl, optionally substituted by a C₃-C₆-cycloalkyl group,
- R⁸: represents C₂-C₄- halogenoalkyl substituted by 1 to 6 fluoro substituents,
are novel.

The compounds of the formula (IVb) 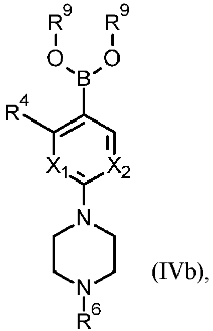 in which R⁴, R⁶, R⁹ and X₁ and X₂ are defined as above are novel and can be prepared

### [C] by reacting compounds of the formula (IVa)

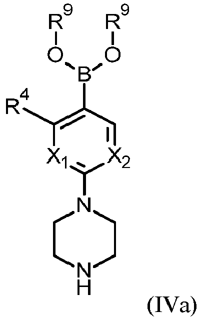

in which R⁴, R⁹ and X₁ and X₂ are defined as above
with compounds of formula (XV)

R^{6a}-CHO (XV)

in which
- R^{6a} represents: C₁-C₅-alkyl, optionally substituted by one or more substituent independently selected from the group consisting of methyl, trifluoromethoxy, nitril, amido, C₂-C₅-halogenoalkyl, substituted by 1 to 5 fluoro substituents, C₃-C₆-cycloalkyl, optionally substituted by 1 to 5 fluoro substituents or a trifluoromethyl group, spiro[2.2]butan-2-ylmethyl or [(3-fluoro-1-bicyclo[1.1.1]butanyl)methyl,
in the presence of a reducing agent, a base and a suitable solvent
or alternatively

### [D] by reacting compounds of the formula (IVa)

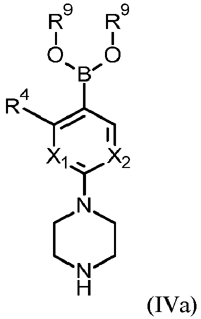

in which R⁴, R⁹ and X₁ and X₂ are defined as above
with compounds of formula (XVI)

R⁶-X (XVI)

in which R⁶ is as defined above and X is Br, OTs, OTf
in the presence of a base and a suitable solvent.
or alternatively

### [F] first by reacting compounds of the formula (IVa)

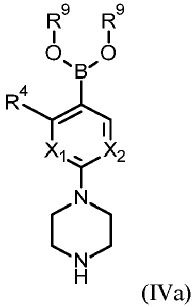

in which R⁴, R⁹ and X₁ and X₂ are defined as above
with compounds of formula (XVII) 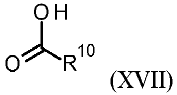 in which
- R¹⁰ represents: C₁-C₅-alkyl, optionally substituted by one or more substituent independently selected from the group consisting of methyl, trifluoromethoxy, nitril, amido, C₂-C₅-halogenoalkyl, substituted by 1 to 5 fluoro substituents, C₃-C₆-cycloalkyl, optionally substituted by 1 to 5 fluoro substituents or a trifluoromethyl group,
spiro[2.2]butan-2-ylmethyl or [(3-fluoro-1-bicyclo[1.1.1]butanyl)methyl,
in the presence of a base, and a suitable solvent
to provide compounds of formula (IVc) 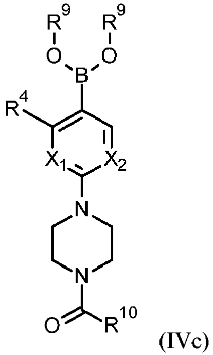 in which R⁴, R⁹, R¹⁰ and X₁ and X₂ are as defined above and

### [E] by further reacting compounds of the formula (IVc)

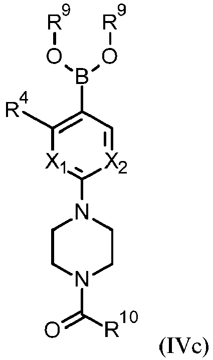

in which R⁴, R⁹, R¹⁰ and X₁ and X₂ are as defined above
in the presence of a reducing agent and a suitable solvent
to provide compounds of formula (IVd) 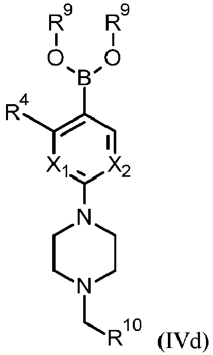 in which R⁴, R⁹, R¹⁰ and X₁ and X₂ are as defined above

Compounds of formula (IVc) will also be utilized in reaction [B] (Suzuki coupling) mentioned above.

### Reaction [C] (reductive amination)

The reaction [C] is generally carried out in inert solvents in the presence of a reducing agent, if appropriate in the presence of a base and or a dehydrating agent, preferably in a temperature range from 0°C to 60°C at atmospheric pressure.

Suitable reducing agents for reductive aminations are alkali metal borohydrides customary for such purposes such as sodium borohydride, sodium cyanoborohydride or sodium triacetoxyborohydride; preference is given to using sodium triacetoxyborohydride.

The addition of an acid, such as acetic acid in particular, and/or of a dehydrating agent, for example molecular sieve or trimethyl orthoformate or triethyl orthoformate, may be advantageous in these reactions.

Bases are, for example organic bases such as trialkylamines, for example triethylamine, N-methylmorpholine, N-methylpiperidine, 4-dimethylaminopyridine or diisopropylethylamin, or pyridine. Bases, such as N,N-diisopropylethylamine and triethylamine in particular, may be advantageous in these reactions.

Suitable solvents for these reactions are especially alcohols such as methanol, ethanol, *n*-propanol or isopropanol, ethers such as diisopropyl ether, methyl *tert-butyl* ether, tetrahydrofuran, 1,4-dioxane or 1,2-dimethoxyethane, polar aprotic solvents such as acetonitrile or *N,N-*dimethylformamide (DMF) or mixtures of such solvents; preference is given to using tetrahydrofuran.

The reactions are generally conducted within a temperature range of 0°C to +60°C.

The aldehydes of formula (XV) are commercial available or can be synthesized from known starting materials by known processes.

The starting material of formula (IVa) is either commercial available, known or available by known processes.

### Reaction [D] (alkylation)

The reaction [D] is generally carried out in a temperature range of from 0°C to +120°C, preferably at from +20°C to +80°C, if appropriate in a microwave. The reaction can be carried out at atmospheric, elevated or reduced pressure (for example from 0.5 to 5 bar).

Suitable inert solvents for the alkylations are, for example, halogenated hydrocarbons such as dichloromethane, trichloromethane, carbon tetrachloride, trichloroethylene or chlorobenzene, ethers such as diethyl ether, dioxane, tetrahydrofuran, glycol dimethyl ether or diethylene glycol dimethyl ether, hydrocarbons such as benzene, toluene, xylene, hexane, cyclohexane or mineral oil fractions, or other solvents such as acetone, methyl ethyl ketone, ethyl acetate, acetonitrile, N,N-dimethylformamide, N,N-dimethylacetamide, dimethyl sulphoxide, N,N'-dimethylpropyleneurea (DMPU), N-methylpyrrolidone (NMP) or pyridine. It is also possible to use mixtures of the solvents mentioned. Preference is given to using dimethylformamide, dimethyl sulphoxide or tetrahydrofuran.

Suitable bases for the alkylations are the customary inorganic or organic bases. These preferably include alkali metal hydroxides, for example lithium hydroxide, sodium hydroxide or potassium hydroxide, alkali metal or alkaline metal carbonates such as lithium carbonate, sodium carbonate, potassium carbonate, calcium carbonate or caesium carbonate, if appropriate with addition of an alkali metal iodide, for example sodium iodide or potassium iodide, alkali metal alkoxides such as sodium methoxide or potassium methoxide, sodium ethoxide or potassium ethoxide or sodium tert-butoxide or potassium tert-butoxide, alkali metal - hydrides such as sodium hydride or potassium hydride, amides such as sodium amide, lithium bis(trimethylsilyl)amide or potassium bis(trimethylsilyl)amide or lithium diisopropylamide, or organic amines such as triethylamine, N-methylmorpholine, N-methylpiperidine, N,N-diisopropylethylamine, pyridine, 1,5-diazabicyclo[4.3.0]non-5-ene (DBN), 4-(N,N-dimethylamino)pyridine (DMAP), 1,8-diazabicyclo[5.4.0]undec-7-ene (DBU) or 1,4-diazabicyclo[2.2.2]octane (DABCO^{®}). Preference is given to using potassium carbonate, caesium carbonate or N,N-diisopropylethylamine.

The alkylating agents of formula ((XVI) are known, commercial available or obtainable by known methods.

The starting material of formula (IVa) is either commercial available, known or available by known processes.

### Reaction [E] (reduction)

The reaction [E] is generally carried out in inert solvents, preferably in a temperature range from 0°C to +65°C, preferably at from 0°C to +40°C, if appropriate in a microwave. The reaction can be carried out at atmospheric, elevated or reduced pressure (for example from 0.5 to 5 bar).

Suitable inert solvents for the reductions are, for example, halogenated hydrocarbons such as dichloromethane, trichloromethane, carbon tetrachloride, trichloroethylene or chlorobenzene, ethers such as diethyl ether, dioxane, tetrahydrofuran, hydrocarbons such as benzene, toluene, xylene, hexane, cyclohexane or mineral oil fractions. It is also possible to use mixtures of the solvents mentioned. Preference is given to using tetrahydrofuran.

Suitable reducing agents for the amide reductions in process steps are, for example lithium aluminium hydride or borane tetrahydrofuran complex. Preference is given to using borane tetrahydrofuran complex.

The starting material of formula (IVc) is either commercial available, known or available by known processes or reaction [F].

### Reaction [F] (amide formation)

The reaction [F] is generally carried out in inert solvents, in presence of a condensing agent preferably in a temperature of from -20°C to +100°C, preferably at from 0°C to +60°C. The reaction can be performed at atmospheric, elevated or at reduced pressure (for example from 0.5 to 5 bar). In general, the reaction is carried out at atmospheric pressure.

Inert solvents for the amide formation are, for example, ethers such as diethyl ether, dioxane, tetrahydrofuran, glycol dimethyl ether or diethylene glycol dimethyl ether, hydrocarbons such as benzene, toluene, xylene, hexane, cyclohexane or mineral oil fractions, halogenated hydrocarbons such as dichloromethane, trichloromethane, carbon tetrachloride, 1,2-dichloroethane, trichloroethylene or chlorobenzene, or other solvents such as acetone, ethyl acetate, acetonitrile, pyridine, dimethyl sulphoxide, N,N-dimethylformamide, N,N-dimethylacetamide, N,N'-dimethylpropyleneurea (DMPU) or N-methylpyrrolidone (NMP). It is also possible to use mixtures of the solvents mentioned. Preference is given to dichloromethane, tetrahydrofuran, dimethylformamide or mixtures of these solvents.

Suitable condensing agents for the amide formation are, for example, carbodiimides such as N,N'-diethyl-, N,N'-dipropyl-, N,N'-diisopropyl-, N,N'-dicyclohexylcarbodiimide (DCC) or N-(3-dimethylaminopropyl)-N'-ethylcarbodiimide hydrochloride (EDC), phosgene derivatives such as N,N'-carbonyldiimidazole (CDI), 1,2-oxazolium compounds such as 2-ethyl-5-phenyl-1,2-oxazolium 3-sulphate or 2-tert-butyl-5-methylisoxazolium perchlorate, acylamino compounds such as 2-ethoxy-1-ethoxycarbonyl-1,2-dihydroquinoline, or isobutyl chloroformate, propanephosphonic anhydride (T3P), 1-chloro-N,N,2-trimethylpropl-ene-1-amine, diethyl cyanophosphonate, bis-(2-oxo-3-oxazolidinyl)phosphoryl chloride, benzotriazol-1-yl-oxytris(dimethylamino)phosphonium hexafluorophosphate, benzotriazol-1-yloxytris(pyrrolidino)-phosphonium hexafluorophosphate (PyBOP), O-(benzotriazol-1-yl)-N,N,N',N'-tetramethyluronium tetrafluoroborate (TBTU), O-(benzotriazol-1-yl)-N,N,N',N'-tetramethyluronium hexafluorophosphate (HBTU), 2-(2-oxo-1-(2H)-pyn*dyl)-1,1,3,3-tetramethyluronium tetrafluoroborate (TPTU), O-(7-azabenzo-triazol-1-yl)-N,N,N',N'-tetramethyluronium hexafluorophosphate (HATU) or O-(1H-6-chlorobenzotriazol-1-yl)-1,1,3,3-tetramethyluronium tetrafluoroborate (TCTU), if appropriate in combination with further auxiliaries such as 1-hydroxybenzotriazole (HOBt) or N-hydroxysuccinimide (HOSu), and also as bases alkali metal carbonates, for example sodium carbonate or potassium carbonate or sodium bicarbonate or potassium bicarbonate, or organic bases such as trialkylamines, for example triethylamine, N-methylmorpholine, N-methylpiperidine or N,N-diisopropylethylamine. Preference is given to using TBTU in combination with N-methylmorpholine, 1-chloro-N,N,2-trimethylprop-1-ene-1amine or HATU in combination with N,N-diisopropylethylamine.

Alternatively, the carboxylic acids can also initially be converted into the corresponding carbonyl chloride and this can then be reacted directly or in a separate reaction with an amine to give the compounds according to the invention. The formation of carbonyl chlorides from carboxylic acids is carried out by methods known to the person skilled in the art, for example by treatment with thionyl chloride, sulphuryl chloride or oxalyl chloride in the presence of a suitable base, for example in the presence of pyridine, and also optionally with addition of dimethylformamide, optionally in a suitable inert solvent.

The starting material of formula (IVc) is either commercial available, known or available by known processes or reaction [F].

The acylating agent of formula (XVII) is either commercial available, known or available by known processes.

### Compounds of the formula (IVf)

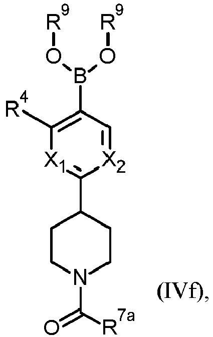

in which R⁴, R⁹ and X₁ and X₂ are defined as above are novel.
in which R^{7a} represents C₁-C₂--alkyl, cyclopropyl
They can be obtained by

### [G] reacting compounds of formula (IVe)

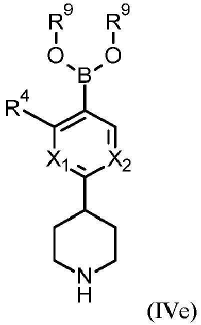

in which R⁴, R⁹ and X₁ and X₂ are defined as above
with compounds of formula (XVIII) 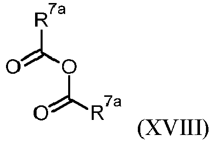 in which R^{7a} represents C₁-C₂--alkyl, cyclopropyl
in the presence of a base, a suitable solvent.

### Reaction [G] (acylation)

The reaction [G] is generally carried out in inert solvents, in presence of a base and and a dehydrating agent preferably in a temperature range of from 0°C to +100°C, preferably at from 0°C to +40°C, if appropriate in a microwave. The reaction can be carried out at atmospheric, elevated or reduced pressure (for example from 0.5 to 5 bar).

Suitable inert solvents for the acylationa are, for example, halogenated hydrocarbons such as dichloromethane, trichloromethane, carbon tetrachloride, trichloroethylene or chlorobenzene, ethers such as diethyl ether, dioxane, tetrahydrofuran, glycol dimethyl ether or diethylene glycol dimethyl ether, hydrocarbons such as benzene, toluene, xylene, hexane, cyclohexane or mineral oil fractions, or other solvents such as acetone, methyl ethyl ketone, ethyl acetate, acetonitrile, N,N-dimethylformamide, N,N-dimethylacetamide, dimethyl sulphoxide, N,N'-dimethylpropyleneurea (DMPU), N-methylpyrrolidone (NMP) or pyridine. It is also possible to use mixtures of the solvents mentioned. Preference is given to using dimethylformamide or dichloromethane.

Suitable bases for the alkylations are the customary inorganic or organic bases. These preferably include alkali metal hydroxides, for example lithium hydroxide, sodium hydroxide or potassium hydroxide, alkali metal or alkaline metal carbonates such as lithium carbonate, sodium carbonate, potassium carbonate, calcium carbonate or caesium carbonate, if appropriate with addition of an alkali metal iodide, for example sodium iodide or potassium iodide, alkali metal alkoxides such as sodium methoxide or potassium methoxide, sodium ethoxide or potassium ethoxide or sodium tert-butoxide or potassium tert-butoxide, alkali metal - hydrides such as sodium hydride or potassium hydride, amides such as sodium amide, lithium bis(trimethylsilyl)amide or potassium bis(trimethylsilyl)amide or lithium diisopropylamide, or organic amines such as triethylamine, N-methylmorpholine, N-methylpiperidine, N,N-diisopropylethylamine, pyridine, 1,5-diazabicyclo[4.3.0]non-5-ene (DBN), 4-(N,N-dimethylamino)pyridine (DMAP), 1,8-diazabicyclo[5.4.0]undec-7-ene (DBU) or 1,4-diazabicyclo[2.2.2]octane (DABCO^{®}). Preference is given to using pyridine, triethylamine or N,N-diisopropylethylamine.

### Compounds of the formula (IVe)

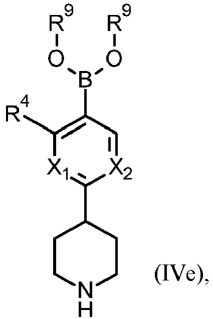

in which R⁴, R⁹ and X₁ and X₂ are defined as above
are known, commercial available or aobtainable by known processes.

### Compounds of the formula (XVIII)

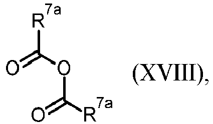

in which R^{7a} is defined as above
are known, commercial available or obtainable by known processes.

### Compounds of the formula (IVi)

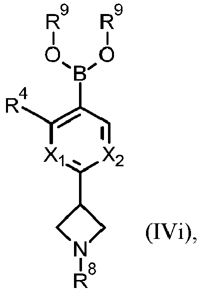

in which R⁴, R⁸, R⁹ and X₁ and X₂ are defined as above are novel and can be obtained by

### [I] first reacting compounds of formula (IVg)

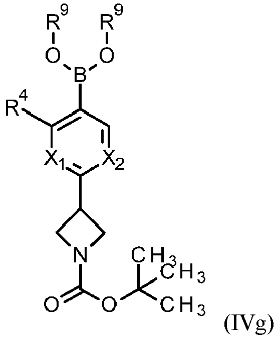

in which R⁴, R⁹ and X₁ and X₂ are defined as above are
with an acid in a suitable solvent
to obtain compounds of formula (IVh) 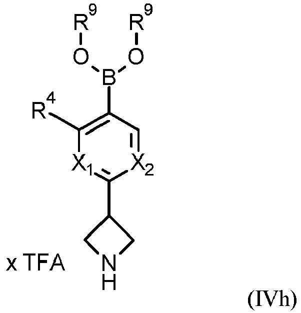 in which R⁴, R⁹ and X₁ and X₂ are defined as above
and

### [H] secondly reacting compounds of formula (IVh)

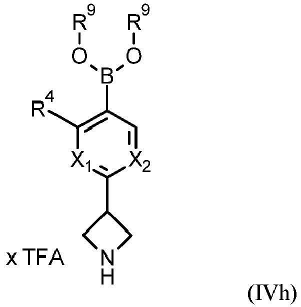

in which R⁴, R⁹ and X₁ and X₂ are defined as above
with compounds of formula ((XVIII)

X-R⁸ (XIX)

in which X is I, OTf
and in which R⁸ is as defined above
in the presence of a base and a suitable solvent
to obtain compounds of formula (IVi) 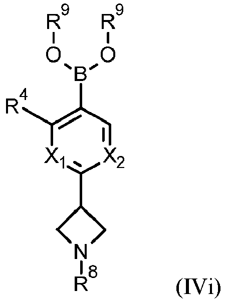 in which R⁴, R⁸, R⁹ and X₁ and X₂ are defined as above.

### Reaction [H] (alkylation)

The reaction [H] is generally carried out in a temperature range of from 0°C to +120°C, preferably at from +20°C to +80°C, if appropriate in a microwave. The reaction can be carried out at atmospheric, elevated or reduced pressure (for example from 0.5 to 5 bar).

Suitable inert solvents for the alkylations are, for example, halogenated hydrocarbons such as dichloromethane, trichloromethane, carbon tetrachloride, trichloroethylene or chlorobenzene, ethers such as diethyl ether, dioxane, tetrahydrofuran, glycol dimethyl ether or diethylene glycol dimethyl ether, hydrocarbons such as benzene, toluene, xylene, hexane, cyclohexane or mineral oil fractions, or other solvents such as acetone, methyl ethyl ketone, ethyl acetate, acetonitrile, N,N-dimethylformamide, N,N-dimethylacetamide, dimethyl sulphoxide, N,N'-dimethylpropyleneurea (DMPU), N-methylpyrrolidone (NMP) or pyridine. It is also possible to use mixtures of the solvents mentioned. Preference is given to using dimethylformamide, dimethyl sulphoxide or tetrahydrofuran.

Suitable bases for the alkylations are the customary inorganic or organic bases. These preferably include alkali metal hydroxides, for example lithium hydroxide, sodium hydroxide or potassium hydroxide, alkali metal or alkaline metal carbonates such as lithium carbonate, sodium carbonate, potassium carbonate, calcium carbonate or caesium carbonate, if appropriate with addition of an alkali metal iodide, for example sodium iodide or potassium iodide, alkali metal alkoxides such as sodium methoxide or potassium methoxide, sodium ethoxide or potassium ethoxide or sodium tert-butoxide or potassium tert-butoxide, alkali metal - hydrides such as sodium hydride or potassium hydride, amides such as sodium amide, lithium bis(trimethylsilyl)amide or potassium bis(trimethylsilyl)amide or lithium diisopropylamide, or organic amines such as triethylamine, N-methylmorpholine, N-methylpiperidine, N,N-diisopropylethylamine, pyridine, 1,5-diazabicyclo[4.3.0]non-5-ene (DBN), 4-(N,N-dimethylamino)pyridine (DMAP), 1,8-diazabicyclo[5.4.0]undec-7-ene (DBU) or 1,4-diazabicyclo[2.2.2]octane (DABCO^{®}). Preference is given to using potassium carbonate, caesium carbonate or N,N-diisopropylethylamine.

### Reaction [I] (Deprotection)

The reaction [I] is generally carried out in inert solvents in the presence of a suitable acid, preferably in a temperature range from 0°C to 60°C at atmospheric pressure.

Acids are, for example organic or inorganic acids such as sulfuric acid, hydrogen chloride/hydrochloric acid, hydrogen bromide/hydrobromic acid, phosphoric acid, acetic acid, trifluoroacetic acid, toluenesulfonic acid, methanesulfonic acid or trifluoromethanesulfonic acid, or mixtures thereof, optionally with addition of water. Preference is given to hydrogen chloride or trifluoroacetic acid.

Suitable solvents for these reactions are especially alcohols such as methanol, ethanol, n-propanol or isopropanol, ethers such as diisopropyl ether, methyl tert-butyl ether, tetrahydrofuran, 1,4-dioxane or 1,2-dimethoxyethane, polar aprotic solvents such as acetonitrile or N,N-dimethylformamide (DMF) or mixtures of such solvents; preference is given to using tetrahydrofuran.

The reactions are generally conducted within a temperature range of 0°C to +60°C.

The alkylating agents of formula ((XVI) are known, commercial available or obtainable by known methods.

The starting material of formula (IVa) is either commercial available, known or available by known processes.

The compounds of the formula (IVg) are known, commercial available or obtainable form known starting materials by known processes.

The compounds of formula (XIX) are known, commercial available or obtainable form known starting materials by known processes.

The compounds of formula (III) 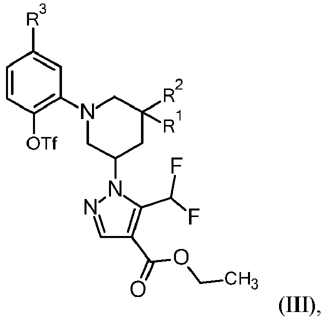 in which R¹, R² and R³ are defined as above are novel and can be prepared

### [J] by reacting compounds of the formula (V)

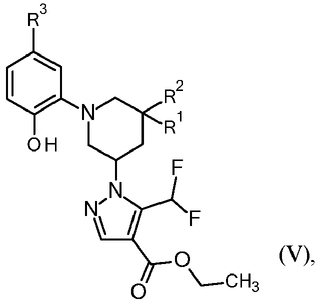

in which R¹, R² and R³ are as defined above,
with triflic acid anhydride in the presence of base and an inert solvent.

### Reaction [J] (triflatization)

The reaction [J] is generally carried out in inert solvents, preferably in a temperature range from room temperature up to reflux of the solvents at atmospheric pressure.

Bases are, for example, organic bases like alkali amines or pyridines or inorganic bases such as sodium hydroxide, lithium hydroxide or potassium hydroxide, or alkali metal carbonates such as caesium carbonate, sodium carbonate or potassium carbonate, or alkoxides such as potassium *tert*-butoxide or sodium *tert-*butoxide, or pyridines such as pyridine or 2,6-lutidine, or alkali amines such as triethylamine or N,N-diisopropylethylamine; preference is given to triethylamine.

Inert solvents are, for example, ethers such as diethyl ether, methyl *tert*-butyl ether, 1,2-dimethoxyethane, dioxane or tetrahydrofuran, or other solvents such as dichloromethane, dimethylformamide, dimethylacetamide, acetonitrile or pyridine, or mixtures of solvents; preference is given to dichloromethane.

The compounds of the formula (V) are novel 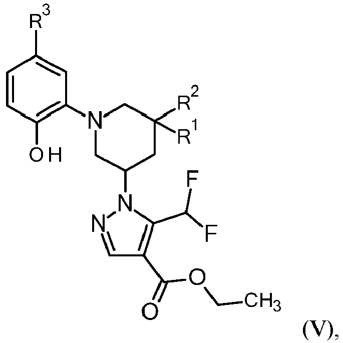 in which R¹, R² and R³ are defined as above.
The compounds of the formula (V) can be prepared

### [K] by reacting compounds of the formula (VI)

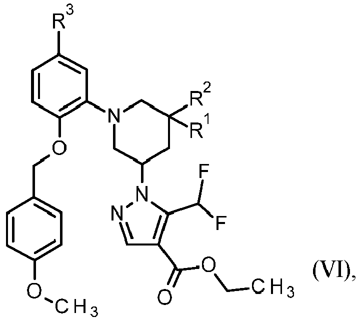

in which R¹, R² and R³ are as defined above,
with an acid optionally in an inert solvent.

### Reaction [K] (acidic deprotection)

The reaction [K] is generally carried out in inert solvents or without solvent, preferably in a temperature range from 0°C up to reflux of the solvents at atmospheric pressure.

Inert solvents are, for example, halogenated hydrocarbons such as dichloromethane, trichloromethane, carbon tetrachloride or 1,2-dichloroethane, alcohols such as methanol or ethanol, ethers such as diethyl ether, methyl *tert*-butyl ether, 1,2-dimethoxyethane, dioxane or tetrahydrofuran, or other solvents such as dimethylformamide, dimethoxy ethane, N-methyl-pyrrolidone, dimethylacetamide, acetonitrile, acetone or pyridine, or mixtures of solvents; preference is given to dichloromethane or dioxane.

Suitable acids for the acidic deprotection are generally sulfuric acid, hydrogen chloride/hydrochloric acid, hydrogen bromide/hydrobromic acid, phosphoric acid, acetic acid, trifluoroacetic acid, toluenesulfonic acid, methanesulfonic acid or trifluoromethanesulfonic acid, or mixtures thereof, optionally with addition of water. Preference is given to hydrogen chloride or trifluoroacetic acid.

### Compounds of the formula (VI)

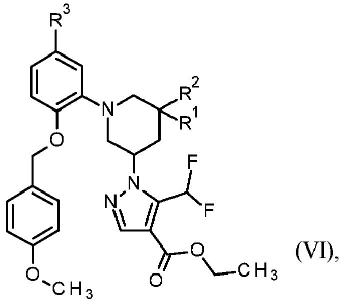

in which R¹, R² and R³ are as defined above are novel.
The compounds of the formula (VI) can be prepared

### [L] by reacting compounds of the formula (VII)

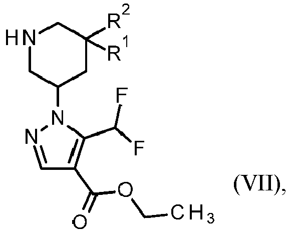

in which R¹ and R² are as defined above,
with compounds of the formula (VIII) 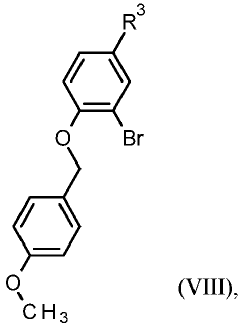 in which R³ is as defined above,
in the presence of a palladium source, a suitable ligand and a base.

### Reaction [L] (Buchwald Hartwig coupling)

The reaction [L] is generally carried out in the presence of a palladium source, a suitable ligand and a base in inert solvents, preferably in a temperature range from room temperature up to reflux of the solvents at atmospheric pressure.

The palladium source and a suitable ligand are, for example, palladium on charcoal, palladium(II)-acetate, tris(dibenzylideneacetone)palladium(0), tetrakis-(triphenylphosphine)-palladium(0), bis-(triphenylphosphine)-palladium(II) chloride, bis-(acetonitrile)-palladium(II) chloride, [1,1'-bis(diphenylphosphino)ferrocene]dichloro palladium (II) and corresponding dichloromethan-complex, optionally in conjunction with additional phosphane ligands like for example 2,2'-Bis(diphenylphosphino)-1,1'-binaphthyl (BINAP), (2-dicyclohexylphosphino-2',4',6'-triisopropyl-1,1'-biphenyl)[2-(2'-amino-1,1 ' -biphenyl)]palladium(II) methanesulfonate (XPhos-Pd-G3, CAS-No: 1445085-55-1), (2-biphenyl)di-*tert*. -butylphosphine, dicyclohexyl[2',4',6'-tris(1-methylethyl)biphenyl-2-yl]phosphane (XPhos, CAS-No: CAS-No: 564483-18-7), Bis(2-phenylphosphinophenyl)ether (DPEphos), or 4,5-bis(diphenyl-phosphino)-9,9-dimethylxanthene (Xantphos: CAS-No: 161265-03-8) [cf. e.g. Hassan J. et al., Chem. Rev. 2002, 102, 1359-1469], 2-(dicyclohexylphosphine)-3,6-dimethoxy-2',4',6'-triisopropyl-1,1'-biphenyl (BrettPhos, CAS-No: 1070663-78-3), 2-dicyclohexylphosphino-2' ,6' -dimethoxybiphenyl (SPhos, CAS-No: 657408-07-6), 2-dicyclohexylphosphino-2' ,6' -diisopropoxybiphenyl (RuPhos, CAS-No: 787618-22-8), 2-(di-tert-butylphosphino)-3-methoxy-6-methyl-2',4',6'-tri-i-propyl-1,1'-biphenyl (RockPhos) and 2-di-tert-butylphosphino-2' ,4' ,6' -triisopropylbiphenyl (*tert*-ButylXPhos). It is also possible to use corresponding precatalysts such as chloro-[2-(dicyclohexylphosphine)-3,6-dimethoxy-2',4',6'-triisopropyl-1,1'-biphenyl][2-(2-aminoethyl)-phenyl]palladium(II) (BrettPhos precatalysts) [cf. e.g. S. L. Buchwald et al., Chem. Sci. 2013, 4, 916] optionally be used in conjunction with additional phosphine ligands such as 2-(dicyclohexylphosphine)-3,6-dimethoxy-2',4',6'-triisopropyl-1,1'-biphenyl (BrettPhos).

Preference is given to 2,2'-Bis(diphenylphosphino)-1,1'-binaphthyl (BINAP), tris(dibenzylideneacetone)palladium(0), or in combination with 4,5-bis(diphenylphosphino)-9,9-dimethyl-xanthen (Xantphos) or dicyclohexyl[2',4',6'-tris(1-methylethyl)biphenyl-2-yl]phosphane (XPhos).

Bases are, for example, suitable inorganic or organic bases like e.g. alkali or earth alkali metal carbonates such as lithium, sodium , potassium, calcium or cesium carbonate, or sodium bicarbonate or potassium bicarbonate, alkali metal hydrogencarbonates such as sodium hydrogencarbonate or potassium hydrogencarbonate, alkali metal or earth alkali hydroxides such as sodium, barium or potassium hydroxide; alkali metal or earth alkali phosphates like potassium phosphate; alkali metal alcoholates like sodium or potassium tert.-butylate and sodium methanolate, alkali metal phenolates like sodium phenolate, potassium acetate, amides like sodium amide, lithium-, sodium- or potassium -bis(trimethylsilyl)amide or lithium diisopropylamide or organic amines like 1,5-diazabicyclo[4.3.0]non-5-ene (DBN), 1,8-diazabicyclo[5.4.0]undec-7-en (DBU). Preference is given to caesium carbonate, sodium carbonate, potassium carbonate or sodium hydrogencarbonate .

Inert solvents are, for example, ethers such as dioxane, diethyl ether, tetrahydrofuran, 2-methyltetrahydrofuran, di-*n*-butylether, cyclopentylmethylether, glycoldimethylether or diethyleneglycoldimethyl-ether, alcohols like *tert.* -butanol or amylalcohols or dimethylformamide, dimethylacetamide, dimethyl sulphoxide, N-methylpyrrolidone, toluene or acetonitrile, or mixtures of the solvents; preference is given to tert.-butanol, 1,4-dioxane and toluene.

The compounds of the formula (VIII) are known or can be synthesized from the corresponding, commercial available starting compounds by known processes.

The compounds of the formula (VII) 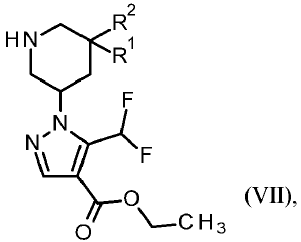 in which R¹ and R² are as defined above are novel.
The compounds of the formula (VII) can be prepared

### [M] by reacting compounds of the formula (IX)

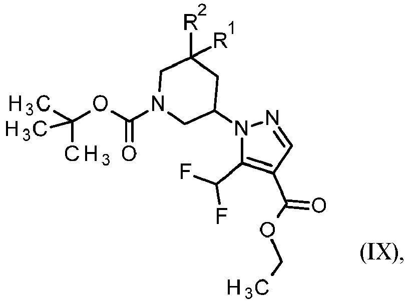

in which R¹ and R² are as defined above,
with an acid in an inert solvent.

### Reaction [M] (debocylation)

The reaction [M] is generally carried out in inert solvents in the presence of a suitable acid, preferably in a temperature range from 0°C to 60°C at atmospheric pressure.

Acids are for example organic or inorganic acids such as sulfuric acid, hydrogen chloride/hydrochloric acid, hydrogen bromide/hydrobromic acid, phosphoric acid, acetic acid, trifluoroacetic acid, toluenesulfonic acid, methanesulfonic acid or trifluoromethanesulfonic acid, or mixtures thereof, optionally with addition of water. Preference is given to hydrogen chloride or trifluoroacetic acid

Inert solvents are alcohols such as methanol, ethanol or isopropanol, ethers such as diethyl ether, diisopropyl ether, methyl *tert-butyl* ether, tetrahydrofuran or 1,4-dioxane, dichloromethane, polar aprotic solvents such as acetonitrile or *N,N*-dimethylformamide (DMF) or mixtures of such solvents; preference is given to using 1,4-dioxane.

The compounds of the formula (IX) 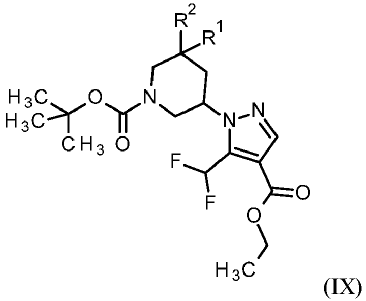 in which R¹ and R² are as defined above, are novel.
The compounds of the formula (IX) can be prepared

### [N] by reacting compounds of the formula (X)

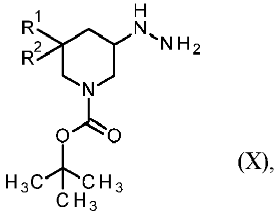

in which R¹ and R² are as defined above,
with compounds of the formula (XI) 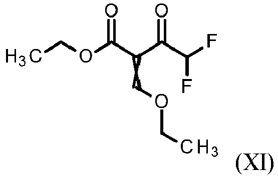 in a solvent.

### Reaction [N] (pyrazole formation)

The reaction [L] is generally carried out in a solvent at temperatures from room temperature to reflux.

Suitable solvents are alcohols such as methanol, ethanol or isopropanol, ethers such as diethyl ether, diisopropyl ether, methyl *tert-butyl* ether, tetrahydrofuran or 1,4-dioxane, dichloromethane, polar aprotic solvents such as acetonitrile or *N,N*-dimethylformamide (DMF) or mixtures of such solvents; preference is given to using ethanol.

The compound of the formula (XI) are known or can be synthesized from the corresponding starting compounds by known processes.

The compounds of the formula (X) 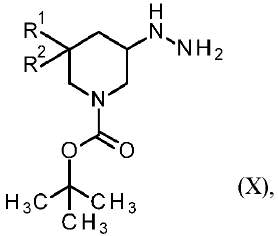 in which R¹ and R² are as defined above are novel.
The compounds of the formula (X) can be prepared

### [O] by reacting compounds of the formula (XII)

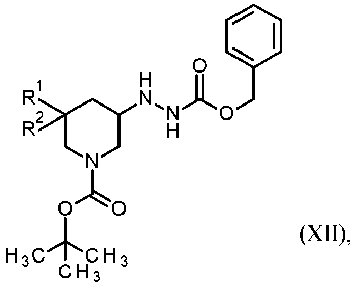

in which R¹ and R² are as defined above
with palladium on charcoal in the presence of hydrogen in a suitable solvent.

### Reaction [O] (Z deprotection)

The reaction [O] is generally carried out in the presence of palladium on charcoal in a suitable solvent at from room temperature to reflux., preferable at 1 bar.

Suitable solvents are alcohols such as methanol, ethanol or isopropanol, ethers such as diethyl ether, diisopropyl ether, methyl *tert-butyl* ether, tetrahydrofuran or 1,4-dioxane, dichloromethane, polar solvents such as acetonitrile, *N,N-*dimethylformamide (DMF), NMP, acetic acid or water or mixtures of such solvents; preference is given to ethanol/acetic acid.

The compounds of the formula (XII) 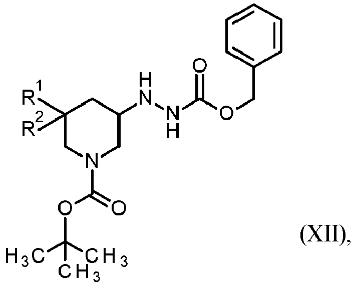 in which R¹ and R² are as defined above are novel.
The compounds of the formula (XII) can be prepared

### [P] by reacting compounds of the formula (XIII)

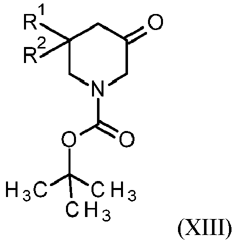

in which R¹ and R² are as defined above
with a compound of the formula (XIV) 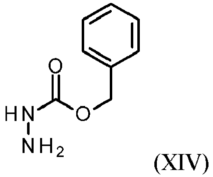 in the presence of a reducing agent and a suitable solvent.

The compound of the formula (XIV) is known and comercial available or can be synthesized from the corresponding starting compounds by known processes.

The compound of the formula (XIII) is known and comercial available or can be synthesized from the corresponding starting compounds by known processes.

The preparation of the starting compounds and of the compounds of the formula (I) can be illustrated by the synthesis schemes 1 to 4 which follow.

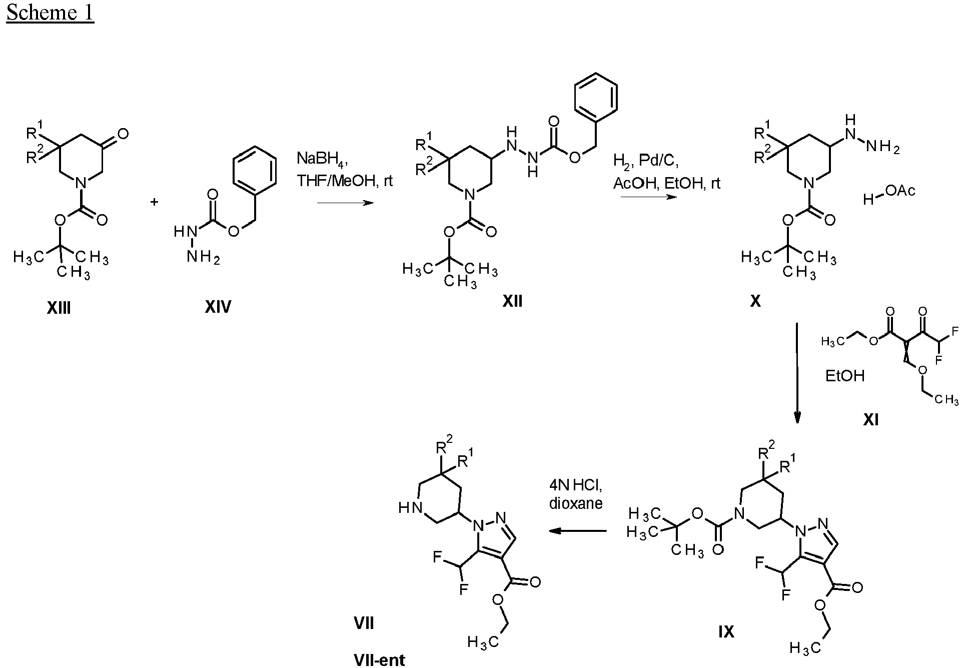

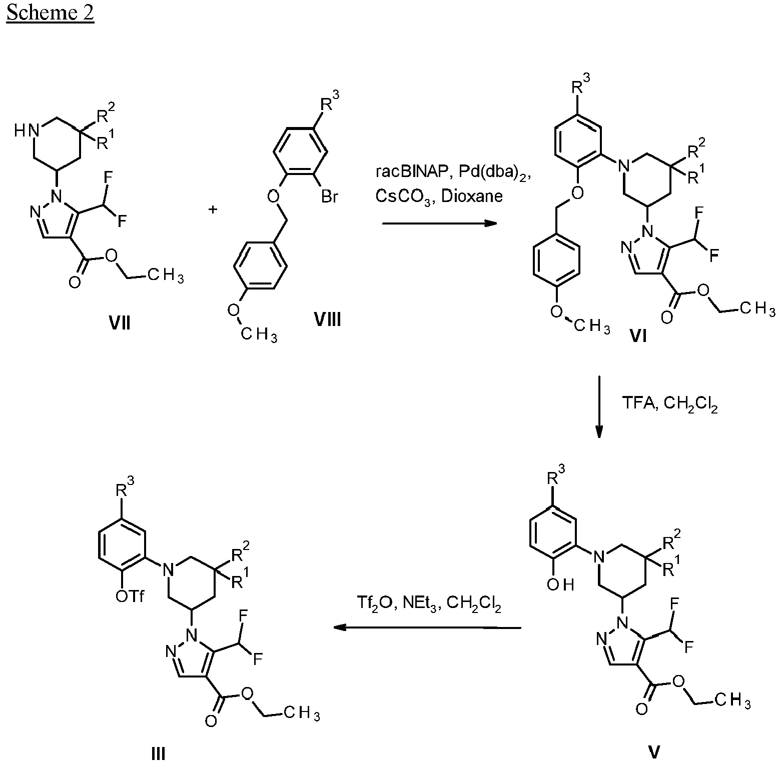

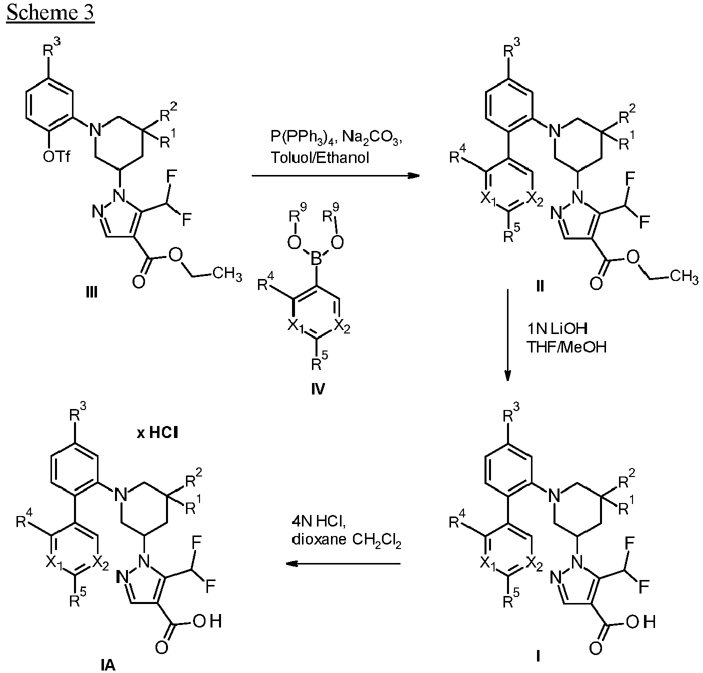

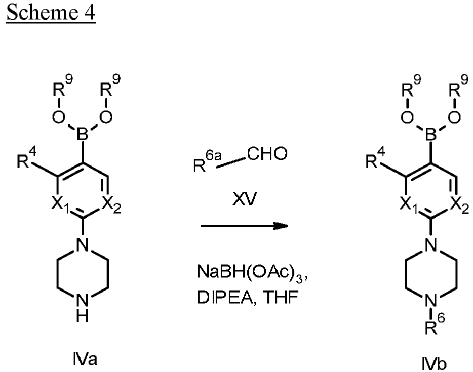

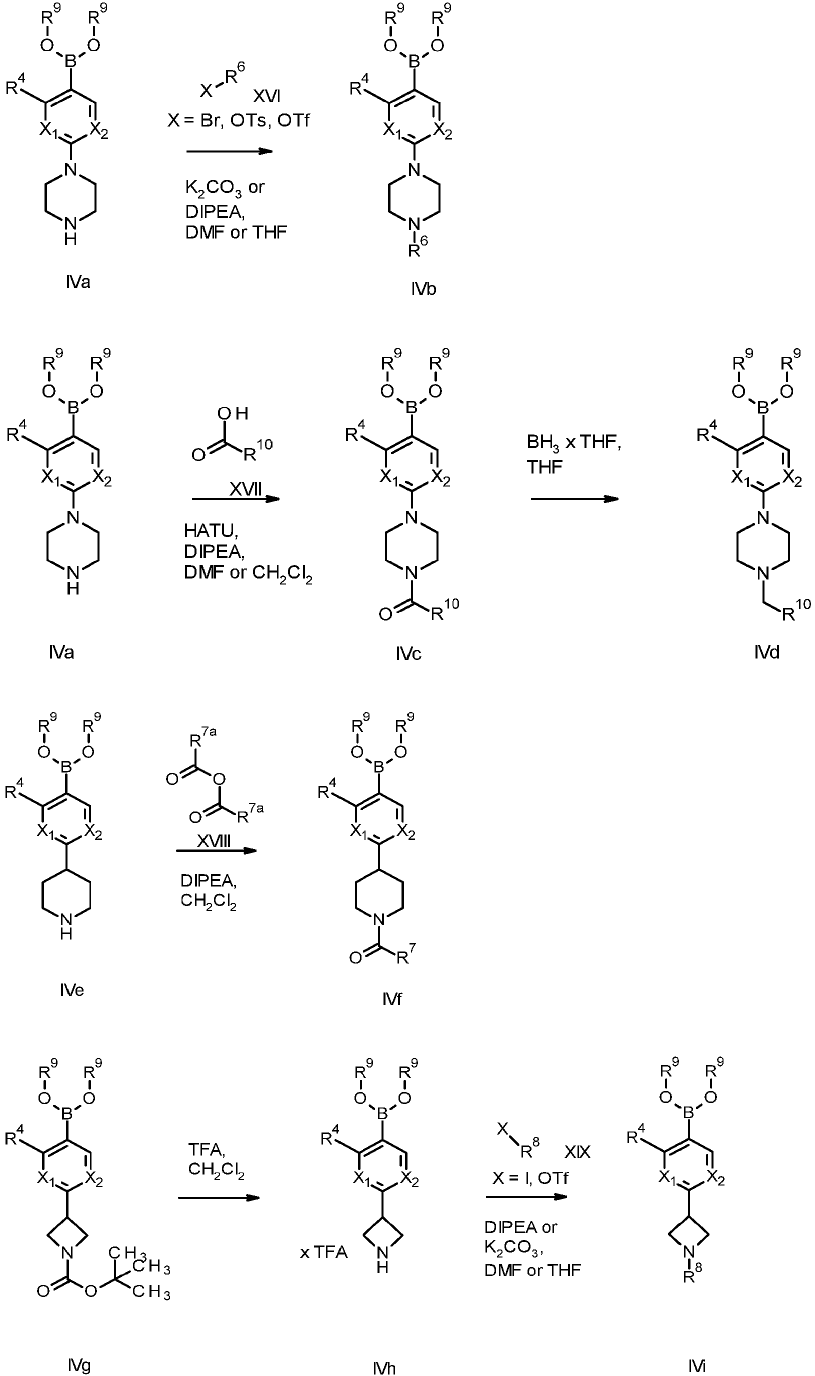

The compounds of the invention have valuable pharmacological properties and can be used for prevention and treatment of diseases in humans and animals.

The compounds according to the invention are potent activators of soluble guanylate cyclase. They lead to vasorelaxation, inhibition of platelet aggregation and lowering of blood pressure and increase of coronary and renal blood flow. These effects are mediated via direct haem-independent activation of soluble guanylate cyclase and an increase of intracellular cGMP.

In addition, the compounds according to the invention have advantageous pharmacokinetic properties, in particular with respect to their bioavailability and/or duration of action after intravenous or oral administration.

The compounds according to the present invention show similar to superior pharmacokinetic (PK) properties in comparison to compounds disclosed in the prior art (WO 2012/058132, e.g. ex. 174) (see experimental part, tables 3 to 4). The shown examples show a lower plasma clearance (CL_{Plasma}) and therefore a higher exposure (AUCₙₒᵣₘ) in comparison to the prior art compound disclosed as example 174 in WO 2012/058132 in rats. Half live and mean residence time (MRT) of most examples are in a comparable range after i.v. application. However example 20 shows a longer half live and mean residence time (MRT). After p.o. application in rats (see experimental part, table 4) most examples (except example 19) do show comparable to higher exposures (AUCₙₒᵣₘ) in comparison to the prior art compound disclosed as example 174 in WO 2012/058132.

The compounds according to the invention have an unforeseeable useful pharmacological activity spectrum and good pharmacokinetic behavior, in particular a sufficient exposure of such a compound in the blood above the minimal effective concentration within a given dosing interval after oral administration. Such a profile results in an improved peak-to-trough ratio (quotient of maximum to minimum concentration) within a given dosing interval, which has the advantage that the compound can be administered less frequently and at a significantly lower dose to achieve an effect. They are compounds that activate soluble guanylate cyclase.

In the context of the present invention, the term "treatment" or "treating" includes inhibition, retardation, checking, alleviating, attenuating, restricting, reducing, suppressing, repelling or healing of a disease, a condition, a disorder, an injury or a health problem, or the development, the course or the progression of such states and/or the symptoms of such states. The term "therapy" is understood here to be synonymous with the term "treatment".

In the context of the present invention, the terms "prevention", "prophylaxis" and "preclusion" are used synonymously and refer to the avoidance or reduction of the risk of contracting, experiencing, suffering from or having a disease, a condition, a disorder, an injury or a health problem, or a development or advancement of such states and/or the symptoms of such states.

The treatment or prevention of a disease, a condition, a disorder, an injury or a health problem may be partial or complete.

In addition, the compounds according to the invention have further advantageous properties, in particular with respect to their pulmoselective action (in contrast to a systemic action), their lung retention time and/or their duration of action following intrapulmonary administration.

The compounds according to the invention are particularly suitable for the treatment and/or prevention of cardiovascular and cardiac diseases, cardio-renal and kidney diseases, cardiopulmonary and lung diseases, thromboembolic diseases, fibrotic disorders and / or wound healing disorders..

The compounds according to the invention are particularly suitable for the treatment and/or prevention of cardiovascular and cardiac diseases, preferably heart failure with reduced and preserved ejection fraction (HFrEF and HFpEF), hypertension (HTN), peripheral arterial diseases (PAD, PAOD), cardio-renal and kidney diseases, preferably chronic and diabetic kidney disease (CKD and DKD), cardiopulmonary and lung diseases, preferable pulmonary hypertension (PH), and other diseases, preferably neurodegenerative diseases and different forms of dementias, fibrotic diseases, systemic sclerosis (SSc), sickle cell disease (SCD), wound healing disorders such as diabetic foot ulcer (DFU).

Accordingly, the compounds according to the invention can be used in medicaments for the treatment and/or prevention of cardiovascular,cardiopulmonary and cardiorenal disorders such as, for example high blood pressure (hypertension), heart failure, coronary heart disease, stable and unstable angina pectoris, pulmonary arterial hypertension (PAH) and secondary forms of pulmonary hypertension (PH), chronic thromboembolic pulmonary hypertension (CTEPH), renal, renovascular and treatment resistant hypertension, disorders of peripheral and cardiac vessels, arrhythmias, atrial and ventricular arrhythmias and impaired conduction such as, for example, grade I-III atrioventricular blocks, supraventricular tachyarrhythmia, atrial fibrillation, atrial flutter, ventricular fibrillation, ventricular flutter, ventricular tachyarrhythmia, Torsade de pointes tachycardia, atrial and ventricular extrasystoles, AV-junctional extrasystoles, sick sinus syndrome, syncopes, AV nodes reentry tachycardia, Wolff-Parkinson-White syndrome, acute coronary syndrome (ACS), autoimmune heart disorders (pericarditis, endocarditis, valvolitis, aortitis, cardiomyopathies), boxer cardiomyopathy, aneurysms, shock such as cardiogenic shock, septic shock and anaphylactic shock, furthermore for the treatment and/or prevention of thromboembolic disorders and ischaemias such as myocardial ischaemia, myocardial infarction, stroke, cardial hypertrophy, transistory and ischaemic attacks, pre-eclampsia, inflammatory cardiovascular disorders, spasms of the coronary arteries and the peripheral arteries, formation of oedemas such as, for example, pulmonary oedema, brain oedema, renal oedema or heart failure-induced oedema, impaired peripheral perfusion, reperfusion damage, arterial and venous thromboses, microalbuminuria, heart failure, endothelial dysfunction, micro- and macrovascular damage (vasculitis), and also for preventing restenoses for example after thrombolysis therapies, percutaneous transluminal angioplasties (PTA), percutaneous transluminal coronary angioplasties (PTCA), heart transplants and bypass operations.

In the context of the present invention, the term "pulmonary hypertension" encompasses both primary and secondary subforms thereof, as defined below by the Dana Point classification according to their respective aetiology [see D. Montana and G. Simonneau, in: A.J. Peacock et al. (Eds.), Pulmonary Circulation. Diseases and their treatment, 3rd edition, Hodder Arnold Publ., 2011, pp. 197-206; M.M. Hoeper et al., J. Am. Coll. Cardiol. 2009, 54 (1), S85-S96]. These include in particular in group 1 pulmonary arterial hypertension (PAH), which, among others, embraces the idiopathic and the familial forms (IPAH and FPAH, respectively). Furthermore, PAH also embraces persistent pulmonary hypertension of the newborn and the associated pulmonary arterial hypertension (APAH) associated with collagenoses, congenital systemic pulmonary shunt lesions, portal hypertension, HIV infections, the intake of certain drugs and medicaments (for example of appetite supressants), with disorders having a significant venous/capillary component such as pulmonary venoocclusive disorder and pulmonary capillary haemangiomatosis, or with other disorders such as disorders of the thyroid, glycogen storage diseases, Gaucher disease, hereditary teleangiectasia, haemoglobinopathies, myeloproliferative disorders and splenectomy. Group 2 of the Dana Point classification comprises PH patients having a causative left heart disorder, such as ventricular, atrial or valvular disorders. Group 3 comprises forms of pulmonary hypertension associated with a lung disorder, for example with chronic obstructive lung disease (COPD), interstitial lung disease (ILD), pulmonary fibrosis (IPF), and/or hypoxaemia (e.g. sleep apnoe syndrome, alveolar hypoventilation, chronic high-altitude sickness, hereditary deformities). Group 4 includes PH patients having chronic thrombotic and/or embolic disorders, for example in the case of thromboembolic obstruction of proximal and distal pulmonary arteries (CTEPH) or non-thrombotic embolisms (e.g. as a result of tumour disorders, parasites, foreign bodies). Less common forms of pulmonary hypertension, such as in patients suffering from sarcoidosis, histiocytosis X or lymphangio-matosis, are summarized in group 5.

In the context of the present invention, the term "heart failure" encompasses both acute and chronic forms of heart failure, and also more specific or related types of disease, such as acute decompensated heart failure, right heart failure, left heart failure, global heart failure, also diastolic heart failure and systolic heart failure, heart failure with reduced ejection fraction (HFrEF), heart failure with preserved ejection fraction (HFpEF), heart failure with mid-range efjection fraction (HFmEF), ischemic cardiomyopathy, dilated cardiomyopathy, hypertrophic cardiomyopathy, idiopathic cardiomyopathy, congenital heart defects and cardiomyopathies, heart valve defects, heart failure associated with heart valve defects, mitral valve stenosis, mitral valve insufficiency, aortic valve stenosis, aortic valve insufficiency, tricuspid valve stenosis, tricuspid valve insufficiency, pulmonary valve stenosis, pulmonary valve insufficiency, combined heart valve defects, myocardial inflammation (myocarditis), chronic myocarditis, acute myocarditis, viral myocarditis, diabetic heart failure, alcoholic cardiomyopathy, cardiac storage disorders.

In addition, the compounds according to the invention can also be used for treatment and/or prevention of arteriosclerosis, disturbed lipid metabolism, hypolipoproteinaemias, dyslipidaemias, hypertriglyceridaemias, hyperlipidaemias, combined hyperlipidaemias, hypercholesterolaemias, abetalipoproteinemia, sitosterolemia, xanthomatosis, Tangier disease, adiposity, obesity, and also of metabolic syndrome.

Furthermore, the compounds according to the invention can be used for treatment and/or prevention of primary and secondary Raynaud's phenomenon, of microcirculation disorders, claudication, hearing disorders, tinnitus, peripheral and autonomic neuropathies, diabetic microangiopathies, diabetic retinopathy, diabetic ulcers at the extremities, gangrene, CREST syndrome, erythematosis, onychomycosis and rheumatic disorders.

Futhermore, the compounds according to the invention can be used for the treatment of sickle cell disease (SCD), sickle cell anemia, and also other SCD-related disease symptoms (for example end organ damage affecting lung brain, kidney or heart) but also vasocclusive events or pain crisis, achalasia, hemolyis-induced vasculopathies for treating malaria, thalassemia, hemolytic uremic syndrome, paroxysmal nocturnal hemoglobinuria, drug-Induced hemolytic anemias or rhabdomyolsis. In addition, since similar above-mentioned pathophysiological mechanisms are effective when blood transfusions (for example by storage etc. with an elevated concentration of free Hb) are administered to patients having a transfusion indication, this compounds could be used for patients receiving a blood transfusion. Finally, in the future the combination of an sGC activator with a synthetic Hb-based oxygen carrier may mitigate the side effects hitherto observed [Weiskopf, Anaesthesia & Analgesia, 110:3; 659-661, 2010] which are caused by reduced availability of NO, thus allowing further clinical applications.

The compounds according to the invention can additionally also be used for preventing ischaemic and/or reperfusion-related damage to organs or tissues and also as additives for perfusion and preservation solutions of organs, organ parts, tissues or tissue parts of human or animal origin, in particular for surgical interventions or in the field of transplantation medicine.

Furthermore, the compounds according to the invention are suitable for treatment and/or prophylaxis of renal disorders, especially of renal insufficiency and kidney failure. In the context of the present invention, the terms renal insufficiency and kidney failure comprise both acute and chronic manifestations (chronic kidney disease; CKD) thereof, as well as underlying or related kidney diseases such as renal hypoperfusion, intradialytic hypotension, obstructive uropathy, glomerulopathies, glomerulonephritis, acute glomerulonephritis, glomerulosclerosis, tubulointerstitial diseases, nephropathic diseases such as primary and congenital kidney disease, nephritis, immunological kidney diseases such as kidney graft rejection and immunocomplex-induced kidney diseases, nephropathy induced by toxic substances, nephropathy induced by contrast agents, diabetic and non-diabetic nephropathy, diabetic kidney diseas (DKD), pyelonephritis, renal cysts and polycystic kidney disease, nephrosclerosis, hypertensive nephrosclerosis and nephrotic syndrome, which can be characterized diagnostically for example by abnormally reduced creatinine and/or water excretion, abnormally raised blood concentrations of urea, nitrogen, potassium and/or creatinine, altered activity of renal enzymes such as, for example, glutamyl synthetase, altered urine osmolarity or urine volume, increased microalbuminuria, macroalbuminuria, lesions on glomerulae and arterioles, tubular dilation, hyperphosphataemia and/or need for dialysis. The present invention also encompasses the use of the compounds according to the invention for treatment and/or prophylaxis of sequelae of renal insufficiency, for example hypertension, pulmonary oedema, heart failure, uremia, anemia, electrolyte disturbances (for example hypercalemia, hyponatremia) and disturbances in bone and carbohydrate metabolism.

In addition, the compounds according to the invention are suitable for treatment and/or prevention of urological disorders, for example benign prostate syndrome (BPS), benign prostate hyperplasia (BPH), benign prostate enlargement (BPE), bladder outlet obstruction (BOO), lower urinary tract syndrome (LUTS), prostatitis, neurogenic overactive bladder (OAB), incontinence, for example mixed, urge, stress or overflow incontinence (MUI, UUI, SUI, OUI), pelvic pain, interstitial cystitis (IC) and also erectile dysfunction and female sexual dysfunction.

The compounds according to the invention are also suitable for treatment and/or prevention of asthmatic disorders, chronic-obstructive pulmonary diseases (COPD), acute respiratory distress syndrome (ARDS) and acute lung injury (ALI), alpha-1 antitrypsin deficiency (AATD), pulmonary fibrosis, pulmonary emphysema (for example pulmonary emphysema induced by cigarette smoke) and cystic fibrosis (CF).

The compounds described in the present invention are also active compounds for control of central nervous system disorders characterized by disturbances of the NO/cGMP system. They are suitable in particular for improving perception, concentration, learning or memory after cognitive impairments like those occurring in particular in association with situations/diseases/syndromes such as mild cognitive impairment, age-associated learning and memory impairments, age-associated memory losses, vascular dementia, craniocerebral trauma, stroke, dementia occurring after strokes (post stroke dementia), post-traumatic craniocerebral trauma, general concentration impairments, concentration impairments in children with learning and memory problems, Alzheimer's disease, Lewy body dementia, dementia with degeneration of the frontal lobes including Pick's syndrome, Parkinson's disease, progressive nuclear palsy, dementia with corticobasal degeneration, amyolateral sclerosis (ALS), Huntington's disease, demyelination, multiple sclerosis, thalamic degeneration, Creutzfeld-Jacob dementia, HIV dementia, schizophrenia with dementia or Korsakoff's psychosis. They are also suitable for the treatment and/or prevention of central nervous system disorders such as states of anxiety, tension and depression, CNS-related sexual dysfunctions and sleep disturbances, and for controlling pathological disturbances of the intake of food, stimulants and addictive substances.

Furthermore, the compounds according to the invention are also suitable for regulation of cerebral blood flow and are thus effective agents for control of migraine. They are also suitable for the prophylaxis and control of sequelae of cerebral infarct (Apoplexia cerebri) such as stroke, cerebral ischaemias and craniocerebral trauma. The compounds according to the invention can likewise be used to control states of pain.

Moreover, the compounds according to the invention have antiinflammatory action and can therefore be used as antiinflammatories for treatment and/or prevention of sepsis (SIRS), multiple organ failure (MODS, MOF), inflammatory disorders of the kidney, chronic bowel inflammations (IBD, Crohn's Disease, UC), pancreatitis, peritonitis, rheumatoid disorders, inflammatory skin disorders and inflammatory eye disorders.

Furthermore, the compounds according to the invention are suitable for the treatment and/or prevention of fibrotic disorders of the internal organs, for example of the lung, of the heart, of the kidneys, of the bone marrow and especially of the liver, and also of dermatological fibroses and fibrotic disorders of the eye. In the context of the present inventions, the term "fibrotic disorders" encompasses especially disorders such as hepatic fibrosis, hepatic cirrhosis, non-alcoholic steato-hepatosis (NASH), pulmonary fibrosis, endomyocardial fibrosis, nephropathy, glomerulonephritis, interstitial renal fibrosis, fibrotic damage resulting from diabetes, myelofibrosis and similar fibrotic disorders, scleroderma, systemic sclerosis, morphea, keloids, hypertrophic scarring, naevi, diabetic retinopathy, proliferative vitreoretinopathy and disorders of the connective tissue (for example sarcoidosis). The compounds according to the invention can likewise be used for promoting wound healing including the healing of digital ulcer and diabeteic foot ulcer, for controlling postoperative scarring, for example resulting from glaucoma operations, and cosmetically for ageing and keratinized skin.

By virtue of their activity profile, the compounds according to the invention are particularly suitable for the treatment and/or prevention of cardiovascular and cardiopulmonary disorders such as primary and secondary forms of pulmonary hypertension, heart failure, angina pectoris and hypertension, and also for the treatment and/or prevention of thromboembolic disorders, ischaemias, vascular disorders, impaired microcirculation, renal insufficiency, fibrotic disorders and arteriosclerosis.

The present invention furthermore provides the use of the compounds according to the invention for the treatment and/or prevention of disorders, in particular the disorders mentioned above.

The present invention furthermore provides the use of the compounds according to the invention for preparing a medicament for the treatment and/or prevention of disorders, in particular the disorders mentioned above.

The present invention furthermore provides a medicament comprising at least one of the compounds according to the invention for the treatment and/or prevention of disorders, in particular the disorders mentioned above.

The present invention furthermore provides the use of the compounds according to the invention in a method for the treatment and/or prevention of disorders, in particular the disorders mentioned above.

The present invention furthermore provides a method for the treatment and/or prevention of disorders, in particular the disorders mentioned above, using an effective amount of at least one of the compounds according to the invention.

They are therefore suitable for use as medicaments for the treatment and/or prophylaxis of diseases in humans and animals.

The present invention further provides for the use of the compounds according to the invention for the treatment and/or prophylaxis of disorders, in particular cardiovascular disorders, preferably thrombotic or thromboembolic disorders and/or thrombotic or thromboembolic complications such as acute coronary syndrome or myocardial infarction or ischemic stroke or peripheral arterial occlusive disease , and/or diabetes, and/or urogenital disorders, in particular those associated with.

For the purpose of the present invention, the "thrombotic or thromboembolic disorders" include disorders which occur preferably in the arterial vasculature and which can be treated with the compounds according to the invention, in particular disorders leading to peripheral arterial occlusive disorders and in the coronary arteries of the heart, such as acute coronary syndrome (ACS), myocardial infarction with ST segment elevation (STEMI) and without ST segment elevation (non-STEMI), stable angina pectoris, unstable angina pectoris, reocclusions and restenoses after coronary interventions such as angioplasty, stent implantation or aortocoronary bypass, but also thrombotic or thromboembolic disorders in cerebrovascular arteries, such as transitory ischaemic attacks (TIA), ischemic strokes including cardioembolic strokes, such as strokes due to atrial fibrillation, non-cardioembolic strokes, such as lacunar stroke, strokes due to large or small artery diseases, or strokes due to undetermined cause, cryptogenic strokes, embolic strokes, embolic strokes of undetermined source, or events of thrombotic and/or thromboembolic origin leading to stroke or TIA.

Moreover, the compounds according to the invention are suitable in particular for the treatment and/or prophylaxis of disorders where, the pro-inflammatory component plays an essential role, including vasculitides like Kawasaki disease, Takayasu arteritis and Thrombangiitis obliterans (Buerger's disease) as well as inflammatory disorders like myocarditis.

Furthermore, the compounds according to the invention are suitable for the treatment and/or prophylaxis of disorders of the urogenital tract like overactive bladder, interstitial cystitis and bladder pain syndrome. Moreover, the compounds according to the invention are suitable for the treatment and/or prophylaxis of diabetes mellitus including its end-organ manifestations like diabetic retinopathy and diabetic nephropathy.

Furthermore, the compounds according to the invention are suitable in particular for the treatment and/or prophylaxis of neurological disorders like neuropathic pain, neurodegenerative disorders and dementias such as vascular dementia or Alzheimer's disease and Parkinson's disease.

Moreover, the compounds according to the invention are suitable in particular for the treatment and/or prophylaxis of pulmonologic disorders like chronic cough, asthma and COPD.

The present invention further provides for the use of the compounds according to the invention for the treatment and/or prophylaxis of disorders, especially the disorders mentioned above.

The present invention further provides for the use of the compounds according to the invention for production of a medicament for the treatment and/or prophylaxis of disorders, especially the disorders mentioned above.

The present invention further provides a method for the treatment and/or prophylaxis of disorders, especially the disorders mentioned above, using a therapeutically effective amount of a compound according to the invention.

The present invention further provides the compounds according to the invention for use in a method for the treatment and/or prophylaxis of disorders, especially the disorders mentioned above, using a therapeutically effective amount of a compound according to the invention.

Particularly the present invention provides the compounds according to the invention for use in a method for the treatment and/or prophylaxis of thrombotic or thromboembolic, in particular atherothrombotic disorders using a therapeutically effective amount of a compound according to the invention.

The present invention further provides medicaments comprising a compound according to the invention and one or more further active compounds.

In addition, the compounds according to the invention can also be used for preventing coagulation ex vivo, for example for the protection of organs to be transplanted against organ damage caused by formation of clots and for protecting the organ recipient against thromboemboli from the transplanted organ, for preserving blood and plasma products, for cleaning/pretreating catheters and other medical auxiliaries and instruments, for coating synthetic surfaces of medical auxiliaries and instruments used in vivo or ex vivo or for biological samples which may comprise factor XIa or plasma kallikrein.

The present invention furthermore provides a method for preventing the coagulation of blood in vitro, in particular in banked blood or biological samples which may comprise factor XIa or plasma kallikrein or both enzymes, which method is characterized in that an anticoagulatory effective amount of the compound according to the invention is added.

The compounds of the invention can act systemically and/or locally. For this purpose, they can be administered in a suitable manner, for example by the oral, parenteral, pulmonal, nasal, sublingual, lingual, buccal, rectal, dermal, transdermal, conjunctival or otic route, or as an implant or stent.

For these administration routes, it is possible for the compounds according to the invention to be administered in suitable administration forms.

For oral administration, it is possible to formulate the compounds according to the invention to dosage forms known in the art that deliver the compounds of the invention rapidly and/or in a modified manner, such as, for example, tablets (uncoated or coated tablets, for example with enteric or controlled release coatings that dissolve with a delay or are insoluble), orally-disintegrating tablets, films/wafers, films/lyophylisates, capsules (for example hard or soft gelatine capsules), sugar-coated tablets, granules, pellets, powders, emulsions, suspensions, aerosols or solutions. It is possible to incorporate the compounds according to the invention in crystalline and/or amorphised and/or dissolved form into said dosage forms.

Parenteral administration can be effected with avoidance of an absorption step (for example intravenous, intraarterial, intracardial, intraspinal or intralumbal) or with inclusion of absorption (for example intramuscular, subcutaneous, intracutaneous, percutaneous or intraperitoneal). Administration forms which are suitable for parenteral administration are, inter alia, preparations for injection and infusion in the form of solutions, suspensions, emulsions, lyophylisates or sterile powders.

Suitable for extraocular (topic) administration are administration forms which operate in accordance with the prior art, which release the active compound rapidly and/or in a modified or controlled manner and which contain the active compound in crystalline and/or amorphized and/or dissolved form such as, for example, eye drops, sprays and lotions (e.g. solutions, suspensions, vesicular/colloidal systems, emulsions, aerosols), powders for eye drops, sprays and lotions (e.g. ground active compound, mixtures, lyophilisates, precipitated active compound), semisolid eye preparations (e.g. hydrogels, in-situ hydrogels, creams and ointments), eye inserts (solid and semisolid preparations, e.g. bioadhesives, films/wafers, tablets, contact lenses).

Intraocular administration includes, for example, intravitreal, subretinal, subscleral, intrachoroidal, subconjunctival, retrobulbar and subtenon administration. Suitable for intraocular administration are administration forms which operate in accordance with the prior art, which release the active compound rapidly and/or in a modified or controlled manner and which contain the active compound in crystalline and/or amorphized and/or dissolved form such as, for example, preparations for injection and concentrates for preparations for injection (e.g. solutions, suspensions, vesicular/colloidal systems, emulsions), powders for preparations for injection (e.g. ground active compound, mixtures, lyophilisates, precipitated active compound), gels for preparations for injection (semisolid preparations, e.g. hydrogels, in-situ hydrogels) and implants (solid preparations, e.g. biodegradable and nonbiodegradable implants, implantable pumps).

Preference is given to oral administration.

Examples which are suitable for other administration routes are pharmaceutical forms for inhalation [inter alia powder inhalers, nebulizers], nasal drops, nasal solutions, nasal sprays; tablets/films/wafers/capsules for lingual, sublingual or buccal administration; suppositories; eye drops, eye ointments, eye baths, ocular inserts, ear drops, ear sprays, ear powders, ear-rinses, ear tampons; vaginal capsules, aqueous suspensions (lotions, mixturae agitandae), lipophilic suspensions, emulsions, ointments, creams, transdermal therapeutic systems (such as, for example, patches), milk, pastes, foams, dusting powders, implants or stents.

The compounds according to the invention can be incorporated into the stated administration forms. This can be effected in a manner known per se by mixing with pharmaceutically suitable excipients. Pharmaceutically suitable excipients include, inter alia,
- fillers and carriers (for example cellulose, microcrystalline cellulose (such as, for example, Avicel^{®}), lactose, mannitol, starch, calcium phosphate (such as, for example, Di-Cafos^{®})),
- ointment bases (for example petroleum jelly, paraffins, triglycerides, waxes, wool wax, wool wax alcohols, lanolin, hydrophilic ointment, polyethylene glycols),
- bases for suppositories (for example polyethylene glycols, cacao butter, hard fat),
- solvents (for example water, ethanol, isopropanol, glycerol, propylene glycol, medium chain-length triglycerides fatty oils, liquid polyethylene glycols, paraffins),
- surfactants, emulsifiers, dispersants or wetters (for example sodium dodecyl sulfate), lecithin, phospholipids, fatty alcohols (such as, for example, Lanette^{®}), sorbitan fatty acid esters (such as, for example, Span^{®}), polyoxyethylene sorbitan fatty acid esters (such as, for example, Tween^{®}), polyoxyethylene fatty acid glycerides (such as, for example, Cremophor^{®}), polyoxethylene fatty acid esters, polyoxyethylene fatty alcohol ethers, glycerol fatty acid esters, poloxamers (such as, for example, Pluronic^{®}),
- buffers, acids and bases (for example phosphates, carbonates, citric acid, acetic acid, hydrochloric acid, sodium hydroxide solution, ammonium carbonate, trometamol, triethanolamine),
- isotonicity agents (for example glucose, sodium chloride),
- adsorbents (for example highly-disperse silicas),
- viscosity-increasing agents, gel formers, thickeners and/or binders (for example polyvinylpyrrolidone, methylcellulose, hydroxypropylmethylcellulose, hydroxypropylcellulose, carboxymethylcellulose-sodium, starch, carbomers, polyacrylic acids (such as, for example, Carbopol^{®}); alginates, gelatine),
- disintegrants (for example modified starch, carboxymethylcellulose-sodium, sodium starch glycolate (such as, for example, Explotab^{®}), cross- linked polyvinylpyrrolidone, croscarmellose-sodium (such as, for example, AcDiSol^{®})),
- flow regulators, lubricants, glidants and mould release agents (for example magnesium stearate, stearic acid, talc, highly-disperse silicas (such as, for example, Aerosil^{®})),
- coating materials (for example sugar, shellac) and film formers for films or diffusion membranes which dissolve rapidly or in a modified manner (for example polyvinylpyrrolidones (such as, for example, Kollidon^{®}), polyvinyl alcohol, hydroxypropylmethylcellulose, hydroxypropylcellulose, ethylcellulose, hydroxypropylmethylcellulose phthalate, cellulose acetate, cellulose acetate phthalate, polyacrylates, polymethacrylates such as, for example, Eudragit^{®})),
- capsule materials (for example gelatine, hydroxypropylmethylcellulose),
- synthetic polymers (for example polylactides, polyglycolides, polyacrylates, polymethacrylates (such as, for example, Fudragit^{®}), polyvinylpyrrolidones (such as, for example, Kollidon^{®}), polyvinyl alcohols, polyvinyl acetates, polyethylene oxides, polyethylene glycols and their copolymers and blockcopolymers),
- plasticizers (for example polyethylene glycols, propylene glycol, glycerol, triacetine, triacetyl citrate, dibutyl phthalate),
- penetration enhancers,
- stabilisers (for example antioxidants such as, for example, ascorbic acid, ascorbyl palmitate, sodium ascorbate, butylhydroxyanisole, butylhydroxytoluene, propyl gallate),
- preservatives (for example parabens, sorbic acid, thiomersal, benzalkonium chloride, chlorhexidine acetate, sodium benzoate),
- colourants (for example inorganic pigments such as, for example, iron oxides, titanium dioxide),
- flavourings, sweeteners, flavour- and/or odour-masking agents.

The present invention furthermore relates to a pharmaceutical composition which comprises at least one compound according to the invention, conventionally together with one or more pharmaceutically suitable excipient(s), and to their use according to the present invention.

An embodiment of the invention are pharmaceutical compositions comprising at least one compound of formula (I) according to the invention, preferably together with at least one inert, non-toxic, pharmaceutically suitable auxiliary, and the use of these pharmaceutical compositions for the above cited purposes.

In accordance with another aspect, the present invention covers pharmaceutical combinations, in particular medicaments, comprising at least one compound of general formula (I) of the present invention and at least one or more further active ingredients, in particular for the treatment and/or prophylaxis of cardiovascular disorders, preferably thrombotic or thromboembolic disorders, and diabetes, and also urogenital and ophthalmic disorders.

The term "combination" in the present invention is used as known to persons skilled in the art, it being possible for said combination to be a fixed combination, a non-fixed combination or a kit-of-parts.

A "fixed combination" in the present invention is used as known to persons skilled in the art and is defined as a combination wherein, for example, a first active ingredient, such as one or more compounds of general formula (I) of the present invention, and a further active ingredient are present together in one unit dosage or in one single entity. One example of a "fixed combination" is a pharmaceutical composition wherein a first active ingredient and a further active ingredient are present in admixture for simultaneous administration, such as in a formulation. Another example of a "fixed combination" is a pharmaceutical combination wherein a first active ingredient and a further active ingredient are present in one unit without being in admixture.

A non-fixed combination or "kit-of-parts" in the present invention is used as known to persons skilled in the art and is defined as a combination wherein a first active ingredient and a further active ingredient are present in more than one unit. One example of a non-fixed combination or kit-of-parts is a combination wherein the first active ingredient and the further active ingredient are present separately. It is possible for the components of the non-fixed combination or kit-of-parts to be administered separately, sequentially, simultaneously, concurrently or chronologically staggered.

The inventive compounds can be employed alone or, if required, in combination with other active ingredients. The present invention further provides medicaments comprising at least one of the inventive compounds and one or more further active ingredients, especially for treatment and/or prophylaxis of the aforementioned disorders. Preferred examples of suitable active ingredient combinations include:
- organic nitrates and NO donors, for example sodium nitroprusside, nitroglycerin, isosorbide mononitrate, isosorbide dinitrate, molsidomine or SIN-1, and inhaled NO;
- compounds which inhibit the breakdown of cyclic guanosine monophosphate (cGMP), for example inhibitors of phosphodiesterases (PDE) 1, 2, 5 and/or 9, especially PDE 5 inhibitors such as sildenafil, vardenafil, tadalafil, udenafil, desantafil, avanafil, mirodenafil, lodenafil or PF-00489791;
- compounds which inhibit the breakdown of cyclic adenosine monophosphate (cAMP), for example inhibitors of phosphodiesterases (PDE) 3 and 4, especially cilostatzole, milrinone, roflumilast, apremilast, or crisaborole;

- hypotensive active ingredients, by way of example and with preference from the group of the calcium antagonists, angiotensin All antagonists, ACE inhibitors, NEP-inhibitors, vasopeptidase-inhibitors, endothelin antagonists, renin inhibitors, alpha-receptor blockers, beta-receptor blockers, mineralocorticoid receptor antagonists, rho-kinase-inhibitors and the diuretics;
- antiarrhythmic agents, by way of example and with preference from the group of sodium channel blocker, beta-receptor blocker, potassium channel blocker, calcium antagonists, If-channel blocker, digitalis, parasympatholytics (vagoliytics), sympathomimetics and other antiarrhythmics as adenosin, adenosine receptor agonists as well as vemakalant;
- positive-inotrop agents, by way of example cardiac glycoside (Dogoxin), beta-adrenergic and dopaminergic agonists, such as isoprenalin, adrenalin, noradrenalin, dopamin or dobutamin;
- vasopressin-receptor-antagonists, by way of example and with preference from the group of conivaptan, tolvaptan, lixivaptan, mozavaptan, satavaptan, SR-121463, RWJ 676070 or BAY 86-8050, as well as the compounds described in WO 2010/105770, WO2011/104322 and WO 2016/071212;
- active ingredients which alter lipid metabolism, for example and with preference from the group of the thyroid receptor agonists, cholesterol synthesis inhibitors such as, by way of example and preferably, HMG-CoA reductase inhibitors or squalene synthesis inhibitors, of ACAT inhibitors, CETP inhibitors, MTP inhibitors, PPAR-alpha, PPAR-gamma and/or PPAR-delta agonists, cholesterol absorption inhibitors, lipase inhibitors, polymeric bile acid adsorbents, bile acid reabsorption inhibitors and lipoprotein(a) antagonists.
- bronchodilatory agents, for example and with preference from the group of the beta-adrenergic rezeptor-agonists, such as, by way of example and preferably, albuterol, isoproterenol, metaproterenol, terbutalin, formoterol or salmeterol, or from the group of the anticholinergics, such as, by way of example and preferably, ipratropiumbromid;
- anti-inflammatory agents, for example and with preference from the group of the glucocorticoids, such as, by way of example and preferably, prednison, prednisolon, methylprednisolon, triamcinolon, dexamethason, beclomethason, betamethason, flunisolid, budesonid or fluticason as well as the non-steroidal anti-inflammatory agents (NSAIDs), by way of example and preferably, acetyl salicylic acid (aspirin), ibuprofen and naproxen, 5-amino salicylic acid-derivates, leukotriene-antagonists, TNFalpha-inhibitors and chemokin-receptor antagonists, such as CCR1, 2 and/or 5 inhibitors;
- agents modulating the immune system, for example immunoglobulins;
- agents that inhibit the signal transductions cascade, for example and with preference from the group of the kinase inhibitors, by way of example and preferably, from the group of the tyrosine kinase- and/or serine/threonine kinase inhibitors;
- agents, that inhibit the degradation and modification of the extracellular matrix, for example and with preference from the group of the inhibitors of the matrix-metalloproteases (MMPs), by way of example and preferably, inhibitors of chymasee, stromelysine, collagenases, gelatinases and aggrecanases (with preference from the group of MMP-1, MMP-3, MMP-8, MMP-9, MMP-10, MMP-11 and MMP-13) as well as of the metallo-elastase (MMP-12) and neutrophil-elastase (HNE), as for example sivelestat or DX-890;
- agents, that block the bindung of serotonin to its receptor, for example and with preference antagonists of the 5-HT2b-receptor;
- organic nitrates and NO-donators, for example and with preference sodium nitroprussid, nitroglycerine, isosorbid mononitrate, isosorbid dinitrate, molsidomine or SIN-1, as well as inhaled NO;
- NO-independent, but heme-dependent stimulators of the soluble guanylate cyclase, for example and with preference the compounds described in WO 00/06568, WO 00/06569, WO 02/42301, WO 03/095451, WO 2011/147809, WO 2012/004258, WO 2012/028647 and WO 2012/059549;
- NO-independent and heme-independent activators of the soluble guanylate cyclase, for example and with preference the compounds described in WO 01/19355, WO 01/19776, WO 01/19778, WO 01/19780, WO 02/070462 and WO 02/070510 beschriebenen Verbindungen;
- agents, that stimulates the synthesis of cGMP, like for example sGC modulators, for example and with preference riociguat, cinaciguat, vericiguat or runcaciguat;
- prostacyclin-analogs, for example and with preference iloprost, beraprost, treprostinil or epoprostenol;
- agents, that inhibit soulble epoxidhydrolase (sEH), for example and with preference N,N'-Dicyclohexyl urea, 12-(3-Adamantan-1-yl-ureido)-dodecanic acid or l-Adamantan-l-yl-3-{5-[2-(2-ethoxyethoxy)ethoxy]pentyl} -urea;
- agents that interact with glucose metabolism, for example and with preference insuline, biguanide, thiazolidinedione, sulfonyl urea, acarbose, DPP4 inhibitors, GLP-1 analogs or SGLT-2 inhibitors, for example empagliflozin, dapagliflozin, canagliflozin;
- natriuretic peptides, for example and with preference atrial natriuretic peptide (ANP), natriuretic peptide type B (BNP, Nesiritid) natriuretic peptide type C (CNP) or urodilatin;
- activators of the cardiac myosin, for example and with preference omecamtiv mecarbil (CK-1827452);
- calcium-sensitizers, for example and with preference levosimendan;
- agents that affect the energy metabolism of the heart, for example and with preference etomoxir, dichloroacetat, ranolazine or trimetazidine, full or partial adenosine A1 receptor agonists such as GS-9667 (formerly known as CVT-3619), capadenoson, neladenoson and neladenoson bialanate;
- agents that affect the heart rate, for example and with preference ivabradin;
- cyclooxygenase inhibitors such as, for example, bromfenac and nepafenac;
- inhibitors of the kallikrein-kinin system such as, for example, safotibant and ecallantide;
- inhibitors of the sphingosine 1-phosphate signal paths such as, for example, sonepcizumab;
- inhibitors of the complement-C5a receptor such as, for example, eculizumab;
- plasminogen activators (thrombolytics/fibrinolytics) and compounds which promote thrombolysis/fibrinolysis such as inhibitors of the plasminogen activator inhibitor (PAI inhibitors) or inhibitors of the thrombin-activated fibrinolysis inhibitor (TAFI inhibitors) such as, for example, tissue plasminogen activator (t-PA, for example Actilyse^{®}), streptokinase, reteplase and urokinase or plasminogen-modulating substances causing increased formation of plasmin;
- anticoagulatory substances (anticoagulants) such as, for example, heparin (UFH), low-molecular-weight heparins (LMW), for example tinzaparin, certoparin, parnaparin, nadroparin, ardeparin, enoxaparin, reviparin, dalteparin, danaparoid, semuloparin (AVE 5026), adomiparin (M118) and EP-42675/ORG42675;
- direct thrombin inhibitors (DTI) such as, for example, Pradaxa (dabigatran), atecegatran (AZD-0837), DP-4088, SSR-182289A, argatroban, bivalirudin and tanogitran (BIBT-986 and prodrug BIBT-1011) and hirudin;
- direct factor Xa inhibitors such as, for example, rivaroxaban, apixaban, edoxaban (DU-176b), betrixaban (PRT-54021), R-1663, darexaban (YM-150), otamixaban (FXV-673/RPR-130673), letaxaban (TAK-442), razaxaban (DPC-906), DX-9065a, LY-517717, tanogitran (BIBT-986, prodrug: BIBT-101 1), idraparinux and fondaparinux;
- inhibitors of coagulation factor XI and XIa such as, for example, FXI ASO-LICA, BAY 121-3790, MAA868, BMS986177, EP-7041 and AB-022;
- substances which inhibit the aggregation of platelets (platelet aggregation inhibitors, thrombocyte aggregation inhibitors), such as, for example, acetylsalicylic acid (such as, for example, aspirin), P2Y12 antagonists such as, for example, ticlopidine (Ticlid), clopidogrel (Plavix), prasugrel, ticagrelor, cangrelor and elinogrel, and PAR-1 antagonists such as, for example, vorapaxar, and PAR-4 antagonists;
- platelet adhesion inhibitors such as GPVI and/or GPIb antagonists such as, for example, Revacept or caplacizumab;
- fibrinogen receptor antagonists (glycoprotein-IIb/IIIa antagonists) such as, for example, abciximab, eptifibatide, tirofiban, lamifiban, lefradafiban and fradafiban;
- recombinant human activated protein C such as, for example, Xigris or recombinant thrombomodulin.

Antithrombotic agents are preferably understood to mean compounds from the group of the platelet aggregation inhibitors, the anticoagulants or the profibrinolytic substances.

In a preferred embodiment of the invention, the inventive compounds are administered in combination with a platelet aggregation inhibitor, by way of example and with preference aspirin, clopidogrel, prasugrel, ticagrelor, ticlopidin or dipyridamole.

In a preferred embodiment of the invention, the inventive compounds are administered in combination with a thrombin inhibitor, by way of example and with preference ximelagatran, dabigatran, melagatran, bivalirudin or clexane.

In a preferred embodiment of the invention, the inventive compounds are administered in combination with a GPIIb/IIIa antagonist such as, by way of example and with preference, tirofiban or abciximab.

In a preferred embodiment of the invention, the inventive compounds are administered in combination with a factor Xa inhibitor, by way of example and with preference rivaroxaban (BAY 59-7939), DU-176b, apixaban, betrixaban, otamixaban, fidexaban, razaxaban, letaxaban, eribaxaban, fondaparinux, idraparinux, PMD-3112, darexaban (YM-150), KFA-1982, EMD-503982, MCM-17, MLN-1021, DX 9065a, DPC 906, JTV 803, SSR-126512 or SSR-128428.

In a preferred embodiment of the invention, the inventive compounds are administered in combination with a factor XI or factor XIa inhibitor, by way of example and with preference FXI ASO-LICA, BAY 121-3790, MAA868, BMS986177, EP-7041 or AB-022.

In a preferred embodiment of the invention, the inventive compounds are administered in combination with heparin or with a low molecular weight (LMW) heparin derivative.

In a preferred embodiment of the invention, the inventive compounds are administered in combination with a vitamin K antagonist, by way of example and with preference coumarin.

Hypotensive agents are preferably understood to mean compounds from the group of the calcium antagonists, angiotensin All antagonists, ACE inhibitors, endothelin antagonists, renin inhibitors, alpha-receptor blockers, beta-receptor blockers, mineralocorticoid receptor antagonists, rho-kinase inhibitors and the diuretics.

In a preferred embodiment of the invention, the inventive compounds are administered in combination with a calcium antagonist, by way of example and with preference nifedipine, amlodipine, verapamil or diltiazem.

In a preferred embodiment of the invention, the inventive compounds are administered in combination with an alpha-1-receptor blocker, by way of example and with preference prazosin.

In a preferred embodiment of the invention, the inventive compounds are administered in combination with a beta-receptor blocker, by way of example and with preference propranolol, atenolol, timolol, pindolol, alprenolol, oxprenolol, penbutolol, bupranolol, metipranolol, nadolol, mepindolol, carazalol, sotalol, metoprolol, betaxolol, celiprolol, bisoprolol, carteolol, esmolol, labetalol, carvedilol, adaprolol, landiolol, nebivolol, epanolol or bucindolol.

In a preferred embodiment of the invention, the inventive compounds are administered in combination with an angiotensin All antagonist, by way of example and with preference losartan, candesartan, valsartan, telmisartan or embusartan or a dual angiotensin All antagonist/neprilysin-inhibitor, by way of example and with preference LCZ696 (valsartan/sacubitril).

In a preferred embodiment of the invention, the inventive compounds are administered in combination with an ACE inhibitor, by way of example and with preference enalapril, captopril, lisinopril, ramipril, delapril, fosinopril, quinopril, perindopril or trandopril.

In a preferred embodiment of the invention, the inventive compounds are administered in combination with an endothelin antagonist, by way of example and with preference bosentan, darusentan, ambrisentan or sitaxsentan.

In a preferred embodiment of the invention, the inventive compounds are administered in combination with a renin inhibitor, by way of example and with preference aliskiren, SPP-600 or SPP-800.

In a preferred embodiment of the invention, the inventive compounds are administered in combination with a mineralocorticoid receptor antagonist, by way of example and with preference spironolactone, AZD9977, finerenone or eplerenone.

In a preferred embodiment of the invention, the inventive compounds are administered in combination with a loop diuretic, for example furosemide, torasemide, bumetanide and piretanide, with potassium-sparing diuretics, for example amiloride and triamterene, with aldosterone antagonists, for example spironolactone, potassium canrenoate and eplerenone, and also thiazide diuretics, for example hydrochlorothiazide, chlorthalidone, xipamide and indapamide.

Lipid metabolism modifiers are preferably understood to mean compounds from the group of the CETP inhibitors, thyroid receptor agonists, cholesterol synthesis inhibitors such as HMG-CoA reductase inhibitors or squalene synthesis inhibitors, the ACAT inhibitors, MTP inhibitors, PPAR-alpha, PPAR-gamma and/or PPAR-delta agonists, cholesterol absorption inhibitors, polymeric bile acid adsorbents, bile acid reabsorption inhibitors, lipase inhibitors and the lipoprotein(a) antagonists.

In a preferred embodiment of the invention, the inventive compounds are administered in combination with a CETP inhibitor, by way of example and with preference dalcetrapib,anacetrapib, torcetrapib (CP-529 414), JJT-705 or CETP vaccine (Avant).

In a preferred embodiment of the invention, the inventive compounds are administered in combination with a thyroid receptor agonist, by way of example and with preference D-thyroxine, 3,5,3'-triiodothyronine (T3), CGS 23425 or axitirome (CGS 26214).

In a preferred embodiment of the invention, the inventive compounds are administered in combination with an HMG-CoA reductase inhibitor from the class of statins, by way of example and with preference lovastatin, simvastatin, pravastatin, fluvastatin, atorvastatin, rosuvastatin or pitavastatin.

In a preferred embodiment of the invention, the inventive compounds are administered in combination with a squalene synthesis inhibitor, by way of example and with preference BMS-188494 or TAK-475.

In a preferred embodiment of the invention, the inventive compounds are administered in combination with an ACAT inhibitor, by way of example and with preference avasimibe, melinamide, pactimibe, eflucimibe or SMP-797.

In a preferred embodiment of the invention, the inventive compounds are administered in combination with an MTP inhibitor, by way of example and with preference implitapide, BMS-201038, R-103757 or JTT-130.

In a preferred embodiment of the invention, the inventive compounds are administered in combination with a PPAR-gamma agonist, by way of example and with preference pioglitazone or rosiglitazone.

In a preferred embodiment of the invention, the inventive compounds are administered in combination with a PPAR-delta agonist, by way of example and with preference GW 501516 or BAY 68-5042.

In a preferred embodiment of the invention, the inventive compounds are administered in combination with a cholesterol absorption inhibitor, by way of example and with preference ezetimibe, tiqueside or pamaqueside.

In a preferred embodiment of the invention, the inventive compounds are administered in combination with a lipase inhibitor, a preferred example being orlistat.

In a preferred embodiment of the invention, the inventive compounds are administered in combination with a polymeric bile acid adsorbent, by way of example and with preference cholestyramine, colestipol, colesolvam, CholestaGel or colestimide.

In a preferred embodiment of the invention, the inventive compounds are administered in combination with a bile acid reabsorption inhibitor, by way of example and with preference ASBT (= IBAT) inhibitors, for example AZD-7806, S-8921, AK-105, BARI-1741, SC-435 or SC-635.

In a preferred embodiment of the invention, the inventive compounds are administered in combination with a lipoprotein(a) antagonist, by way of example and with preference, gemcabene calcium (CI-1027) or nicotinic acid.

In a preferred embodiment of the invention, the inventive compounds are administered in combination with a lipoprotein(a) antagonist, by way of example and with preference, gemcabene calcium (CI-1027) or nicotinic acid.

In a preferred embodiment of the invention, the inventive compounds are administered in combination with sGC modulators, by way of example and with preference, riociguat, cinaciguat or vericiguat.

In a preferred embodiment of the invention, the inventive compounds are administered in combination with an agent affecting the glucose metabolism, by way of example and with preference, insuline, a sulfonyl urea, acarbose, DPP4 inhibitors, GLP-1 analogs or SGLT-1 inhibitors empagliflozin, dapagliflozin, canagliflozin.

In a preferred embodiment of the invention, the compounds according to the invention are administered in combination with a TGFbeta antagonist, by way of example and with preference pirfenidone or fresolimumab.

In a preferred embodiment of the invention, the compounds according to the invention are administered in combination with a CCR2 antagonist, by way of example and with preference CCX-140.

In a preferred embodiment of the invention, the compounds according to the invention are administered in combination with a TNFalpha antagonist, by way of example and with preference adalimumab.

In a preferred embodiment of the invention, the compounds according to the invention are administered in combination with a galectin-3 inhibitor, by way of example and with preference GCS-100.

In a preferred embodiment of the invention, the compounds according to the invention are administered in combination with a Nrf-2 inhibitor, by way of example and with preference bardoxolone In a preferred embodiment of the invention, the compounds according to the invention are administered in combination with a BMP-7 agonist, by way of example and with preference THR-184.

In a preferred embodiment of the invention, the compounds according to the invention are administered in combination with a NOXl/4 inhibitor, by way of example and with preference GKT-137831.

In a preferred embodiment of the invention, the compounds according to the invention are administered in combination with a medicament which affects the vitamin D metabolism, by way of example and with preference calcitriol, alfacalcidol, doxercalciferol, maxacalcitol, paricalcitol, cholecalciferol or paracalcitol.

In a preferred embodiment of the invention, the compounds according to the invention are administered in combination with a cytostatic agent, by way of example and with preference cyclophosphamide.

In a preferred embodiment of the invention, the compounds according to the invention are administered in combination with an immunosuppressive agent, by way of example and with preference ciclosporin.

In a preferred embodiment of the invention, the compounds according to the invention are administered in combination with a phosphate binder, by way of example and with preference colestilan, sevelamer hydrochloride and sevelamer carbonate, Lanthanum and lanthanum carbonate.

In a preferred embodiment of the invention, the compounds according to the invention are administered in combination with renal proximal tubule sodium-phosphate co-transporter, by way of example and with preference, niacin or nicotinamide.

In a preferred embodiment of the invention, the compounds according to the invention are administered in combination with a calcimimetic for therapy of hyperparathyroidism.

In a preferred embodiment of the invention, the compounds according to the invention are administered in combination with agents for iron deficit therapy, by way of example and with preference iron products.

In a preferred embodiment of the invention, the compounds according to the invention are administered in combination with agents for the therapy of hyperurikaemia, by way of example and with preference allopurinol or rasburicase.

In a preferred embodiment of the invention, the compounds according to the invention are administered in combination with glycoprotein hormone for the therapy of anaemia, by way of example and with preference erythropoietin daprodustat, molidustat, roxadustat, vadadustat, desidustat..

In a preferred embodiment of the invention, the compounds according to the invention are administered in combination with biologics for immune therapy, by way of example and with preference abatacept, rituximab, eculizumab or belimumab.

In a preferred embodiment of the invention, the compounds according to the invention are administered in combination with vasopressin antagonists (group of the vaptanes) for the treatment of heart failure, by way of example and with preference tolvaptan, conivaptan, lixivaptan, mozavaptan, satavaptan or relcovaptan.

In a preferred embodiment of the invention, the compounds according to the invention are administered in combination with Jak inhibitors, by way of example and with preference ruxolitinib, tofacitinib, baricitinib, CYT387, GSK2586184, lestaurtinib, pacritinib (SB1518) or TG101348.

In a preferred embodiment of the invention, the compounds according to the invention are administered in combination with prostacyclin analogs for therapy of microthrombi.

In a preferred embodiment of the invention, the compounds according to the invention are administered in combination with an alkali therapy, by way of example and with preference sodium bicarbonate.

In a preferred embodiment of the invention, the compounds according to the invention are administered in combination with an mTOR inhibitor, by way of example and with preference everolimus or rapamycin.

In a preferred embodiment of the invention, the compounds according to the invention are administered in combination with an NHE3 inhibitor, by way of example and with preference AZD1722 or tenapanor.

In a preferred embodiment of the invention, the compounds according to the invention are administered in combination with an eNOS modulator, by way of example and with preference sapropterin.

In a preferred embodiment of the invention, the compounds according to the invention are administered in combination with a CTGF inhibitor, by way of example and with preference FG-3019.

The total amount of the active ingredient to be administered will generally range from about 0.001 mg/kg to about 200 mg/kg body weight per day, and preferably from about 0.01 mg/kg to about 50 mg/kg body weight per day, and more preferably from about 0.01 mg/kg to about 20 mg/kg body weight per day. Clinically useful dosing schedules will range from one to three times a day dosing to once every four weeks dosing. In addition, it is possible for "drug holidays", in which a patient is not dosed with a drug for a certain period of time, to be beneficial to the overall balance between pharmacological effect and tolerability. It is possible for a unit dosage to contain from about 0.5 mg to about 1500 mg of active ingredient, and can be administered one or more times per day or less than once a day. The average daily dosage for administration by injection, including intravenous, intramuscular, subcutaneous and parenteral injections, and use of infusion techniques will preferably be from 0.01 to 200 mg/kg of total body weight. The average daily rectal dosage regimen will preferably be from 0.01 to 200 mg/kg of total body weight. The average daily vaginal dosage regimen will preferably be from 0.01 to 200 mg/kg of total body weight. The average daily topical dosage regimen will preferably be from 0.1 to 200 mg administered between one to four times daily. The transdermal concentration will preferably be that required to maintain a daily dose of from 0.01 to 200 mg/kg. The average daily inhalation dosage regimen will preferably be from 0.01 to 100 mg/kg of total body weight.

Of course the specific initial and continuing dosage regimen for each patient will vary according to the nature and severity of the condition as determined by the attending diagnostician, the activity of the specific compound employed, the age and general condition of the patient, time of administration, route of administration, rate of excretion of the drug, drug combinations, and the like. The desired mode of treatment and number of doses of a compound of the present invention or a pharmaceutically acceptable salt or ester or composition thereof can be ascertained by those skilled in the art using conventional treatment tests.

Nevertheless, it may optionally be necessary to deviate from the stated amounts, namely depending on body weight, route of administration, individual response to the active substance, type of preparation and time point or interval when application takes place. Thus, in some cases it may be sufficient to use less than the aforementioned minimum amount, whereas in other cases the stated upper limit must be exceeded. When applying larger amounts, it may be advisable to distribute these in several individual doses throughout the day.

According to a further embodiment, the compounds of formula (I) according to the invention are administered orally once or twice or three times a day. According to a further embodiment, the compounds of formula (I) according to the invention are administered orally once or twice a day. According to a further embodiment, the compounds of formula (I) according to the invention are administered orally once a day. For the oral administration, a rapid release or a modified release dosage form may be used.

Unless stated otherwise, the percentages in the tests and examples which follow are percentages by weight; parts are parts by weight. Solvent ratios, dilution ratios and concentration data for the liquid/liquid solutions are based in each case on volume, "w/v" means "weight/volume". For example, "10% w/v" means: 100 ml of solution or suspension comprise 10 g of substance.

### EXPERIMENTAL SECTION

### EXPERIMENTAL SECTION - GENERAL PART

NMR peak forms are stated as they appear in the spectra, possible higher order effects have not been considered.

The 1H-NMR data of selected compounds are listed in the form of 1H-NMR peaklists. For each signal peak the δ value in ppm is given, followed by the signal intensity, reported in round brackets. The δ value-signal intensity pairs from different peaks are separated by commas. Therefore, a peaklist is described by the general form: δ1 (intensity1), δ2 (intensity2), ... , δi (intensityi), ... , δn (intensityn).

The intensity of a sharp signal correlates with the height (in cm) of the signal in a printed NMR spectrum. When compared with other signals, this data can be correlated to the real ratios of the signal intensities. In the case of broad signals, more than one peak, or the center of the signal along with their relative intensity, compared to the most intense signal displayed in the spectrum, are shown. A 1H-NMR peaklist is similar to a classical 1H-NMR readout, and thus usually contains all the peaks listed in a classical NMR interpretation. Moreover, similar to classical 1H-NMR printouts, peaklists can show solvent signals, signals derived from stereoisomers of target compounds (also the subject of the invention), and/or peaks of impurities. The peaks of stereoisomers, and/or peaks of impurities are typically displayed with a lower intensity compared to the peaks of the target compounds (e.g., with a purity of >90%). Such stereoisomers and/or impurities may be typical for the particular manufacturing process, and therefore their peaks may help to identify the reproduction of our manufacturing process on the basis of "by-product fingerprints". An expert who calculates the peaks of the target compounds by known methods (MestReC, ACD simulation, or by use of empirically evaluated expectation values), can isolate the peaks of target compounds as required, optionally using additional intensity filters. Such an operation would be similar to peak-picking in classical 1H-NMR interpretation. A detailed description of the reporting of NMR data in the form of peaklists can be found in the publication "Citation of NMR Peaklist Data within Patent Applications" (cf. Research Disclosure Database Number 605005, 2014, 01 Aug 2014, or http://www.researchdisclosure.com/searching-disclosures). In the peak picking routine, as described in the Research Disclosure Database Number 605005, the parameter "MinimumHeight" can be adjusted between 1% and 4%. Depending on the chemical structure and/or depending on the concentration of the measured compound it may be reasonable to set the parameter "MinimumHeight" <1%.

Chemical names were generated using the ACD/Name software from ACD/Labs. In some cases generally accepted names of commercially available reagents were used in place of ACD/Name generated names.

The following table 1 lists the abbreviations used in this paragraph and in the Examples section as far as they are not explained within the text body. Other abbreviations have their meanings customary per se to the skilled person.

**Table 1: Abbreviations**

| The following table lists the abbreviations used herein. | |
|---|---|
| BH₃· THF | Borane-tetrahydrofuran |
| BINAP | 2,2'-Bis(diphenylphosphino)-1,1'-binaphthyl |
| br | broad (¹H-NMR signal) |
| CI | chemical ionisation |
| d | doublet (¹H-NMR signal) |
| d | day(s) |
| DAD | diode array detector |
| dd | double-doublet |
| DMF | N,N-dimethylformamide |
| DMSO | dimethylsulfoxide |
| ESI | electrospray (ES) ionisation |
| EtOAc | Ethyl acetate |
| h | hour(s) |
| HATU | 1-[Bis(dimethylamino)methylene]-1H-1,2,3-triazolo[4,5 - b]pyridinium 3-oxide hexafluorophosphate,CAS 148893-10-1 |
| HPLC | high performance liquid chromatography |
| LC-MS | liquid chromatography mass spectrometry |
| m | multiplet (¹H-NMR signal) |
| M | molar |
| min | minute(s) |
| MS | mass spectrometry |
| MTBE | methyl-tert-butylether |
| NaBH₄ | Sodium borohydride, sodium tetrahydroborate |
| NaHCO₃ | Sodium hydrogen carbonate |
| Na₂SO₄ | Sodium sulphate |
| NMR | nuclear magnetic resonance spectroscopy: chemical shifts (δ) are given in ppm. The chemical shifts were corrected by setting the DMSO signal to 2.50 ppm unless otherwise stated. |
| PDA | Photo Diode Array |
| Pd₂dba₃ | Tris(dibenzylideneacetone)dipalladium (0), CAS 51364-51-3 |
| Pd(PPh₃)₄ | Tetrakis(triphenylphosphane)palladium(0), CAS 14221-01-3 |
| quant. | quantitative |
| rac | racemic |
| Rₜ, Rt | retention time (as measured either with HPLC or UPLC) in minutes |
| RuPhos Pd G3 | (2-Dicyclohexylphosphino-2',6'-diisopropoxy-1,1'-biphenyl)[2-(2'-amino-1,1'-biphenyl)]palladium(II) methanesulfonate, CAS 1445085-77-7 |
| s | singlet (¹H-NMR signal) |
| SFC | Supercritical Fluid Chromatography |
| SQD | Single-Quadrupole-Detector |
| t | triplet (¹H-NMR signal) |
| td | triple-doublet (¹H-NMR signal) |
| TFA | trifluoroacetic acid |
| THF | tetrahydrofuran |
| UPLC | ultra performance liquid chromatography |
| X-Phos | 2-Dicyclohexylphosphino-2' ,4' ,6' -triisopropylbiphenyl, CAS 564483-18-7 |

The various aspects of the invention described in this application are illustrated by the following examples which are not meant to limit the invention in any way.

The example testing experiments described herein serve to illustrate the present invention and the invention is not limited to the examples given.

All reagents, for which the synthesis is not described in the experimental part, are either commercially available, or are known compounds or may be formed from known compounds by known methods by a person skilled in the art.

The compounds and intermediates produced according to the methods of the invention may require purification. Purification of organic compounds is well known to the person skilled in the art and there may be several ways of purifying the same compound. In some cases, no purification may be necessary. In some cases, the compounds may be purified by crystallization. In some cases, impurities may be stirred out using a suitable solvent. In some cases, the compounds may be purified by chromatography, particularly flash column chromatography, using for example prepacked silica gel cartridges, e.g. Biotage SNAP cartidges KP-Sil^{®} or KP-NH^{®} in combination with a Biotage autopurifier system (SP4^{®} or Isolera Four^{®}) and eluents such as gradients of hexane/ethyl acetate or DCM/methanol. In some cases, the compounds may be purified by preparative HPLC using for example a Waters autopurifier equipped with a diode array detector and/or on-line electrospray ionization mass spectrometer in combination with a suitable prepacked reverse phase column and eluents such as gradients of water and acetonitrile which may contain additives such as trifluoroacetic acid, formic acid or aqueous ammonia.

In some cases, purification methods as described above can provide those compounds of the present invention which possess a sufficiently basic or acidic functionality in the form of a salt, such as, in the case of a compound of the present invention which is sufficiently basic, a trifluoroacetate or formate salt for example, or, in the case of a compound of the present invention which is sufficiently acidic, an ammonium salt for example. A salt of this type can either be transformed into its free base or free acid form, respectively, by various methods known to the person skilled in the art, or be used as salts in subsequent biological assays. It is to be understood that the specific form (e.g. salt, free base etc.) of a compound of the present invention as isolated and as described herein is not necessarily the only form in which said compound can be applied to a biological assay in order to quantify the specific biological activity.

In the case of the synthesis intermediates and working examples of the invention described hereinafter, any compound specified in the form of a salt of the corresponding base or acid is generally a salt of unknown exact stoichiometric composition, as obtained by the respective preparation and/or purification process. Unless specified in more detail, additions to names and structural formulae, such as "hydrochloride", "trifluoroacetate", "sodium salt" or "x HCl", "x CF₃COOH", "x Na⁺" should not therefore be understood in a stoichiometric sense in the case of such salts, but have merely descriptive character with regard to the salt-forming components present therein.

This applies correspondingly if synthesis intermediates or working examples or salts thereof were obtained in the form of solvates, for example hydrates, of unknown stoichiometric composition (if they are of a defined type) by the preparation and/or purification processes described.

### HPLC and LC-MS methods:

### Method 1 (LC-MS)

MS instrument type: SHIMADZU LCMS-2020, Column: Kinetex EVO C18 30^{∗}2.1mm, 5um, mobile phase A: 0.0375% TFA in water (v/v), B: 0.01875% TFA in Acetonitrile (v/v), gradient: 0.0 min 0% B→0.8 min 95% B→1.2 min 95% B→1.21 min 5% B→1.55 min 5% B, flow rate: 1.5 mL/min, oven temperature: 50 °C; UV detection: 220 nm & 254 nm.

### Method 2 (LC-MS)

HPLC instrument type: SHIMADZU LCMS-2020, Column: Kinetex EVO C18 50^{∗}4.6mm, 5um, mobile phase A: 0.0375% TFA in water (v/v), B: 0.01875% TFA in Acetonitrile (v/v), gradient: 0.0 min 10% B→ 2.4 min 80% B→3.7 min 80% B→3.71 min 10% B→4.0 min 10% B, flow rate: 1.5 mL/min, oven temperature: 50 °C; UV detection: 220 nm & 215 nm & 254 nm.

### Method 3 (LC-MS)

Instrument: Waters ACQUITY SQD UPLC System; Column: Waters Acquity UPLC HSS T3 1.8 µm 50 x 1 mm; Eluent A: 11 water + 0.25 ml formic acid, Eluent B: 11 Acetonitrile + 0.25 ml formic acid; Gradient: 0.0 min 90% A → 1.2 min 5% A → 2.0 min 5% A; oven: 50°C; flow rate: 0.40 ml/min; UV-Detection: 210 nm.

### Method 4 (LC-MS)

Instrument MS: Thermo Scientific FT-MS; Instrument type UHPLC+: Thermo Scientific UltiMate 3000; Column: Waters, HSST3, 2.1 x 75 mm, C18 1.8 µm; Eluent A: 1 1 water + 0.01% formic acid; Eluent B: 11 Acetonitrile + 0.01% formic acid; Gradient: 0.0 min 10% B → 2.5 min 95% B → 3.5 min 95% B; oven: 50°C; flow rate: 0.90 ml/min; UV-Detection: 210 nm/ Optimum Integration Path 210-300 nm.

### Method 5 (LC-MS)

Instrument: Waters ACQUITY SQD UPLC System; Column: Waters Acquity UPLC HSS T3 1.8 µm 50 x 1 mm; Eluent A: 11 water + 0.25 ml formic acid, Eluent B: 11 Acetonitrile + 0.25 ml formic acid; Gradient: 0.0 min 95% A → 6.0 min 5% A → 7.5 min 5% A; oven: 50°C; flow rate: 0.35 ml/min; UV-Detection: 210 nm.

### Method 6 (LC-MS)

Instrument: Agilent MS Quad 6150;HPLC: Agilent 1290; Column: Waters Acquity UPLC HSS T3 1.8 µm 50 x 2.1 mm; Eluent A: 1 1 water + 0.25 ml formic acid, Eluent B: 1 1 Acetonitrile + 0.25 ml formic acid; Gradient: 0.0 min 90% A → 0.3 min 90% A → 1.7 min 5% A → 3.0 min 5% A oven: 50°C; flow rate: 1,20 ml/min; UV-Detection: 205 - 305 nm.

### Method 7 (LC-MS)

System MS: Waters TOF instrument; System UPLC: Waters Acquity I-CLASS; Column: Waters Acquity UPLC HSS T3 1.8 µm 50 x 1 mm; Eluent A: 1 1 Water + 0.100 ml 99%ige Formic acid, Eluent B: 1 1 Acetonitrile + 0.100 ml 99%ige Formic acid; Gradient: 0.0 min 90% A →1.2 min 5% A → 2.0 min 5% A Oven: 50°C; Flow: 0.40 ml/min; UV-Detection: 210 nm.

### Method 8 (LC-MS)

System MS: Waters TOF instrument; System UPLC: Waters Acquity I-CLASS; Column: Waters, HSST3, 2.1 x 50 mm, C18 1.8 µm; Eluent A: 1 1 Water + 0.01% Formic acid; Eluent B: 1 1 Acetonitrile + 0.01% Formic acid; Gradient: 0.0 min 2% B → 0.5 min 2% B → 7.5 min 95% B → 10.0 min 95% B; Oven: 50°C; Flow: 1.00 ml/min; UV-Detection: 210 nm

### Method 9 (preparative HPLC)

Instrument: Waters Prep LC/MS System, column: Phenomenex Kinetex C18 5µm 100x30 mm, UV-detection 200-400 nm, room temperature, At-Column Injection (complete injection), eluent A : water, eluent B : acetonitrile, eluent C : 2 % formic acid in water, eluent D : acetonitrile/water (80 vol.% / 20 vol.%); flow: 80 ml/min, gradient profil: 0 to 2 min : eluent A 55 ml/min, eluent B 15 ml/min; 2 to 10 min : eluent A from 55 ml/min to 31 ml/min, eluent B from 15 ml/min to 39 ml/min; 10 to 12 min eluent A 0 ml/min and eluent B 70 ml/min; eluent C and eluent D have a constant flow of 5 ml/min each over the whole running time.

### Method 10 (preparative HPLC)

Instrument: Waters Prep LC/MS System, column: XBridge C18 5µm 100x30 mm, UV-detection 200-400 nm, room temperature, At-Column Injection (complete injection), eluent A : water, eluent B : acetonitrile, eluent C : 2 % ammonia in water, eluent D : acetonitrile/water (80 vol.% / 20 vol.%); flow: 80 ml/min, gradient profil: 0 to 2 min : eluent A 55 ml/min, eluent B 15 ml/min; 2 to 10 min : eluent A from 55 ml/min to 31 ml/min, eluent B from 15 ml/min to 39 ml/min; 10 to 12 min eluent A 0 ml/min and eluent B 70 ml/min; eluent C and eluent D have a constant flow of 5 ml/min each over the whole running time.

### Method 11 (preparative HPLC)

Instrument: Waters Prep LC/MS System, column: Phenomenex Kinetex C18 5µm 100x30 mm, UV-detection 200-400 nm, room temperature, At-Column Injection (complete injection), eluent A : water, eluent B : acetonitrile, eluent C : 2 % formic acid in water, eluent D : acetonitrile/water (80 vol.% / 20 vol.%); flow: 80 ml/min, gradient profil: 0 to 2 min : eluent A 47 ml/min, eluent B 23 ml/min; 2 to 10 min : eluent A from 47 ml/min to 23 ml/min, eluent B from 23 ml/min to 47 ml/min; 10 to 12 min eluent A 0 ml/min and eluent B 70 ml/min; eluent C and eluent D have a constant flow of 5 ml/min each over the whole running time.

### Method 12 (preparative HPLC)

Instrument: Waters Prep LC/MS System, column: Phenomenex Kinetex C18 5µm 100x30 mm, UV-detection 200-400 nm, room temperature, At-Column Injection (complete injection), eluent A : water, eluent B : acetonitrile, eluent C : 2 % formic acid in water, eluent D : acetonitrile/water (80 vol.% / 20 vol.%); flow: 80 ml/min, gradient profil: 0 to 2 min : eluent A 23 ml/min, eluent B 47 ml/min; 2 to 10 min : eluent A from 23 ml/min to 0 ml/min, eluent B from 47 ml/min to 70 ml/min; 10 to 12 min eluent A 0 ml/min and eluent B 70 ml/min; eluent C and eluent D have a constant flow of 5 ml/min each over the whole running time.

### Method 13 (Preparative HPLC)

Instrument: Waters Prep LC/MS System, Säule: Phenomenex Kinetex C18 5µm 100x30 mm, UV-detection 200-400 nm, room temperature, At-Column Injection (complete injection), eluent A : Water, eluent B : acetonitrile, eluent C : 2 % formic acid in Water, eluent D : acetonitrile/Water (80 vol.% / 20 vol.%) flow: 80 ml/min , gradient profil: eluent A 0 bis 2 min 70 ml/min, eluent B 0 bis 2min 0 ml/min, eluent A 2 bis 10 min von 70 ml/min to 0 ml/min and eluent B von 0 ml/min to 70 ml/min, 10 bis 12 min 0 ml/min eluent A und 70 ml/min eluent B; eluent C and eluent D have a constant flow of 5 ml/min over the whole running time.

Microwave: The microwave reactor used was an Initiator⁺ microwave system with robot sixty from Biotage^{®}.

When compounds according to the invention are purified by preparative HPLC by the above-described methods in which the eluents contain additives, for example trifluoroacetic acid, formic acid or ammonia, the compounds according to the invention may be obtained in salt form, for example as trifluoroacetate, formate or ammonium salt, if the compounds according to the invention contain a sufficiently basic or acidic functionality. Such a salt can be converted to the corresponding free base or acid by various methods known to the person skilled in the art.

In the case of the synthesis intermediates and working examples of the invention described hereinafter, any compound specified in the form of a salt of the corresponding base or acid is generally a salt of unknown exact stoichiometric composition, as obtained by the respective preparation and/or purification process. Unless specified in more detail, additions to names and structural formulae, such as "hydrochloride", "trifluoroacetate", "sodium salt" or "x HCl", "x CF₃COOH", "x Na⁺" should not therefore be understood in a stoichiometric sense in the case of such salts, but have merely descriptive character with regard to the salt-forming components present therein.

This applies correspondingly if synthesis intermediates or working examples or salts thereof were obtained in the form of solvates, for example hydrates, of unknown stoichiometric composition (if they are of a defined type) by the preparation and/or purification processes described.

Enantiomer 1 is an enantiomer which eluted first out of the column.

Enantiomer 2 is an enantiomer which eluted second out of the column.

Diastereomeric mixture 1 defines a compound where its starting material is defined as Enantiomer 1 and is reacted with a building block containing at least one chiral center and where the configuration is not defined

Diastereomeric mixture 2 defines a compound where its starting material is defined as Enantiomer 2 and is reacted with a building block containing at least one chiral center and where the configuration is not defined

Diastereomer 1 and Diastereomer 2 defines the two compounds resulting from the chiral separation of the *diastereomeric mixture 1* described above.

Diastereomer 3 and Diastereomer 4 defines the two compounds resulting from the chiral separation of the *diastereomeric mixture* 2 described above.

Stereoisomer 1 defines a compound where its starting material is defined as Enantiomer 1 and is reacted with a building block containing at least one chiral center and where the configuration is defined

Stereoisomer 2 defines a compound where its starting material is defined as Enantiomer 2 and is reacted with a building block containing at least one chiral center and where the configuration is defined

### EXPERIMENTAL SECTION - STARTING MATERIALS AND INTERMEDIATES

### Example 1A

### Tert-butyl 3-{2-[(benzyloxy)carbonyl]hydrazino}piperidine-1-carboxylate (Racemate)

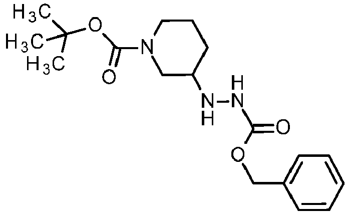

To a solution of tert-butyl 3-oxopiperidine-1-carboxylate [CAS No. 989-36-7] (300 g, 1.51 mol) in tetrahydrofuran (1.50 L) and methanol (300 mL) was added benzyl hydrazinecarboxylate [CAS No. 5331-43-1] (250 g, 1.51 mol) at 25°C. The mixture was stirred at 25°C for 1 h. Afterwards sodium borohydride (114 g, 3.01 mol) was added in portions to the mixture at 25°C and stirred at 25°C for 2 h. The reaction mixture was cooled to 10°C and saturated NH₄Cl was added dropwise to pH∼6. The mixture was extracted with EtOAc (300 mL x 2) and concentrated in vacuo. The residue was dissolved in MTBE (300 mL) and petroleum ether (300 mL) was added. The mixture was filtered off and the precipitate was washed with petroleum ether (100 mL) to give the title compound (400 g, 1.14 mol, 76.0% yield) as a white solid.

LC-MS: (Method 1) Rt = 0.832 min, MS (M-100+1) = 250.4.

### Example 2A

### Tert-butyl 3-hydrazinopiperidine-1-carboxylate acetic acid (Racemate)

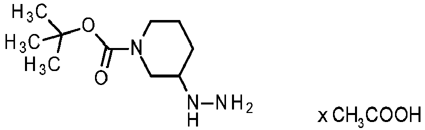

To a solution of tert-butyl 3-{2-[(benzyloxy)carbonyl]hydrazino}piperidine-1-carboxylate (prepared in analogy to Example 1A, 1.20 kg, 3.43 mol) in ethanol (11.0 L) was added acetic acid (415 g, 6.91 mol, 395 mL) and Pd/C (120 g, 20% purity) under H₂ (15 Psi). The mixture was stirred at 25 °C for 12 h. The mixture was filtered and the precipitate was washed with ethanol (11.0 L) to give a solution of the title compound in ethanol (945 g, acetic acid salt) as a black liquid. The filtrate was used for the next step without purification.
¹H-NMR (400 MHz, CDCl₃) δ [ppm]: 7.52 (s, 5H), 3.59 (d, *J* = 6.0 Hz, 12H), 3.30 - 3.24 (m, 2H), 2.75 - 2.71 (m, 2H), 1.38 - 1.34 (m, 1H), 1.20 - 1.18 (m, 1H), 1.10 (s, 9H).
LC-MS: (Method 1) Rt = 0.263 min, MS (M-HOAc-56+1) = 160.2

### Example 3A

### Ethyl 2-(ethoxymethylene)-4,4-difluoro-3-oxobutanoate

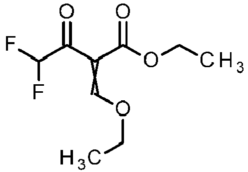

A solution of ethyl 4,4-difluoro-3-oxobutanoate (120 g, 722 mmol) and (diethoxymethoxy)ethane (240 ml, 1.4 mol) in acetic acid anhydride (200 ml, 2.2 mol) was stirred overnight at 140°C and evaporated to drynees affording 155 g (quant.) of the title compound which was used in the next step without further purification.
¹H-NMR (600 MHz, CDCl₃) δ [ppm]: 1.306 (6.05), 1.318 (16.00), 1.330 (14.48), 1.341 (4.56), 1.428 (5.99), 1.436 (5.01), 1.440 (12.20), 1.448 (9.25), 1.451 (6.31), 1.460 (4.48), 2.095 (1.59), 2.225 (1.56), 4.247 (1.97), 4.260 (5.79), 4.271 (5.85), 4.277 (1.55), 4.283 (2.00), 4.289 (4.40), 4.301 (4.37), 4.308 (2.03), 4.313 (1.64), 4.320 (5.74), 4.332 (5.78), 4.340 (1.60), 4.344 (2.01), 4.351 (4.21), 4.364 (4.20), 4.375 (1.37), 6.262 (1.79), 6.339 (1.35), 6.352 (3.56), 6.429 (2.63), 6.442 (1.72), 6.519 (1.28), 7.867 (5.48), 7.880 (7.31).

### Example 4A

### Tert-butyl 3-[5-(difluoromethyl)-4-(ethoxycarbonyl)-1H-pyrazol-1-yl]piperidine-1-carboxylate (Racemate)

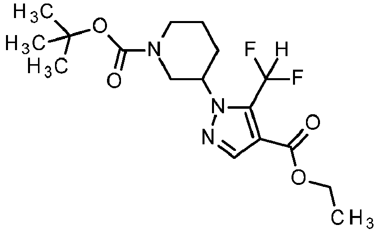

To a mixture of tert-butyl 3-hydrazinopiperidine-1-carboxylate acetic acid (Example 2A, 945 g, 3.43 mol) in ethanol (20.0 L) was added ethyl 2-(ethoxymethylene)-4,4-difluoro-3-oxobutanoate (prepared in analogy to Example 3A, 840 g, 3.78 mol). The mixture was stirred at 25°C for 12 h. The reaction mixture was concentrated. The residue was poured into saturated NaHCO₃ aqueous solution (10.0 L) and extracted with Ethyl acetate (10.0 L x 2). The combined organic layer was washed with brine (10.0 L), dried over Na₂SO₄, filtered and concentrated. The residue was purified by column chromatography on silica gel eluted with Petroleum ether: Ethyl acetate (50:1-25:1-10:1, R_{f} = 0.3) affording 530 g (41.4% yield) of the title compound.
¹H-NMR (400 MHz, CDCl₃) δ [ppm]: 7.84 (s, 1H), 7.51 (t, *J* = 12.8 Hz, 1H), 4.47 - 4.41 (m, 1H), 4.30 - 4.10 (m, 4H), 3.19 - 3.13 (m, 1H), 2.69 (s, 1H), 2.15 - 2.10 (m, 2H), 1.83 - 1.78 (m, 1H), 1.60 - 1.55 (m, 1H), 1.40 (s, 9H), 1.32 - 1.29 (m, 3H)
LC-MS (Method 1) Rt = 0.992 min, MS (M-56+1) = 318.0

### Example 5A

### Ethyl 5-(difluoromethyl)-1-(piperidin-3-yl)-1H-pyrazole-4-carboxylate (Racemate)

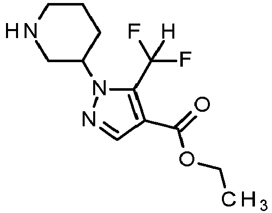

Tert-butyl 3-[5-(difluoromethyl)-4-(ethoxycarbonyl)-1H-pyrazol-1-yl]piperidine-1-carboxylate (prepared in analogy to Example 4A, 593 g, 1.59 mol) was added to a solution of hydrogen chloride in dioxane (4 M, 2.50 L), the mixture was stirred at 25°C for 12 h. The mixture was evaporated and the residue was dissolved in 1.00 L water and extracted with MTBE 500 mL. The aqueous phase was separated and adjusted pH to 8 - 9 with NaHCO₃. The aqueous phase was extracted with dichloromethane (1.00 L x 2), and the combined organic phase were washed with brine (1.00 L), dried over Na₂SO₄ and concentrated to give 350 g (80.6% yield) of the title compound.
¹H-NMR (400 MHz, CDCl₃) δ [ppm]: 7.87 (s, 1H), 7.54 (t, *J* = 12.8 Hz, 1H), 4.55 - 4.54 (m, 1H), 4.34 - 4.28 (m, 2H), 3.25 - 3.03 (m, 3H), 2.71 - 2.65 (m, 1H), 2.19 - 1.86 (m, 4H), 1.63 - 1.60 (m, 1H), 1.35 (t, *J* = 7.2 Hz, 3H)
LC-MS: (Method 1) Rt = 0.644 min, MS (M+1) = 274.6

In analogy to Example 5A, ethyl 5-(difluoromethyl)-1-(piperidin-3-yl)-1H-pyrazole-4-carboxylate (Racemate) was prepared using different protecting groups. The two enantiomers were separated by SFC [sample preparation: 20 g were dissolved in 500 ml methanol; injection volume: 15 ml; column: Daicel AZ SCF 20µm, 400 x 50 mm; eluent: carbon dioxide/methanol/aqueous ammonia (1%) 80:19:1 to 60:39:1; flow rate: 400 ml/min; temperature: 40°C; UV detection: 220 nm]. After separation, 8.1 g of enantiomer 1 (Example 6A), which eluted first, and 8.0g of enantiomer 2 (Example 7A), which eluted later, were isolated.

### Example 6A

### Ethyl 5-(difluoromethyl)-1-(piperidin-3-yl)-1H-pyrazole-4-carboxylate (Enantiomer 1)

For separation conditions see Example 5A.

Analytical SFC: Rt = 0.980 min, e.e. = 100% [Column Chiralpak IC-3: 50 x 4.6 mm; eluent: CO2/[methanol + 0.2% diethyl amine]: 90:10 flow rate: 3.0 ml/min; temperature: 25°C; UV detection: 220 nm].
¹H-NMR (400 MHz, DMSO-d6) δ [ppm]: 8.00 (s, 1H), 7.75-7.44 (m, 1H), 4.50-4.36 (m, 1H), 4.33-4.18 (m, 2H), 3.10-2.95 (m, 1H), 2.91-2.76 (m, 2H), 2.48-2.33 (m, 2H), 2.08-1.94 (m, 2H), 1.81-1.66 (m, 1H), 1.62-1.40 (m, 1H), 1.37-1.21 (m, 3H).

### Example 7A

### Ethyl 5-(difluoromethyl)-1-(piperidin-3-yl)-1H-pyrazole-4-carboxylate (Enantiomer 2)

### For separation conditions see Example 5A.

Analytical SFC: Rt = 1.227 min, e.e. = 97% [Column Chiralpak IC-3: 50 x 4.6 mm; eluent: CO2/[methanol + 0.2% diethyl amine]: 90:10 flow rate: 3.0 ml/min; temperature: 25°C; UV detection: 220 nm].
¹H-NMR (400 MHz, DMSO-d6) δ [ppm]: 8.01 (s, 1H), 7.75-7.43 (m, 1H), 4.50-4.37 (m, 1H), 4.27 (q, 2H), 3.09-2.97 (m, 1H), 2.94-2.81 (m, 2H), 2.47-2.34 (m, 2H), 2.06-1.92 (m, 2H), 1.79-1.66 (m, 1H), 1.60-1.41 (m, 1H), 1.29 (t, 3H).

### Example 8A

### 2-Bromo-4-chloro-1-[(4-methoxyphenyl)methoxy]benzene

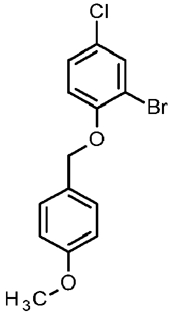

A solution of 2-bromo-4-chlorophenol (10.0 g, 48.2 mmol) in acetone (75 ml) was treated with potassium carbonate (13.3 g, 96.4 mmol), potassium iodide (12.0 g, 72.3 mmol) and 1-(chloromethyl)-4-methoxybenzene (7.55 g, 48.2 mmol). The resulting mixture was stirred ∼19 hours at 70°C. The reaction mixture was diluted with water and extracted twice with ethyl acetate. The combined organic layers were dried over sodium sulphate and evaporated. The residue was purified by flash chromatography (silica gel, cyclohexane/ethyl acetate gradient) affording 13.78 g (86 % yield) of the title compound.
LC-MS (Method 3): Rₜ = 2.48 min; MS (ESIneg): m/z = 324 [M-H]⁻
¹H-NMR (600 MHz, DMSO-d6) δ [ppm]: 3.349 (10.98), 5.124 (16.00), 6.949 (0.87), 6.954 (8.36), 6.957 (2.68), 6.965 (2.83), 6.968 (8.92), 6.973 (1.00), 7.218 (5.23), 7.233 (6.21), 7.380 (0.90), 7.384 (7.80), 7.399 (7.44), 7.402 (4.47), 7.406 (3.89), 7.417 (3.04), 7.421 (3.07), 7.697 (6.51), 7.702 (6.34).

### Example 9A

### Ethyl 1-[1-{5-chloro-2-[(4-methoxyphenyl)methoxy]phenyl}piperidin-3-yl]-5-(difluoromethyl)-1H-pyrazole-4-carboxylate (Enantiomer 1)

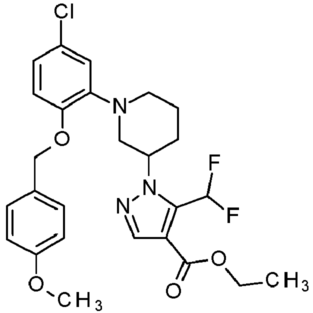

Under argon, a solution of 2-bromo-4-chloro-1-[(4-methoxyphenyl)methoxy]benzene (prepared in analogy to Example 8A, 10.0 g, 30.5 mmol) and ethyl 5-(difluoromethyl)-1-[piperidin-3-yl]-1H-pyrazole-4-carboxylate (prepared in analogy to Example 6A, Enantiomer 1, 8.34 g, 30.5 mmol) in 1,4-dioxane (100 ml) was treated with caesium carbonate (29.8 g, 91.6 mmol), Pd₂dba₃ (2.80 g, 3.05 mmol) and rac-BINAP (3.80 g, 6.10 mmol) and the resulting mixture was stirred overnight at 100°C. The reaction mixture was combined with a 500 mg test reaction, filtered over Celite, rinsed with ethyl acetate and evaporated. The residue was retaken in water and extracted three times with ethyl acetate. The combined organic layers were washed with a saturated solution of sodium chloride, dried over sodium sulphate and evaporated. The residue was purified by flash chromatography (silica gel, cyclohexane / ethyl acetate gradient) affording 10.1 g (60 % yield) of the title compound.
LC-MS (Method 4): Rₜ = 1.44 min; MS (ESIpos): m/z = 520 [M+H]⁺

### Example 10A

### Ethyl 1-[1-(5-chloro-2-hydroxyphenyl)piperidin-3-yl]-5-(difluoromethyl)-1H-pyrazole-4-carboxylate (Enantiomer 1)

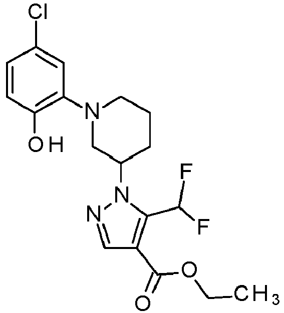

A solution of ethyl 1-[1-{5-chloro-2-[(4-methoxyphenyl)methoxy]phenyl}piperidin-3-yl]-5-(difluoromethyl)-1H-pyrazole-4-carboxylate (Example 9A, Enantiomer 1, 10.1 g, 19.4 mmol) in dichloromethane (200 ml) was treated with trifluoroacetic acid and stirred over night at room temperature. The reaction mixture was evaporated. The residue was retaken in ethyl acetate and washed once with water, once with a saturated solution of sodium hydrogen carbonate and finally once with a saturated solution of sodium chloride. The organic phase was dried over sodium sulphate and evaporated. The residue was purified by flash chromatography (silica gel, cyclohexane / ethyl acetate gradient) affording 7.17 g (83 % purity, 77 % yield) of the title compound.
LC-MS (Method 8): Rₜ = 1.26 min; MS (ESIpos): m/z = 400 [M+H]⁺

### Example 11A

### Ethyl 1-[1-{5-chloro-2-[(trifluoromethanesulfonyl)oxy]phenyl}piperidin-3-yl]-5-(difluoromethyl)-1H-pyrazole-4-carboxylate (Enantiomer 1)

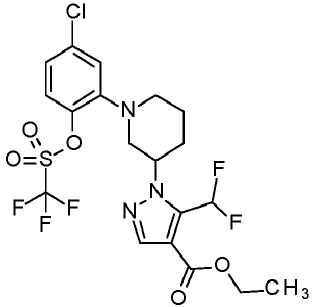

Under argon, a solution of ethyl 1-[1-(5-chloro-2-hydroxyphenyl)piperidin-3-yl]-5-(difluoromethyl)-1H-pyrazole-4-carboxylate (Example 10A, Enantiomer 1, 7.17 g, 83 % purity, 14.9 mmol) in dichloromethane (160 ml) was treated with triethylamine (5.2 ml, 37 mmol) and cooled to 0°C. Trifluoromethanesulfonic anhydride was added dropwise and the resulting mixture was stirred 45 minutes at 0°C. The reaction mixture was diluted with dichloromethane (150 ml) and washed three times with water. The organic phase was dried over sodium sulphate and evaporated. The residue was purified by flash chromatography (silica gel, cyclohexane / ethyl acetate gradient) affording 7.89 g (quant.) of the title compound.
LC-MS (Method 4): Rₜ = 1.47 min; MS (ESIpos): m/z = 532 [M+H]⁺

### Example 12A

### Tert-butyl 4-(4'-chloro-2'-{3-[5-(difluoromethyl)-4-(ethoxycarbonyl)-1H-pyrazol-1-yl]piperidin-1-yl}[1,1'-biphenyl]-4-yl)piperazine-1-carboxylate (Enantiomer 1)

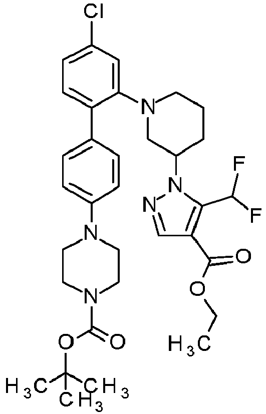

Under argon, a solution of ethyl 1-[1-{5-chloro-2-[(trifluoromethanesulfonyl)oxy]phenyl}piperidin-3-yl]-5-(difluoromethyl)-1H-pyrazole-4-carboxylate (prepared in analogy to Example 11A, Enantiomer 1, 665 mg, 1.25 mmol) and tert-butyl 4-[4-(4,4,5,5-tetramethyl-1,3,2-dioxaborolan-2-yl)phenyl]piperazine-1-carboxylate (583 mg, 1.50 mmol) in toluene/ethanol mixture 1:1 (14 ml) was treated with an aqueous solution of sodium carbonate (1.9 ml, 2.0 M, 3.8 mmol) and Pd(PPh₃)₄ (72.2 mg, 62.5 µmol) and stirred 2 hours at 100°C. The reaction mixture was cooled to room temperature, filtered over Celite and rinsed with ethyl acetate. The filtrate was evaporated and the residue was purified by flash chromatography (silica gel, cyclohexane/ethyl acetate gradient) affording 760 mg (94 % yield) of the title compound.
LC-MS (Method 3): Rₜ = 3.13 min; MS (ESIpos): m/z = 644 [M+H]⁺

### Example 13A

### Ethyl 1-{1-[4-chloro-4'-(piperazin-1-yl)[1,1'-biphenyl]-2-yl]piperidin-3-yl}-5-(difluoromethyl)-1H-pyrazole-4-carboxylate hydrochloride (Enantiomer 1)

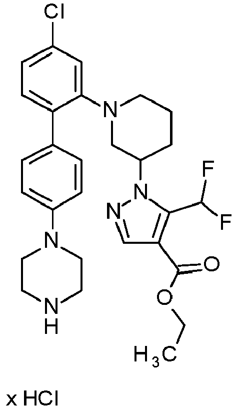

A solution of tert-butyl 4-(4'-chloro-2'-{3-[5-(difluoromethyl)-4-(ethoxycarbonyl)-1H-pyrazol-1-yl]piperidin-1-yl}[1,1'-biphenyl]-4-yl)piperazine-1-carboxylate (Example 12A, Enantiomer 1, 760mg, 1.18 mmol) in dichloromethane (2.9 ml) was treated with a solution of hydrogen chloride in dioxane (2.9 ml, 4.0 M, 12 mmol) and stirred 30 minutes at room temperature. The reaction mixture was evaporated affording 626 mg (70 % yield) of the title compound which was used in the next steps without further purification.
LC-MS (Method 4): Rₜ = 0.98 min; MS (ESIpos): m/z = 544 [M+H]⁺

### Example 14A

### Ethyl 1-[1-{5-chloro-2-[(4-methoxyphenyl)methoxy]phenyl}piperidin-3-yl]-5-(difluoromethyl)-1H-pyrazole-4-carboxylate (Enantiomer 2)

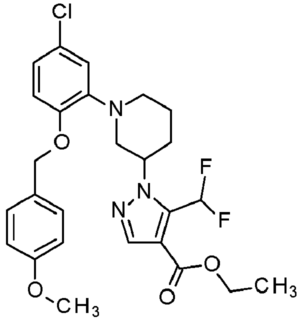

Under argon, a solution of ethyl 5-(difluoromethyl)-1-[piperidin-3-yl]-1H-pyrazole-4-carboxylate (prepared in analogy to Example 7A, Enantiomer 2, 43.6 g, 160 mmol) and 2-bromo-4-chloro-1-[(4-methoxyphenyl)methoxy]benzene (prepared in analogy to Example 8A, 52.3 g, 160 mmol) in 1,4-dioxane (680 ml) was treated with Pd₂(dba)₃ (14.6 g, 16.0 mmol), rac-BINAP (19.9 g, 31.9 mmol) and freshly ground caesium carbonate (156 g, 479 mmol) and stirred 18 hours at 100°C. The reaction mixture was diluted with ethyl acetate and a 10% solution of sodium chloride, filtered over Celite and rinsed with ethyl acetate. The aqueous phase of the filtrate was extracted with ethyl acetate. The combined organic layers were washed with a 10% solution of sodium chloride, dried over sodium sulphate and evaporated. The residue was purified flash chromatography over silica gel (dichloromethane/ petrol ether 4:1) affording 42 g (82% yield) of the title compound.
LC-MS (Method 3): Rₜ = 2.78 min; MS (ESIpos): m/z = 520 [M+H]⁺
¹H-NMR (400 MHz, DMSO-d6) δ [ppm]: 1.272 (3.65), 1.290 (7.68), 1.307 (3.76), 1.686 (0.44), 1.717 (0.54), 1.852 (0.73), 1.885 (0.50), 1.989 (0.47), 2.019 (0.56), 2.058 (0.99), 2.084 (0.61), 2.587 (0.51), 2.616 (0.89), 2.642 (0.45), 3.030 (0.76), 3.057 (1.51), 3.084 (0.83), 3.447 (0.72), 3.474 (0.69), 3.613 (0.74), 3.640 (0.67), 3.737 (16.00), 4.251 (1.13), 4.269 (3.48), 4.287 (3.45), 4.304 (1.12), 4.624 (0.40), 4.639 (0.48), 4.650 (0.76), 4.661 (0.51), 5.035 (6.45), 6.872 (3.47), 6.893 (5.67), 6.947 (0.98), 6.952 (0.85), 6.968 (1.72), 6.974 (1.67), 7.017 (2.84), 7.039 (1.57), 7.305 (3.66), 7.326 (3.43), 7.340 (0.56), 7.380 (0.41), 7.439 (0.93), 7.463 (0.64), 7.476 (0.48), 7.569 (1.65), 7.699 (0.76), 8.044 (3.66).

### Example 15A

### Ethyl 1-[1-(5-chloro-2-hydroxyphenyl)piperidin-3-yl]-5-(difluoromethyl)-1H-pyrazole-4-carboxylate (Enantiomer 2)

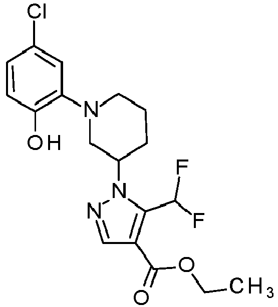

A solution of ethyl 1-[1-{5-chloro-2-[(4-methoxyphenyl)methoxy]phenyl}piperidin-3-yl]-5-(difluoromethyl)-1H-pyrazole-4-carboxylate (prepared in analogy to Example 14A, Enantiomer 2, 67.5 g, 130 mmol) in dichloromethane (1.0 1) was treated with trifluoroacetic acid (100 ml, 1.3 mol) and stirred overnight at room temperature. The reaction mixture was diluted with water (750 ml) and carefully treated with a 10% solution of sodium carbonate (450 ml) until no more carbone dioxide was generated. The organic phase was dried over sodium sulphate and evaporated affording 52 g (90% yield) of the title compound which was used in the next step without further purification.
LC-MS (Method 3): Rₜ = 2.42 min; MS (ESIpos): m/z = 400 [M+H]⁺

### Example 16A

### Ethyl 1-[1-{5-chloro-2-[(trifluoromethanesulfonyl)oxy]phenyl}piperidin-3-yl]-5-(difluoromethyl)-1H-pyrazole-4-carboxylate (Enantiomer 2)

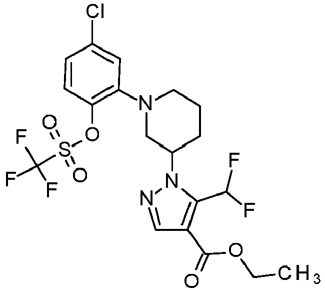

A solution of ethyl 1-[1-(5-chloro-2-hydroxyphenyl)piperidin-3-yl]-5-(difluoromethyl)-1H-pyrazole-4-carboxylate (Example 15A, Enantiomer 2, 52.0 g, 117 mmol) and triethylamine (49 ml, 350 mmol) in dichloromethane (330 ml) was cooled to -50°C. Trifluoromethanesulfonic acid (28 ml, 160 mmol) was added dropwise and the resulting mixture was stirred 1 hour at -50°C. The reaction mixture was then diluted with dichloromethane (330 ml) and water (370 ml). The aqueous phase was extracted with dichloromethane (330 ml). The combined organic layers were washed with (370 ml), dried over sodium sulphate and evaporated. The resulting mixture was purified by flash chromatography (silica gel, dichloromethane/petrol ether 6:4) affording 60 g (96 % yield) of the title compound.
LC-MS (Method 3): Rₜ = 2.74 min; MS (ESIpos): m/z = 532 [M+H]⁺
¹H-NMR (600 MHz, DMSO-d6) δ [ppm]: -0.021 (0.65), 1.082 (0.51), 1.270 (7.69), 1.282 (16.00), 1.294 (7.63), 1.772 (0.48), 1.780 (0.51), 1.787 (0.63), 1.793 (0.66), 1.801 (0.62), 1.808 (0.60), 1.910 (1.25), 1.914 (0.99), 1.927 (0.67), 1.932 (0.89), 2.068 (0.72), 2.075 (1.03), 2.086 (2.45), 2.091 (2.40), 2.100 (1.41), 2.792 (0.71), 2.796 (0.83), 2.812 (1.48), 2.816 (1.50), 2.832 (0.83), 2.836 (0.72), 3.142 (1.17), 3.161 (1.04), 3.201 (1.21), 3.219 (2.80), 3.237 (1.83), 3.278 (1.37), 3.285 (1.56), 4.251 (2.26), 4.263 (7.09), 4.275 (7.06), 4.287 (2.20), 4.755 (0.50), 4.765 (0.90), 4.773 (0.89), 4.781 (0.90), 4.791 (0.49), 5.734 (2.17), 7.261 (2.19), 7.265 (2.27), 7.275 (2.69), 7.279 (2.82), 7.391 (4.65), 7.406 (3.75), 7.431 (4.73), 7.435 (4.51), 7.492 (1.26), 7.579 (2.61), 7.666 (1.07), 8.026 (6.37).

### Example 17A

### Tert-butyl 4-(4'-chloro-2'-{3-[5-(difluoromethyl)-4-(ethoxycarbonyl)-1H-pyrazol-1-yl]piperidin-1-yl}[1,1'-biphenyl]-4-yl)piperazine-1-carboxylate (Enantiomer 2)

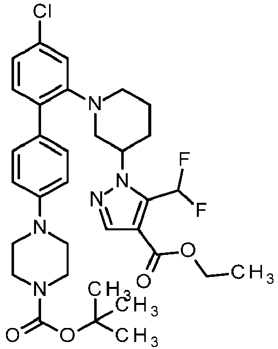

Under argon, a solution of ethyl 1-[1-{5-chloro-2-[(triffuoromethanesulfonyl)oxy]phenyl}piperidin-3-yl]-5-(difluoromethyl)-1H-pyrazole-4-carboxylate (Example 16A, Enantiomer2, 57.0 g, 107 mmol) and tert-butyl 4-[4-(4,4,5,5-tetramethyl-1,3,2-dioxaborolan-2-yl)phenyl]piperazine-1-carboxylate (49.9 g, 129 mmol) in toluene (600 ml) and ethanol (600 ml) was treated with an aqueous solution of sodium carbonate (160 ml, 2.0 M, 320 mmol) and Pd(Ph₃)₄ (6.19 g, 5.36 mmol). The resulting mixture was stirred 4 hours at 100°C The reaction mixture was cooled to room temperature, filtered over Celite, washed with ethyl acetate and evaporated. The residue was purified by flash chromatography (silica gel, petrol ether/ethyl acetate 9:1 to 8:2) affording 62 g (89 % yield) of the title compound.
LC-MS (Method 3): Rₜ = 3,15 min; MS (ESIpos): m/z = 644 [M+H]⁺

### Example 18A

### Ethyl 1-{1-[4-chloro-4'-(piperazin-1-yl)[1,1'-biphenyl]-2-yl]piperidin-3-yl}-5-(difluoromethyl)-1H-pyrazole-4-carboxylate hydrochloride (Enantiomer 2)

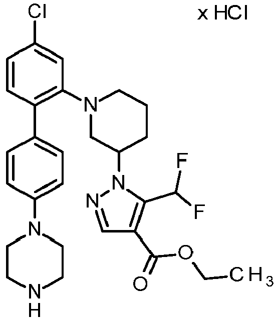

A solution of tert-butyl 4-(4'-chloro-2'-{(3-[5-(difluoromethyl)-4-(ethoxycarbonyl)-1H-pyrazol-1-yl]piperidin-1-yl}[1,1'-biphenyl]-4-yl)piperazine-1-carboxylate (Example 17A, Enantiomer 2, 60.0 g, 93.1 mmol) in dichloromethane (250 ml) was treated with a solution of hydrogen chloride in dioxane (230 ml, 4.0 M, 930 mmol). The resulting mixture was stirred 3 hours at room temperature and evaporated. The residue was co-evaporated twice with diethyl ether (250 ml x 2), stirred 4 days in diisopropyl ether. The suspension was filtered, the solid was washed twice with diisopropyl ether affording 57 g (quant.) of the title compound.
LC-MS (Method 3): Rₜ = 1.78 min; MS (ESIpos): m/z = 544 [M+H]⁺
¹H-NMR (400 MHz, DMSO-d6) δ [ppm]: 1.029 (13.49), 1.044 (13.77), 1.262 (7.53), 1.280 (16.00), 1.297 (7.81), 1.496 (0.79), 1.506 (0.62), 1.527 (0.91), 1.559 (0.40), 1.716 (1.24), 1.749 (0.95), 1.888 (0.84), 1.897 (0.78), 1.918 (0.98), 1.926 (0.93), 1.966 (1.38), 1.995 (0.69), 2.580 (1.54), 2.606 (0.83), 2.992 (1.21), 3.018 (2.69), 3.044 (2.33), 3.063 (1.24), 3.435 (5.96), 3.448 (7.25), 3.460 (5.00), 3.570 (5.78), 3.586 (0.87), 3.601 (1.12), 3.616 (0.85), 4.227 (5.38), 4.238 (6.62), 4.256 (9.26), 4.273 (7.97), 4.291 (2.70), 4.444 (0.41), 4.455 (0.77), 4.470 (0.89), 4.481 (1.31), 4.491 (0.92), 4.507 (0.68), 7.045 (6.02), 7.067 (6.86), 7.074 (5.10), 7.079 (5.42), 7.099 (2.25), 7.104 (1.49), 7.120 (3.55), 7.125 (3.10), 7.164 (6.27), 7.185 (3.37), 7.383 (1.62), 7.483 (6.90), 7.505 (6.40), 7.513 (3.75), 7.643 (1.34), 8.005 (5.77), 9.399 (1.97).

### Example 19A

### Ethyl 1-{1-[4-chloro-4'-(4-ethylpiperazin-1-yl)[1,1'-biphenyl]-2-yl]piperidin-3-yl}-5-(difluoromethyl)-1H-pyrazole-4-carboxylate trifluoro acetic acid (Enantiomer 1)

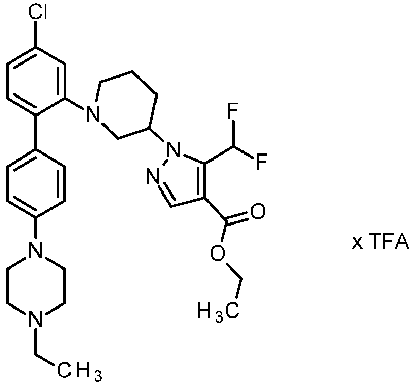

Ethyl 1-[1-{5-chloro-2-[(trifluoromethanesulfonyl)oxy]phenyl}piperidin-3-yl]-5-(difluoromethyl)-1H-pyrazole-4-carboxylate (prepared in analogy to Example 11A, Enantiomer 1, 100 mg, 96 % purity, 180 µmol) and 1-ethyl-4-[4-(4,4,5,5-tetramethyl-1,3,2-dioxaborolan-2-yl)phenyl]piperazine (68.5 mg, 217 µmol) were placed in 1.8 ml DMF, mixed with tetrakis(triphenylphosphine)palladium(0) (10.4 mg, 9.02 µmol) and 2 M sodium carbonate solution (270 µl, 2.0 M, 540 µmol) and stirred at 130°C for 2.5 h. The reaction mixture was filtered through Celite, washed with acetonitrile and the filtrate was purified by preparative HPLC (RP18 column, mobile phase: acetonitrile/water gradient with the addition of 0.1% TFA). The product fractions were combined and the acetonitrile was evaporated and then lyophilized. 51 mg of the target compound were obtained (41% of theory).
LC-MS (Method 3): Rₜ = 1.93 min; MS (ESIpos): m/z = 572 [M+H]⁺

### Example 20A

### Ethyl 1-{1-[4-chloro-4'-(4-ethylpiperazin-1-yl)[1,1'-biphenyl]-2-yl]piperidin-3-yl}-5-(difluoromethyl)-1H-pyrazole-4-carboxylate (Enantiomer 2)

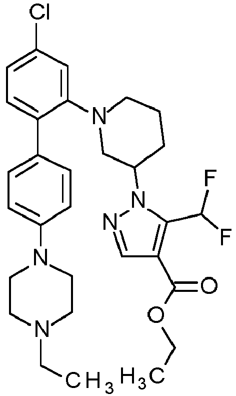

Under argon, a solution of ethyl 1-[1-{5-chloro-2-[(triffuoromethanesulfonyl)oxy]phenyl}piperidin-3-yl]-5-(difluoromethyl)-1H-pyrazole-4-carboxylate (prepared in analogy to Example 16A, Enantiomer 2, 300 mg, 95 % purity, 536 µmol) and 1-ethyl-4-[4-(4,4,5,5-tetramethyl-1,3,2-dioxaborolan-2-yl)phenyl]piperazine (203 mg, 643 µmol) in toluene/ethanol mixture 1:1 (6.0 ml) was treated with an aqueous solution of sodium carbonate (800 µl, 2.0 M, 1.6 mmol) and Pd(PPh₃)₄ (31.0 mg, 26.8 µmol) and stirred 4 hours at 100°C. The reaction mixture was cooled to room temperature, diluted with ethyl acetate and filtered over celite. The filtrate was diluted with water. The aqueous phase was extracted with ethyl acetate. The combined organic layers were washed with a saturated solution of sodium chloride, dried over magnesium sulfate and evaporated. The residue was purified by flash chromatography (amino phase silica gel, cyclohexane/ethyl acetate gradient) affording 236 mg (90 % purity, 69 % yield) of the title compound.
LC-MS (Method 3): Rₜ = 1.88 min; MS (ESIpos): m/z = 572 [M+H]⁺

### Example 21A

### 1-Propyl-4-[4-(4,4,5,5-tetramethyl-1,3,2-dioxaborolan-2-yl)phenyl]piperazine

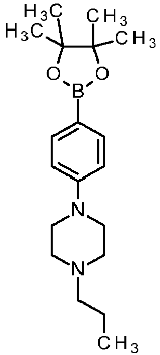

1-[4-(4,4,5,5-tetramethyl-1,3,2-dioxaborolan-2-yl)phenyl]piperazine (300 mg, 1.04 mmol) was placed in 6.4 ml of THF and N,N-diisopropylethylamine (270 ul, 1.6 mmol) was added. Propanal (242 mg, 4.16 mmol) was then added and the mixture was stirred for 10 min. Then sodium triacetoxyborohydride (662 mg, 3.12 mmol) was added and the mixture was stirred at 55°C for 1.5 h. The reaction mixture was cooled to room temperature, saturated aqueous sodium bicarbonate solution was added and the mixture was extracted three times with ethyl acetate. The combined organic phases were washed once with saturated aqueous sodium chloride solution, dried over sodium sulfate, filtered and evaporated. The mixture was purified by means of silica gel chromatography (dichloromethane/methanol 100/1, then isocratic dichloromethane/methanol: 50/1). 186 mg of the target compound (53% of theory) were obtained.
LC-MS (Method 6): Rₜ = 0.97 min; MS (ESIpos): m/z = 331 [M+H]⁺
¹H-NMR (500 MHz, DMSO-d6) δ [ppm]: 0.856 (1.10), 0.871 (2.41), 0.886 (1.18), 1.070 (6.41), 1.258 (16.00), 1.457 (0.59), 1.472 (0.58), 2.250 (0.49), 2.265 (0.64), 2.279 (0.45), 2.453 (0.86), 2.462 (1.15), 2.472 (0.89), 3.181 (0.94), 3.192 (1.13), 3.201 (0.86), 3.916 (1.09), 6.877 (1.01), 6.894 (1.02), 7.490 (1.17), 7.507 (1.04).

### Example 22A

### Ethyl 1-{1-[4-chloro-4'-(4-propylpiperazin-1-yl)[1,1'-biphenyl]-2-yl]piperidin-3-yl}-5-(difluoromethyl)-1H-pyrazole-4-carboxylate trifluoroacetic acid (Enantiomer 2)

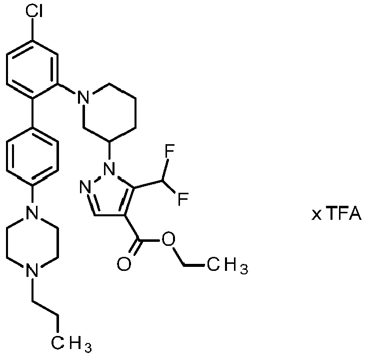

Ethyl 1-[1-{5-chloro-2-[(trifluoromethanesulfonyl)oxy]phenyl}piperidin-3-yl]-5-(difluoromethyl)-1H-pyrazole-4-carboxylate (prepared in analogy to Example 16A, Enantiomer 2, 90.0 mg, 169 µmol) and 1-propyl-4-[4-(4,4,5,5-tetramethyl-1,3,2-dioxaborolan-2-yl)phenyl]piperazine (Example 21A, 67.1 mg, 203 µmol) were placed under argon in toluene/ethanol (940 µl/940 µl), 2 M sodium carbonate solution (250 µl) and tetrakis(triphenylphosphine)palladium(0) (9.78 mg, 8.46 µmol) were added and the mixture was stirred at 100°C overnight. Tetrakis(triphenylphosphine)palladium(0) (9.78 mg, 8.46 µmol) was added to the mixture, flushed with argon and stirred at 100°C. for 3 h. The reaction mixture was diluted with ethyl acetate and water. The aqueous phase was acidified with 1M hydrochloric acid. The phases were separated and the aqueous phase was extracted twice with ethyl acetate. The combined organic phases were dried over sodium sulfate, filtered and evaporated. The residue was purified by preparative HPLC (RP18 column, mobile phase: acetonitrile/water gradient with the addition of 0.1% trifluoroacetic acid). 43 mg of the target compound were obtained (36% of theory).
LC-MS (Method 3): Rₜ = 2.07 min; MS (ESIpos): m/z = 586 [M+H]⁺

### Example 23A

### Ethyl 1-[1-{4-chloro-4'-[4-(propan-2-yl)piperazin-1-yl][1,1'-biphenyl]-2-yl}piperidin-3-yl]-5-(difluoromethyl)-1H-pyrazole-4-carboxylate (Enantiomer 2)

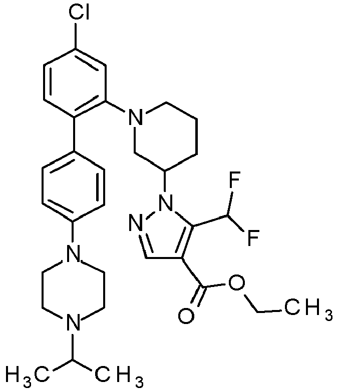

Ethyl 1-[1-{5-chloro-2-[(trifluoromethanesulfonyl)oxy]phenyl}piperidin-3-yl]-5-(difluoromethyl)-1H-pyrazole-4-carboxylate (prepared in analogy to Example 16A, Enantiomer 2, 100 mg, 184 µmol) and 1-(propan-2-yl)-4-[4-(4,4,5,5-tetramethyl-1,3,2-dioxaborolan-2-yl)phenyl]piperazine (73.0 mg, 221 µmol) were dissolved under Argon in toluene/ethanol (1/1 ml). 2 M sodium carbonate solution (280 µl) and tetrakis(triphenylphosphine)palladium(0) (10.6 mg, 9.21 µmol) were added and the mixture was stirred at 100°C overnight. The reaction mixture was diluted with ethyl acetate, filtered through Celite, washed well with ethyl acetate and the filtrate was evaporated. The residue was taken up in acetonitrile/water/formic acid and purified by preparative HPLC (RP18 column, acetonitrile/water gradient with addition of 0.1% formic acid). 20 mg (17% of theory, purity 92%) of the target compound were obtained.
LC-MS (Method 4): Rₜ = 1.02 min; MS (ESIpos): m/z = 586 [M+H]⁺

### Example 24A

### 1-Cyclopropyl-4-[4-(4,4,5,5-tetramethyl-1,3,2-dioxaborolan-2-yl)phenyl]piperazine

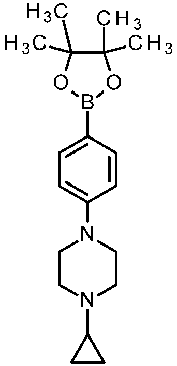

1-[4-(4,4,5,5-tetramethyl-1,3,2-dioxaborolan-2-yl)phenyl]piperazine (700 mg, 2.43 mmol) was dissolved in 12 ml of methanol. Successively 3A mol sieve (∼200 mg), 1.4 ml acetic acid, [(1-ethoxycyclopropyl)oxy](trimethyl)silane (2.9 ml, 15 mmol) and sodium cyanoborohydride (687 mg, 10.9 mmol) were added. The mixture was stirred at room temperature for 3 days. The molecular sieve was filtered off through Celite, washed well with ethyl acetate and the organic phase was then washed twice with water. The organic phase was dried over sodium sulfate, filtered and evaporated. The crude product was purified using silica gel (dichloromethane/methanol gradient: 100/1 to 50/1). 270 mg of the target compound (30% of theory, purity 88%) were obtained.
LC-MS (Method 4): Rₜ = 0.66 min; MS (ESIpos): m/z = 329 [M+H]⁺

### Example 25A

### Ethyl 1-{1-[4-chloro-4'-(4-cyclopropylpiperazin-1-yl)[1,1'-biphenyl]-2-yl]piperidin-3-yl}-5-(difluoromethyl)-1H-pyrazole-4-carboxylate trifluoroacetic acid (Enantiomer 1)

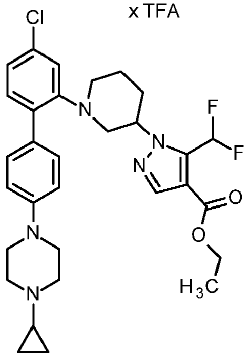

Ethyl 1-[1-{5-chloro-2-[(trifluoromethanesulfonyl)oxy]phenyl}piperidin-3-yl]-5-(difluoromethyl)-1H-pyrazole-4-carboxylate (prepared in analogy to Example 11A, Enantiomer 1, 100 mg, 93 % purity, 175 µmol) and 1-cyclopropyl-4-[4-(4,4,5,5-tetramethyl-1,3,2-dioxaborolan-2-yl)phenyl]piperazine (Example 24A, 71.7 mg, 88 % purity, 192 µmol) were dissolved in 1.7 ml DMF, mixed with tetrakis(triphenylphosphine)palladium(0) (10.1 mg, 8.74 µmol) and 2 M sodium carbonate solution (260 µl) and stirred at 130°C for 2.5 h. The reaction mixture was filtered through Celite, washed with acetonitrile and the filtrate was purified by preparative HPLC (RP18 column, acetonitrile/water gradient with the addition of 0.1% TFA). 22 mg of the target compound were obtained as a TFA adduct (15% of theory, purity 83%).
LC-MS (Method 4): Rₜ = 1.01 min; MS (ESIpos): m/z = 584 [M+H]⁺

### Example 26A

### Ethyl 1-[1-[5-chloro-2-[4-(4-cyclopropylpiperazin-1-yl)phenyl]phenyl]-3-piperidyl]-5-(difluoromethyl)pyrazole-4-carboxylate trifluoroacetic acid (Enantiomer 2)

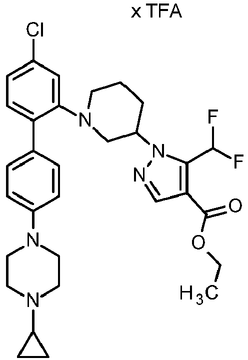

Ethyl 1-[1-{5-chloro-2-[(trifluoromethanesulfonyl)oxy]phenyl}piperidin-3-yl]-5-(difluoromethyl)-1H-pyrazole-4-carboxylate (prepared in analogy to Example 11A, Enantiomer 2, 100 mg, 95 % purity, 179 µmol) and 1-cyclopropyl-4-[4-(4,4,5,5-tetramethyl-1,3,2-dioxaborolan-2-yl) phenyl] piperazines (Example 24A, 73.3 mg, 88% purity, 196 µmol) were dissolved in 1.7 ml DMF, mixed with tetrakis (triphenylphosphine)palladium(0) (10.3 mg, 8.93 µmol) and 2 M sodium carbonate solution (270 µl, 2.0 M) and stirred at 130°C for 2.5 h. The reaction mixture was filtered through Celite, washed with acetonitrile and the filtrate was purified by preparative HPLC (RP18 column, acetonitrile/water gradient with the addition of 0.1% TFA). 24.4 mg of the target compound were obtained as a TFA adduct (14% of theory, purity ∼71%).
LC-MS (Method 4): Rₜ = 1.05 min; MS (ESIpos): m/z = 584 [M+H]⁺

### Example 27A

### 1-(Cyclopropylmethyl)-4-[4-(4,4,5,5-tetramethyl-l,3,2-dioxaborolan-2-yl)phenyl]piperazine

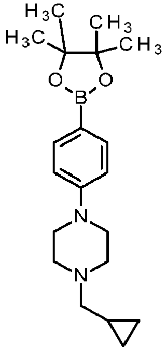

1-[4-(4,4,5,5-tetramethyl-1,3,2-dioxaborolan-2-yl)phenyl]piperazine (380 mg, 1.32 mmol) was dissolved in 8 ml THF and N,N-diisopropylethylamine (340 µl, 2.0 mmol) was added. Then cyclopropanecarbaldehyde (370 mg, 5.27 mmol) was added and the mixture was stirred for 10 min. Then sodium triacetoxyborohydride (838 mg, 3.96 mmol) was added and the mixture was stirred at 55°C for 4 h. The reaction mixture was cooled to room temperature, saturated aqueous sodium bicarbonate solution was added and the mixture was extracted three times with ethyl acetate. The combined organic phases were washed once with saturated aqueous sodium chloride solution, dried over sodium sulfate, filtered and evaporated. 519 mg of the target compound (98% of theory, purity 85%) were obtained.
LC-MS (Method 3): Rₜ = 1.18 min; MS (ESIpos): m/z = 343 [M+H]⁺
¹H-NMR (600 MHz, DMSO-d6) δ [ppm]: 0.089 (0.56), 0.096 (0.58), 0.471 (0.53), 0.483 (0.55), 1.158 (0.59), 1.175 (0.52), 1.259 (16.00), 1.989 (1.00), 3.210 (0.89), 3.216 (0.92), 3.226 (0.55), 6.885 (0.93), 6.900 (0.95), 7.494 (1.10), 7.509 (1.02).

### Example 28A

### 1-{1-[4-Chloro-4'-(piperazin-1-yl)[1,1'-biphenyl]-2-yl]piperidin-3-yl}-5-(difluoromethyl)-1H-pyrazole-4-carboxylic acid (Enantiomer 2)

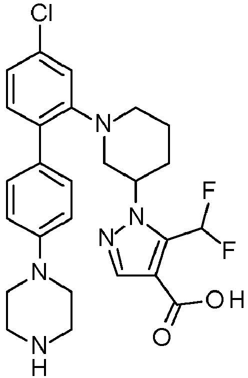

An aqueous solution of lithium hydroxide (4.0 ml, 1.0 M, 4.0 mmol) was added to a solution of ethyl 1-{1-[4-chloro-4'-(piperazin-1-yl)[1,1'-biphenyl]-2-yl]piperidin-3-yl}-5-(difluoromethyl)-1H-pyrazole-4-carboxylate hydrochloride (prepared in analogy to Example 18A, Enantiomer 2, 281 mg, 82 % purity, 396 µmol) in a THF/methanol mixture 10:1 (8.8 ml). The resulting mixture was stirred 2 hours at room temperature. The reaction mixture was acidified with an aqueous solution of hydrogen chloride (2 N) and evaporated. The residue was purified by preparative HPLC (RP18 column, eluent: Acetonitrile/water gradient) affording 175 mg (86 % yield) of the title compound.
LC-MS (Method 4): Rₜ = 0.83 min; MS (ESIpos): m/z = 516 [M+H]⁺

### Example 29A

### 1-(2,2-Dimethylpropyl)-4-[4-(4,4,5,5-tetramethyl-1,3,2-dioxaborolan-2-yl)phenyl]piperazine

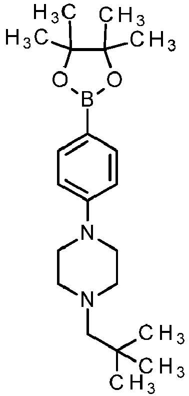

1-[4-(4,4,5,5-tetramethyl-1,3,2-dioxaborolan-2-yl)phenyl]piperazine (300 mg, 1.04 mmol) was placed in 6.4 ml THF and N,N-diisopropylethylamine (270 µl, 1.6 mmol) was added. Then 2,2-dimethylpropanal (450 µl, 4.2 mmol) was added and the mixture was stirred for 10 min. Then sodium triacetoxyborohydride (662 mg, 3.12 mmol) was added and the mixture was stirred at 55°C. for 4 h. The reaction mixture was cooled to room temperature, saturated aqueous sodium bicarbonate solution was added and the mixture was extracted three times with ethyl acetate. The combined organic phases were washed once with saturated, aqueous sodium chloride solution, dried over sodium sulfate, filtered and evaporated. 366 mg of the target compound (96% of theory) were obtained.
LC-MS (Method 3): Rₜ = 1.38 min; MS (ESIpos): m/z = 359 [M+H]⁺
¹H-NMR (600 MHz, DMSO-d6) δ [ppm]: 0.874 (11.03), 1.259 (16.00), 1.358 (0.55), 2.094 (2.28), 2.582 (1.32), 2.589 (1.74), 2.598 (1.38), 3.177 (1.08), 3.186 (1.41), 3.193 (1.12), 6.868 (1.03), 6.882 (1.05), 7.490 (1.08), 7.504 (0.99).

### Example 30A

### Ethyl 1-[1-{4-chloro-4'-[4-(2,2-dimethylpropyl)piperazin-1-yl][1,1'-biphenyl]-2-yl}piperidin-3-yl]-5-(difluoromethyl)-1H-pyrazole-4-carboxylate trifluoroacetic acid (Enantiomer 1)

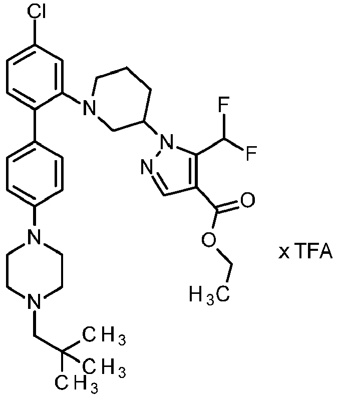

Ethyl 1-[1-{5-chloro-2-[(trifluoromethanesulfonyl)oxy]phenyl}piperidin-3-yl]-5-(difluoromethyl)-1H-pyrazole-4-carboxylate (prepared in analogy to Example 11A, Enantiomer 1, 30.0 mg, 55.3 µmol) and 1-(2,2-dimethylpropyl)-4-[4-(4,4,5,5-tetramethyl-1,3,2-dioxaborolan-2-yl)phenyl]piperazine (Example 29A, 21.8 mg, 60.8 µmol) were dissolved under argon in toluene/ethanol (0.31/0.31 ml). Tetrakis(triphenylphosphine)palladium(0) (3.19 mg, 2.76 µmol) and 2 M sodium carbonate solution (83 µl, 2.0 M, 170 µmol) were added and the mixture was stirred at 100°C for 2.5 h. The reaction mixture was filtered through a Millipore filter, washed with ethyl acetate and the filtrate was evaporated. The residue was dissolved in acetonitrile/TFA/water and purified by preparative HPLC (RP18 column, mobile phase: acetonitrile/water gradient with addition of 0. 1% TFA). 26 mg of the target compound were obtained (64% of theory).
LC-MS (Method 3): Rₜ = 2.23 min; MS (ESIpos): m/z = 614 [M-TFA+H]⁺

### Example 31A

### Ethyl 1-[1-{4-chloro-4'-[4-(2,2-dimethylpropyl)piperazin-1-yl][1,1'-biphenyl]-2-yl}piperidin-3-yl]-5-(difluoromethyl)-1H-pyrazole-4-carboxylate trifluoroacetic acid (Enantiomer 2)

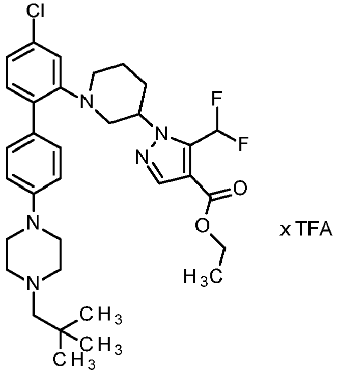

Ethyl 1-[1-{5-chloro-2-[(trifluoromethanesulfonyl)oxy]phenyl}piperidin-3-yl]-5-(difluoromethyl)-1H-pyrazole-4-carboxylate (prepared in analogy to Example 16A, Enantiomer 2, 50.0 mg, 93.1 µmol) 1 and 1-(2,2-dimethylpropyl)-4-[4-(4,4,5,5-tetramethyl-1,3,2-dioxaborolan-2-yl)phenyl]piperazine (Example 29A, 36.7 mg, 102 µmol) were dissolved under argon in toluene/ethanol (0.52/0.52 ml). Tetrakis(triphenylphosphine)palladium(0) (5.38 mg, 4.65 µmol) and 2 M sodium carbonate solution (140 µl, 280 µmol) were added and stirred at 100°C for 2.5 h. The reaction mixture was filtered through a Millipore filter, washed with ethyl acetate and the filtrate was evaporated. The residue was dissolved in acetonitrile/TFA/water and purified by preparative HPLC (RP18 column, acetonitrile/water gradient with addition of 0.1% TFA). 39 mg of the target compound (57% of theory) were received.
LC-MS (Method 3): Rₜ = 2.24 min; MS (ESIpos): m/z = 614 [M-TFA+H]⁺

### Example 32A

### 1-(Cyclobutylmethyl)-4-[4-(4,4,5,5-tetramethyl-1,3,2-dioxaborolan-2-yl)phenyl]piperazine

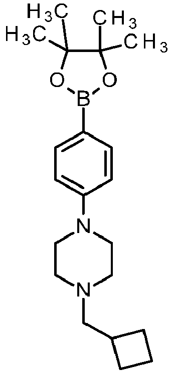

1-[4-(4,4,5,5-tetramethyl-1,3,2-dioxaborolan-2-yl)phenyl]piperazine (350 mg, 1.21 mmol) was placed in 7.4 ml THF and N,N-diisopropylethylamine (320 µl, 1.8 mmol) was added. Then cyclobutanecarbaldehyde (409 mg, 4.86 mmol) was added and the mixture was stirred for 10 min. Sodium triacetoxyborohydride (772 mg, 3.64 mmol) was added and the mixture was stirred at 55°C. for 4 h. The reaction mixture was cooled to room temperature, saturated aqueous sodium bicarbonate solution was added and the mixture was extracted three times with ethyl acetate. The combined organic phases were washed once with saturated, aqueous sodium chloride solution, dried over sodium sulfate, filtered and evaporated. The residue was purified by silica gel chromatography (dichloromethane/methanol: 50/1). 394 mg of the target compound (91% of theory) were obtained.
LC-MS (Method 3): Rₜ = 1.37 min; MS (ESIpos): m/z = 357 [M+H]^{+ 1}H-NMR (600 MHz, DMSO-d6) δ [ppm]: 1.071 (2.26), 1.257 (16.00), 1.650 (0.55), 1.666 (0.53), 2.013 (0.48), 2.027 (0.46), 2.357 (1.03), 2.369 (1.16), 2.441 (1.51), 2.517 (0.56), 3.164 (1.16), 3.172 (1.51), 3.180 (1.10), 5.744 (1.42), 6.865 (1.06), 6.879 (1.11), 7.486 (1.12), 7.500 (1.06).

### Example 33A

### Ethyl 1-[1-{4-chloro-4'-[4-(cyclobutylmethyl)piperazin-1-yl][1,1'-biphenyl]-2-yl}piperidin-3-yl]-5-(difluoromethyl)-1H-pyrazole-4-carboxylat trifluoroacetic acid (Enantiomer 2)

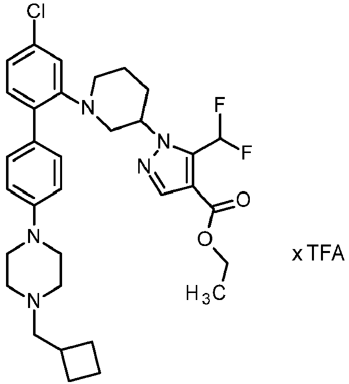

Ethyl 1-[1-{5-chloro-2-[(trifluoromethanesulfonyl)oxy]phenyl}piperidin-3-yl]-5-(difluoromethyl)-1H-pyrazole-4-carboxylate (prepared in analogy to Example 16A, Enantiomer 2, 47.0 mg, 87.5 µmol) and 1-(cyclobutylmethyl)-4-[4-(4,4,5,5-tetramethyl-1,3,2-dioxaborolan-2-yl)phenyl]piperazine (Example 32A, 34.3 mg, 96.2 µmol) were dissolved under argon in toluene/ethanol (0.48/0.48 ml). Tetrakis(triphenylphosphine)palladium(0) (5.05 mg, 4.37 µmol) and 2 M sodium carbonate solution (130 µl, 260 µmol) were added and stirred at 100°C for 2 h. The reaction mixture was filtered through a Millipore filter, washed with ethyl acetate and the filtrate evaporated. The residue was dissolved in acetonitrile/TFA/water and purified by preparative HPLC (RP18 column, acetonitrile/water gradient with addition of 0.1% TFA). 43 mg of the target compound (68% of theory) were received.
LC-MS (Method 3): Rₜ = 2.07 min; MS (ESIpos): m/z = 612 [M-TFA+H]⁺

### Example 34A

### [4-[4-[(3,3-Difluorocyclobutyl)methyl]piperazin-1-yl]phenyl]boronic acid

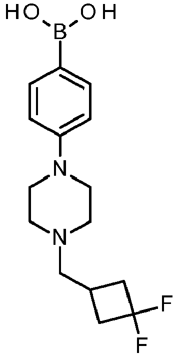

A solution of 1-[4-(4,4,5,5-tetramethyl-1,3,2-dioxaborolan-2-yl)phenyl]piperazine (700 mg, 2.43 mmol) in *N,N-*dimethylformamide (30 ml) was treated with 3-(bromomethyl)-1,1-difluorocyclobutane (674 mg, 3.64 mmol) and potassium carbonate (1.01 g, 7.29 mmol). The resulting mixture was stirred overnight at 80 °C then the reaction was diluted with water and extracted with dichloromethane. The organic phase was washed with water, dried, concentrated and purified by preparative HPLC (RP18 column, eluent: Acetonitrile/water + 0.1% formic acid gradient) affording 384 mg (88 % purity, 45 % yield) of the title compound.
LC-MS (Method 3): Rₜ = 0.60 min; MS (ESIpos): m/z = 311 [M+H]⁺

### Example 35A

### Ethyl 1-[1-(4-chloro-4'-{4-[(3,3-difluorocyclobutyl)methyl]piperazin-1-yl}[1,1'-biphenyl]-2-yl)piperidin-3-yl]-5-(difluoromethyl)-1H-pyrazole-4-carboxylate (Enantiomer 1)

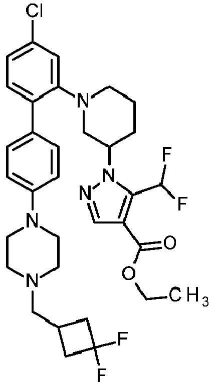

Under argon, a solution of ethyl 1-[1-{5-chloro-2-[(trifluoromethanesulfonyl)oxy]phenyl}piperidin-3-yl]-5-(difluoromethyl)-1H-pyrazole-4-carboxylate (prepared in analogy to Example 11A, Enantiomer 1, 100 mg, 188 µmol) and (4-{4-[(3,3-difluorocyclobutyl)methyl]piperazin-1-yl}phenyl)boronic acid (Example 34A, 79.5 mg, 88 % purity, 226 µmol) in toluene/ethanol (1:1, 2.6 ml) was treated with Pd(PPh₃)₄ (10.9 mg, 9.40 µmol)and an aqueous solution of sodium carbonate (280 µl, 2 N, 560 µmol) and the reaction was stirred at 100°C overnight. The reaction mixture was filtered over celite and the filtrate was concentrated. The residue was purified by preparative HPLC (RP18 column, eluent: Acetonitrile/water + 0.1% formic acid gradient) affording 49.0 mg (40 % yield) of the title compound.
LC-MS (Method 3): Rₜ = 2.15 min; MS (ESIpos): m/z = 648 [M+H]⁺
¹H-NMR (600 MHz, DMSO-d6) δ [ppm]: 1.265 (8.00), 1.276 (16.00), 1.288 (7.85), 1.506 (1.15), 1.527 (1.21), 1.710 (1.61), 1.732 (1.30), 1.878 (0.48), 1.893 (1.24), 1.913 (1.31), 1.934 (0.59), 1.980 (1.64), 2.293 (1.05), 2.385 (1.18), 2.423 (1.20), 2.558 (2.94), 2.577 (1.80), 2.613 (0.78), 2.652 (0.85), 2.701 (1.42), 3.008 (1.88), 3.026 (3.64), 3.044 (3.67), 3.065 (1.85), 3.158 (2.25), 3.244 (2.80), 4.242 (2.48), 4.254 (7.20), 4.266 (6.91), 4.278 (2.19), 4.478 (0.92), 4.496 (1.57), 4.514 (0.81), 6.975 (2.59), 6.986 (2.60), 7.055 (4.85), 7.086 (2.34), 7.100 (3.21), 7.151 (5.87), 7.165 (3.90), 7.382 (1.58), 7.443 (3.94), 7.456 (3.77), 7.469 (3.40), 7.556 (1.45), 7.996 (7.92), 8.131 (0.44).

### Example 36A

### [4-(4-Bromophenyl)piperazin-1-yl](1-fluorocyclobutyl)methanone

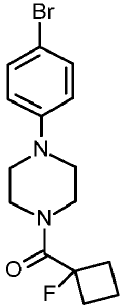

A solution of 1-fluorocyclobutane-1-carboxylic acid (269 mg, 2.28 mmol) in *N,N-*dimethylformamide (10 ml) was treated with HATU (788 mg, 2.07 mmol), *N,N-*diisopropylethylamine (720 µl, 4.1 mmol) and stirred 10 minutes at room temperature. 1-(4-bromophenyl)piperazine (500 mg, 2.07 mmol) was then added, the resulting mixture was stirred overnight at room temperature. The reaction mixture was diluted with water, extracted with ethyl acetate and the oganic phase was washed with water and evaporated. The residue was purified by preparative HPLC (RP18 column, eluent: Acetonitrile/water + 0.1% formic acid gradient) affording 440 mg (62 % yield) of the title compound.
LC-MS (Method 3): Rₜ = 2.15 min; MS (ESIpos): m/z = 341 [M+H]⁺
¹H-NMR (600 MHz, DMSO-d6) δ [ppm]: 1.486 (1.46), 1.501 (2.95), 1.515 (3.10), 1.519 (2.12), 1.530 (1.62), 1.533 (1.37), 1.834 (0.81), 1.841 (0.96), 1.846 (1.02), 1.852 (2.29), 1.859 (2.37), 1.864 (1.39), 1.870 (2.33), 1.876 (2.27), 1.883 (1.00), 1.887 (0.92), 1.894 (0.81), 2.069 (1.17), 2.348 (1.27), 2.353 (0.79), 2.365 (2.52), 2.370 (2.33), 2.380 (2.35), 2.385 (3.91), 2.403 (3.62), 2.408 (2.35), 2.418 (2.12), 2.423 (2.93), 2.440 (1.33), 2.644 (1.60), 2.651 (2.10), 2.659 (2.10), 2.665 (4.14), 2.671 (3.29), 2.682 (3.14), 2.687 (3.75), 2.694 (1.77), 2.702 (1.60), 2.709 (1.21), 3.148 (11.61), 3.156 (16.00), 3.164 (11.71), 3.260 (0.42), 3.555 (4.79), 3.634 (4.79), 6.903 (1.50), 6.909 (14.56), 6.924 (15.31), 6.930 (1.50), 7.351 (1.60), 7.356 (15.88), 7.371 (14.65), 7.377 (1.29).

### Example 37A

### 1-(4-Bromophenyl)-4-[(1-fluorocyclobutyl)methyl]piperazine

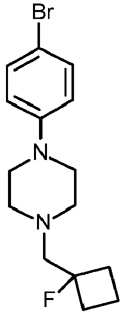

Under argon, a solution of 4-(4-bromophenyl)piperazin-1-yl](1-fluorocyclobutyl)methanone (Example 36A 440 mg, 1.29 mmol) in THF (8.9 ml) was treated dropwise with a solution of BH₃·THF complex (26 ml, 1 N, 26 mmol) and the resulting mixture was stirred overnight at room temperature. The reaction mixture was carefully quenched with methanol and evaporated. The residue was dissolved ethyl acetate and purified by preparative HPLC (RP18 column, eluent: Acetonitrile/water + 0.1% formic acid gradient) affording 300 mg (71 % yield) of the title compound.
LC-MS (Method 3): Rₜ = 1.18 min; MS (ESIpos): m/z = 327 [M+H]⁺
¹H-NMR (600 MHz, DMSO-d6) δ [ppm]: 1.493 (1.79), 1.508 (1.95), 1.764 (2.01), 2.184 (7.41), 2.383 (0.42), 2.604 (13.67), 2.659 (5.19), 3.111 (16.00), 3.245 (0.40), 3.256 (0.53), 3.323 (0.56), 6.868 (7.05), 6.882 (7.58), 7.3211 (6.99), 7.334 (6.82).

### Example 38A

### 1-[(1-Fluorocyclobutyl)methyl]-4-[4-(4,4,5,5-tetramethyl-1,3,2-dioxaborolan-2-yl)phenyl]piperazine

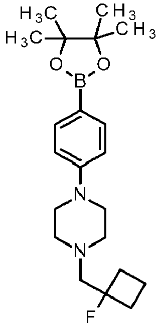

Under argon, a solution of 1-(4-bromophenyl)-4-[(1-fluorocyclobutyl)methyl]piperazine (Example 39A, 200 mg, 611 µmol) and 4,4,4',4',5,5,5',5'-octamethyl-2,2'-bi-1,3,2-dioxaborolane (232 mg, 917 µmol) in 1,4-dioxane (6.0 ml) was treated with Pd₂dba₃ (16.8 mg, 18.0 µmol), X-Phos (17.5 mg, 37.0 µmol) and potassium acetate (180 mg, 1.83 mmol). The mixture was stirred overnight at 105°C then filtered through celite and concentrated. The residue was purified with flash chromatography (silica gel, dichloromethane/methanol gradient) affording 228 mg (quant.) of the title compound.
LC-MS (Method 3): Rₜ = 1.42 min; MS (ESIpos): m/z = 375 [M+H]⁺

### Example 39A

### Ethyl 1-[1-(4-chloro-4'-{4-[(1-fluorocyclobutyl)methyl]piperazin-1-yl}[1,1'-biphenyl]-2-yl)piperidin-3-yl]-5-(difluoromethyl)-1H-pyrazole-4-carboxylate (Enantiomer 2)

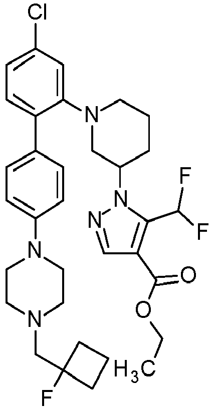

Under argon, a solution of ethyl 1-[1-{5-chloro-2-[(trifluoromethanesulfonyl)oxy]phenyl}piperidin-3-yl]-5-(difluoromethyl)-1H-pyrazole-4-carboxylate (prepared in analogy to Example 16A, Enantiomer 2, 92.0 mg, 173 µmol) and (4-{4-[(1-fluorocyclobutyl)methyl]piperazin-1-yl}phenyl)boronic acid (Example 38A, 60.6 mg, 208 µmοl) in toluene/ethanol (1:1, 2.4 ml) was treated with Pd(PPh₃)₄ (9.99 mg, 8.65 µmol) and an aqueous solution of sodium carbonate (260 µl, 2 N, 520 µmol) and the reaction was stirred at 100°C for 3 hours. The reaction mixture was diluted with acetonitrile and purified by preparative HPLC (RP18 column, eluent: Acetonitrile/water + 0.1% formic acid gradient) affording 73.0 mg (61 % purity, 41 % yield) of the title compound, which was used without further purification.
LC-MS (Method 3): Rₜ = 2.28 min; MS (ESIpos): m/z = 630 [M+H]⁺

### Example 40A

### (3-Fluorobicyclo[1.1.1]pentan-1-yl){4-[4-(4,4,5,5-tetramethyl-1,3,2-dioxaborolan-2-yl)phenyl]piperazin-1-yl}methanone

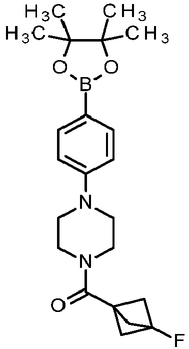

A solution of 3-fluorobicyclo[1.1.1]pentane-1-carboxylic acid (248 mg, 1.91 mmol) in DMF (8.0 ml) was treated with HATU (660 mg, 1.73 mmol), N,N-diisopropylethylamine (600 µl, 3.5 mmol) and stirred 10 minutes at room temperature. 1-[4-(4,4,5,5-tetramethyl-1,3,2-dioxaborolan-2-yl)phenyl]piperazine (500 mg, 1.73 mmol) was then added, the resulting mixture was stirred overnight at room temperature. The reaction mixture was diluted with water, extracted with ethyl acetate and the oganic phase was washed with water and evaporated. The residue was purified by preparative HPLC (RP18 column, eluent: Acetonitrile/water + 0.1% formic acid gradient) affording 450 mg (64 % yield) of the title compound.
LC-MS (Method 3): Rₜ = 2.12 min; MS (ESIpos): m/z = 401 [M+H]⁺
¹H-NMR (600 MHz, DMSO-d6) δ [ppm]: 1.262 (11.68), 2.404 (3.66), 2.408 (3.63), 3.217 (0.58), 3.287 (16.00), 6.891 (0.74), 6.906 (0.79), 7.521 (0.82), 7.535 (0.78).

### Example 41A

### 1-[(3-Fluorobicyclo[1.1.1]pentan-1-yl)methyl]-4-[4-(4,4,5,5-tetramethyl-1,3,2-dioxaborolan-2-yl)phenyl]piperazine

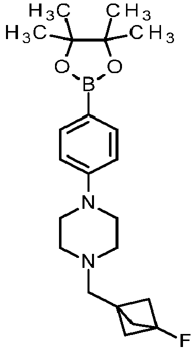

Under argon, a solution of (3-fluorobicyclo[1.1.1]pentan-1-yl){4-[4-(4,4,5,5-tetramethyl-1,3,2-dioxaborolan-2-yl)phenyl]piperazin-1-yl}methanone (Example 40A, 340 mg, 849 µmol) in THF (6.0 ml) was treated dropwise with a solution of BH₃·THF complex (17 ml, 1 N, 17 mmol) and the resulting mixture was stirred overnight at room temperature. The reaction mixture was carefully quenched with methanol and evaporated. The residue was dissolved ethyl acetate and purified by flash chromatograpthy (silica gel dichloromethane/methanol gradient) affording 353 mg (quant.) of the title compound, which was used without further purification
LC-MS (Method 3): Rₜ = 2.41 min; MS (ESIpos): m/z = 387 [M+H]⁺
¹H-NMR (400 MHz, DMSO-d6) δ [ppm]: 1.263 (16.00), 2.178 (4.94), 2.185 (4.66), 2.963 (0.91), 2.977 (1.65), 2.989 (0.88), 3.247 (1.91), 3.405 (0.53), 3.417 (0.80), 3.430 (0.69), 3.444 (0.71), 3.458 (0.41), 6.905 (1.08), 6.927 (1.06), 7.523 (1.25), 7.545 (1.06).

### Example 42A

### Ethyl 1-[1-(4-chloro-4'-{4-[(3-fluorobicyclo[1.1.1]pentan-1-yl)methyl]piperazin-1-yl}[1,1'-biphenyl]-2-yl)piperidin-3-yl]-5-(difluoromethyl)-1H-pyrazole-4-carboxylate (Enantiomer 2)

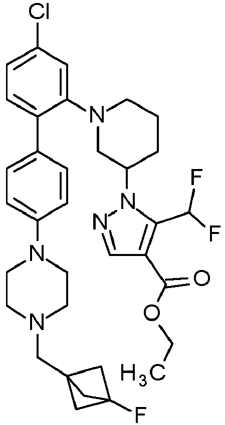

Under argon, a solution of ethyl 1-[1-{5-chloro-2-[(trifluoromethanesulfonyl)oxy]phenyl}piperidin-3-yl]-5-(difluoromethyl)-1H-pyrazole-4-carboxylate (prepared in analogy to Example 16A, Enantiomer 2, 180 mg, 338 µmol) and 1-[(3-fluorobicyclo[1.1.1]pentan-1-yl)methyl]-4-[4-(4,4,5,5-tetramethyl-1,3,2-dioxaborolan-2-yl)phenyl]piperazine (Example 41A, 157 mg, 406 µmol) in toluene/ethanol (1:1, 5.0 ml) was treated with Pd(PPh₃)₄ (19.6 mg, 16.9 µmol) and an aqueous solution of sodium carbonate (510 µl, 2 N, 1.0 mmol) and the reaction was stirred at 100°C for 3 hours. The reaction mixture was concentrated and the residue was purified by flash chromatography (silica gel, dichloromethane/methanol, gradient). The product containing fractions were then purified by preparative HPLC (RP18 column, eluent: Acetonitrile/water + 0.1% formic acid gradient) affording 64.0 mg (28 % yield) of the title compound.
LC-MS (Method 3): Rₜ = 2.08 min; MS (ESIpos): m/z = 642 [M+H]⁺
¹H-NMR(600 MHz, DMSO-d6) δ [ppm]: 1.071 (4.26), 1.265 (4.72), 1.277 (9.86), 1.289 (4.88), 1.502 (0.45), 1.524 (0.48), 1.704 (0.59), 1.726 (0.49), 1.889 (0.49), 1.896 (0.46), 1.909 (0.49), 1.979 (0.61), 2.016 (16.00), 2.020 (15.80), 2.559 (3.85), 2.568 (3.09), 2.667 (5.76), 3.010 (0.73), 3.027 (1.46), 3.045 (1.36), 3.061 (0.60), 3.158 (2.44), 3.164 (3.22), 3.168 (3.24), 3.175 (2.33), 3.250 (0.75), 3.262 (0.77), 3.268 (0.80), 3.899 (0.66), 4.243 (1.32), 4.254 (4.04), 4.266 (3.85), 4.278 (1.21), 4.485 (0.42), 4.502 (0.66), 6.953 (3.26), 6.968 (3.39), 7.045 (2.50), 7.048 (2.86), 7.081 (1.44), 7.085 (1.18), 7.095 (1.92), 7.098 (1.80), 7.150 (3.57), 7.164 (2.36), 7.387 (0.73), 7.431 (3.86), 7.445 (3.50), 7.474 (1.49), 7.560 (0.67), 7.998 (3.96).

### Example 43A

### 1-(2,2-Difluoroethyl)-4-[4-(4,4,5,5-tetramethyl-1,3,2-dioxaborolan-2-yl)phenyl]piperazine

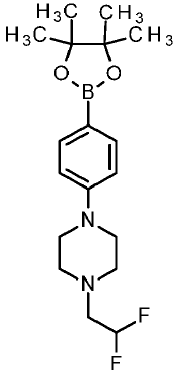

Under argon, a solution of 1-[4-(4,4,5,5-tetramethyl-1,3,2-dioxaborolan-2-yl)phenyl]piperazine (500 mg, 1.73 mmol) in DMF (8.1 ml) was treated with N,N-diisopropylethylamine (940 µl, 5.4 mmol) and 2,2-difluoroethyl trifluoromethanesulfonate (1.04 g, 4.86 mmol). The mixture was stirred overnight at room temperature, then the reaction was diluted with water and extracted with ethyl actetate. The organic phase was washed with brine, dried with sodium sulfate and concentrated affording 582 mg (92 % yield) of the title compound, which was used without further purification.
LC-MS (Method 3): Rₜ = 2.03 min; MS (ESIpos): m/z = 353 [M+H]⁺
¹H-NMR (600 MHz, DMSO-d6) δ [ppm]: 1.257 (16.00), 2.636 (1.04), 2.644 (1.30), 2.653 (1.08), 2.772 (0.61), 2.779 (0.61), 3.194 (1.08), 3.202 (1.25), 3.210 (0.99), 6.887 (1.05), 6.902 (1.05), 7.496 (1.17), 7.511 (1.05).

### Example 44A

### Ethyl 1-[1-{4-chloro-4'-[4-(2,2-difluoroethyl)piperazin-1-yl][1,1'-biphenyl]-2-yl}piperidin-3-yl]-5-(difluoromethyl)-1H-pyrazole-4-carboxylate (Enantiomer 1)

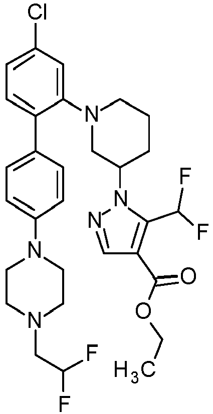

Under argon, a solution of ethyl 1-[1-{5-chloro-2-[(tnfluoromethanesulibnyl)oxy]phenyl}pipendin-3-yl]-5-(difluoromethyl)-1H-pyrazole-4-carboxylate (prepared in analogy to Example 11A, Enantiomer 1, 113 mg, 213 µmol) and 1-(2,2-difluoroethyl)-4-[4-(4,4,5,5-tetramethyl-1,3,2-dioxaborolan-2-yl)phenyl]piperazine (Example 43A, 90.0 mg, 256 µmol) in toluene/ethanol (1:1, 3.0 ml) was treated with Pd(PPh₃)₄ (12.3 mg, 10.6 µmol) and an aqueous solution of sodium carbonate (320 µl, 2.0 M, 640 µmol) and the reaction was stirred at 100°C for 3 hours. The reaction mixture was acidified with formic acid and the mixture was filtered through an Extrelut cartridge. The filtrate was concentrated affording 87.0 mg (65 % yield) of the title compound, which was used without further purification.
LC-MS (Method 3): Rₜ = 2.90 min; MS (ESIpos): m/z = 608 [M+H]⁺

### Example 45A

### Ethyl 1-[1-{4-chloro-4'-[4-(2,2-difluoroethyl)piperazin-1-yl][1,1'-biphenyl]-2-yl}piperidin-3-yl]-5-(difluoromethyl)-1H-pyrazole-4-carboxylate (Enantiomer 2)

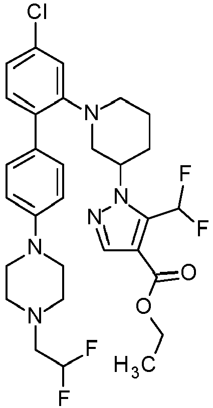

Under argon, a solution of ethyl 1-[1-{5-chloro-2-[(trifluoromethanesulflonyl)oxy]phenyl}piperidin-3-yl]-5-(difluoromethyl)-1H-pyrazole-4-carboxylate (prepared in analogy to Example 16A, Enantiomer 2, 113 mg, 213 µmol) and 1-(2,2-difluoroethyl)-4-[4-(4,4,5,5-tetramethyl-1,3,2-dioxaborolan-2-yl)phenyl]piperazine (Example 43A, 90.0 mg, 256 µmol) in toluene/ethanol (1:1, 3.0 ml) was treated with Pd(PPh₃)₄ (12.3 mg, 10.6 µmol) and an aqueous solution of sodium carbonate (320 µl, 2.0 M, 640 µmol) and the reaction was stirred at 100°C for 3 hours. The reaction mixture was acidified with formic acid and the mixture was filtered through an Extrelut cartridge. The filtrate was concentrated affording 93.0 mg (91 % purity, 65 % yield) of the title compound, which was used without further purification.
LC-MS (Method 3): Rₜ = 2.91 min; MS (ESIpos): m/z = 608 [M+H]⁺

### Example 46A

### Tert-butyl 4-(2'-bromo-4'-chloro[1,1'-biphenyl]-4-yl)piperazine-1-carboxylate

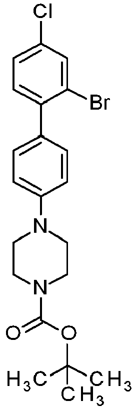

Under argon, a suspension of 2-bromo-4-chloro-1-iodobenzene (518 mg, 1.63 mmol), {4-[4-(tert-butoxycarbonyl)piperazin-1-yl]phenyl}boronic acid (500 mg, 1.63 mmol), Pd(PPh₃)₄ (94.4 mg, 81.7 µmol) was treated with an aqueous solution of sodium carbonate (2.4 ml, 2.0 M, 4.9 mmol) and heated overnight at 85°C. The reaction mixture was cooled to room temperature, diluted with water and extracted three times with ethyl acetate. The combined organic layers were dried over magnesium sulfate and evaporated. The residue was purified by flash chromatography (silica gel, cyclohexane/ethyl acetate gradient) affording 390 mg (52 % yield) of the title compound.
LC-MS (Method 3): Rₜ = 2.82 min; MS (ESIpos): m/z = 451 [M+H]⁺
¹H-NMR (400 MHz, DMSO-d6) δ [ppm]: -0.008 (0.43), 0.008 (0.47), 1.419 (1.16), 1.428 (16.00), 3.166 (0.88), 3.179 (1.25), 3.192 (1.05), 3.458 (0.93), 3.471 (1.13), 3.483 (0.78), 7.006 (1.04), 7.028 (1.24), 7.258 (1.42), 7.280 (1.16), 7.355 (0.92), 7.376 (1.19), 7.488 (0.68), 7.493 (0.69), 7.508 (0.50), 7.514 (0.52), 7.820 (1.11), 7.826 (1.09).

### Example 47A

### 1-(2'-Bromo-4'-chloro[1,1'-biphenyl]-4-yl)piperazine hydrochloride

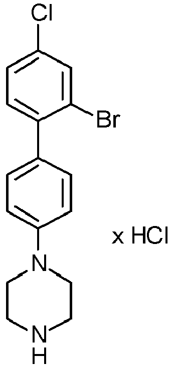

A solution of tert-butyl 4-(2'-bromo-4'-chloro[1,1'-biphenyl]-4-yl)piperazine-1-carboxylate (prepared in analogy to Example 46A, 664 mg, 1.47 mmol) in dichloromethane (8.0 ml) was treated with a solution of hydrogen chloride in dioxane (3.7 ml, 4.0 M, 15 mmol), stirred 2.5 hours and evaporated. The residue was triturated in diethyl ether. The solid was filtered off affording 602 mg (quant.) of the title compound.
LC-MS (Method 3): Rₜ = 1.46 min; MS (ESIpos): m/z = 351 [M+H]⁺
¹H-NMR(400 MHz, DMSO-d6) δ [ppm]: 0.146 (0.66), 1.596 (1.27), 2.329 (0.80), 2.671 (0.82), 3.360 (1.28), 3.437 (12.88), 3.451 (14.96), 3.463 (10.26), 3.568 (1.96), 4.670 (3.35), 5.756 (2.57), 7.002 (0.87), 7.055 (12.43), 7.077 (14.87), 7.294 (16.00), 7.315 (12.89), 7.362 (11.21), 7.382 (14.09), 7.502 (7.29), 7.507 (7.43), 7.522 (5.26), 7.528 (5.74), 7.835 (12.20), 7.840 (11.66), 9.202 (2.59).

### Example 48A

### 1-(2'-Bromo-4'-chloro[1,1'-biphenyl]-4-yl)-4-(2,2,2-trifluoroethyl)piperazine

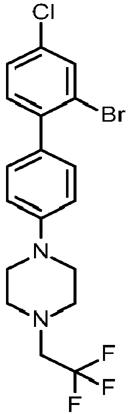

Under argon, a solution of 1-(2'-bromo-4'-chloro[1,1'-biphenyl]-4-yl)piperazine hydrochloride (Example 47A, 600 mg, 1.55 mmol) in DMF (7.8 ml) was treated with N,N-diisopropylethylamine (1.6 ml, 9.3 mmol) and 2,2,2-trifluoroethyl trifluoromethanesulfonate (670 µl, 4.6 mmol) and stirred overnight at room temperature. The reaction mixture was diluted with water and extracted three times with ethyl acetate. The combined organic layers were dried over magnesium sulfate and evaporated. The residue was purified by flash chromatography (silica gel, cyclohexane/ethyl acetate gradient) affording 536 mg (80 % yield) of the title compound.
LC-MS (Method 4): Rₜ = 1.42 min; MS (ESIpos): m/z = 433 [M+H]⁺ (isotope 1) m/z = 435 [M+H]⁺ (isotope 2)
¹H-NMR (400 MHz, DMSO-d6) δ [ppm]: -0.007 (1.98), 0.008 (2.22), 2.740 (0.98), 2.758 (11.29), 2.770 (14.79), 2.782 (12.18), 3.115 (0.79), 3.128 (0.89), 3.140 (0.72), 3.205 (12.89), 3.212 (13.88), 3.218 (16.00), 3.230 (12.50), 3.236 (11.79), 3.262 (9.39), 3.288 (3.13), 6.914 (0.51), 6.935 (0.60), 6.990 (11.06), 7.011 (13.08), 7.204 (0.54), 7.246 (13.70), 7.267 (11.56), 7.354 (8.19), 7.375 (10.92), 7.485 (5.59), 7.490 (5.77), 7.506 (4.15), 7.511 (4.36), 7.817 (7.70), 7.823 (7.57).

### Example 49A

### Ethyl 1-[1-{4-chloro-4'-[4-(2,2,2-trifluoroethyl)piperazin-1-yl][1,1'-biphenyl]-2-yl}piperidin-3-yl]-5-(difluoromethyl)-1H-pyrazole-4-carboxylate (Enantiomer 1)

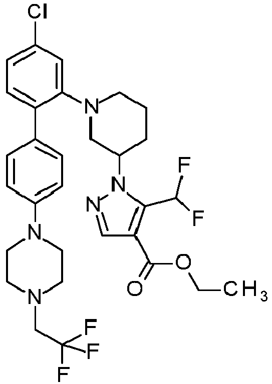

Under argon, a solution of 1-(2'-bromo-4'-chloro[1,1'-biphenyl]-4-yl)-4-(2,2,2-trifluoroethyl)piperazine (Example 48A, 150 mg, 346 µmol) and ethyl 5-(difluoromethyl)-1-[piperidin-3-yl]-1H-pyrazole-4-carboxylate (prepared in analogy to Example 6A, Enantiomer 1, 94.5 mg, 346 µmol) in toluene (3.0 ml) was treated with caesium carbonate (282 mg, 865 µmol) and RuPhos Pd G3 (57.9 mg, 69.2 µmol) and stirred 16 hours at 100°C. The reaction mixture was cooled to room temperature, diluted with ethyl acetate, filtered over celite and evaporated. The residue was purified by preparative HPLC (RP18 column, eluent: Acetonitrile/water + 0.5% formic acid gradient) affording 62.7 mg (29 % yield) of the title compound.
LC-MS (Method 4): Rₜ = 1.58 min; MS (ESIpos): m/z = 626 [M+H]⁺

### Example 50A

### Ethyl 1-[1-{4-chloro-4'-[4-(2,2,2-trifluoroethyl)piperazin-1-yl][1,1'-biphenyl]-2-yl}piperidin-3-yl]-5-(difluoromethyl)-1H-pyrazole-4-carboxylate (Enantiomer 2)

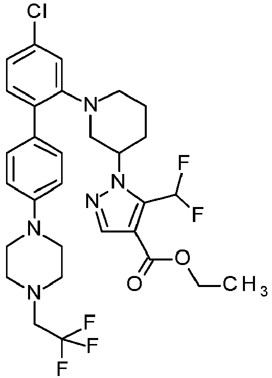

Under argon, a solution of 1-(2'-bromo-4'-chloro[1,1'-biphenyl]-4-yl)-4-(2,2,2-trifluoroethyl)piperazine (Example 48A, 150 mg, 346 µmol) and ethyl 5-(difluoromethyl)-1-[piperidin-3-yl]-1H-pyrazole-4-carboxylate (prepared in analogy to Example 7A, Enantiomer 2, 94.5 mg, 346 µmol) in toluene (3.0 ml) was treated with caesium carbonate (282 mg, 865 µmol) and RuPhos Pd G3 (57.9 mg, 69.2 µmol) and stirred 16 hours at 100°C. The reaction mixture was cooled to room temperature, diluted with ethyl acetate, filtered over celite and evaporated. The residue was purified by preparative HPLC (RP18 column, eluent: Acetonitrile/water + 0.5% formic acid gradient) affording 66.6 mg (30 % yield) of the title compound.
LC-MS (Method 4): Rₜ = 1.58 min; MS (ESIpos): m/z = 626 [M+H]⁺

### Example 51A

### 3,3-Difluoropropyl 4-methylbenzene-1-sulfonate

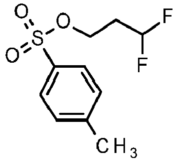

Under argon, a solution of 3,3-difluoropropan-1-ol (100 mg, 1.04 mmol) in dichloromethane (970 µl) and pyridine (680 µl) was stirred 30 minutes at room temperature and cooled to -10°C. 4-methylbenzene-1-sulfonyl chloride (238 mg, 1.25 mmol) was then added slowly and the resulting mixture was stirred 2.5 hours at 0°C and overnight at room temperature. The reaction mixture was diluted with dichloromethane, washed once with water followed by a saturated solution of sodium chloride. The organic layer was dried over sodium sulfate and evaporated affording 202 mg (77 % yield) of the title compound which was used in the next steps without further purification.
¹H-NMR(600 MHz, DMSO-d6) δ [ppm]: 2.173 (0.86), 2.181 (0.93), 2.192 (1.13), 2.202 (1.71), 2.209 (1.72), 2.220 (1.14), 2.230 (0.86), 2.238 (0.85), 2.287 (7.61), 2.431 (16.00), 4.126 (3.53), 4.136 (6.70), 4.146 (3.44), 4.800 (0.70), 4.811 (1.36), 4.823 (0.67), 6.006 (0.79), 6.092 (0.79), 6.099 (1.59), 6.106 (0.78), 6.192 (0.81), 6.200 (0.42), 7.103 (1.84), 7.116 (1.98), 7.467 (2.27), 7.480 (2.14), 7.494 (3.88), 7.508 (4.20), 7.798 (5.08), 7.812 (4.60), 8.170 (0.48), 8.182 (0.75), 8.193 (0.51), 8.629 (0.50), 8.845 (0.48), 9.123 (0.91), 9.132 (0.90).

### Example 52A

### 1-(3,3-Difluoropropyl)-4-[4-(4,4,5,5-tetramethyl-1,3,2-dioxaborolan-2-yl)phenyl]piperazine

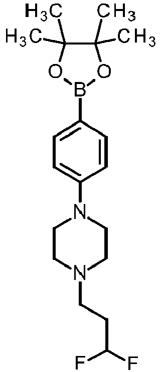

A solution of 1-[4-(4,4,5,5-tetramethyl-1,3,2-dioxaborolan-2-yl)phenyl]piperazine (80.0 mg, 278 µmol) in acetonitrile (3.5 ml) was treated with 3,3-difluoropropyl 4-methylbenzene-1-sulfonate (Example 51A, 83.4 mg, 333 µmol), potassium carbonate (192 mg, 1.39 mmol) and potassium iodide (4.61 mg, 27.8 µmol) and stirred overnight at 70°C. The reaction mixture was combined with a 100 mg test reaction, filtered over celite and rinsed with acetonitrile. The filtrate was evaporated and the residue was purified by flash chromatography (silica gel, dichloromethane/methanol gradient) affording 152 mg (66 % yield) of the title compound.
LC-MS (Method 3): Rₜ = 1.20 min; MS (ESIpos): m/z = 367 [M+H]⁺

### Example 53A

### Ethyl 1-[1-{4-chloro-4'-[4-(3,3-difluoropropyl)piperazin-1-yl][1,1'-biphenyl]-2-yl}piperidin-3-yl]-5-(difluoromethyl)-1H-pyrazole-4-carboxylate (Enantiomer 2)

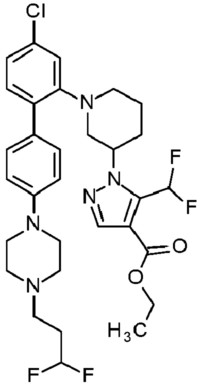

Under argon, a solution of ethyl 1-[1-{5-chloro-2-[(triffuoromethanesulfonyl)oxy]phenyl}piperidin-3-yl]-5-(difluoromethyl)-1H-pyrazole-4-carboxylate (prepared in analogy to Example 16A, Enantiomer 2, 91.4 mg, 172 µmol) and 1-(3,3-difluoropropyl)-4-[4-(4,4,5,5-tetramethyl-1,3,2-dioxaborolan-2-yl)phenyl]piperazine (Example 52A, 75.5 mg, 206 µmol) in toluene (960 µl) and ethanol (960 µl) was treated with an aqueous solution of sodium carbonate (260 µl, 2.0 M, 520 µmol) and Pd(PPh₃)₄ (9.93 mg, 8.59 µmol) and stirred 2 hours at 100°C. The reaction mixture was cooled to room temperature, filtered over celite and washed with ethyl acetate. The filtrate was evaporated. The residue was purified by preparative HPLC (RP18 column, eluent: Acetonitrile/water + 0.1% formic acid gradient) affording 76.0 mg (71 % yield) of the title compound.
LC-MS (Method 3): Rₜ = 2.19 min; MS (ESIpos): m/z = 622 [M+H]⁺

### Example 54A

### 1-[4-(4,4,5,5-Tetramethyl-1,3,2-dioxaborolan-2-yl)phenyl]-4-(3,3,3-trifluoropropyl)piperazine

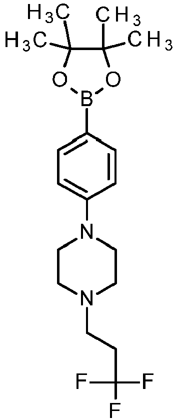

A solution of 1-[4-(4,4,5,5-tetramethyl-1,3,2-dioxaborolan-2-yl)phenyl]piperazine (300 mg, 1.04 mmol) in THF (6.4 ml) was treated with N,N-diisopropylethylamine (270 µl, 1.6 mmol) and 3,3,3-trifluoropropanal (481 mg, 4.16 mmol). The resulting mixture was stirred 10 minutes at room temperature. Sodium triacetoxyborohydride (662 mg, 3.12 mmol) was added and the resulting mixture was stirred 4 hours at 55°C. The reaction mixture was cooled to room temperature, diluted with a saturated solution of sodium hydrogencarbonate and extracted three times with ethyl acetate. The combined organic layers were dried over sodium sulfate and evaporated affording 482 mg (73 % purity, 88 % yield) of the title compound which was used in the next step without further purification.
LC-MS (Method 3): Rₜ = 1.54 min; MS (ESIpos): m/z = 385 [M+H]⁺

### Example 55A

### Ethyl 1-[1-{4-chloro-4'-[4-(3,3,3-trifluoropropyl)piperazin-1-yl][1,1'-biphenyl]-2-yl}piperidin-3-yl]-5-(difluoromethyl)-1H-pyrazole-4-carboxylate (Enantiomer 2)

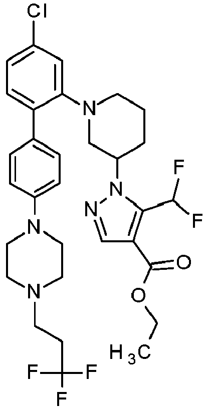

Under argon, a solution of ethyl 1-[1-{5-chloro-2-[(trifluoromethanesulfonyl)oxy]phenyl}piperidin-3-yl]-5-(difluoromethyl)-1H-pyrazole-4-carboxylate (prepared in analogy to Example 16A, Enantiomer 2, 100 mg, 188 µmol) and 1-[4-(4,4,5,5-tetramethyl-1,3,2-dioxaborolan-2-yl)phenyl]-4-(3,3,3-trifluoropropyl)piperazine (Example 54A, 119 mg, 73 % purity, 226 µmol) in toluene/ethanol mixture 1:1 (2 ml) was treated with an aqueous solution of sodium carbonate (280 µl, 2.0 M, 560 µmol) and Pd(PPh₃)₄ (10.9 mg, 9.40 µmol) and stirred 4 hours at 100°C. The reaction mixture was cooled to room temperature, acidified with formic acid and filtered over an EXtrelut NT 3 cartridge. The filtrated was evaporated and the residue purified by preparative HPLC (RP18 column, eluent: Acetonitrile/water gradient) affording 66.0 mg (55 % yield) of the title compound.
LC-MS (Method 3): Rₜ = 2.54 min; MS (ESIpos): m/z = 640 [M+H]⁺

### Example 56A

### 2,2-Difluoro-1-{4-[4-(4,4,5,5-tetramethyl-1,3,2-dioxaborolan-2-yl)phenyl]piperazin-1-yl}propan-1-one

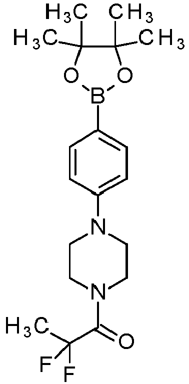

A solution of 2,2-difluoropropanoic acid (147 mg, 1.34 mmol) in dichloromethane (5.0 ml) was treated with HATU (462 mg, 1.21 mmol), N,N-diisopropylethylamine (420 µl, 2.4 mmol) and finally 1-[4-(4,4,5,5-tetramethyl-1,3,2-dioxaborolan-2-yl)phenyl]piperazine (350 mg, 1.21 mmol) was added. The resulting mixture was stirred overnight at room temperature then diluted with water and dichloromethane. The organic phase was washed with water, dried with sodium sulfate and concentrated. The residue was purified by flash chromatography (silica gel, cylcohexane/ethyl acetate gradient) affording 287 mg (62 % yield) of the title compound
LC-MS (Method 3): Rₜ = 2.25 min; MS (ESIpos): m/z = 381 [M+H]⁺
¹H-NMR (600 MHz, DMSO-d6) δ [ppm]: 1.263 (16.00), 1.795 (0.88), 1.829 (1.80), 1.863 (0.81), 3.284 (1.31), 3.670 (0.58), 3.771 (0.57), 6.920 (1.05), 6.935 (1.14), 7.526 (1.12), 7.540 (1.11).

### Example 57A

### 1-(2,2-Difluoropropyl)-4-[4-(4,4,5,5-tetramethyl-1,3,2-dioxaborolan-2-yl)phenyl]piperazine

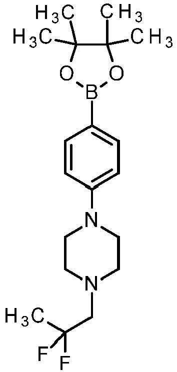

Under argon, a solution of 2,2-difluoro-1-{4-[4-(4,4,5,5-tetramethyl-1,3,2-dioxaborolan-2-yl)phenyl]piperazin-1-yl}propan-1-one (Example 56A, 286 mg, 752 µmol) in THF (1.4 ml) was treated dropwise with a solution of BH₃·THF complex (7.5 ml, 1 N, 7.5 mmol) and the resulting mixture was stirred for 2 hours at room temperature. The reaction mixture was carefully quenched with methanol and evaporated. The residue was purified by flash chromatograpthy (silica gel cyclohexane/ethyl acetate gradient) affording 212 mg (77 % yield) of the title compound.
LC-MS (Method 3): Rₜ = 2.19 min; MS (ESIpos): m/z = 367 [M+H]⁺
¹H-NMR (600 MHz, DMSO-d6) δ [ppm]: 1.260 (16.00), 1.612 (0.81), 1.644 (1.67), 1.676 (0.77), 2.648 (1.06), 2.656 (1.41), 2.664 (1.20), 2.739 (0.48), 2.762 (0.99), 2.786 (0.47), 3.197 (1.10), 3.206 (1.37), 3.214 (1.12), 6.882 (1.02), 6.897 (1.09), 7.497 (1.10), 7.511 (1.06).

### Example 58A

### Ethyl 1-[1-{4-chloro-4'-[4-(2,2-difluoropropyl)piperazin-1-yl][1,1'-biphenyl]-2-yl}piperidin-3-yl]-5-(difluoromethyl)-1H-pyrazole-4-carboxylate (Enantiomer 1)

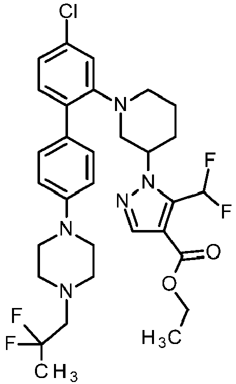

Under argon, a solution of ethyl 1-[1-{5-chloro-2-[(trifluoromethanesulfonyl)oxy]phenyl}piperidin-3-yl]-5-(difluoromethyl)-1H-pyrazole-4-carboxylate (prepared in analogy to Example 11A, Enantiomer 1, 84.7 mg, 159 µmol) and 1-(2,2-difluoropropyl)-4-[4-(4,4,5,5-tetramethyl-1,3,2-dioxaborolan-2-yl)phenyl]piperazine (Example 57A, 70.0 mg, 191 µmol) in toluene/ethanol (1:1, 2.3 ml) was treated with Pd(PPh₃)₄ (9.20 mg, 7.96 µmol) and an aqueous solution of sodium carbonate (240 µl, 2 N, 480 µmol) and the reaction was stirred at 100°C overnight. The reaction mixture was filtered over celite and the filtrate was concentrated. The residue was purified by preparative HPLC (RP18 column, eluent: Acetonitrile/water + 0.1% formic acid gradient) affording 70 mg (71 % yield) of the title compound.
LC-MS (Method 3): Rₜ = 3.00 min; MS (ESIpos): m/z = 622 [M+H]⁺
¹H-NMR (600 MHz, DMSO-d6) δ [ppm]: -0.022 (1.04), 0.025 (0.49), 1.235 (3.16), 1.264 (8.16), 1.276 (16.00), 1.288 (7.88), 1.504 (1.18), 1.524 (1.27), 1.546 (0.60), 1.710 (2.76), 1.731 (1.97), 1.880 (0.53), 1.895 (1.20), 1.916 (1.31), 1.936 (0.62), 1.980 (1.68), 2.384 (0.45), 2.423 (0.60), 2.559 (1.72), 2.577 (0.97), 2.613 (0.46), 2.652 (0.81), 2.707 (1.27), 3.012 (1.84), 3.030 (3.65), 3.047 (3.25), 3.061 (1.76), 3.195 (2.81), 3.246 (2.79), 3.265 (2.79), 4.243 (2.44), 4.255 (7.22), 4.266 (7.02), 4.278 (2.26), 4.481 (0.92), 4.499 (1.60), 4.517 (0.83), 6.987 (2.89), 6.999 (3.04), 7.057 (4.82), 7.089 (2.23), 7.102 (3.20), 7.155 (5.91), 7.168 (3.92), 7.391 (1.48), 7.451 (3.77), 7.464 (3.69), 7.478 (3.34), 7.565 (1.34), 7.998 (7.89).

### Example 59A

### Ethyl 1-[1-{4-chloro-4'-[4-(2,2-difluoropropyl)piperazin-1-yl][1,1'-biphenyl]-2-yl}piperidin-3-yl]-5-(difluoromethyl)-1H-pyrazole-4-carboxylate (Enantiomer 2)

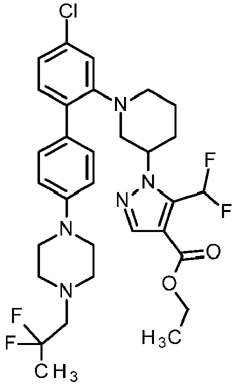

Under argon, a solution of ethyl 1-[1-{5-chloro-2-[(trifluoromethanesulfonyl)oxy]phenyl}piperidin-3-yl]-5-(difluoromethyl)-1H-pyrazole-4-carboxylate (prepared in analogy to Example 16A, Enantiomer 2, 84.7 mg, 159 µmol) and 1-(2,2-difluoropropyl)-4-[4-(4,4,5,5-tetramethyl-1,3,2-dioxaborolan-2-yl)phenyl]piperazine (Example 57A 70.0 mg, 191 µmol) in toluene/ethanol (1:1, 2.3 ml) was treated with Pd(PPh₃)₄ (9.20 mg, 7.96 µmol) and an aqueous solution of sodium carbonate (240 µl, 2 N, 480 µmol) and the reaction was stirred at 100°C overnight. The reaction mixture was filtered over celite and the filtrate was concentrated. The residue was purified by preparative HPLC (RP18 column, eluent: Acetonitrile/water + 0.1% formic acid gradient) affording 74.0 mg (75 % yield) of the title compound.
LC-MS (Method 3): Rₜ = 2.99 min; MS (ESIpos): m/z = 622 [M+H]⁺
¹H-NMR (600 MHz, DMSO-d6) δ [ppm]: 1.175 (0.61), 1.235 (0.79), 1.264 (7.86), 1.276 (16.00), 1.288 (8.01), 1.505 (0.90), 1.526 (0.99), 1.628 (4.72), 1.660 (9.79), 1.692 (4.44), 1.707 (1.32), 1.728 (1.05), 1.891 (0.96), 1.897 (0.91), 1.911 (1.03), 1.917 (1.01), 1.932 (0.52), 1.987 (2.08), 2.423 (0.51), 2.567 (1.06), 2.612 (0.43), 2.652 (0.56), 2.681 (5.88), 2.689 (8.80), 2.697 (6.56), 2.758 (2.80), 2.781 (5.55), 2.804 (2.59), 3.011 (1.49), 3.028 (2.94), 3.046 (2.77), 3.063 (1.25), 3.174 (5.36), 3.184 (6.96), 3.190 (5.25), 3.250 (1.68), 3.271 (1.80), 4.242 (2.38), 4.254 (7.08), 4.266 (6.88), 4.277 (2.19), 4.485 (0.83), 4.503 (1.32), 4.521 (0.76), 6.966 (6.15), 6.980 (6.44), 7.046 (4.64), 7.049 (5.19), 7.083 (2.44), 7.086 (1.98), 7.097 (3.30), 7.100 (2.99), 7.153 (5.92), 7.166 (4.02), 7.388 (1.38), 7.436 (7.03), 7.451 (6.40), 7.475 (2.81), 7.562 (1.17), 7.998 (7.50).

### Example 60A

### Ethyl 1-[1-(4-chloro-4'-{4-[2-(trifluoromethoxy)ethyl]piperazin-1-yl}[1,1'-biphenyl]-2-yl)piperidin-3-yl]-5-(difluoromethyl)-1H-pyrazole-4-carboxylate (Enantiomer 2)

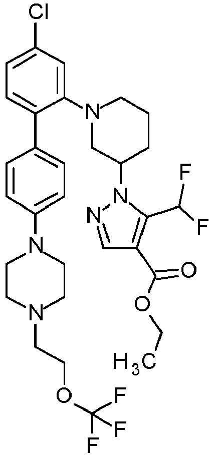

A solution of ethyl 1-{1-[4-chloro-4'-(piperazin-1-yl)[1,1'-biphenyl]-2-yl]piperidin-3-yl}-5-(difluoromethyl)-1H-pyrazole-4-carboxylate (prepared in analogy to Example 18A, Enantiomer 2, 150 mg, 276 µmol) in DMF (2.7 ml) was treated with 1-bromo-2-(trifluoromethoxy)ethane (48 µl, 410 µmol) and potassium carbonate (114 mg, 827 µmol). The resulting mixture was stirred overnight at room temperature. A further portion of potassium carbonate (38.1 mg, 276 µmol) and 1-bromo-2-(trifluoromethoxy)ethane (16 µl, 140 µmol) was added and the mixture was stirred at room temperature for 4 days. The reaction was diluted with water and extracted with dichloromethane. The organic phase was concentrated and purified by preparative HPLC (RP18 column, eluent: Acetonitrile/water + 0.1% formic acid gradient) affording 31.9 mg (18 % yield) of the title compound.
LC-MS (Method 3): Rₜ = 2.47 min; MS (ESIpos): m/z = 656 [M+H]⁺
¹H-NMR (600 MHz, DMSO-d6) δ [ppm]: -0.021 (0.71), 1.235 (1.56), 1.263 (7.61), 1.275 (16.00), 1.287 (7.76), 1.511 (0.79), 1.532 (0.83), 1.710 (1.05), 1.733 (0.88), 1.891 (0.81), 1.896 (0.76), 1.911 (0.92), 1.917 (0.86), 1.982 (1.06), 2.423 (0.42), 2.557 (1.82), 2.576 (0.90), 2.600 (5.04), 2.608 (7.34), 2.616 (5.61), 2.652 (0.40), 2.691 (2.72), 2.700 (5.27), 2.710 (2.88), 3.009 (1.30), 3.027 (2.50), 3.045 (2.29), 3.068 (1.14), 3.176 (4.80), 3.185 (6.06), 3.192 (4.72), 3.245 (1.41), 3.262 (1.57), 3.309 (1.81), 4.198 (3.71), 4.207 (7.14), 4.216 (3.71), 4.242 (2.15), 4.254 (6.35), 4.266 (6.09), 4.277 (1.92), 4.482 (0.68), 4.501 (1.15), 4.519 (0.64), 6.971 (5.57), 6.985 (5.81), 7.048 (4.20), 7.051 (4.88), 7.083 (2.32), 7.086 (1.88), 7.097 (3.15), 7.100 (2.91), 7.153 (5.77), 7.166 (3.91), 7.382 (1.20), 7.436 (6.44), 7.450 (5.93), 7.469 (2.60), 7.556 (1.03), 7.995 (6.66).
LC-MS (Method 3): Rₜ = 2.46 min; MS (ESIpos): m/z = 656 [M+H]⁺

### Example 61A

### Ethyl 1-[1-(4-chloro-4'-{4-[2,2-difluorocyclopropane-1-carbonyl]piperazin-1-yl}[1,1'-biphenyl]-2-yl)piperidin-3-yl]-5-(difluoromethyl)-1H-pyrazole-4-carboxylate (Diastereomeric mixture 1)

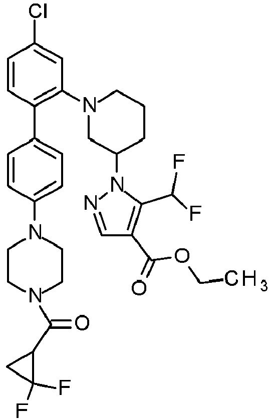

A solution of 2,2-difluorocyclopropane-1-carboxylic acid (43.5 mg, 357 µmol) in dichloromethane (1.3 ml) was treated with HATU (123 mg, 324 µmol) and N,N-diisopropylethylamine (230 µl, 1.3 mmol). Ethyl 1-{1-[4-chloro-4'-(piperazin-1-yl)[1,1'-biphenyl]-2-yl]piperidin-3-yl}-5-(difluoromethyl)-1H-pyrazole-4-carboxylate hydrochloride (prepared in analogy to Example 18A, Enantiomer 1, 200 mg, 324 µmol) was then added and the resulting mixture was stirred 1 hour at room temperature. The reaction mixture was evaporated and the residue purified by preparative HPLC (RP18 column, eluent: Acetonitrile/water gradient) affording 136 mg (64 % yield) of the title compound.
LC-MS (Method 4): Rₜ = 1.45 min; MS (ESIpos): m/z = 648 [M+H]⁺

The two diastereomers were separated by preparative chiral HPLC [sample preparation: 136 mg were dissolved in 4 ml ethanol + 2 ml n-heptane; injection volume: 360 µl; column: Daicel Chiralpak IH 5 µm, 250 x 20 mm; eluent: n-heptane/ethanol 75:25; flow rate: 18 ml/min; temperature: 35°C; UV detection: 235 nm]. After separation, 62 mg of diastereomer 1 (Example 62A), which eluted first, and 63 mg of diastereomer 2 (Example 63A), which eluted later, were isolated.

### Example 62A

### Ethyl 1-[1-(4-chloro-4'-{4-[2,2-difluorocyclopropane-1-carbonyl]piperazin-1-yl}[1,1'-biphenyl]-2-yl)piperidin-3-yl]-5-(difluoromethyl)-1H-pyrazole-4-carboxylate (Diastereomer 1)

For separation conditions see Example 61A.

Analytical chiral HPLC: Rₜ = 5.518 min, e.e. = 100% [column: Daicel Chiralpak IH 5 µm, 250 x 4.6 mm; eluent: n-heptane/ethanol 75 : 25 + 0.2% diethylamine; flow rate: 1.0 ml/min; temperature: 35°C; UV detection: 235 nm].

### Example 63A

### Ethyl 1-[1-(4-chloro-4'-{4-[2,2-difluorocyclopropane-1-carbonyl]piperazin-1-yl}[1,1'-biphenyl]-2-yl)piperidin-3-yl]-5-(difluoromethyl)-1H-pyrazole-4-carboxylate (Diastereomer 2)

For separation conditions see Example 61A.

Analytical chiral HPLC: Rₜ = 7.122 min, e.e. = 100% [column: Daicel Chiralpak IH 5 µm, 250 x 4.6 mm; eluent: n-heptane/ethanol 75 : 25 + 0.2% diethylamine; flow rate: 1.0 ml/min; temperature: 35°C; UV detection: 235 nm].

### Example 64A

### Ethyl 1-{1-[4-chloro-4'-(4-{[2,2-difluorocyclopropyl]methyl}piperazin-1-yl)[1,1'-biphenyl] -2-yl]piperidin-3-yl}-5-(difluoromethyl)-1H-pyrazole-4-carboxylate (Diastereomer 1)

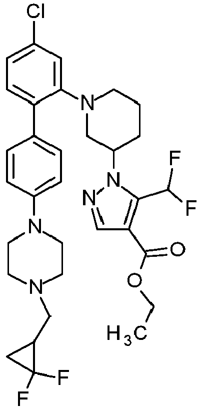

Under argon, a solution of ethyl 1-[1-(4-chloro-4'-{4-[2,2-difluorocyclopropane-1-carbonyl]piperazin-1-yl}[1,1'-biphenyl]-2-yl)piperidin-3-yl]-5-(difluoromethyl)-1H-pyrazole-4-carboxylate (Example 62A, Diastereomer 1, 62 mg, 96.9 µmol) in THF (940 µl) was treated dropwise with a solution of BH₃·THF complex in THF (970 µl, 1.0 M, 970 µmol) and the resulting mixture was stirred 3 days at room temperature. The reaction mixture was carefully quenched with water and purified by preparative HPLC (RP18 column, eluent: Acetonitrile/water gradient) affording 19.0 mg (31 % yield) of the title compound.
LC-MS (Method 4): Rₜ = 1.11 min; MS (ESIpos): m/z = 634 [M+H]⁺

### Example 65A

### Ethyl 1-{1-[4-chloro-4'-(4-{[2,2-difluorocyclopropyl]methyl}piperazin-1-yl)[1,1'-biphenyl]-2-yl]piperidin-3-yl}-5-(difluoromethyl)-1H-pyrazole-4-carboxylate (Diastereomer 2)

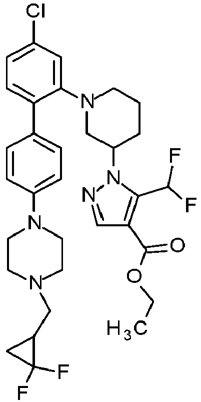

Under argon, a solution of ethyl 1-[1-(4-chloro-4'-{4-[2,2-difluorocyclopropane-1-carbonyl]piperazin-1-yl}[1,1'-biphenyl]-2-yl)piperidin-3-yl]-5-(difluoromethyl)-1H-pyrazole-4-carboxylate (Example 63A, Diastereomer 2, 63 mg, 97.7 µmol) in THF (950 µl) was treated dropwise with a solution of BH₃·THF complex in THF (980 µl, 1.0 M, 980 µmol) and the resulting mixture was stirred 3 days at room temperature. The reaction mixture was carefully quenched with water and purified by preparative HPLC (RP18 column, eluent: Acetonitrile/water gradient) affording 21.0 mg (34% yield) of the title compound.
LC-MS (Method 4): Rₜ = 1.11 min; MS (ESIpos): m/z = 634 [M+H]⁺

### Example 66A

### Ethyl 1-[1-(4-chloro-4'-{4-[2,2-difluorocyclopropane-1-carbonyl]piperazin-1-yl}[1,1'-biphenyl]-2-yl)piperidin-3-yl]-5-(difluoromethyl)-1H-pyrazole-4-carboxylate (Diastereomeric mixture 2)

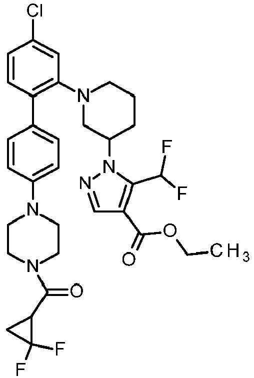

A solution of 2,2-difluorocyclopropane-1-carboxylic acid (54.4 mg, 446 µmol) in dichloromethane (1.6 ml) was treated with HATU (154 mg, 405 µmol) and N,N-diisopropylethylamine (280 µl, 1.6 mmol). Ethyl 1-{1-[4-chloro-4'-(piperazin-1-yl)[1,1'-biphenyl]-2-yl]piperidin-3-yl}-5-(difluoromethyl)-1H-pyrazole-4-carboxylate hydrochloride (prepared in analogy to Example 18A, Enantiomer 2, 250 mg, 405 µmol) was then added and the resulting mixture was stirred 1 hour at room temperature. The reaction mixture was evaporated and the residue purified by preparative HPLC (RP18 column, eluent: Acetonitrile/water gradient) affording 191 mg (73 % yield) of the title compound.
LC-MS (Method 3): Rₜ = 2.79 min; MS (ESIpos): m/z = 648 [M+H]⁺

Another batch of Example 66A (diastereomeric mixture 2) was prepared and the two diastereomers were separated by preparative chiral HPLC [sample preparation: 108 mg were dissolved in 3 ml ethanol + 3.5 ml n-heptane; injection volume: 440 µl; column: Daicel Chiralpak IG 5 µm, 250 x 20 mm; eluent: n-heptane/ethanol 80:20; flow rate: 17 ml/min; temperature: 40°C; UV detection: 220 nm]. After separation, 52 mg of diastereomer 3 (Example 67A), which eluted first, and 53 mg of diastereomer 4 (Example 68A), which eluted later, were isolated.

### Example 67A

### Ethyl 1-[1-(4-chloro-4'-{4-[2,2-difluorocyclopropane-1-carbonyl]piperazin-1-yl}[1,1'-biphenyl]-2-yl)piperidin-3-yl]-5-(difluoromethyl)-1H-pyrazole-4-carboxylate (Diastereomer 3)

For separation conditions see Example 66A.

Analytical chiral HPLC: Rₜ = 10.834 min, e.e. = 100% [column: Daicel Chiralpak IG 5 µm, 250 x 4.6 mm; eluent: n-heptane/ethanol 80 : 20 + 0.2% diethylamine; flow rate: 1.0 ml/min; temperature: 40°C; UV detection: 235 nm].

### Example 68A

### Ethyl 1-[1-(4-chloro-4'-{4-[2,2-difluorocyclopropane-1-carbonyl]piperazin-1-yl}[1,1'-biphenyl]-2-yl)piperidin-3-yl]-5-(difluoromethyl)-1H-pyrazole-4-carboxylate (Diastereomer 4)

For separation conditions see Example 66A.

Analytical chiral HPLC: Rₜ = 12.298 min, e.e. = 100% [column: Daicel Chiralpak IG 5 µm, 250 x 4.6 mm; eluent: n-heptane/ethanol 80 : 20 + 0.2% diethylamine; flow rate: 1.0 ml/min; temperature: 40°C; UV detection: 235 nm].

### Example 69A

### Ethyl 1-{1- [4-chloro-4'-(4-{[2,2-difluorocyclopropyl]methyl}piperazin-1-yl)[1,1'-biphenyl] -2-yl]piperidin-3-yl}-5-(difluoromethyl)-1H-pyrazole-4-carboxylate (Diastereomeric mixture 2)

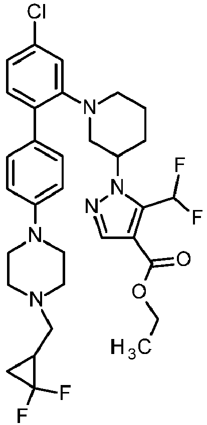

Under argon, a solution of ethyl 1-[1-(4-chloro-4'-{4-[2,2-difluorocyclopropane-1-carbonyl]piperazin-1-yl}[1,1'-biphenyl]-2-yl)piperidin-3-yl]-5-(difluoromethyl)-1H-pyrazole-4-carboxylate (Example 66A, diatereomeric mixture 2, 191 mg, 295 µmol) in THF (17 ml) was treated dropwise with a solution of BH₃·THF complex (2.9 ml, 1.0 M, 2.9 mmol) and the resulting mixture was stirred overnight at room temperature. The reaction mixture was carefully quenched with water and evaporated. The residue was retaken in water and extracted three times with ethyl acetate. The combined organic layers were dried over sodium sulfate and evaporated. The residue was purified by preparative HPLC (RP18 column, eluent: Acetonitrile/water gradient) affording 140 mg (75 % yield) of the title compound.
LC-MS (Method 3): Rₜ = 2.07 min; MS (ESIpos): m/z = 634 [M+H]⁺

### Example 70A

### Ethyl 1-{1-[4-chloro-4'-(4-{[2,2-difluorocyclopropyl]methyl}piperazin-1-yl)[1,1'-biphenyl]-2-yl]piperidin-3-yl}-5-(difluoromethyl)-1H-pyrazole-4-carboxylate (Diasteromer 3)

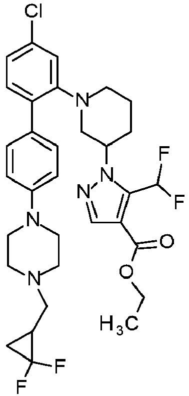

Under argon, a solution of ethyl 1-[1-(4-chloro-4'-{4-[2,2-difluorocyclopropane-1-carbonyl]piperazin-1-yl}[1,1'-biphenyl]-2-yl)piperidin-3-yl]-5-(difluoromethyl)-1H-pyrazole-4-carboxylate (prepared in analogy to Example 67A, Diastereomer 3, 85.3 mg, 132 µmol) in THF (7.4 ml) was treated dropwise with a solution of BH₃·THF complex in THF (1.3 ml, 1.0 M, 1.3 mmol) and the resulting mixture was stirred overnight at room temperature. A second portion of the solution of BH₃·THF complex in THF (1.3 ml, 1.0 M, 1.3 mmol) was added and the resulting mixture was stirred overnight at room temperature. The reaction mixture was carefully quenched with water and purified by preparative HPLC (RP18 column, eluent: Acetonitrile/water gradient) affording 35.0 mg (42 % yield) of the title compound.
LC-MS (Method 4): Rₜ = 1.07 min; MS (ESIpos): m/z = 634 [M+H]⁺

### Example 71A

### Ethyl 1-{1-[4-chloro-4'-(4-{[2,2-difluorocyclopropyl]methyl}piperazin-1-yl)[1,1'-biphenyl]-2-yl]piperidin-3-yl}-5-(difluoromethyl)-1H-pyrazole-4-carboxylate (Diastereomer 4)

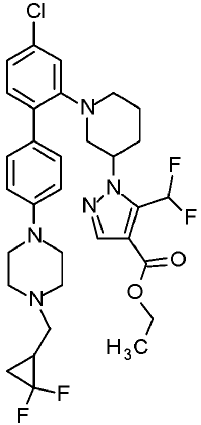

Under argon, a solution of ethyl 1-1-(4-chloro-4'-{4-[2,2-difluorocyclopropane-1-carbonyl]piperazin-1-yl}[1,1'-biphenyl]-2-yl)piperidin-3-yl]-5-(difluoromethyl)-1H-pyrazole-4-carboxylate (prepared in analogy to Example 68A, Diastereomer 4, 88.1 mg, 136 µmol) in THF (7.7 ml) was treated dropwise with a solution of BH₃·THF complex in THF (1.4 ml, 1.0 M, 1.4 mmol) and the resulting mixture was stirred overnight at room temperature. A second portion of the solution of BH₃·THF complex in THF (1.4 ml, 1.0 M, 1.4 mmol) was added and the resulting mixture was stirred overnight at room temperature. The reaction mixture was carefully quenched with water and purified by preparative HPLC (RP18 column, eluent: Acetonitrile/water gradient) affording 58.8 mg (68 % yield) of the title compound.
LC-MS (Method 3): Rₜ = 2.07 min; MS (ESIpos): m/z = 634 [M+H]⁺

### Example 72A

### Ethyl 1-[1-(4-chloro-4'-{4-[trans-2-fluorocyclopropane-1-carbonyl]piperazin-1-yl}[1,1'-biphenyl]-2-yl)piperidin-3-yl]-5-(difluoromethyl)-1H-pyrazole-4-carboxylate (Diastereomeric mixture 2)

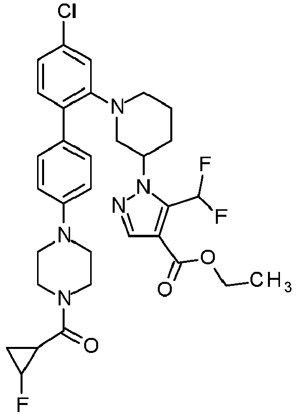

A solution of trans-2-fluorocyclopropane-1-carboxylic acid (25.3 mg, 243 µmol) in DMF (2.3 ml) was treated with HATU (126 mg, 331 µmol) and N,N-diisopropylethylamine (120 µl, 660 µmol). After stirring 10 minutes at room temperature, ethyl 1-{1-[4-chloro-4'-(piperazin-1-yl)[1,1'-biphenyl]-2-yl]piperidin-3-yl}-5-(difluoromethyl)-1H-pyrazole-4-carboxylate (prepared in analogy to Example 18A, Enantiomer 2, 120 mg, 221 µmol) was added and the resulting mixture was stirred overnight at room temperature. Purification by preparative HPLC (RP18 column, eluent: Acetonitrile/water + 0.1% formic acid gradient) afforded 96.0 mg (69 % yield) of the title compound.
LC-MS (Method 3): Rₜ = 2.77 min; MS (ESIpos): m/z = 630 [M+H]⁺
¹H-NMR (600 MHz, DMSO-d6) δ [ppm]: 1.153 (0.62), 1.164 (1.73), 1.174 (2.23), 1.185 (2.28), 1.195 (1.65), 1.206 (0.61), 1.235 (0.40), 1.263 (7.77), 1.274 (16.00), 1.286 (7.88), 1.382 (0.61), 1.387 (0.60), 1.392 (0.73), 1.399 (0.84), 1.405 (0.71), 1.410 (0.68), 1.415 (0.69), 1.419 (0.73), 1.424 (0.62), 1.429 (0.72), 1.436 (0.85), 1.441 (0.72), 1.447 (0.64), 1.452 (0.55), 1.511 (0.88), 1.532 (0.97), 1.553 (0.44), 1.711 (1.17), 1.734 (0.98), 1.895 (0.96), 1.901 (0.85), 1.915 (0.96), 1.922 (0.93), 1.936 (0.49), 1.982 (1.17), 1.999 (0.79), 2.559 (1.72), 2.563 (1.72), 2.579 (0.84), 2.624 (0.67), 2.638 (0.68), 2.642 (0.75), 2.655 (1.24), 2.666 (0.72), 2.673 (0.74), 2.684 (0.67), 3.014 (1.40), 3.032 (2.80), 3.049 (2.64), 3.065 (1.19), 3.171 (1.28), 3.245 (2.32), 3.264 (2.54), 3.312 (0.92), 3.614 (2.10), 3.847 (2.47), 4.241 (2.34), 4.253 (7.07), 4.265 (6.83), 4.276 (2.15), 4.484 (0.72), 4.503 (1.26), 4.521 (0.69), 4.759 (0.75), 4.764 (0.90), 4.768 (0.95), 4.772 (0.86), 4.777 (0.73), 4.878 (0.92), 4.881 (0.89), 5.744 (0.77), 7.012 (6.39), 7.027 (6.73), 7.057 (4.80), 7.061 (5.61), 7.091 (2.70), 7.094 (2.18), 7.104 (3.67), 7.108 (3.33), 7.162 (6.64), 7.175 (4.54), 7.390 (1.15), 7.463 (7.58), 7.477 (9.06), 7.564 (1.01), 7.996 (7.65).

The two diastereoisomers were separated by preparative chiral HPLC [sample preparation: 96 mg; injection volume: 40 µl; column: Daicel Chiralcel OX-H 5 µm, 250 x 20 mm; eluent: n-heptane/ethanol 92.5:7.5; flow rate: 20 ml/min; temperature: 30°C; UV detection: 220 nm]. After separation, 37 mg of diastereomer 3 (Example 73A), which eluted first, and 38 mg of diastereomer 4 (Example 74A), which eluted later, were isolated.

### Example 73A

### Ethyl 1-[1-(4-chloro-4'-{4-[trans-2-fluorocyclopropane-1-carbonyl]piperazin-1-yl}[1,1'-biphenyl]-2-yl)piperidin-3-yl]-5-(difluoromethyl)-1H-pyrazole-4-carboxylate (Diastereomer 3)

For separation conditions see Example 72A.

Analytical chiral HPLC: Rₜ = 3.505 min, e.e. = 100% [column: Daicel Chiralpak OX 3 µm, 50 x 4.6 mm; eluent: n-heptane/ethanol 90:10 + 0.2% diethylamine; flow rate: 1.0 ml/min; temperature: 23°C; UV detection: 220 nm].
LC-MS (Method 3): Rₜ = 2.77 min; MS (ESIpos): m/z = 630 [M+H]⁺
¹H-NMR (600 MHz, DMSO-d6) δ [ppm]: 1.128 (1.25), 1.141 (2.63), 1.153 (1.70), 1.162 (1.51), 1.173 (1.93), 1.184 (1.96), 1.194 (1.49), 1.204 (0.54), 1.235 (0.45), 1.262 (7.66), 1.273 (16.00), 1.285 (7.73), 1.381 (0.54), 1.387 (0.47), 1.392 (0.65), 1.399 (0.77), 1.404 (0.61), 1.409 (0.59), 1.415 (0.59), 1.418 (0.62), 1.423 (0.52), 1.428 (0.63), 1.436 (0.79), 1.441 (0.61), 1.446 (0.55), 1.451 (0.53), 1.510 (0.77), 1.531 (0.81), 1.710 (1.02), 1.733 (0.83), 1.894 (0.78), 1.902 (0.74), 1.915 (0.85), 1.936 (0.43), 1.981 (1.03), 2.559 (1.51), 2.580 (0.79), 2.624 (0.60), 2.634 (0.68), 2.641 (0.68), 2.655 (1.09), 2.665 (0.65), 2.672 (0.65), 2.683 (0.56), 2.875 (0.79), 2.887 (0.80), 3.013 (1.22), 3.031 (2.42), 3.049 (2.28), 3.064 (1.04), 3.167 (1.86), 3.249 (2.41), 3.314 (0.87), 3.318 (0.89), 3.613 (2.19), 3.847 (2.41), 4.240 (2.07), 4.252 (6.22), 4.263 (5.92), 4.275 (1.83), 4.484 (0.67), 4.502 (1.11), 4.520 (0.60), 4.764 (0.80), 4.767 (0.83), 4.772 (0.75), 4.877 (0.79), 4.880 (0.79), 7.011 (5.48), 7.026 (5.65), 7.057 (4.17), 7.060 (4.70), 7.090 (2.36), 7.094 (1.90), 7.104 (3.13), 7.107 (2.82), 7.162 (5.68), 7.175 (3.85), 7.391 (1.14), 7.462 (6.47), 7.477 (8.05), 7.564 (0.96), 7.996 (6.51).

### Example 74A

### Ethyl 1-[1-(4-chloro-4'-{4-[trans-2-fluorocyclopropane-1-carbonyl]piperazin-1-yl}[1,1'-biphenyl]-2-yl)piperidin-3-yl]-5-(difluoromethyl)-1H-pyrazole-4-carboxylate (Diastereomer 4)

For separation conditions see Example 72A.

Analytical chiral HPLC: Rₜ = 3.935 min, e.e. = 99.4% [column: Daicel Chiralpak OX 3 µm, 50 x 4.6 mm; eluent: n-heptane/ethanol 90:10 + 0.2% diethylamine; flow rate: 1.0 ml/min; temperature: 23°C; UV detection: 220 nm].
LC-MS (Method 3): Rₜ = 2.77 min; MS (ESIpos): m/z = 630 [M+H]⁺
¹H-NMR(600 MHz, DMSO-d6) δ [ppm]: 1.129 (1.63), 1.141 (3.35), 1.153 (2.12), 1.163 (1.46), 1.174 (1.89), 1.185 (1.91), 1.195 (1.43), 1.206 (0.54), 1.262 (7.70), 1.274 (16.00), 1.286 (7.61), 1.382 (0.52), 1.387 (0.47), 1.392 (0.61), 1.399 (0.68), 1.405 (0.57), 1.409 (0.56), 1.415 (0.56), 1.418 (0.58), 1.424 (0.49), 1.429 (0.60), 1.436 (0.72), 1.442 (0.57), 1.447 (0.55), 1.452 (0.48), 1.510 (0.73), 1.531 (0.76), 1.711 (0.92), 1.733 (0.74), 1.894 (0.75), 1.901 (0.68), 1.915 (0.76), 1.921 (0.72), 1.983 (0.93), 1.998 (0.60), 2.559 (1.32), 2.563 (1.29), 2.579 (0.71), 2.624 (0.58), 2.627 (0.55), 2.635 (0.60), 2.638 (0.56), 2.642 (0.64), 2.645 (0.58), 2.655 (1.02), 2.666 (0.63), 2.669 (0.58), 2.673 (0.63), 2.676 (0.56), 2.684 (0.55), 2.863 (0.41), 2.875 (1.20), 2.887 (1.19), 2.899 (0.41), 3.013 (1.20), 3.031 (2.33), 3.048 (2.18), 3.065 (0.91), 3.149 (0.79), 3.185 (0.83), 3.247 (1.99), 3.265 (2.36), 3.615 (1.39), 3.847 (1.77), 4.241 (2.03), 4.252 (6.08), 4.264 (5.82), 4.276 (1.79), 4.483 (0.64), 4.494 (0.64), 4.501 (1.04), 4.508 (0.66), 4.519 (0.59), 4.759 (0.63), 4.761 (0.71), 4.764 (0.75), 4.767 (0.75), 4.772 (0.72), 4.777 (0.56), 4.867 (0.61), 4.870 (0.70), 4.872 (0.70), 4.875 (0.74), 4.880 (0.72), 4.882 (0.64), 4.885 (0.59), 7.011 (5.34), 7.026 (5.48), 7.057 (4.07), 7.060 (4.66), 7.090 (2.38), 7.094 (1.80), 7.104 (3.08), 7.107 (2.76), 7.162 (5.68), 7.175 (3.81), 7.389 (1.14), 7.462 (6.38), 7.477 (7.35), 7.563 (0.96), 7.995 (6.41).

### Example 75A

### Ethyl 1-{1-[4-chloro-4'-(4-{ [trans-2-fluorocyclopropyl]methyl[1,1'-biphenyl]-2-yl]piperidin-3-yl}-5-(difluoromethyl)-1H-pyrazole-4-carboxylate (Diastereomer 3)

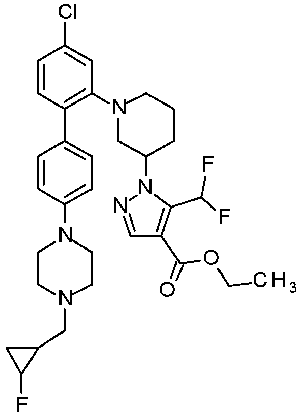

A solution of ethyl 1-[1-(4-chloro-4'-{4-[trans-2-fluorocyclopropane-1-carbonyl]piperazin-1-yl}[1,1'-biphenyl]-2-yl)piperidin-3-yl]-5-(difluoromethyl)-1H-pyrazole-4-carboxylate (Example 73A, Diastereomer 3, 37.0 mg, 58.7 µmol) in THF (1.4 ml) was treated with a solution of BH₃·THF complex in THF (1.2 ml, 1.0 M, 1.2 mmol) and stirred overnight at 35°C. The reaction mixture was quenched with methanol, diluted with etyl acetate and purified by preparative HPLC (RP18 column, eluent: Acetonitrile/water + 0.1% formic acid gradient) affording 16.0 mg (44 % yield) of the title compound.
LC-MS (Method 3): Rₜ = 1.96 min; MS (ESIpos): m/z = 616 [M+H]⁺

### Example 76A

### Ethyl 1-{(1-[4-chloro-4'-(4-{[trans-2-fluorocyclopropyl]methyl)[1,1'-biphenyl]-2-yl]piperidin-3-yl}-5-(difluoromethyl)-1H-pyrazole-4-carboxylate (Diasteromer 4)

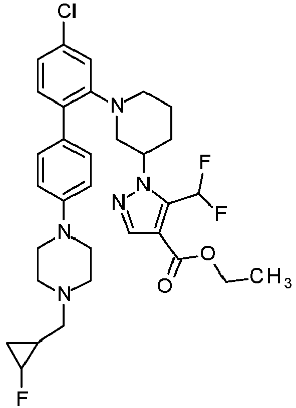

A solution of ethyl 1-[1-(4-chloro-4'-{4-[trans-2-fluorocyclopropane-1-carbonyl]piperazin-1-yl}[1,1'-biphenyl]-2-yl)piperidin-3-yl]-5-(difluoromethyl)-1H-pyrazole-4-carboxylate (Example 74A, Diastereomer 4, 38.0 mg, 60.3 µmol) in THF (1.4 ml) was treated with a solution of BH₃·THF complex in THF (1.2 ml, 1.0 M, 1.2 mmol) and stirred overnight at 35°C. The reaction mixture was quenched with methanol, diluted with etyl acetate and purified by preparative HPLC (RP18 column, eluent: Acetonitrile/water + 0.1% formic acid gradient) affording 22.0 mg (59 % yield) of the title compound.
LC-MS (Method 3): Rₜ = 1.98 min; MS (ESIpos): m/z = 616 [M+H]⁺

### Example 77A

### [(1S,2S)-2-Fluorocyclopropyl]{4-[4-(4,4,5,5-tetramethyl-1,3,2-dioxaborolan-2-yl)phenyl]piperazin-1-yl}methanone

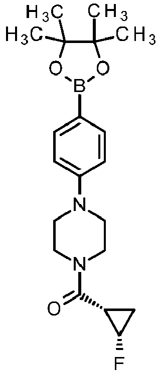

A solution of (1S,2S)-2-fluorocyclopropane-1-carboxylic acid (278 mg, 2.67 mmol) in DMF (10 ml) was treated with HATU (924 mg, 2.43 mmol), N,N-diisopropylethylamine (850 µl, 4.9 mmol) and stirred 10 minutes at room temperature. 1-[4-(4,4,5,5-tetramethyl-1,3,2-dioxaborolan-2-yl)phenyl]piperazine (700 mg, 2.43 mmol) was then added, the resulting mixture was stirred overnight at room temperature. The reaction mixture was diluted with water, extracted with ethyl acetate and the oganic phase was washed with water and evaporated. The residue was purified by preparative HPLC (RP18 column, eluent: Acetonitrile/water + 0.1% formic acid gradient) affording 759 mg (79 % yield) of the title compound.
LC-MS (Method 3): Rₜ = 1.92 min; MS (ESIpos): m/z = 375 [M+H]⁺
¹H-NMR (400 MHz, DMSO-d6) δ [ppm]: 1.263 (16.00), 6.922 (1.07), 6.944 (1.07), 7.518 (1.24), 7.540 (1.07).

### Example 78A

### 1-{[(1S,2S)-2-fluorocyclopropyl]methyl}-4-[4-(4,4,5,5-tetramethyl-1,3,2-dioxaborolan-2-yl)phenyl]piperazine

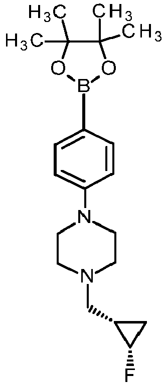

Under argon, a solution of [(1S,2S)-2-fluorocyclopropyl]{4-[4-(4,4,5,5-tetramethyl-1,3,2-dioxaborolan-2-yl)phenyl]piperazin-1-yl}methanone (Example 77A, 759 mg, 2.03 mmol) in THF (18 ml) was treated dropwise with a solution of BH₃·THF complex (41 ml, 1 N, 41 mmol) and the resulting mixture was stirred overnight at room temperature. The reaction mixture was carefully quenched with methanol and evaporated. The residue was dissolved ethyl acetate and purified by flash chromatograpthy (silica gel dichloromethane/methanol gradient) affording 250 mg (31 % yield) of the title compound, which was used without further purification
LC-MS (Method 3): Rₜ = 1.23 min; MS (ESIpos): m/z = 361 [M+H]⁺
¹H-NMR (600 MHz, DMSO-d6) δ [ppm]: 0.853 (0.99), 0.865 (2.16), 0.877 (1.05), 1.159 (2.02), 1.260 (16.00), 1.264 (10.64), 1.290 (0.41), 3.041 (0.44), 3.220 (0.52), 3.378 (0.65), 3.387 (0.66), 4.281 (0.72), 6.481 (0.97), 6.892 (0.54), 6.907 (0.57), 6.918 (0.64), 6.932 (0.63), 7.498 (0.74), 7.512 (0.70), 7.526 (0.69), 7.540 (0.64).

### Example 79A

### Ethyl 1-{1-[4-chloro-4'-(4-{[(1S,2S)-2-fluorocyclopropyl]methyl}piperazin-1-yl)[1,1'-biphenyl]-2-yl]piperidin-3-yl}-5-(difluoromethyl)-1H-pyrazole-4-carboxylate (Stereisomer 2)

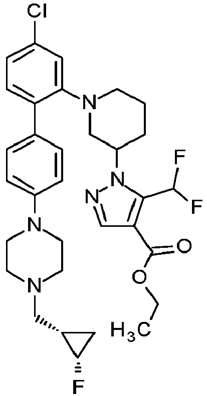

Under argon, a solution of ethyl 1-[1-{5-chloro-2-[(trifluoromethanesulfonyl)oxy]phenyl}piperidin-3-yl]-5-(difluoromethyl)-1H-pyrazole-4-carboxylate (prepared in analogy to Example 16A, Stereoisomer 2, 120 mg, 226 µmol) and 1-{[(1S,2S)-2-fluorocyclopropyl]methyl}-4-[4-(4,4,5,5-tetramethyl-1,3,2-dioxaborolan-2-yl)phenyl]piperazine (Example 78A, 97.5 mg, 271 µmol) in toluene/ethanol (1:1, 3.3 ml) was treated with Pd(PPh₃)₄ (13.0 mg, 11.3 µmol) and an aqueous solution of sodium carbonate (340 µl, 2 N, 680 µmol) and the reaction was stirred at 100°C overnight. The reaction mixture was concentrated and the residue was dissolved in dichloromethane and purified by flash chromatography (silica gel, dichloromethane/methanol, gradient) affording 78.0 mg (91 % purity, 51 % yield) of the title compound, which was used without further purification.
LC-MS (Method 3): Rₜ = 1.90 min; MS (ESIpos): m/z = 616 [M+H]⁺
¹H-NMR(600 MHz, DMSO-d6) δ [ppm]: 0.539 (0.54), 0.543 (0.53), 0.550 (1.02), 0.554 (0.99), 0.562 (0.61), 0.567 (0.59), 0.577 (0.54), 0.581 (0.54), 0.588 (1.04), 0.593 (0.98), 0.600 (0.60), 0.605 (0.56), 0.810 (0.52), 0.821 (0.96), 0.830 (0.88), 0.838 (1.40), 0.847 (0.99), 0.857 (1.10), 0.867 (0.58), 1.009 (0.46), 1.022 (0.92), 1.032 (1.02), 1.048 (0.78), 1.263 (7.72), 1.275 (16.00), 1.286 (7.87), 1.515 (0.86), 1.536 (0.92), 1.558 (0.41), 1.713 (1.18), 1.735 (0.92), 1.888 (0.91), 1.894 (0.84), 1.908 (0.95), 1.915 (0.93), 1.929 (0.45), 1.984 (1.17), 1.999 (0.77), 2.378 (1.49), 2.391 (1.46), 2.399 (1.64), 2.412 (1.60), 2.564 (2.03), 2.574 (3.14), 2.583 (3.18), 2.591 (1.58), 2.624 (1.51), 2.632 (3.16), 2.640 (3.90), 2.651 (3.32), 2.661 (1.87), 2.670 (1.32), 3.007 (1.38), 3.024 (2.76), 3.042 (1.75), 3.053 (1.26), 3.073 (1.19), 3.189 (5.38), 3.198 (9.13), 3.206 (5.27), 3.249 (2.17), 4.241 (2.12), 4.253 (6.37), 4.265 (6.18), 4.276 (1.93), 4.483 (0.75), 4.501 (1.26), 4.520 (0.72), 4.724 (0.64), 4.728 (0.68), 4.734 (1.19), 4.738 (1.20), 4.744 (0.65), 4.748 (0.60), 4.834 (0.58), 4.839 (0.62), 4.844 (1.18), 4.848 (1.16), 4.854 (0.67), 4.858 (0.58), 6.973 (5.96), 6.988 (6.14), 7.048 (4.50), 7.051 (5.15), 7.083 (2.36), 7.087 (1.90), 7.097 (3.29), 7.101 (2.94), 7.152 (5.84), 7.166 (3.97), 7.385 (1.33), 7.438 (6.88), 7.452 (6.26), 7.472 (2.72), 7.540 (0.42), 7.549 (1.11), 7.560 (1.63), 7.566 (0.92), 7.604 (1.13), 7.616 (1.14), 7.624 (1.51), 7.637 (0.97), 7.995 (7.28), 8.142 (1.00).

### Example 80A

### [Trans-2-Fluorocyclopropyl]{4-[4-(4,4,5,5-tetramethyl-1,3,2-dioxaborolan-2-yl)phenyl]piperazin-1-yl}methanone (Racemate)

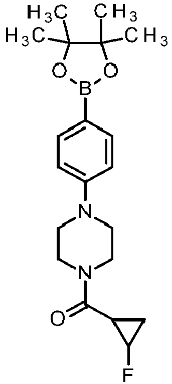

A solution of *trans*-2-fluorocyclopropane-1-carboxylic acid (278 mg, 2.67 mmol) in N,N-dimethylformamide (10 ml) was treated with HATU (924 mg, 2.43 mmol) and N,N-diisopropylethylamine (850 µl, 4.9 mmol) and stirred for 10 minutes at room temperature. 1-[4-(4,4,5,5-tetramethyl-1,3,2-dioxaborolan-2-yl)phenyl]piperazine (700 mg, 2.43 mmol) was added and the reaction was stirred overnight at room temperature then water was added and the mixture was extracted with ethyl acetate. The organic phase was washed with water, concentrated and the residue was purified with preparative HPLC (RP18 column, eluent: Acetonitrile/water + 0.1% formic acid gradient) affording 696 mg (74 % yield) the title compound.
LC-MS (Method 3): Rₜ = 2.04 min; MS (ESIpos): m/z = 375 [M+H]⁺
¹H-NMR (400 MHz, DMSO-d6) δ [ppm]: 1.264 (16.00), 3.210 (0.41), 3.583 (0.56), 3.821 (0.61), 6.919 (1.02), 6.941 (1.11), 7.518 (1.18), 7.539 (1.10).

### Example 81A

### 1-{[Trans-2-fluorocyclopropyl]methyl}-4-[4-(4,4,5,5-tetramethyl-1,3,2-dioxaborolan-2-yl)phenyl]piperazine (Racemate)

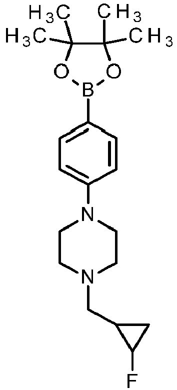

Under argon, a solution of [2-*trans*-fluorocyclopropyl]{4-[4-(4,4,5,5-tetramethyl-1,3,2-dioxaborolan-2-yl)phenyl]piperazin-1-yl}methanone (Example 80A, racemate, 696 mg, 1.86 mmol) in THF (17 ml) was treated dropwise with a solution of BH₃·THF complex (37 ml, 1 M, 37 mmol) and the resulting mixture was stirred for 30 minutes at room temperature. The reaction was carefully quenched with methanol and concentrated. The residue was dissolved in ethyl acetate and purified with flash chromatography (silica gel, dichloromethane/methanol gradient) affording 422 mg (57 % yield) of the title compound
LC-MS (Method 3): Rₜ = 2.29 min; MS (ESIpos): m/z = 361 [M+H]⁺
¹H-NMR (600 MHz, DMSO-d6) δ [ppm]: 1.260 (3.81), 1.264 (16.00), 3.024 (0.51), 3.034 (0.82), 3.042 (0.55), 3.448 (0.58), 3.455 (0.40), 3.461 (0.51), 6.919 (1.01), 6.933 (1.04), 7.526 (1.15), 7.541 (1.04).

### Example 82A

### Ethyl 1-{1-[4-chloro-4'-(4-{[trans-2-fluorocyclopropyl]methyl}piperazin-1-yl)[1,1'-biphenyl]-2-yl]piperidin-3-yl}-5-(difluoromethyl)-1H-pyrazole-4-carboxylate (Diastereomeric mixture 2)

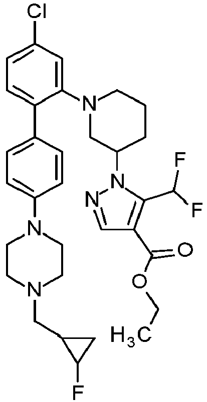

Under argon, a solution of ethyl 1-[1-{5-chloro-2-[(trifluoromethanesulfonyl)oxy]phenyl}piperidin-3-yl]-5-(trifluoromethyl)-1H-pyrazole-4-carboxylate (prepared in analogy to Example 16A, Enantiomer 2, 120 mg, 226 µmol) and 1-{[trans-2-fluorocyclopropyl]methyl}-4-[4-(4,4,5,5-tetramethyl-1,3,2-dioxaborolan-2-yl)phenyl]piperazine (Example 81A, racemate, 97.5 mg, 271 µmol) in toluene/ethanol (1:1, 3.3 ml) was treated with Pd(PPh₃)₄ (13.0 mg, 11.3 µmol) and an aqueous solution of sodium carbonate (340 µl, 2 M, 680 µmol) and the reaction was stirred at 100°C fro 3 hours. The reaction mixture was concentrated and the residue was purified by flash chromatography (silica gel, dichloromethane/methanol, gradient) followed by preparative HPLC (RP18 column, eluent: Acetonitrile/water + 0.1% formic acid gradient) affording 41.0 mg (27 % yield) of the title compound
LC-MS (Method 3): Rₜ = 1.97 min; MS (ESIpos): m/z = 616 [M+H]⁺
¹H-NMR (400 MHz, DMSO-d6) δ [ppm]: -0.149 (1.55), 0.146 (1.56), 0.499 (0.43), 0.515 (1.24), 0.531 (1.32), 0.541 (1.33), 0.557 (1.24), 0.574 (0.47), 1.000 (0.52), 1.005 (0.48), 1.016 (0.55), 1.027 (0.70), 1.033 (0.64), 1.044 (0.61), 1.050 (0.65), 1.055 (0.68), 1.061 (0.53), 1.072 (0.56), 1.083 (0.72), 1.089 (0.62), 1.099 (0.60), 1.105 (0.56), 1.235 (0.47), 1.257 (7.43), 1.275 (16.00), 1.293 (7.59), 1.339 (0.43), 1.369 (0.53), 1.386 (0.65), 1.404 (0.57), 1.433 (0.42), 1.504 (0.64), 1.537 (0.74), 1.706 (1.00), 1.738 (0.77), 1.879 (0.72), 1.909 (0.77), 1.977 (1.03), 2.114 (0.83), 2.133 (0.84), 2.141 (1.18), 2.160 (1.11), 2.269 (1.06), 2.285 (0.86), 2.300 (0.75), 2.323 (0.70), 2.327 (0.89), 2.366 (1.53), 2.524 (4.07), 2.570 (4.57), 2.586 (4.85), 2.598 (2.54), 2.614 (0.97), 2.665 (0.69), 2.669 (0.89), 2.674 (0.69), 2.710 (1.53), 2.999 (1.03), 3.025 (2.21), 3.051 (2.20), 3.075 (0.98), 3.180 (4.13), 3.192 (7.38), 3.204 (4.11), 3.247 (1.45), 4.233 (2.08), 4.251 (6.42), 4.268 (6.17), 4.286 (1.92), 4.472 (1.44), 4.487 (1.58), 4.501 (1.06), 4.633 (0.98), 4.648 (1.01), 5.754 (3.02), 6.970 (5.05), 6.993 (5.42), 7.048 (3.87), 7.053 (4.77), 7.082 (2.09), 7.087 (1.47), 7.102 (3.29), 7.107 (2.99), 7.151 (5.99), 7.171 (3.40), 7.350 (1.34), 7.437 (6.12), 7.459 (5.46), 7.480 (2.70), 7.547 (0.95), 7.556 (0.77), 7.565 (1.08), 7.573 (0.91), 7.597 (1.36), 7.610 (1.78), 7.626 (1.56), 7.643 (0.77), 8.003 (5.40).

### Example 83A

### Ethyl 1-[1-(4-chloro-4'-{4-[(1R,2R)-2-fluorocyclopropane-1-carbonyl]piperazin-1-yl}[1,1'-biphenyl]-2-yl)piperidin-3-yl]-5-(difluoromethyl)-1H-pyrazole-4-carboxylate (Stereoisomer 2)

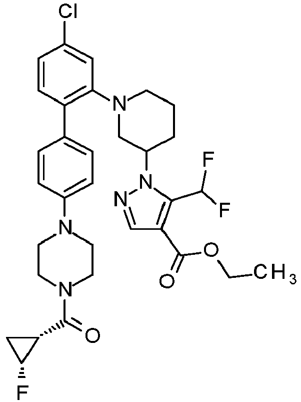

A solution of (1R,2R)-2-fluorocyclopropane-1-carboxylic acid (24.1 mg, 232 µmol) in DMF (930 µl) was treated with HATU (120 mg, 316 µmol) and N,N-diisopropylethylamine (110 µl, 630 µmol). ethyl 1-{1-[4-chloro-4'-(piperazin-1-yl)[1,1'-biphenyl]-2-yl]piperidin-3-yl}-5-(difluoromethyl)-1H-pyrazole-4-carboxylate hydrochloride (prepared in analogy to Example 18A, Enantiomer 2, 130 mg, 211 µmol) was then added and the resulting mixture was stirred 1 hour at room temperature. The reaction mixture was evaporated and the residue purified by preparative HPLC (RP18 column, eluent: Acetonitrile/water + 0.1% formic acid gradient) affording 104 mg (78 % yield) of the title compound.
LC-MS (Method 3): Rₜ = 2.71 min; MS (ESIpos): m/z = 630 [M+H]⁺
¹H-NMR (400 MHz, DMSO-d6) δ [ppm]: 0.993 (0.50), 1.008 (1.06), 1.016 (0.71), 1.024 (1.19), 1.032 (1.20), 1.039 (1.17), 1.047 (1.22), 1.055 (0.76), 1.063 (1.04), 1.078 (0.55), 1.253 (7.69), 1.271 (16.00), 1.289 (7.91), 1.503 (1.33), 1.512 (1.71), 1.521 (1.80), 1.530 (1.69), 1.539 (1.34), 1.553 (1.04), 1.561 (1.07), 1.571 (1.46), 1.579 (1.22), 1.588 (0.71), 1.596 (0.57), 1.706 (1.34), 1.738 (1.00), 1.881 (0.91), 1.911 (1.04), 1.976 (1.41), 2.086 (0.43), 2.182 (0.59), 2.199 (1.52), 2.217 (1.65), 2.222 (1.68), 2.240 (1.43), 2.256 (0.47), 2.328 (0.45), 2.366 (0.58), 2.523 (2.15), 2.587 (1.07), 2.670 (0.50), 2.710 (0.60), 2.998 (1.32), 3.024 (3.00), 3.051 (2.73), 3.069 (1.96), 3.151 (1.22), 3.168 (1.16), 3.239 (2.24), 3.257 (1.47), 3.604 (1.09), 3.622 (1.08), 3.688 (1.16), 3.819 (2.78), 4.229 (2.26), 4.247 (7.11), 4.264 (6.98), 4.282 (2.21), 4.466 (0.78), 4.493 (1.40), 4.520 (0.71), 4.840 (0.63), 4.848 (0.73), 4.856 (1.10), 4.864 (1.07), 4.871 (0.68), 4.879 (0.57), 5.007 (0.56), 5.015 (0.65), 5.022 (1.09), 5.030 (1.05), 5.038 (0.71), 5.046 (0.58), 5.754 (8.09), 7.012 (6.34), 7.034 (6.84), 7.057 (4.80), 7.062 (5.75), 7.089 (2.36), 7.094 (1.63), 7.110 (3.75), 7.114 (3.23), 7.160 (6.56), 7.181 (3.72), 7.340 (1.64), 7.460 (7.72), 7.470 (4.11), 7.481 (6.85), 7.600 (1.38), 8.002 (6.29).

### Example 84A

### Ethyl 1-{1-[4-chloro-4'-(4-{[(1R,2R)-2-fluorocyclopropyl]methyl}piperazin-1-yl)[1,1'-biphenyl]-2-yl]piperidin-3-yl}-5-(difluoromethyl)-1H-pyrazole-4-carboxylate (Stereoisomer 2)

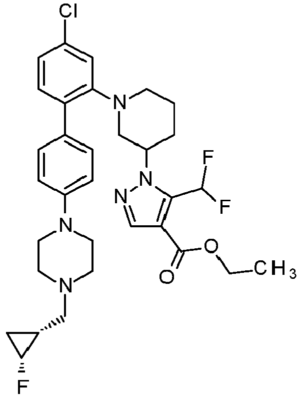

Under argon, a solution of ethyl 1-[1-(4-chloro-4'-{4-[(1R,2R)-2-fluorocyclopropane-1-carbonyl]piperazin-1-yl}[1,1'-biphenyl]-2-yl)piperidin-3-yl]-5-(difluoromethyl)-1H-pyrazole-4-carboxylate (prepared in analogy to Example 83A, Steroisomer 2, 62.0 mg, 98.4 µmol) in THF (2.1 ml) was treated with a solution of BH₃·THF complex in THF (2.0 ml, 1.0 M, 2.0 mmol). The resulting mixtre was stirred overnight at 35°C and cooled to room temperature. The reaction mixture was quenched with methanol, diluted with ethyl acetate and evaporated. The residue was purified by preparative HPLC (RP18 column, eluent: Acetonitrile/water + 0.1% formic acid gradient) affording 17.5 mg (29 % yield) of the title compound.
LC-MS (Method 3): Rₜ = 1.94 min; MS (ESIpos): m/z = 616 [M+H]⁺

### Example 85A

### [4-(4-Bromophenyl)piperazin-1-yl][cis-2-(trifluoromethyl)cyclopropyl] (Racemate)

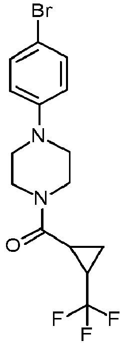

A solution of *cis*-2-(trifluoromethyl)cyclopropane-1-carboxylic acid (351 mg, 2.28 mmol) in N,N-dimethylformamide (10 ml) was treated with HATU (788 mg, 2.07 mmol), N,N-diisopropylethylamine (720 µl, 4.1 mmol) and stirred 10 minutes at room temperature. 1-(4-bromophenyl)piperazine (500 mg, 2.07 mmol) was then added, the resulting mixture was stirred for 30 minutes at room temperature. The reaction mixture was diluted with water, extracted with ethyl acetate and the oganic phase was washed with water and evaporated. The residue was purified by flash chromatography (silica gel, cyclohexane/ethyl acetate gradient) affording 462 mg (59 % yield) of the title compound.
LC-MS (Method 3): Rₜ = 2.09 min; MS (ESIpos): m/z = 377 [M+H]⁺
¹H-NMR (600 MHz, DMSO-d6) δ [ppm]: 1.195 (1.56), 1.204 (4.03), 1.213 (5.13), 1.219 (6.66), 1.228 (5.40), 1.235 (3.81), 1.243 (1.35), 1.400 (16.00), 1.987 (0.48), 2.194 (0.97), 2.205 (1.74), 2.215 (2.30), 2.226 (1.93), 2.238 (1.09), 2.558 (2.88), 2.652 (0.43), 3.117 (3.10), 3.125 (3.67), 3.131 (3.34), 3.202 (5.17), 3.210 (5.18), 3.250 (0.76), 3.262 (0.89), 3.326 (3.03), 3.607 (4.94), 3.614 (4.98), 3.814 (6.01), 6.916 (12.83), 6.931 (13.62), 7.358 (13.72), 7.373 (12.99).

### Example 86A

### 1-(4-Bromophenyl)-4-{[cis-2-(trifluoromethyl)cyclopropyl]methyl} (Racemate)

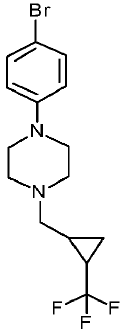

Under argon, a solution of [4-(4-bromophenyl)piperazin-1-yl]-cis-2-(trifluoromethyl)cyclopropyl]methanone (Example 85A, racemate 461 mg, 1.22 mmol) in THF (2.4 ml) was treated dropwise with a solution of BH₃·THF complex (12 ml, 1 M, 12 mmol) and the resulting mixture was stirred overnight at room temperature. A second portion of BH₃·THF complex (6.1 ml, 1 M, 6.1 mmol) was added and the reaction was stirred for a further 4 hours at room temperature. A third portion of BH₃·THF complex (6.1 ml, 1 M, 6.1 mmol) was added and the reaction was stirred for a further 24 hours at room temperature. The reaction was carefully quenched with methanol and concentrated. The residue was purified with flash chromatography (silca gel, cyclohexane/ethyl acetate gradient) then by preparative HPLC (RP18 column, eluent: Acetonitrile/water + 0.1% formic acid gradient) affording 44.9 mg (10 % yield) of the title compound.
LC-MS (Method 3): Rₜ = 1.25 min; MS (ESIpos): m/z = 363 [M+H]⁺
¹H-NMR(600 MHz, DMSO-d6) δ [ppm]: 0.718 (1.40), 0.727 (2.91), 0.741 (2.96), 0.751 (1.56), 0.941 (1.94), 0.950 (3.74), 0.957 (3.35), 0.965 (4.00), 0.973 (1.93), 1.281 (0.51), 1.291 (1.50), 1.302 (2.21), 1.309 (2.19), 1.314 (2.12), 1.324 (1.38), 1.335 (0.44), 1.721 (2.29), 2.283 (1.55), 2.295 (1.73), 2.305 (2.35), 2.315 (2.17), 2.383 (2.74), 2.393 (2.58), 2.403 (1.83), 2.414 (1.69), 3.123 (11.54), 3.131 (16.00), 3.139 (10.92), 6.880 (14.22), 6.895 (15.05), 7.314 (1.64), 7.320 (15.65), 7.335 (14.39).

### Example 87A

### 1-[4-(4,4,5,5-Tetramethyl-1,3,2-dioxaborolan-2-yl)phenyl]-4-{[cis-2-(trifluoromethyl)cyclopropyl]methyl} (Racemate)

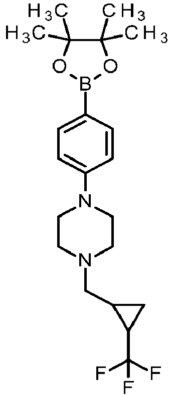

Under argon, a solution of 1-(4-bromophenyl)-4-[[cis-2-(trifluoromethyl)cyclopropyl]methyl]piperazine (prepared in analogy to Example 86A, racemate, 193 mg, 531 µmol), 4,4,4',4',5,5,5',5'-octamethyl-2,2'-bi-1,3,2-dioxaborolane (202 mg, 797 µmol), Pd₂dba₃ (14.6 mg, 15.9 µmol), X-Phos (15.2 mg, 31.9 µmol) and potassium acetate (156 mg, 1.59 mmol) in cyclopentylmethylether (5.9 ml) was stirred overnight at 105°C. The reaction mixture was filtered over Celite, rinsed with ethyl acetate and the filtrate evaporated affording 451 mg (75 % purity, quant.) of the title compound, which was used without further purification.
LC-MS (Method 3): Rₜ = 1.45 min; MS (ESIpos): m/z = 411 [M+H]⁺
¹H-NMR (600 MHz, DMSO-d6) δ [ppm]: 1.072 (0.65), 1.160 (16.00), 1.168 (9.14), 1.259 (7.01), 1.295 (2.53), 1.897 (0.44), 2.568 (0.51), 3.150 (0.78), 3.206 (0.54), 3.214 (0.71), 3.223 (0.51), 6.889 (0.46), 6.903 (0.47), 7.495 (0.51), 7.509 (0.47).

### Example 88A

### Ethyl 1-{1-[4-chloro-4'-(4-{[cis-2-(trifluoromethyl)cyclopropyl]methyl}piperazin-1-yl)[1,1'-biphenyl]-2-yl]piperidin-3-yl}-5-(difluoromethyl)-1H-pyrazole-4-carboxylate (Diastereomeric mixture 2)

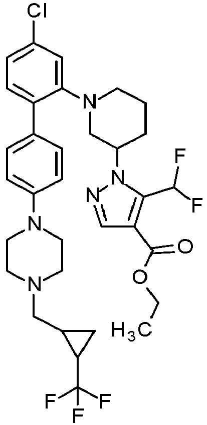

Under argon, a solution of ethyl 1-[1-{5-chloro-2-[(trifluoromethanesulfonyl)oxy]phenyl}piperidin-3-yl]-5-(difluoromethyl)-1H-pyrazole-4-carboxylate (prepared in analogy to Example 16A, Enantiomer 2, 70.0 mg, 132 µmol) and 1-[4-(4,4,5,5-tetramethyl-1,3,2-dioxaborolan-2-yl)phenyl]-4-[[cis-2-(trifluoromethyl)cyclopropyl]methyl]piperazine (Example 87A, racemate, 86.4 mg, 75 % purity, 158 µmol) in toluene/ethanol (1:1, 1.9 ml) was treated with Pd(PPh₃)₄ (7.60 mg, 6.58 µmol) and an aqueous solution of sodium carbonate (200 µl, 2 N, 390 µmol) and the reaction was stirred at 100°C overnight. The reaction mixture was concentrated and the residue was purified by preparative HPLC (RP18 column, eluent: Acetonitrile/water + 0.1% formic acid gradient) affording 39.0 mg (44% yield) of the title compound.
LC-MS (Method 3): Rₜ = 2.09 min; MS (ESIpos): m/z = 666 [M+H]⁺
¹H-NMR (600 MHz, DMSO-d6) δ [ppm]: 0.761 (0.51), 0.987 (0.63), 1.263 (7.71), 1.275 (16.00), 1.287 (7.84), 1.347 (0.47), 1.510 (0.81), 1.531 (0.86), 1.713 (1.15), 1.734 (1.17), 1.890 (0.85), 1.898 (0.78), 1.911 (0.89), 1.932 (0.42), 1.982 (1.09), 1.997 (0.73), 2.384 (0.52), 2.423 (0.71), 2.558 (1.79), 2.574 (1.62), 2.609 (1.62), 2.652 (0.74), 3.009 (1.34), 3.026 (2.57), 3.044 (2.29), 3.068 (1.12), 3.189 (2.33), 3.247 (1.53), 3.259 (1.46), 3.333 (0.83), 4.242 (2.12), 4.253 (6.34), 4.265 (6.03), 4.277 (1.84), 4.481 (0.65), 4.499 (1.12), 4.516 (0.62), 6.982 (2.31), 6.996 (2.40), 7.050 (3.60), 7.054 (3.99), 7.086 (2.02), 7.089 (1.66), 7.099 (2.79), 7.103 (2.50), 7.153 (5.69), 7.166 (3.76), 7.385 (1.24), 7.442 (3.62), 7.456 (3.37), 7.472 (2.51), 7.559 (1.02), 7.998 (6.89).

### Example 89A

### {4-[4-(4,4,5,5-Tetramethyl-1,3,2-dioxaborolan-2-yl)phenyl]piperazin-1-yl}[1-(trifluoromethyl)cyclopropyl]methanone

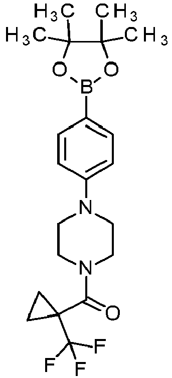

A solution of 1-(trifluoromethyl)cyclopropane-1-carboxylic acid (206 mg, 1.34 mmol) in dichloromethane (5.0 ml) was treated with HATU (462 mg, 1.21 mmol), N,N-diisopropylethylamine (420 µl, 2.4 mmol) and finally 1-[4-(4,4,5,5-tetramethyl-1,3,2-dioxaborolan-2-yl)phenyl]piperazine (350 mg, 1.21 mmol) was added. The resulting mixture was stirred overnight at room temperature then water was added and the mixture was extracted with dichloromethane. The organic phase was washed with brine, dried and concentrated. The residue was purified by flash chromatography (silica gel, cylcohexane/ethyl acetate gradient) affording 347 mg (67 % yield) of the title compound.
LC-MS (Method 3): Rₜ = 2.22 min; MS (ESIpos): m/z = 425

### Example 90A

### 1-[4-(4,4,5,5-Tetramethyl-1,3,2-dioxaborolan-2-yl)phenyl]-4-{[1-(trifluoromethyl)cyclopropyl]methyl}piperazine

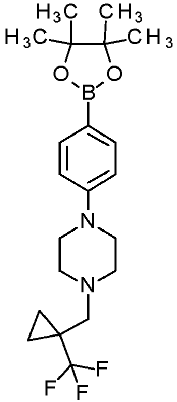

Under argon, a solution of {4-[4-(4,4,5,5-tetramethyl-1,3,2-dioxaborolan-2-yl)phenyl]piperazin-1-yl}[1-(trifluoromethyl)cyclopropyl]methanone (Example 89A, 346 mg, 816 µmol) in THF (1.4 ml) was treated dropwise with a solution of BH₃ · THF complex (8.2 ml, 1 N, 8.2 mmol) and the resulting mixture was stirred overnight at room temperature. The reaction mixture was carefully quenched with methanol and evaporated. The residue was purified by flash chromatograpthy (silica gel cyclohexane/ethyl acetate gradient) affording 265 mg (79 % yield) of the title compound.
LC-MS (Method 4): Rₜ = 0.87 min; MS (ESIpos): m/z = 411

### Example 91A

### Ethyl 1-{1-[4-chloro-4'-(4-{[1-(trifluoromethyl)cyclopropyl]methyl}piperazin-1-yl)[1,1'-biphenyl]-2-yl]piperidin-3-yl}-5-(difluoromethyl)-1H-pyrazole-4-carboxylate (Enantiomer 2)

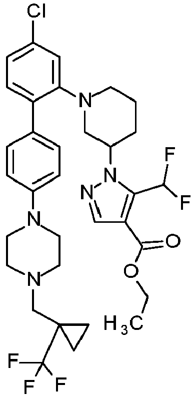

Under argon, a solution of ethyl 1-[1-{5-chloro-2-[(triffuoromethanesulfonyl)oxy]phenyl}piperidin-3-yl]-5-(difluoromethyl)-1H-pyrazole-4-carboxylate (prepared in analogy to Example 16A, Enantiomer 2, 100 mg, 188 µmol) and 1-[4-(4,4,5,5-tetramethyl-1,3,2-dioxaborolan-2-yl)phenyl]-4-{[1-(trifluoromethyl)cyclopropyl]methyl}piperazine (prepared in analogy to Example 90A, 145 mg, 86 % purity, 282 µmol) in toluene/ethanol (1:1, 2.3 ml) was treated with Pd(PPh₃)₄ (10.9 mg, 9.40 µmol) and an aqueous solution of sodium carbonate (280 µl, 2.0 M, 560 µmol) and the reaction was stirred at 100°C overnight. The reaction mixture was filtered over celite and the filtrate was concentrated. The residue was purified by preparative HPLC (RP18 column, eluent: Acetonitrile/water + 0.1% formic acid gradient) affording 10.4 mg (8 % yield) of the title compound.
LC-MS (Method 3): Rₜ = 2.96 min; MS (ESIpos): m/z = 666 [M+H]⁺

### Example 92A

### Ethyl 1-[1-{4-chloro-4'-[4-(spiro[2.2]pentane-1-carbonyl)piperazin-1-yl][1,1'-biphenyl]-2-yl}piperidin-3-yl]-5-(difluoromethyl)-1H-pyrazole-4-carboxylate (Enantiomer 2)

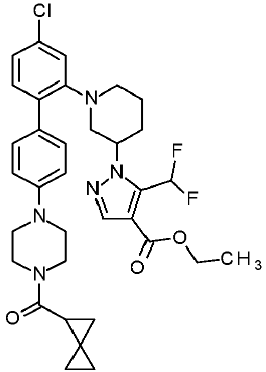

A solution of spiro[2.2]pentane-1-carboxylic acid (30.0 mg, 267 µmol) in dichloromethane (980 µl) was treated with HATU (102 mg, 267 µmol) and N,N-diisopropylethylamine (170 µl, 970 µmol). ethyl 1-{1-[4-chloro-4'-(piperazin-1-yl)[1,1'-biphenyl]-2-yl]piperidin-3-yl}-5-(difluoromethyl)-1H-pyrazole-4-carboxylate hydrochloride (prepared in analogy to Example 18A, Enantiomer 2, 150 mg, 243 µmol) was then added and the resulting mixture was stirred overnight at room temperature. The reaction mixture was evaporated and the residue purified by preparative HPLC (RP18 column, eluent: Acetonitrile/water + 0.1% formic acid gradient) affording 123 mg (80 % yield) of the title compound.
LC-MS (Method 3): Rₜ = 2.86 min; MS (ESIpos): m/z = 638 [M+H]⁺
¹H-NMR(600 MHz, DMSO-d6) δ [ppm]: 0.690 (1.40), 0.781 (0.76), 0.789 (1.36), 0.796 (1.89), 0.804 (2.42), 0.811 (1.46), 0.830 (0.90), 0.841 (1.57), 0.845 (1.40), 0.850 (1.43), 0.918 (1.01), 0.926 (1.70), 0.933 (1.84), 0.941 (1.52), 0.948 (0.71), 1.200 (1.85), 1.206 (2.28), 1.212 (2.17), 1.218 (2.04), 1.262 (7.84), 1.274 (16.00), 1.286 (8.01), 1.414 (1.96), 1.420 (3.36), 1.427 (1.94), 1.511 (0.92), 1.531 (1.02), 1.554 (0.42), 1.713 (1.30), 1.734 (1.06), 1.890 (1.01), 1.896 (0.96), 1.910 (1.08), 1.916 (1.04), 1.931 (0.52), 1.979 (1.32), 1.996 (0.88), 2.354 (1.65), 2.365 (1.89), 2.373 (1.60), 2.565 (1.84), 2.584 (0.90), 3.007 (1.21), 3.024 (2.60), 3.043 (3.03), 3.065 (2.40), 3.182 (0.81), 3.237 (1.82), 3.254 (2.13), 3.320 (0.74), 3.580 (0.88), 3.627 (0.85), 3.678 (1.79), 4.241 (2.07), 4.252 (6.24), 4.264 (6.15), 4.276 (2.02), 4.473 (0.67), 4.491 (1.24), 4.507 (0.67), 6.991 (6.64), 7.006 (7.01), 7.057 (4.98), 7.060 (5.89), 7.088 (2.81), 7.092 (2.29), 7.102 (3.82), 7.105 (3.49), 7.156 (6.63), 7.170 (4.46), 7.377 (0.89), 7.381 (0.91), 7.454 (7.90), 7.469 (8.63), 7.554 (0.83), 7.993 (8.04).

### Example 93A

### Ethyl 1-[1-(4-chloro-4'-{4-[(spiro[2.2]pentan-1-yl)methyl]piperazin-1-yl}[1,1'-biphenyl]-2-yl)piperidin-3-yl]-5-(difluoromethyl)-1H-pyrazole-4-carboxylate (Diastereomeric mixture 2)

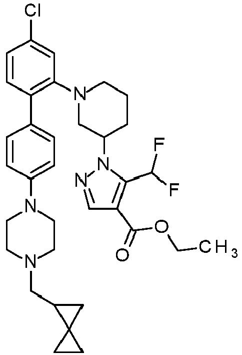

Under argon, a solution of ethyl 1-[(1-{4-chloro-4'-[4-(spiro[2.2]pentane-1-carbonyl)piperazin-1-yl][1,1'-biphenyl]-2-yl}piperidin-3-yl]-5-(difluoromethyl)-1H-pyrazole-4-carboxylate (Example 92A, Enantiomer 2, 120 mg, 188 µmol) in THF (11 ml) was treated dropwise with a solution of BH₃·THF complex trihydrido(oxolane)boron (1.9 ml, 1 N, 1.9 mmol) and the resulting mixture was stirred overnight at room temperature. The reaction mixture was carefully quenched with water and evaporated. The residue was retaken in water and extracted three times with ethyl acetate. The combined organic layers were dried over sodium sulfate and evaporated. The residue was purified by flash chromatography (silica gel, cyclohexane / ethyl acetate gradient) affording 76.0 mg (62 % yield) of the title compound.
LC-MS (Method 9): Rₜ = 5.80 min; MS (ESIpos): m/z = 624 [M+H]⁺
¹H-NMR (600 MHz, DMSO-d6) δ [ppm]: 0.702 (0.82), 0.711 (0.67), 0.724 (1.00), 0.732 (1.76), 0.739 (1.00), 0.786 (1.31), 0.797 (1.35), 0.826 (0.92), 0.831 (0.86), 0.838 (0.92), 0.846 (0.62), 1.121 (0.96), 1.235 (0.68), 1.262 (4.01), 1.274 (7.86), 1.285 (3.99), 1.357 (9.69), 1.398 (16.00), 1.505 (0.65), 1.526 (0.74), 1.732 (1.34), 1.896 (0.48), 1.917 (0.55), 1.978 (0.73), 2.183 (1.35), 2.562 (1.01), 2.582 (0.50), 2.749 (0.65), 2.762 (0.67), 2.770 (0.85), 2.783 (0.80), 2.898 (0.67), 2.917 (0.52), 2.970 (1.76), 3.010 (0.70), 3.027 (1.40), 3.045 (1.59), 3.066 (1.84), 3.071 (1.86), 3.238 (0.77), 3.251 (0.81), 3.257 (0.83), 3.426 (1.04), 3.434 (1.60), 3.443 (2.01), 3.451 (1.58), 3.462 (1.09), 4.239 (1.11), 4.250 (3.39), 4.262 (3.33), 4.274 (1.12), 4.496 (0.62), 6.600 (0.53), 6.871 (0.84), 6.998 (2.91), 7.013 (3.05), 7.057 (2.26), 7.060 (2.57), 7.091 (1.21), 7.095 (1.10), 7.105 (1.66), 7.108 (1.53), 7.162 (2.82), 7.176 (1.95), 7.384 (0.66), 7.462 (3.44), 7.477 (3.38), 7.559 (0.59), 7.998 (3.44).

### Example 94A

### 1-(Oxetan-3-yl)-4-[4-(4,4,5,5-tetramethyl-1,3,2-dioxaborolan-2-yl)phenyl]piperazine

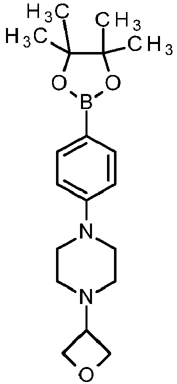

1-[4-(4,4,5,5-tetramethyl-1,3,2-dioxaborolan-2-yl)phenyl]piperazine (80.0 mg, 278 µmol) was placed in 1.7 ml THF and N,N-diisopropylethylamine (73 µl, 420 µmol) was added. Oxetan-3-one (100 mg, 1.39 mmol) was then added and the mixture was stirred for 10 min. Then sodium triacetoxyborohydride (176 mg, 833 µmol) was added and the mixture was stirred at 55°C for 4 h. The reaction mixture was cooled to room temperature, saturated aqueous sodium bicarbonate solution was added and the mixture was extracted three times with ethyl acetate. The combined organic phases were dried over sodium sulfate, filtered and evaporated. 89 mg of the target compound (92% of theory) were obtained.
LC-MS (Method 3): Rₜ = 1.26 min; MS (ESIpos): m/z = 345 [M+H]⁺
¹H-NMR (500 MHz, DMSO-d6) δ [ppm]: 1.259 (16.00), 2.370 (0.91), 2.379 (1.14), 2.389 (0.92), 3.219 (0.94), 3.230 (1.08), 3.239 (0.87), 4.453 (0.62), 4.465 (1.23), 4.477 (0.73), 4.548 (0.78), 4.561 (1.30), 4.574 (0.59), 6.891 (0.99), 6.909 (1.02), 7.497 (1.16), 7.515 (1.05).

### Example 95A

### Ethyl 1-[1-{4-chloro-4'-[4-(oxetan-3-yl)piperazin-1-yl][1,1'-biphenyl]-2-yl}piperidin-3-yl]-5-(difluoromethyl)-1H-pyrazole-4-carboxylate trifluoroacetic acid (Enantiomer 1)

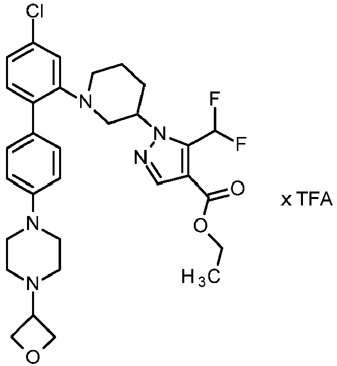

Ethyl 1-[1-{5-chloro-2-[(trifluoromethanesulfonyl)oxy]phenyl}piperidin-3-yl]-5-(difluoromethyl)-1H-pyrazole-4-carboxylate (prepared in analogy to Example 11A, Enantiomer 1, 150 mg, 93 % purity, 262 µmol) and 1-(oxetan-3-yl)-4-[4-(4,4,5,5-tetramethyl-1,3,2-dioxaborolan-2-yl)phenyl]piperazine (prepared in analogy to Example 94A, 99.3 mg, 288 µmol) were placed in 2.6 ml DMF Tetrakis (triphenylphosphine)palladium(0) (15.2 mg, 13.1 µmol) and 2 M sodium carbonate solution (390 µl, 790 µmol) were added and the mixture was stirred for 2.5 hours at 130°C. The reaction mixture was made slightly acidic with 6M hydrochloric acid and extracted three times with ethyl acetate. The organic phase was dried over sodium sulfate, filtered and evaporated. The residue was dissolved in acetonitrile/water/TFA and purified by preparative HPLC (RP18 column, acetonitrile/water gradient with addition of 0.1% TFA). The product fraction was purified again by means of thick layer chromatography (dichloromethane/methanol/formic acid: 10/1/0.1). 120 mg of the target compound (54% of theory, purity 85%) were obtained.
LC-MS (Method 3): Rₜ = 2.29 min; MS (ESIpos): m/z = 600 [M-TFA+H]⁺

### Example 96A

### Ethyl 1-[1-{4-chloro-4'-[4-(oxetan-3-yl)piperazin-1-yl][1,1'-biphenyl]-2-yl}piperidin-3-yl]-5-(difluoromethyl)-1H-pyrazole-4-carboxylate trifluoroacetic acid (Enantiomer 2)

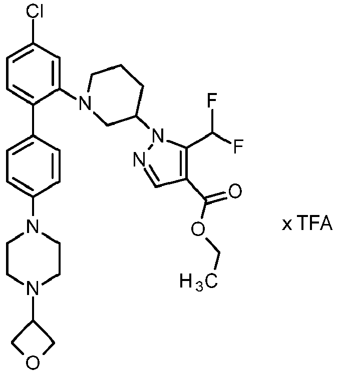

Ethyl 1-[1-{5-chloro-2-[(trifluoromethanesulfonyl)oxy]phenyl}piperidin-3-yl]-5-(difluoromethyl)-1H-pyrazole-4-carboxylate (prepared in analogy to Example 16A, Enantiomer 2, 200 mg, 95 % purity, 357 µmol) and 1-(oxetan-3-yl)-4-[4-(4,4,5,5-tetramethyl-1,3,2-dioxaborolan-2-yl)phenyl]piperazine (prepared in analogy to Example 94A, 135 mg, 393 µmol) were placed in 3.5 ml in DMF. Tetrakis (triphenylphosphine)palladium(0) (20.6 mg, 17.9 µmol) and 2 M sodium carbonate solution (540 µl, 1.1 mmol) were added and the mixture was stirred for 2.5 hours at 130°C. The reaction mixture was made slightly acidic with 6M hydrochloric acid and extracted three times with ethyl acetate. The organic phase was dried over sodium sulfate, filtered and evaporated. The residue was dissolved in acetonitrile/water/TFA and purified by preparative HPLC (RP18 column, acetonitrile/water gradient with the addition of 0.1% TFA). The product fraction was purified by means of thick layer chromatography (dichloromethane/methanol/formic acid: 10/1/0.1). 108 mg of the target compound (41% of theory, purity 96%) were obtained.
LC-MS (Method 3): Rₜ = 2.28 min; MS (ESIpos): m/z = 600 [M+H]⁺

### Example 97A

### Ethyl 1-{1-[4-chloro-4'-(pyrrolidin-1-yl)[1,1'-biphenyl]-2-yl]piperidin-3-yl}-5-(difluoromethyl)-1H-pyrazole-4-carboxylate (Enantiomer 1)

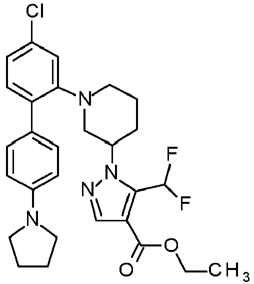

Under argon, a solution of ethyl 1-[1-{5-chloro-2-[(triffuoromethanesulfonyl)oxy]phenyl}piperidin-3-yl]-5-(difluoromethyl)-1H-pyrazole-4-carboxylate (prepared in analogy to Example 11A, Enantiomer 1, 38.5 mg, 69 % purity, 49.7 µmol) in DMF (550 µl) was treated with 1-[4-(4,4,5,5-tetramethyl-1,3,2-dioxaborolan-2-yl)phenyl]pyrrolidine (16.3 mg, 59.7 µmol), sodium carbonate (75 µl, 2.0 M, 150 µmol) and Pd(PPh₃)₄ (3 mg, 2.0 µmol). The resulting mixture was stirred 1.5 hours at 130°C and cooled to room temperature. The reaction mixture was diluted with water (30 ml) and extracted three times with ethyl acetate. The combined organic layers were dried over sodium sulfate and evaporated. The residue was purified by flash chromatography (silica gel, cyclohexane / ethyl acetate gradient) affording 10.0 mg (84 % purity, 32 % yield) of the title compound.
LC-MS (Method 3): Rₜ = 3.21 min; MS (ESIpos): m/z = 529 [M+H]⁺
¹H-NMR (600 MHz, DMSO-d6) δ [ppm]: 1.251 (16.00), 1.264 (3.28), 1.276 (6.28), 1.288 (3.22), 1.398 (1.39), 1.528 (0.41), 1.550 (0.46), 1.695 (0.60), 1.717 (0.47), 1.895 (0.46), 1.917 (0.53), 1.938 (1.22), 1.948 (2.52), 1.958 (2.74), 1.968 (4.72), 1.978 (2.08), 3.020 (0.64), 3.038 (1.22), 3.055 (0.72), 3.073 (0.62), 3.092 (0.55), 3.198 (0.43), 3.226 (1.14), 3.236 (2.50), 3.255 (4.20), 3.265 (1.85), 3.703 (0.40), 4.242 (0.91), 4.253 (2.64), 4.265 (2.62), 4.277 (0.86), 4.539 (0.59), 5.747 (1.01), 6.483 (1.07), 6.497 (1.07), 6.581 (2.60), 6.596 (2.56), 7.012 (1.94), 7.016 (2.04), 7.063 (0.95), 7.067 (0.79), 7.077 (1.31), 7.080 (1.15), 7.136 (2.34), 7.150 (1.66), 7.382 (0.65), 7.415 (2.87), 7.429 (2.67), 7.454 (1.15), 7.468 (2.07), 7.555 (0.55), 8.001 (2.85).

### Example 98A

### Ethyl 1-11-[4-chloro-4'-(pyrrolidin-1-yl)[1,1'-biphenyl]-2-yl]piperidin-3-yl}-5-(difluoromethyl)-1H-pyrazole-4-carboxylate (Enantiomer 2)

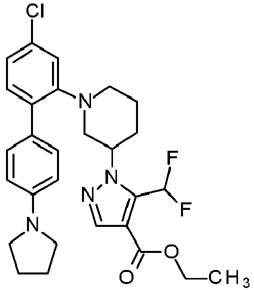

Under argon, a solution of ethyl 1-[1-{5-chloro-2-[(triffuoromethanesulfonyl)oxy]phenyl}piperidin-3-yl]-5-(difluoromethyl)-1H-pyrazole-4-carboxylate (prepared in analogy to Example 16A, Enantiomer 2, 167 mg, 300 µmol) in DMF (3.3 ml) was treated with 1-[4-(4,4,5,5-tetramethyl-1,3,2-dioxaborolan-2-yl)phenyl]pyrrolidine (98.3 mg, 360 µmol) sodium carbonate (17.3 mg, 15.0 µmol) and Pd(PPh₃)₄ (17.3 mg, 15.0 µmol). The resulting mixture was stirred 1.5 hours at 130°C and cooled to room temperature. The reaction mixture was diluted with water (30 ml) and extracted three times with ethyl acetate. The combined organic layers were dried over sodium sulfate and evaporated. The residue was purified by preparative HPLC (RP18 column, eluent: Acetonitrile/water gradient) affording 70.2 mg (44 % yield) of the title compound.
LC-MS (Method 3): Rₜ = 3.20 min; MS (ESIpos): m/z = 529 [M+H]⁺
¹H-NMR (600 MHz, DMSO-d6) δ [ppm]: 1.229 (0.96), 1.263 (7.73), 1.276 (16.00), 1.287 (7.94), 1.525 (0.79), 1.546 (0.86), 1.690 (1.09), 1.712 (0.86), 1.891 (0.87), 1.897 (0.83), 1.911 (0.92), 1.918 (0.90), 1.932 (0.51), 1.939 (0.55), 1.955 (3.56), 1.961 (4.53), 1.966 (9.65), 1.977 (4.12), 1.987 (1.48), 2.005 (0.79), 2.484 (0.88), 2.520 (1.06), 3.020 (1.31), 3.037 (2.56), 3.055 (1.50), 3.072 (1.17), 3.091 (1.11), 3.242 (3.36), 3.253 (8.27), 3.264 (3.41), 3.280 (1.36), 3.295 (1.10), 4.241 (2.14), 4.253 (6.27), 4.265 (5.95), 4.277 (1.87), 4.525 (0.68), 4.536 (0.73), 4.543 (1.17), 4.550 (0.74), 4.561 (0.66), 6.581 (5.73), 6.595 (5.89), 7.013 (4.40), 7.016 (4.85), 7.068 (2.30), 7.072 (1.97), 7.082 (3.20), 7.085 (2.99), 7.137 (5.89), 7.151 (3.95), 7.399 (1.23), 7.423 (6.61), 7.438 (6.18), 7.485 (2.44), 7.572 (1.00), 8.016 (6.96).

### Example 99A

### 3-[4-(4-Bromophenyl)piperazin-1-yl]propanenitrile

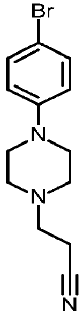

Under argon, a solution of 1-bromo-4-iodobenzene (500 mg, 1.77 mmol) and 3-(piperazin-1-yl)propanenitrile (295 mg, 2.12 mmol) in 1,4-dioxane (8.0 ml) was treated with Pd₂dba₃ (40.5 mg, 44.2 µmol), Xantphos (77.7 mg, 134 µmol) and caesium carbonate (806 mg, 2.47 mmol) and stirred overnight at 100°C. The reaction mixture was cooled, diluted with water, extracted three times with ethyl acetate and concentrated. The crude residue was purified by preparative HPLC (RP18 column, eluent: Acetonitrile/water + 0.1% formic acid gradient) affording 94 mg (18 % yield) of the title compound.
LC-MS (Method 3): Rₜ = 1.10 min; MS (ESIpos): m/z = 294 [M+H]⁺
¹H-NMR (400 MHz, DMSO-d6) δ [ppm]: 2.366 (0.59), 2.567 (14.25), 2.593 (4.36), 2.610 (12.81), 2.625 (9.11), 2.670 (0.45), 2.694 (9.38), 2.710 (14.56), 2.726 (4.38), 2.729 (4.12), 3.113 (13.70), 3.126 (15.62), 3.138 (12.62), 3.276 (0.51), 5.754 (0.55), 6.877 (1.51), 6.885 (14.59), 6.890 (4.73), 6.902 (5.25), 6.908 (16.00), 6.916 (1.60), 7.314 (1.61), 7.323 (15.82), 7.328 (4.62), 7.340 (4.66), 7.345 (14.13), 7.354 (1.24).

### Example 100A

### 3-{4-[4-(4,4,5,5-Tetramethyl-1,3,2-dioxaborolan-2-yl)phenyl]piperazin-1-yl}propanenitrile

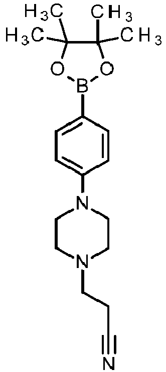

Under argon, a solution of 3-[4-(4-bromophenyl)piperazin-1-yl]propanenitrile (prepared in analogy to Example 99A, 579 mg, 1.97 mmol) and 4,4,4',4',5,5,5',5'-octamethyl-2,2'-bi-1,3,2-dioxaborolane (750 mg, 2.95 mmol) in 1,4-dioxane (14 ml) was treated with Pd₂dba₃ (54.1 mg, 59.0 µmol), X-Phos (56.3 mg, 118 µmol) and potassium acetate (579 mg, 5.90 mmol). The reaction was stirred overnight at 105°C then filtered over celite. The filtrate was concentrated and the residue was purified by flash chromatography (silica gel, dichloromethan/methanol gradient) and then by preparative HPLC (RP18 column, eluent: Acetonitrile/water, gradient) affording 321 mg (48 % yield) of the title compound.
LC-MS (Method 3): Rₜ = 1.37 min; MS (ESIpos): m/z = 342 [M+H]⁺
¹H-NMR (600 MHz, DMSO-d6) δ [ppm]: 1.260 (16.00), 2.560 (1.06), 2.600 (0.43), 2.612 (1.09), 2.623 (0.70), 2.695 (0.75), 2.706 (1.19), 2.718 (0.46), 3.203 (1.05), 3.211 (1.29), 3.219 (1.00), 6.894 (1.03), 6.908 (1.07), 7.498 (1.13), 7.512 (1.05).

### Example 101A

### Ethyl 1-[1-{4-chloro-4'-[4-(2-cyanoethyl)piperazin-1-yl][1,1'-biphenyl]-2-yl}piperidin-3-yl]-5-(difluoromethyl)-1H-pyrazole-4-carboxylate (Enantiomer 2)

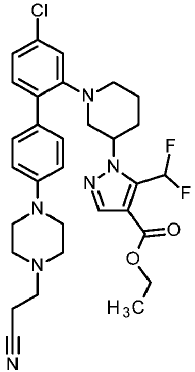

Under argon, a solution of ethyl 1-[1-{5-chloro-2-[(triffuoromethanesulfonyl)oxy]phenyl}piperidin-3-yl]-5-(difluoromethyl)-1H-pyrazole-4-carboxylate (prepared in analogy to Example 16A, Enantiomer 2, 90.0 mg, 169 µmol) and 3-{4-[4-(4,4,5,5-tetramethyl-1,3,2-dioxaborolan-2-yl)phenyl]piperazin-1-yl}propanenitrile (Example 100A, 69.3 mg, 203 µmol) in toluene/ethanol (1:1, 2.5 ml) was treated with Pd(PPh₃)₄ (9.78 mg, 8.46 µmol) and an aqueous solution of sodium carbonate (250 µl, 2 N, 510 µmol) and the reaction was stirred at 100°C for 3 hours. The reaction mixture diluted with dichloromethane and purified by flash chromatography (silica gel, dichloromethane/methanol, gradient) affording 100 mg (89 % yield) of the title compound.
LC-MS (Method 3): Rₜ = 2.41 min; MS (ESIpos): m/z = 597 [M+H]⁺
¹H-NMR (600 MHz, DMSO-d6) δ [ppm]: 1.071 (16.00), 1.160 (1.32), 1.236 (0.73), 1.264 (3.39), 1.275 (6.57), 1.287 (3.37), 1.508 (0.49), 1.530 (0.49), 1.708 (0.60), 1.731 (0.50), 1.890 (0.44), 1.910 (0.49), 1.981 (0.69), 1.998 (0.47), 2.579 (3.06), 2.587 (4.61), 2.594 (3.52), 2.622 (1.40), 2.633 (3.17), 2.644 (2.11), 2.694 (0.48), 2.714 (2.24), 2.725 (3.34), 2.737 (1.32), 2.953 (0.42), 3.010 (0.64), 3.028 (1.28), 3.046 (1.28), 3.066 (0.59), 3.189 (3.60), 3.250 (0.70), 3.263 (0.72), 3.897 (2.68), 4.242 (0.93), 4.254 (2.74), 4.266 (2.70), 4.277 (0.86), 4.502 (0.62), 5.747 (1.90), 6.979 (2.78), 6.993 (2.86), 7.048 (2.04), 7.051 (2.26), 7.084 (1.02), 7.087 (0.86), 7.098 (1.43), 7.101 (1.27), 7.154 (2.44), 7.168 (1.64), 7.385 (0.65), 7.439 (3.07), 7.454 (2.79), 7.472 (1.23), 7.540 (0.44), 7.549 (0.78), 7.560 (1.02), 7.604 (0.75), 7.616 (0.79), 7.624 (1.02), 7.637 (0.68), 7.997 (3.14), 8.765 (0.48).

### Example 102A

### Ethyl 1-[1-{4-chloro-4'-[4-(2-methylpropanoyl)piperazin-1-yl][1,1'-biphenyl]-2-yl}piperidin-3-yl]-5-(difluoromethyl)-1H-pyrazole-4-carboxylate (Enantiomer 2)

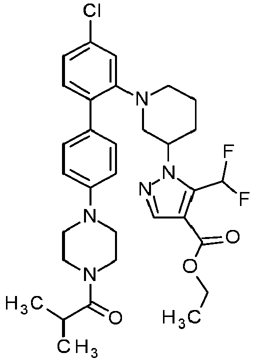

A solution of 2-methylpropanoic acid (15 µl, 160 µmol) in DMF (1.4 ml) was treated with HATU (55.5 mg, 146 µmol]) and N,N-diisopropylethylamine (76 µl, 440 µmol). Ethyl 1-{1-[4-chloro-4'-(piperazin-1-yl)[1,1'-biphenyl]-2-yl]piperidin-3-yl}-5-(difluoromethyl)-1H-pyrazole-4-carboxylate hydrochloride (prepared in analogy to Example 18A, Enantiomer 2, 90.0 mg, 146 µmol) was then added and the resulting mixture was stirred overnight at room temperature. The reaction mixture was evaporated and the residue purified by flash chromatography (silica gel, cyclohexane / ethyl acetate gradient) affording 71 mg (79 % yield) of the title compound.
LC-MS (Method 3): Rₜ = 2.84 min; MS (ESIpos): m/z = 614 [M+H]⁺

### Example 103A

### Ethyl 1-[1-{4-chloro-4'-[4-(2-fluoro-2-methylpropanoyl)piperazin-1-yl][1,1'-biphenyl]-2-yl}pipendin-3-yl]-5-(difluoromethyl)-1H-pyrazole-4-carboxylate (Enantiomer 2)

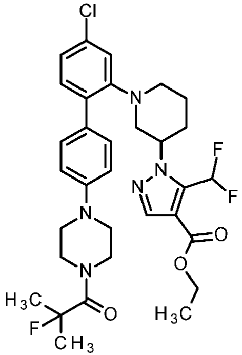

A solution of 2-fluoro-2-methylpropanoic acid (17.0 mg, 160 µmol) in DMF (700 µl) was treated with HATU (55.5 mg, 146 µmol) and N,N-diisopropylethylamine (76 µl, 440 µmol). Ethyl 1-{1-[4-chloro-4'-(piperazin-1-yl)[1,1'-biphenyl]-2-yl]piperidin-3-yl}-5-(difluoromethyl)-1H-pyrazole-4-carboxylate hydrochloride (prepared in analogy to Example 18A, Enantiomer 2, 90.0 mg, 146 µmol) was then added and the resulting mixture was stirred overnight at room temperature. A pre-stirred (10 minutes) solution of 2-fluoro-2-methylpropanoic acid (17.0 mg, 160 µmol), HATU (55.5 mg, 146 µmol) and N,N-diisopropylethylamine (76 µl, 440 µmol) in DMF (0.5 ml) was added and the resulting mixture was stirred overnight at room temperature. The reaction mixture was evaporated and the residue purified by flash chromatography (silica gel, cyclohexane / ethyl acetate gradient) affording 68.0 mg (72 % yield) of the title compound.
LC-MS (Method 3): Rₜ = 2.91 min; MS (ESIpos): m/z = 632 [M+H]⁺

### Example 104A

### Ethyl 1-[1-{4-chloro-4'-[4-(cyclopropanecarbonyl)piperazin-1-yl][1,1'-biphenyl]-2-yl}piperidin-3-yl]-5-(difluoromethyl)-1H-pyrazole-4-carboxylate (Enantiomer 2)

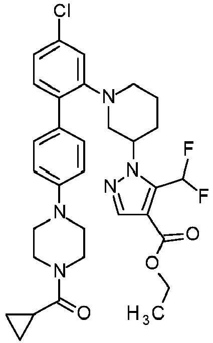

A solution of cyclopropanecarboxylic acid (13 µl, 160 µmol) in DMF (1.4 ml) was treated with HATU (55.5 mg, 146 µmol]) and N,N-diisopropylethylamine (76 µl, 440 µmol). Ethyl 1-{1-[4-chloro-4'-(piperazin-1-yl)[1,1'-biphenyl]-2-yl]piperidin-3-yl}-5-(difluoromethyl)-1H-pyrazole-4-carboxylate hydrochloride (prepared in analogy to Example 18A, Enantiomer 2, 90.0 mg, 146 µmol) was then added and the resulting mixture was stirred overnight at room temperature. The reaction mixture was evaporated and the residue purified by flash chromatography (silica gel, cyclohexane / ethyl acetate gradient) affording 74.0 mg (83 % yield) of the title compound.
LC-MS (Method 3): Rₜ = 2.79 min; MS (ESIpos): m/z = 612 [M+H]⁺

### Example 105A

### Ethyl 1-[1-{4-chloro-4'-[4-(3-methylbutanoyl)piperazin-1-yl][1,1'-biphenyl]-2-yl}piperidin-3-yl]-5-(difluoromethyl)-1H-pyrazole-4-carboxylate (Enantiomer 2)

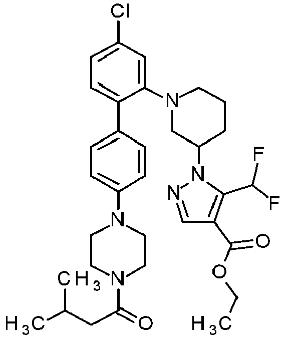

A solution of 3-methylbutanoic acid (18 µl, 160 µmol) in DMF (1.4 ml) was treated with HATU (55.5 mg, 146 µmol]) and N,N-diisopropylethylamine (76 µl, 440 µmol]). Ethyl 1-{1-[4-chloro-4'-(piperazin-1-yl)[1,1'-biphenyl]-2-yl]piperidin-3-yl}-5-(difluoromethyl)-1H-pyrazole-4-carboxylate hydrochloride (prepared in analogy to Example 18A, Enantiomer 2, 90.0 mg, 146 µmol) was then added and the resulting mixture was stirred overnight at room temperature. The reaction mixture was evaporated and the residue purified by flash chromatography (silica gel, cyclohexane / ethyl acetate gradient) affording 64.0 mg (70 % yield) of the title compound.
LC-MS (Method 3): Rₜ = 2.91 min; MS (ESIpos): m/z = 628 [M+H]⁺

### Example 106A

### Tert-butyl 5-{2-[(benzyloxy)carbonyl]hydrazinyl}-3,3-difluoropiperidine-1-carboxylate (Racemate)

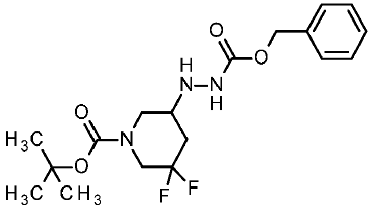

A solution of tert-butyl 3,3-difluoro-5-oxopiperidine-1-carboxylate (7.00 g, 29.8 mmol) in methanol (280 ml) was treated with benzyl hydrazinecarboxylate (4.95 g, 29.8 mmol) and stirred 1 hour at room temperature. The reaction mixture was cooled to 0°C and sodium triacetoxyborohydride was added. The resulting mixture was slowly warm to room temperature and stirred overnight. The reaction mixture was diluted with water and extracted three times with ethyl acetate. The combined organic layers were washed with a saturated solution of sodium chloride, dried over magnesium sulfate and evaporated. The residue was purified by flash chromatography (silica gel, cyclohexane/ethyl acetate gradient) affording 6.30 g (51 % yield) of the title compound.
LC-MS (Method 5): Rₜ = 3.14 min; MS (ESIneg): m/z = 384 [M-H]⁻
¹H-NMR (600 MHz, DMSO-d6) δ [ppm]: 1.163 (0.75), 1.175 (1.46), 1.187 (0.75), 1.381 (16.00), 1.396 (2.96), 1.406 (1.75), 1.415 (0.91), 1.419 (1.19), 1.427 (0.52), 1.432 (0.48), 1.986 (2.73), 2.258 (0.47), 2.985 (0.42), 4.013 (0.49), 4.025 (0.97), 4.036 (1.00), 4.048 (0.61), 4.960 (1.05), 5.062 (5.04), 5.137 (0.48), 7.302 (0.48), 7.307 (0.55), 7.313 (1.17), 7.317 (0.87), 7.324 (0.89), 7.327 (0.98), 7.343 (1.20), 7.353 (8.44), 7.364 (2.81), 7.377 (0.68), 7.391 (0.52), 8.676 (0.44).

### Example 107A

### Tert-butyl 3,3-difluoro-5-hydrazinylpiperidine-1-carboxylate hydrochloride (Racemate)

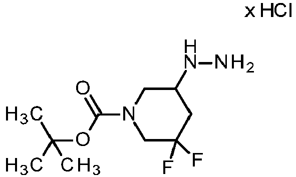

A solution of tert-butyl 5-{2-[(benzyloxy)carbonyl]hydrazinyl}-3,3-difluoropiperidine-1-carboxylate (Example 106A, 6.30 g, 16.3 mmol) in ethanol (350 ml) was treated with 10% Pd/C and stirred 48 hours under 1 atmosphere of hydrogen. The reaction mixture was filtered over Celite. A solution of hydrogen chloride in dioxane (4.9 ml, 4.0 M, 20 mmol) was added to the filtrate and the resulting solution was evaporated. The residue was co-evaporated three times with diethyl ether affording 4.23 g (89 % yield) of the title compound which was used in the next step without further purification.
¹H-NMR (600 MHz, DMSO-d6) δ [ppm]: 1.378 (0.48), 1.386 (0.76), 1.396 (4.76), 1.411 (16.00), 1.432 (0.50), 1.496 (0.45), 3.436 (0.62), 3.447 (0.53), 7.186 (0.46), 7.270 (0.52), 7.356 (0.45).

### Example 108A

### Tert-butyl 5-[5-(difluoromethyl)-4-(ethoxycarbonyl)-1H-pyrazol-1-yl]-3,3-difluoropiperidine-1-carboxylate (Racemate)

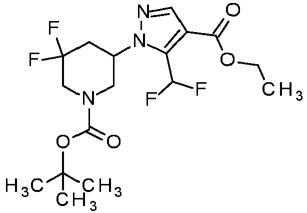

A solution of tert-butyl 3,3-difluoro-5-hydrazinylpiperidine-1-carboxylate hydrochloride (prepared in analogy to Example 108A, 1.07 g, 3.72 mmol) in ethanol (32 ml) was treated with 2-(ethoxymethylidene)-4,4-difluoro-3-oxobutanoate (prepared in analogy to Example 3A, 770 µl, 4.1 mmol) and N,N-diisopropylethylamine (1.3 ml, 7.4 mmol). The resulting mixture was stirred 1 hour at 80°C and evaporated. The residue was purified by flash chromatography (silica gel, cyclohexane/ethyl acetate gradient) affording 717 mg (44 % yield) of the title compound.

The two enantiomers were separated by preparative chiral HPLC [sample preparation: 717 mg were dissolved in 9.5 ml acetonitrile/ethanol (1:1); injection volume: 80 µl; column: Daicel Chiralpak AY-H 5 µm, 250 x 20 mm; eluent: n-heptane/ethanol 85:15; flow rate: 20 ml/min; temperature: 30°C; UV detection: 220 nm]. After separation, 289 mg of enantiomer 1 (Example 109A), which eluted first, and 249 mg of enantiomer 2 (Example 110A), which eluted later, were isolated.

### Example 109A

### Tert-butyl 5-[5-(difluoromethyl)-4-(ethoxycarbonyl)-1H-pyrazol-1-yl]-3,3-difluoropiperidine-1-carboxylate (Enantiomer 1)

For separation conditions see Example 108A.

Analytical chiral HPLC: Rt = 0.752 min, e.e. = 99% [column: Daicel Chiralpak AY 3 µm, 50 x 4.6 mm; eluent: n-heptane/ethanol 70 : 30; flow rate: 1.0 ml/min; temperature: 30°C; UV detection: 220 nm].
LC-MS (Method 3): Rt = 2.33 min; MS (ESIpos): m/z = 410 [M+H]+

### Example 110A

### Tert-butyl 5-[5-(difluoromethyl)-4-(ethoxycarbonyl)-1H-pyrazol-1-yl]-3,3-difluoropiperidine-1-carboxylate (Enantiomer 2)

For separation conditions see Example 108A.

Analytical chiral HPLC: Rt = 0.943 min, e.e. = 98% [column: Daicel Chiralpak AY 3 µm, 50 x 4.6 mm; eluent: n-heptane/ethanol 70 : 30; flow rate: 1.0 ml/min; temperature: 30°C; UV detection: 220 nm].
LC-MS (Method 3): Rt = 2.33 min; MS (ESIpos): m/z = 410 [M+H]+

### Example 111A

### Ethyl 5-(difluoromethyl)-1-[5,5-difluoropiperidin-3-yl]-1H-pyrazole-4-carboxylate hydrochloride (Enantiomer 1)

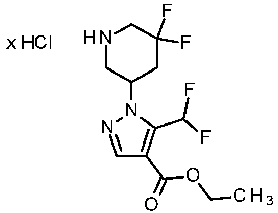

A solution of tert-butyl 5-[5-(difluoromethyl)-4-(ethoxycarbonyl)-1H-pyrazol-1-yl]-3,3-difluoropiperidine-1-carboxylate (Example 109A, Enantiomer 1, 289 mg, 706 µmol) in dichloromethane (7.0 ml) was treated with a solution of hydrogen chloride in dioxane (1.8 ml, 4.0 M, 7.1 mmol) and stirred 3 hours at room temperature. The reaction mixture was co-evaporated three times with acetonitrile affording 249 mg (quant.) of the title compound which was used in the next step without further purification.
LC-MS (Method 3): Rₜ = 1.38 min; MS (ESIpos): m/z = 310 [M+H]⁺

### Example 112A

### Ethyl 5-(difluoromethyl)-1-[5,5-difluoropiperidin-3-yl]-1H-pyrazole-4-carboxylate (Enantiomer 1)

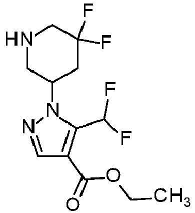

A solution of ethyl 5-(difluoromethyl)-1-[5,5-difluoropiperidin-3-yl]-1H-pyrazole-4-carboxylate hydrochloride (Example 110A, Enantiomer 1, 179 mg, 518 µmol) in dichloromethane was washed once with a saturated solution of sodium hydrogencarbonate. The aqueous phase was extracted twice with dichloromethane. The combined organic layers were dried over sodium sulfate and evaporated affording 153 mg (96 % yield) of the title compound which was used in the next step without further purification.
¹H-NMR (400 MHz, DMSO-d6) δ [ppm]: 1.271 (7.41), 1.289 (16.00), 1.307 (7.62), 1.407 (0.55), 2.572 (0.55), 2.582 (0.51), 2.602 (0.64), 2.613 (0.62), 2.821 (0.40), 2.846 (0.63), 2.879 (0.98), 2.900 (1.18), 2.927 (1.97), 2.954 (3.35), 2.978 (0.94), 3.060 (1.48), 3.090 (1.90), 4.250 (2.32), 4.268 (7.13), 4.286 (7.06), 4.304 (2.21), 4.653 (0.55), 4.666 (0.72), 4.679 (0.95), 4.692 (0.81), 4.706 (0.51), 7.472 (1.79), 7.602 (3.47), 7.732 (1.63), 8.066 (5.92).

### Example 113A

### Ethyl 1-[1-{5-chloro-2-[(4-methoxyphenyl)methoxy]phenyl}-5,5-difluoropiperidin-3-yl]-5-(difluoromethyl)-1H-pyrazole-4-carboxylate (Enantiomer 1)

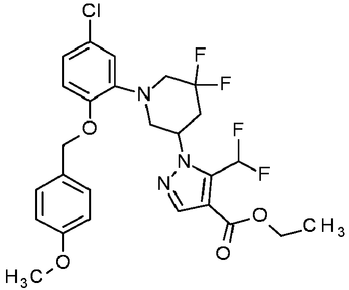

Under argon, a solution of 2-bromo-4-chloro-1-[(4-methoxyphenyl)methoxy]benzene (prepared in analogy to Example 8A, 161 mg, 491 µmol) and ethyl 5-(difluoromethyl)-1-[5,5-difluoropiperidin-3-yl]-1H-pyrazole-4-carboxylate (Example 112A, Enantiomer 1, 152 mg, 491 µmol) in 1,4-dioxane (5.1 ml) was treated with Pd₂dba₃ (225 mg, 246 µmol), rac-BINAP (306 mg, 491 µmol) and caesium carbonate (400 mg, 1.23 mmol). The resulting mixture was stirred overnight at 100°C. Extra 2-bromo-4-chloro-1-[(4-methoxyphenyl)methoxy]benzene (Example 8A, 81 mg, 246 µmol) Pd₂dba₃ (90 mg, 98 µmol), rac-BINAP (122 mg, 196 µmol) and caesium carbonate (200 mg, 0.61 mmol) were added to the reaction mixture and the resulting mixture was stirred overnight at 100°C. The reaction mixture was filtered over Celite, rinsed with ethyl acetate and evaporated. The residue was purified by preparative HPLC (RP18 column, eluent: Acetonitrile/water gradient) affording 134 mg (87 % purity, 42 % yield) of the tilte compound.
LC-MS (Method 3): Rₜ = 2.69 min; MS (ESIpos): m/z = 556 [M+H]⁺

### Example 114A

### Ethyl 1-[1-(5-chloro-2-hydroxyphenyl)-5,5-difluoropiperidin-3-yl]-5-(difluoromethyl)-1H-pyrazole-4-carboxylate (Enantiomer 1)

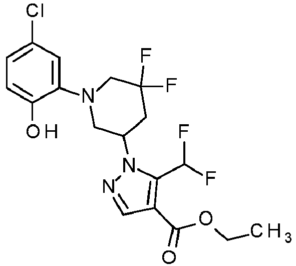

A solution of ethyl 1-[1-{5-chloro-2-[(4-methoxyphenyl)methoxy]phenyl}-5,5-difluoropiperidin-3-yl]-5-(difluoromethyl)-1H-pyrazole-4-carboxylate (Example 113A, Enantiomer 1, 134 mg, 87 % purity, 210 µmol) in dichloromethane (1.4 ml) was treated with trifluoroacetic acid (160 µl, 2.1 mmol) and the resulting mixture was sitrred 2.5 hours at room temperature. The reaction mixture was evaporated and the residue retaken in ethyl acetate. The organic solution was washed once with water, once with a saturated solution of sodium hydrogen carbonate and once with a saturated solution of sodium chloride. The organic phase was dried over sodium sulfate and evaporated. The residue was purified by flash chromatography (silica gel, cyclohexane / ethyl acetate gradient) affording 57 mg (56 % yield) oft he title compound.
LC-MS (Method 3): Rₜ = 2.31 min; MS (ESIpos): m/z = 436 [M+H]⁺

### Example 115A

### Ethyl 1-[1-{5-chloro-2-[(trifluoromethanesulfonyl)oxy]phenyl}-5,5-difluoropiperidin-3-yl]-5-(difluoromethyl)-1H-pyrazole-4-carboxylate (Enantiomer 1)

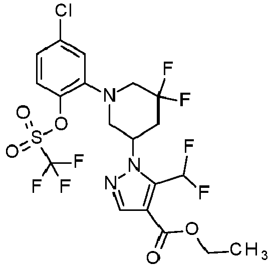

Under argon, a solution of ethyl 1-[1-(5-chloro-2-hydroxyphenyl)-5,5-difluoropiperidin-3-yl]-5-(difluoromethyl)-1H-pyrazole-4-carboxylate (Example 114A, Enantiomer 1, 57.0 mg, 131 µmol) in dichloromethane (1.4 ml) was treated with triethylamine (46 µl, 330 µmol) and cooled to 0°C. Trifluoromethanesulfonic anhydride (27 µl, 160 µmol) was added and the resulting mixture was stirred 45 minutes at 0°C. The reaction mixture was diluted with dichloromethane and washed three times with water. The organic layer was dried over sodium sulfate and evaporated. The residue was purified by flash chromatography (silica gel, cyclohexane / ethyl acetate gradient) affording 58.0 mg (67 % yield) of the title compound.
LC-MS (Method 3): Rₜ = 2.58 min; MS (ESIpos): m/z = 568 [M+H]⁺

### Example 116A

### Tert-butyl 4-(4'-chloro-2'-{5-[5-(difluoromethyl)-4-(ethoxycarbonyl)-1H-pyrazol-1-yl]-3,3-difluoropiperidin-1-yl}[1,1'-biphenyl]-4-yl)piperazine-1-carboxylate (Enantiomer 1)

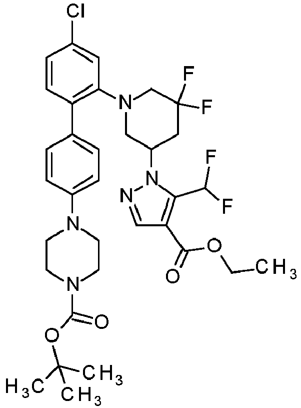

Under argon, a solution of ethyl 1-[1-{5-chloro-2-[(trifluoromethanesulfonyl)oxy]phenyl}-5,5-difluoropiperidin-3-yl]-5-(difluoromethyl)-1H-pyrazole-4-carboxylate (Example 115A, Enantiomer 1, 58.0 mg, 102 µmol) and tert-butyl 4-[4-(4,4,5,5-tetramethyl-1,3,2-dioxaborolan-2-yl)phenyl]piperazine-1-carboxylate (47.6 mg, 123 µmol) in toluene/ethanol mixture (1:1) (800 µl) was treated with an aqueous solution of sodium carbonate (150 µl, 2.0 M, 310 µmol) and Pd(PPh₃)₄ (5.90 mg, 5.11 µmol) and stirred 2 hours at 100°C. The reaction mixture was cooled to room temperature, filtered over celite and rinsed with ethyl acetate. The filtrate was evaporated and the residue purified by flash chromatography (silica gel, cyclohexane/ethyl acetate gradient) affording 52.0 mg (70 % yield) of the title compound.
LC-MS (Method 3): Rₜ = 2.98 min; MS (ESIpos): m/z = 680 [M+H]⁺

### Example 117A

### 1-[1-{4'-[4-(Tert-butoxycarbonyl)piperazin-1-yl]-4-chloro[1,1'-biphenyl]-2-yl}-5,5-difluoropiperidin-3-yl]-5-(difluoromethyl)-1H-pyrazole-4-carboxylic acid (Enantiomer 1)

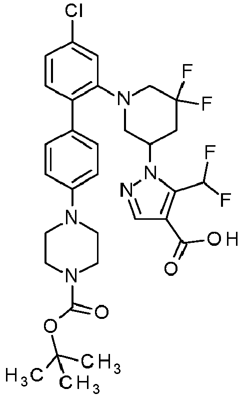

An aqueous solution of lithium hydroxide (760 µl, 1.0 M, 760 µmol) was added to a solution of tert-butyl 4-(4'-chloro-2'-{5-[5-(difluoromethyl)-4-(ethoxycarbonyl)-1H-pyrazol-1-yl]-3,3-difluoropiperidin-1-yl}[1,1'-biphenyl]-4-yl)piperazine-1-carboxylate (Example 116A, Enantiomer 1, 52.0 mg, 76.5 µmol) in a THF/methanol mixture 10:1 (1.5 ml). The resulting mixture was stirred 2 hours at room temperature. The reaction mixture was acidified with an aqueous solution of hydrogen chloride (4 N) and purified by preparative HPLC (RP18 column, eluent: Acetonitrile/water gradient) affording 32.0 mg (64 % yield) of the title compound.
LC-MS (Method 3): Rₜ = 2.66 min; MS (ESIpos): m/z = 652 [M+H]⁺

### Example 118A

### 1-{1-[4-Chloro-4'-(piperazin-1-yl)[1,1'-biphenyl]-2-yl]-5,5-difluoropiperidin-3-yl}-5-(difluoromethyl)-1H-pyrazole-4-carboxylic acid-hydrochloride (Enantiomer 1)

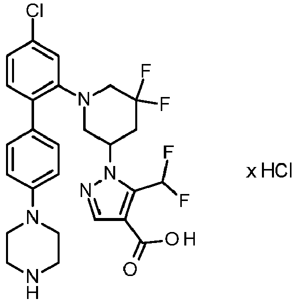

A solution of 1-[1-{4'-[4-(tert-butoxycarbonyl)piperazin-1-yl]-4-chloro[1,1'-biphenyl]-2-yl}-5,5-difluoropiperidin-3-yl]-5-(difluoromethyl)-1H-pyrazole-4-carboxylic acid (Example 117A, Enantiomer 1, 32.0 mg, 49.1 µmol) in dichloromethane (1.0 ml) was treated with a solution of hydrogen chloride in dioxane (120 µl, 4.0 M, 490 µmol) and stirred 3 days at room temperature. The reaction mixture was evaporated affording 34.0 mg (quant.) of the title compound which was used in the next steps without further purification.
LC-MS (Method 3): Rₜ = 1.53 min; MS (ESIpos): m/z = 552 [M+H]⁺

### Example 119A

### Ethyl 5-(difluoromethyl)-1-[5,5-difluoropiperidin-3-yl]-1H-pyrazole-4-carboxylate hydrochloride (Enantiomer 2)

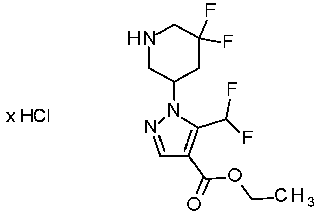

A solution of tert-butyl 5-[5-(difluoromethyl)-4-(ethoxycarbonyl)-1H-pyrazol-1-yl]-3,3-difluoropiperidine-1-carboxylate (Example 110A, Enantiomer 2, 249 mg, 608 µmol) in dichloromethane (6.0 ml) was treated with a solution of hydrogen chloride in dioxane (1.5 ml, 4.0 M, 6.1 mmol) and stirred 3 hours at room temperature. The reaction mixture was co-evaporated three times with acetonitrile affording 209 mg (quant.) of the title compound which was used in the next step without further purification..
LC-MS (Method 3): Rₜ = 1.38 min; MS (ESIpos): m/z = 310 [M+H]⁺

### Example 120A

### Ethyl 5-(difluoromethyl)-1-[5,5-difluoropiperidin-3-yl]-1H-pyrazole-4-carboxylate (Enantiomer 2)

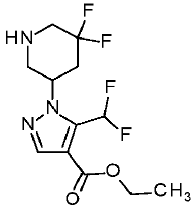

A solution of ethyl 5-(difluoromethyl)-1-[5,5-difluoropiperidin-3-yl]-1H-pyrazole-4-carboxylate hydrochloride (Example 119A, Enantiomer 2,209 mg, 605 µmol) in dichloromethane was washed once with a saturated solution of sodium hydrogencarbonate. The aqueous phase was extracted twice with dichloromethane. The combined organic layers were dried over sodium sulfate and evaporated affording 183 mg (98 % yield) of the title compound which was used in the next step without further purification.
¹H-NMR (400 MHz, DMSO-d6) δ [ppm]: 0.008 (2.49), 1.271 (7.50), 1.288 (16.00), 1.306 (7.78), 1.407 (0.77), 2.571 (0.64), 2.582 (0.59), 2.602 (0.70), 2.612 (0.69), 2.820 (0.46), 2.846 (0.72), 2.854 (0.60), 2.878 (1.11), 2.900 (1.35), 2.927 (2.28), 2.953 (3.85), 2.977 (1.10), 3.059 (1.67), 3.089 (2.17), 4.250 (2.39), 4.268 (7.38), 4.286 (7.32), 4.303 (2.33), 4.653 (0.65), 4.666 (0.84), 4.679 (1.09), 4.691 (0.92), 4.705 (0.59), 7.471 (1.91), 7.601 (3.73), 7.731 (1.76), 8.066 (5.44).

### Example 121A

### Ethyl 1-[1-{5-chloro-2-[(4-methoxyphenyl)methoxy]phenyl}-5,5-difluoropiperidin-3-yl]-5-(difluoromethyl)-1H-pyrazole-4-carboxylate (Enantiomer 2)

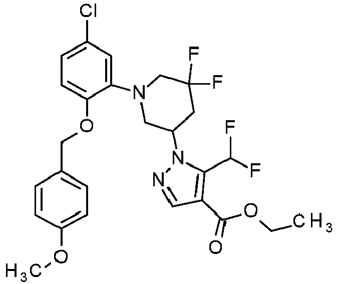

Under argon, a solution of 2-bromo-4-chloro-1-[(4-methoxyphenyl)methoxy]benzene (prepared in analogy to Example 8A, 193 mg, 588 µmol) and ethyl 5-(difluoromethyl)-1-[5,5-difluoropiperidin-3-yl]-1H-pyrazole-4-carboxylate (Example 120A, Enantiomer 2, 182 mg, 588 µmol) in 1,4-dioxane (6.1 ml) was treated with Pd₂dba₃ (269 mg, 294 µmol), rac-BINAP (366 mg, 588 µmol) and caesium carbonate (479 mg, 1.47 mmol). The resulting mixture was stirred overnight at 100°C. Addional 2-bromo-4-chloro-1-[(4-methoxyphenyl)methoxy]benzene (Example 8A, 0.45 mg, 294 µmol), Pd₂dba₃ (107 mg, 117 µmol), rac-BINAP (144 mg, 235 µmol) and caesium carbonate (240 mg, 0.73 mmol) were added to the reaction mixture and the resulting mixture was stirred overnigh at 100°C. The reaction mixture was filtered over Celite, rinsed with ethyl acetate and evaporated. The residue was purified by preparative HPLC (RP18 column, eluent: Acetonitrile/water gradient) affording 128 mg (89 % purity, 35 % yield) of the tilte compound.
LC-MS (Method 3): Rₜ = 2.69 min; MS (ESIpos): m/z = 556 [M+H]⁺

### Example 122A

### Ethyl 1-[1-(5-chloro-2-hydroxyphenyl)-5,5-difluoropiperidin-3-yl]-5-(difluoromethyl)-1H-pyrazole-4-carboxylate (Enantiomer 2)

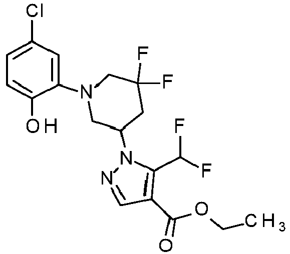

A solution of ethyl 1-[1-{5-chloro-2-[(4-methoxyphenyl)methoxy]phenyl}-5,5-difluoropiperidin-3-yl]-5-(difluoromethyl)-1H-pyrazole-4-carboxylate (Example 121A, Enantiomer 2, 128 mg, 89 % purity, 205 µmol) in dichloromethane (1.4 ml) was treated with trifluoroacetic acid (160 µl, 2.0 mmol) and the resulting mixture was sitrred 2.5 hours at room temperature. The reaction mixture was evaporated and the residue retaken in ethyl acetate. The organic solution was washed once with water, once with a saturated solution of sodium hydrogen carbonate and once with a saturated solution of sodium chloride. The organic phase was dried over sodium sulfate and evaporated. The residue was purified by flash chromatography (silica gel, cyclohexane / ethyl acetate gradient) affording 78 mg (78 % yield) oft he title compound.
LC-MS (Method 3): Rₜ = 2.31 min; MS (ESIpos): m/z = 436 [M+H]⁺

### Example 123A

### Ethyl 1-[1-{5-chloro-2-[(trifluoromethanesulfonyl)oxy]phenyl}-5,5-difluoropiperidin-3-yl]-5-(difluoromethyl)-1H-pyrazole-4-carboxylate (Enantiomer 2)

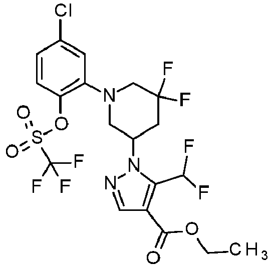

Under argon, a solution of ethyl 1-[1-(5-chloro-2-hydroxyphenyl)-5,5-difluoropiperidin-3-yl]-5-(difluoromethyl)-1H-pyrazole-4-carboxylate (Example 122A, Enantiomer 2, 78.0 mg, 179 µmol) in dichloromethane (1.9 ml) was treated with triethylamine (62 µl, 450 µmol) and cooled to 0°C. Trifluoromethanesulfonic anhydride (36 µl, 210 µmol) and the resulting mixture was stirred 45 minutes at 0°C. The reaction mixture was diluted with dichloromethane and washed three times with water. The organic layer was dried over sodium sulfate and evaporated. The residue was purified by flash chromatography (silica gel, cyclohexane / ethyl acetate gradient) affording 80.0 mg (68 % yield) of the title compound.
LC-MS (Method 3): Rₜ = 2.57 min; MS (ESIpos): m/z = 568 [M+H]⁺

### Example 124A

### Tert-butyl 4-(4'-chloro-2'-{5-[5-(difluoromethyl)-4-(ethoxycarbonyl)-1H-pyrazol-1-yl]-3,3-difluoropiperidin-1-yl}[1,1'-biphenyl]-4-yl)piperazine-1-carboxylate (Enantiomer 2)

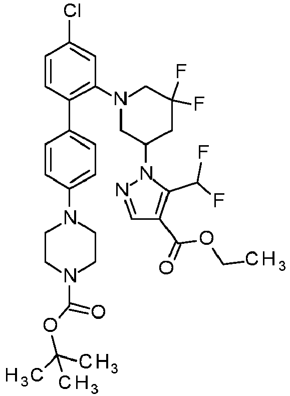

Under argon, a solution of ethyl 1-[1-{5-chloro-2-[(trifluoromethanesulfonyl)oxy]phenyl}-5,5-difluoropiperidin-3-yl]-5-(difluoromethyl)-1H-pyrazole-4-carboxylate (Example 123A, Enantiomer 2, 80.0 mg, 141 µmol) and tert-butyl 4-[4-(4,4,5,5-tetramethyl-1,3,2-dioxaborolan-2-yl)phenyl]piperazine-1-carboxylate (65.6 mg, 169 µmol) in toluene/ethanol mixture (1:1) (1.1 ml) was treated with an aqueous solution of sodium carbonate (210 µl, 2.0 M, 420 µmol) and Pd(PPh₃)₄ (8.14 mg, 7.04 µmol) and stirred 2 hours at 100°C. The reaction mixture was cooled to room temperature, filtered over celite and rinsed with ethyl acetate. The filtrate was evaporated and the residue purified by flash chromatography (silica gel, cyclohexane/ethyl acetate gradient) affording 83.0 mg (74 % yield) of the title compound.
LC-MS (Method 3): Rₜ = 2.99 min; MS (ESIpos): m/z = 680 [M+H]⁺

### Example 125A

### 1-[1-{4'-[4-(Tert-butoxycarbonyl)piperazin-1-yl]-4-chloro[1,1'-biphenyl]-2-yl}-5,5-difluoropiperidin-3-yl]-5-(difluoromethyl)-1H-pyrazole-4-carboxylic acid (Enantiomer 2)

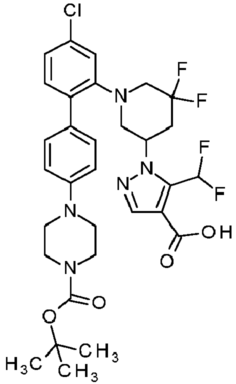

An aqueous solution of lithium hydroxide (1.2 ml, 1.0 M, 1.2 mmol) was added to a solution of tert-butyl 4-(4'-chloro-2'-{5-[5-(difluoromethyl)-4-(ethoxycarbonyl)-1H-pyrazol-1-yl]-3,3-difluoropiperidin-1-yl}[1,1'-biphenyl]-4-yl)piperazine-1-carboxylate (Example 124A, Enantiomer 2, 83.0 mg, 122 µmol) in a THF/methanol mixture 10:1 (2.5 ml). The resulting mixture was stirred 2 hours at room temperature. The reaction mixture was acidified with an aqueous solution of hydrogen chloride (4 N) and purified by preparative HPLC (RP18 column, eluent: Acetonitrile/water gradient) affording 44.0 mg (55 % yield) of the title compound.
LC-MS (Method 3): Rₜ = 2.66 min; MS (ESIpos): m/z = 652 [M+H]⁺

### Example 126A

### 1-{1-[4-Chloro-4'-(piperazin-1-yl)[1,1'-biphenyl]-2-yl]-5,5-difluoropiperidin-3-yl}-5-(difluoromethyl)-1H-pyrazole-4-carboxylic acid hydrochloride (Enantiomer 2)

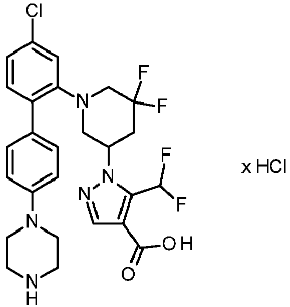

A solution of 1-[1-{4'-[4-(tert-butoxycarbonyl)piperazin-1-yl]-4-chloro[1,1'-biphenyl]-2-yl}-5,5-difluoropiperidin-3-yl]-5-(difluoromethyl)-1H-pyrazole-4-carboxylic acid (Example 125A, Enantiomer 2, 44.0 mg, 67.5 µmol) in dichloromethane (1.4 ml) was treated with a solution of hydrogen chloride in dioxane (170 µl, 4.0 M, 670 µmol) and the resulting mixture was stirred 3 days at room temperature. The reaction mixture was evaporated affordin 50.0 mg (quant.) of the title compound.
LC-MS (Method 3): Rₜ = 1.54 min; MS (ESIpos): m/z = 552 [M+H]⁺

### Example 127A

### 1-{4-[4-(4,4,5,5-Tetramethyl-1,3,2-dioxaborolan-2-yl)phenyl]piperidin-1-yl}propan-1-one

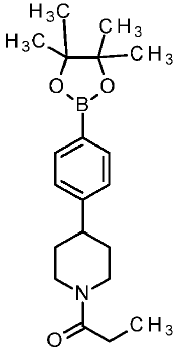

4-[4-(4,4,5,5-tetramethyl-1,3,2-dioxaborolan-2-yl)phenyl]piperidine hydrogen chloride (150 mg, 463 µmol) was dissolved in 4.6 ml dichloromethane under argon. N-ethyl-N-(propan-2-yl)propan-2-amine (400 µl, 2.3 mmol) was added to the mixture and cooled to 0°C. At this temperature propanoic anhydride (65 µl, 510 µmol) was slowly added. After the addition, the mixture was stirred overnight. The reaction solution was diluted with dichloromethane and washed once each with saturated, aqueous sodiumhydrogen carbonate solution and saturated aqueous sodiumchloride solution. The organic phase was dried over sodium sulfate, filtered and evaporated. 154 mg of the target compound (96% of theory) were obtained.
LC-MS (Method 3): Rₜ = 2.13 min; MS (ESIpos): m/z = 344 [M+H]⁺
¹H-NMR (600 MHz, DMSO-d6) δ [ppm]: 0.994 (1.12), 1.006 (2.38), 1.019 (1.15), 1.279 (16.00), 2.333 (0.47), 2.335 (0.48), 2.345 (0.46), 2.348 (0.46), 7.246 (0.94), 7.259 (1.00), 7.600 (1.08), 7.613 (0.96).

### Example 128A

### Ethyl 1-{1-[4-chloro-4'-(1-propanoylpiperidin-4-yl)[1,1'-biphenyl]-2-yl]piperidin-3-yl}-5-(difluoromethyl)-1H-pyrazole-4-carboxylate trifluoroacetic acid (Enantiomer 2)

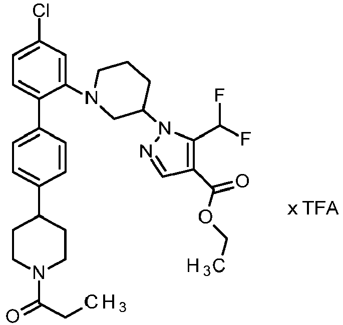

Ethyl 1-[1-{5-chloro-2-[(trifluoromethanesulfonyl)oxy]phenyl}piperidin-3-yl]-5-(difluoromethyl)-1H-pyrazole-4-carboxylate (prepared in analogy to Example 16A, Enantiomer 2, 50.0 mg, 93.1 µmol) and 1-{4-[4-(4,4,5,5-tetramethyl-1,3,2-dioxaborolan-2-yl)phenyl]piperidin-1-yl}propan-1-one (Example 127A, 35.1 mg, 102 µmol) were dissolved in toluene/ethanol (0.52 / 0.52 ml) under argon. Tetrakis(triphenylphosphine)palladium(0) (5.38 mg, 4.65 µmol) and 2 M sodium carbonate solution (140 µl, 280 µmol) were added and the mixture was stirred at 100°C for 2.5 h. The reaction mixture was filtered through a Millipore filter, washed with ethyl acetate and the filtrate was evaporated. The residue was dissolved in acetonitrile/TFA/water and purified by preparative HPLC (RP18 column, acetonitrile/water gradient with addition of 0.1% TFA). 37 mg of the target compound (55% of theory) were obtained.
LC-MS (Method 4): Rₜ = 1.48 min; MS (ESIpos): m/z = 599 [M-TFA+H]⁺

### Example 129A

### Cyclopropyl{4-[4-(4,4,5,5-tetramethyl-1,3,2-dioxaborolan-2-yl)phenyl]piperidin-1-yl}methanone

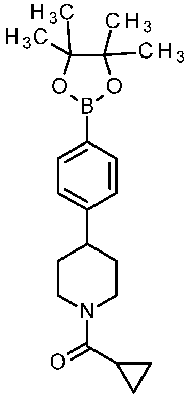

4-[4-(4,4,5,5-tetramethyl-1,3,2-dioxaborolan-2-yl)phenyl]piperidine hydrogen chloride (120 mg, 371 µmol) was dissolved in 3.7 ml dichloromethane under argon. N-ethyl-N-(propan-2-yl)propan-2-amine (320 µl, 1.9 mmol) was added to the mixture and cooled to 0°C. At this temperature cyclopropanecarboxylic anhydride (62.9 mg, 408 µmol) was added. After the addition, the mixture was stirred for 3 h at room temperature. Cyclopropanecarboxylic anhydride (62.9 mg, 408 µmol) and N-ethyl-N-(propan-2-yl)propan-2-amine (130 µl, 1.3 mmol) were added again and the mixture was stirred overnight at room temperature. The reaction solution was diluted with dichloromethane and washed once each with saturated, aqueous sodiumhydrogen carbonate solution and saturated aqueous sodiumchloride solution. The organic phase was dried over sodium sulfate, filtered and evaporated. 123 mg of the target compound (91% of theory) were obtained.
LC-MS (Method 3): Rₜ = 2.19 min; MS (ESIpos): m/z = 356 [M+H]⁺
¹H-NMR (600 MHz, DMSO-d6) δ [ppm]: 0.691 (0.53), 0.704 (0.57), 1.281 (16.00), 7.256 (1.00), 7.270 (1.01), 7.602 (1.06), 7.616 (0.94).

### Example 130A

### Ethyl 1-[1-{4-chloro-4'-[1-(cyclopropanecarbonyl)piperidin-4-yl][1,1'-biphenyl]-2-yl}piperidin-3-yl]-5-(difluoromethyl)-1H-pyrazole-4-carboxylate trifluoroacetic acid (Enantiomer 2)

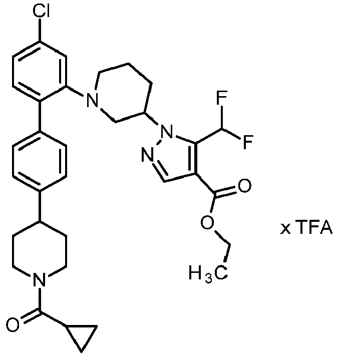

Ethyl 1-[1-{5-chloro-2-[(trifluoromethanesulfonyl)oxy]phenyl}piperidin-3-yl]-5-(difluoromethyl)-1H-pyrazole-4-carboxylate (prepared in analogy to Example 18A, Enantiomer 2, 50.0 mg, 93.1 µmol) and cyclopropyl{4-[4-(4,4,5,5-tetramethyl-1,3,2-dioxaborolan-2-yl)phenyl]piperidin-1-yl}methanone (Example 129A, 36.4 mg, 102 µmol) were dissolved under argon in toluene/ethanol (0.52/0.52 ml). Tetrakis(triphenylphosphine)palladium(0) (5.38 mg, 4.65 µmol) and 2 M sodium carbonate solution (140 µl, 280 µmol) were added and stirred at 100°C for 2.5 h. The reaction mixture was filtered through a Millipore filter, washed with ethyl acetate and the filtrate was evaporated. The residue was dissolved in acetonitrile/TFA/water and purified by preparative HPLC (RP18 column, acetonitrile/water gradient with addition of 0.1% TFA). 32 mg of the target compound (47% of theory) were received.
LC-MS (Method 4): Rₜ = 1.50 min; MS (ESIpos): m/z = 611 [M+H]⁺

### Example 131A

### 3-[4-(4,4,5,5-Tetramethyl-1,3,2-dioxaborolan-2-yl)phenyl]azetidine trifluoroacetic acid

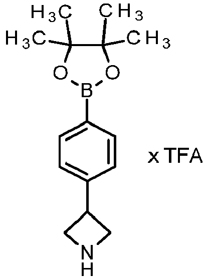

tert-butyl 3-[4-(4,4,5,5-tetramethyl-1,3,2-dioxaborolan-2-yl)phenyl]azetidine-1-carboxylate (100 mg, 278 µmol) was dissolved in 2.2 ml dichloromethane and TFA (540 µl, 7.0 mmol) was added. The reaction solution was stirred for 2 hours at room temperature. The reaction solution was evaporated at 30°C. water bath temperature and dried in a high vacuum. 137 mg of the target compound (quant., purity 75%) were obtained.
LC-MS (Method 3): Rₜ = 1.04 min; MS (ESIpos): m/z = 260 [M-TFA+H]⁺

### Example 132A

### 3-[4-(4,4,5,5-Tetramethyl-1,3,2-dioxaborolan-2-yl)phenyl]-1-(2,2,2-trifluoroethyl)azetidine

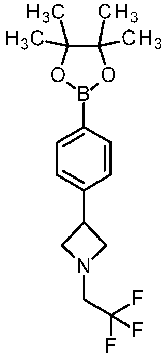

3-[4-(4,4,5,5-tetramethyl-1,3,2-dioxaborolan-2-yl)phenyl]azetidine trifluoroacetic acid (Example 131A, 79.0 mg, purity 75%, 212 µmol) was dissolved in 2.2 ml of DMF. N,N-diisopropylethylamine (110 µl, 660 µmol) and 2,2,2-trifluoroethyl trifluoromethanesulfonate (85 µl, 590 µmol) were added and the mixture was stirred at room temperature for 3 h. The reaction solution was diluted with ethyl acetate and washed twice with saturated aqueous sodium chloride solution. The combined organic phases were dried over sodium sulfate, filtered and evaporated. 80 mg of the target compound (55% of theory, purity ∼50%) were obtained.
LC-MS (Method 3): Rₜ = 2.20 min; MS (ESIpos): m/z = 342 [M+H]⁺

### Example 133A

### 3-[4-(4,4,5,5-Tetramethyl-1,3,2-dioxaborolan-2-yl)phenyl]-1-(3,3,3-trifluoropropyl)azetidine

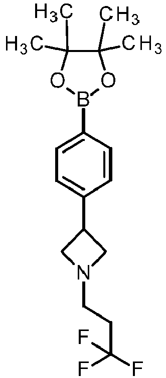

3-[4-(4,4,5,5-tetramethyl-1,3,2-dioxaborolan-2-yl)phenyl]azetidine trifluoroacetic acid (167 mg, 448 µmol) acid (prepared in analogy to Example 131A, 167 mg, 448 µmol) was dissolved in 4.6 ml THF, potassium carbonate (247 mg, 1.79 mmol) and 1,1,1-trifluoro-3-iodopropane (79 µl, 670 µmol) were added and stirred overnight at room temperature. Potassium carbonate (61.8 mg, 448 µmol) and 1,1,1-trifluoro-3-iodopropane (16 µl, 130 µmol) were added and stirring was continued for 2 days. The reaction was stopped here. The reaction mixture was filtered through a Millipore filter, washed with THF and the filtrate was evaporated at 30 °C. The residue was redissolved in 3.8 ml of THF, with potassium carbonate (247 mg, 1.79 mmol) and 1,1,1-trifluoro-3-iodopropane (79 µl, 670 µmol) were added and the mixture was stirred at room temperature for 2 days. 1,1,1-trifluoro-3-iodopropane (26 µl, 220 µmol) and potassium carbonate (61.8 mg, 448 µmol) were added and stirring was continued for 4 h. It was filtered through a Millipore filter, washed with THF and the filtrate was evaporated at 30°C. 153 mg of the target compound (80% of theory, purity 83%) were obtained.
LC-MS (Method 3): Rₜ = 1.30 min; MS (ESIpos): m/z = 356 [M+H]⁺

### Example 134A

### 1-[5-(4,4,5,5-tetramethyl-1,3,2-dioxaborolan-2-yl)pyridin-2-yl]piperazine trifluoroacetatic acid

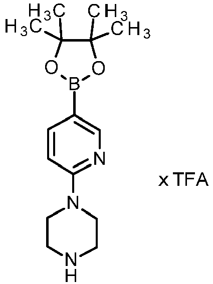

Tert-butyl 4-[5-(4,4,5,5-tetramethyl-1,3,2-dioxaborolan-2-yl)pyridin-2-yl]piperazine-1-carboxylate (500 mg, 1.28 mmol) was dissolved in 10 ml dichloromethane and TFA (2.5 ml, 32 mmol) was added. The reaction solution was stirred at room temperature for 1 h. The reaction solution was evaporated at 30°C and dried in a high vacuum. 994 mg of the target compound (quant., purity ∼80%) were obtained as raw material.
LC-MS (Method 4): Rₜ = 0.54 min; MS (ESIpos): m/z = 290 [M-TFA+H]⁺
¹H-NMR (600 MHz, DMSO-d6) δ [ppm]: 1.070 (0.60), 1.159 (0.96), 1.168 (0.45), 1.277 (16.00), 3.186 (1.09), 3.785 (1.12), 3.794 (1.42), 3.802 (1.03), 6.935 (0.58), 6.950 (0.58), 7.792 (0.49), 7.795 (0.45), 7.806 (0.44), 7.809 (0.42), 8.356 (0.80), 8.358 (0.77).

### Example 135A

### 1-Ethyl-4-[5-(4,4,5,5-tetramethyl-1,3,2-dioxaborolan-2-yl)pyridin-2-yl]piperazine

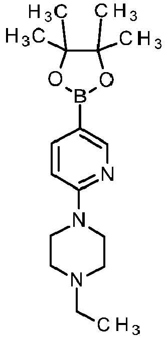

1-[5 -(4,4,5,5-tetramethyl-1,3,2-dioxaborolan-2-yl)pyridin-2-yl]piperazine trifluoroacetic acid (Example 134A; 50.0 mg, 80 % purity, 99.2 µmol) was dissolved in 1.0 THF, potassium carbonate (41.1 mg, 298 µmol) and iodoethane (12 µl, 150 µmol) were added and the mixture was stirred overnight at room temperature. It was filtered through a Millipore filter, washed with THF and the filtrate was evaporated at 30°C. 71 mg of the target compound (90% of theory, purity ∼40%) were obtained.
LC-MS (Method 4): Rₜ = 0.58 min; MS (ESIpos): m/z = 318 [M+H]⁺
¹H-NMR(600 MHz, DMSO-d6) δ [ppm]: 1.012 (0.91), 1.024 (1.94), 1.036 (0.94), 1.070 (1.86), 1.159 (0.62), 1.265 (16.00), 1.356 (5.99), 2.184 (0.84), 2.340 (0.91), 2.352 (0.89), 2.409 (1.21), 2.417 (0.95), 2.738 (0.41), 3.532 (0.77), 3.541 (0.97), 3.549 (0.76), 6.769 (0.46), 6.783 (0.47), 6.871 (0.53), 7.671 (0.48), 7.674 (0.49), 8.318 (0.56), 8.320 (0.57).

### Example 136A

### Ethyl 1-[1-{5-chloro-2-[6-(4-ethylpiperazin-1-yl)pyridin-3-yl]phenyl}pipendin-3-yl]-5-(difluoromethyl)-1H-pyrazole-4-carboxylate (Enantiomer 2)

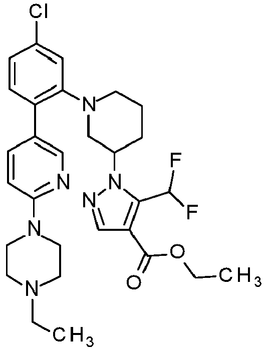

Ethyl 1-[1-{5-chloro-2-[(trifluoromethanesulfonyl)oxy]phenyl}piperidin-3-yl]-5-(difluoromethyl)-1H-pyrazole-4-carboxylate (prepared in analogy to Example 16A, Enantiomer 2, 34.7 mg, 65.1 µmol) and 1-ethyl-4-[5-(4,4,5,5-tetramethyl-1,3,2-dioxaborolan-2-yl)pyridin-2-yl]piperazine (Example 135A, 62.0 mg, 40 % purity, 78.2 µmol) were dissolved under argon in toluene/ethanol (0.36/0.36 ml). Tetrakis(triphenylphosphine)palladium(0) (3.76 mg, 3.26 µmol) and 2 M sodium carbonate solution (98 µl, 200 µmol) were added and stirred at 100°C overnight. The reaction mixture was filtered through a Millipore filter, washed with ethyl acetate and the filtrate was evaporated. The residue was dissolved in acetonitrile/TFA/water and purified by preparative HPLC (RP18 column, acetonitrile/water gradient with addition of 0.1% formic acid). 10 mg of the target compound (25% of theory, purity 95%) were received.
LC-MS (Method 4): Rₜ = 0.97 min; MS (ESIpos): m/z = 573 [M+H]⁺

### Example 137A

### [6-(4-propylpiperazin-1-yl)pyridin-3-yl]boronic acid trifluoroacetic acid

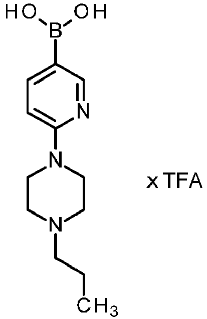

1-[5-(4,4,5,5-tetramethyl-1,3,2-dioxaborolan-2-yl)pyridin-2-yl]piperazine trifluoroacetatic acid (Example 134A, 250 mg, 80 % purity, 496 µmol) was dissolved in 3.0 ml THF and N,N-diisopropylethylamine (260 µl, 1.5 mmol) was added. Propanal (53 µl, 740 µmol) was then added and the mixture was stirred for 10 min. Then sodium triacetoxyborohydride (315 mg, 1.49 mmol) was added and the mixture was stirred at 40°C for 2 h. The mixture was evaporated and dissolved in acetonitrile/TFA and purified by preparative HPLC (RP18 column, acetonitrile/water gradient with addition of 0.1% TFA). 525 mg of the target compound (quant., purity ∼34%) were obtained.
LC-MS (Method 4): Rₜ = 0.14 min; MS (ESIpos): m/z = 250 [M-TFA+H]⁺

### Example 138A

### Ethyl 1-[1-{5-chloro-2-[6-(4-propylpiperazin-1-yl)pyridin-3-yl]phenyl}piperidin-3-yl]-5-(difluoromethyl)-1H-pyrazole-4-carboxylate (Enantiomer 2)

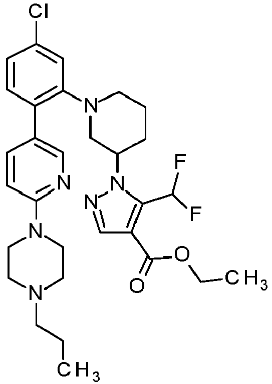

Ethyl 1-[1-{5-chloro-2-[(trifluoromethanesulfonyl)oxy]phenyl}piperidin-3-yl]-5-(difluoromethyl)-1H-pyrazole-4-carboxylate (Example 16A, Enantiomer 2, 74.7 mg, 140 µmol) and [6-(4-propylpiperazin-1-yl)pyridin-3-yl]boronic acid trifluoroacetic acid (Example 137A, 525 mg, 34 % purity, 492 µmol) were dissolved under argon in toluene/ethanol (0.78/0.78 ml). Tetrakis(triphenylphosphine)palladium(0) (8.11 mg, 7.02 µmol) and 2 M sodium carbonate solution (250 µl, 494 µmol) were added and stirred at 100°C for 2 h. The reaction mixture was diluted with ethyl acetate and water. The aqueous phase was acidified with 1 M hydrochloric acid. The phases were separated and the aqueous phase was extracted twice with ethyl acetate. The combined organic phases were dried over sodium sulfate, filtered and evaporated. The residue was purified by means of thick layer chromatography (eluent: dichloromethane/methanol/formic acid: 10/1/0.1). 50 mg of the target compound (47% of theory, purity 78%) were obtained.
LC-MS (Method 3): Rₜ = 2.06 min; MS (ESIpos): m/z = 587 [M+H]⁺
¹H-NMR (600 MHz, DMSO-d6) δ [ppm]: 0.929 (5.74), 0.942 (12.28), 0.954 (5.88), 1.265 (7.80), 1.277 (16.00), 1.288 (7.80), 1.522 (0.88), 1.543 (1.00), 1.736 (1.65), 1.750 (2.64), 1.763 (2.96), 1.776 (2.71), 1.789 (1.22), 1.926 (0.92), 1.947 (1.06), 1.979 (1.52), 2.425 (1.03), 2.575 (0.49), 2.637 (0.88), 2.654 (2.40), 2.674 (0.87), 2.995 (1.41), 3.013 (2.81), 3.031 (2.00), 3.070 (3.77), 3.078 (4.12), 3.097 (3.21), 3.168 (1.55), 3.181 (1.35), 3.299 (1.98), 3.321 (2.05), 3.585 (3.57), 3.604 (3.10), 4.243 (1.92), 4.254 (5.37), 4.266 (5.13), 4.277 (1.68), 4.421 (2.88), 4.440 (3.38), 7.070 (1.49), 7.085 (1.52), 7.154 (5.57), 7.159 (3.40), 7.173 (3.51), 7.176 (2.79), 7.220 (5.17), 7.233 (3.32), 7.439 (1.31), 7.526 (2.75), 7.551 (1.78), 7.563 (1.34), 7.568 (1.41), 7.604 (1.94), 7.615 (2.61), 7.622 (1.98), 7.927 (1.44), 7.943 (1.46), 8.013 (7.38), 8.333 (4.51), 8.336 (4.48), 10.458 (0.71).

### Example 139A

### [6-(4-isobutylpiperazin-1-yl)pyridin-3-yl]boronic acid trifluoroacetatic acid

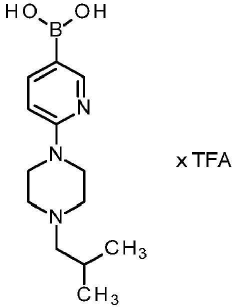

1-[5-(4,4,5,5-tetramethyl-1,3,2-dioxaborolan-2-yl)pyridin-2-yl]piperazine trifluoroacetatic acid (Example 134A, 200 mg, 80 % purity, 397 µmol) was dissolved in 2.4 ml THF and N,N-diisopropylethylamine (210 µl, 1.2 mmol) was added. Then 2-methylpropanal (140 µl, 1.6 mmol) was added and the mixture was stirred for 10 min. Then sodium triacetoxyborohydride (252 mg, 1.19 mmol) was added and the mixture was stirred at 55°C. for 4 h. The reaction mixture was evaporated at 30°C, mixed with acetonitrile, water and a little TFA and purified by means of preparative HPLC (RP18 column, acetonitrile/water gradient with the addition of 0.1% TFA). 382 mg of the target compound (quant., purity ∼ 39%) were obtained.
LC-MS (Method 4): Rₜ = 0.15 min; MS (ESIpos): m/z = 264 [M-TFA+H]⁺

### Example 140A

### {6-[4-(cyclopropylmethyl)piperazin-1-yl]pyridin-3-yl}boronic acid trifluoroacetatic acid

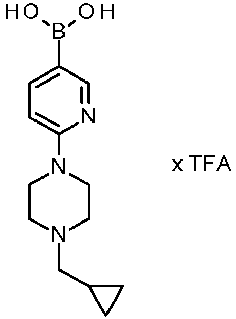

1-[5-(4,4,5,5-tetramethyl-1,3,2-dioxaborolan-2-yl)pyridin-2-yl]piperazine trifluoroacetatic acid (Example 134A 200 mg, 80 % purity, 397 µmol) was dissolved in 2.4 ml THF and N,N-diisopropylethylamine (210 µl, 1.2 mmol) was added. Then cyclopropanecarbaldehyde (120 µl, 1.6 mmol) was added and the mixture was stirred for 10 minutes. Sodium triacetoxyborohydride (252 mg, 1.19 mmol) was added and the mixture was stirred at 55°C for 4 h. The reaction mixture was evaporated at 30°C, dissolved in acetonitrile, water and a little TFA and purified by means of preparative HPLC (RP18 column, acetonitrile/water gradient with the addition of 0.1% TFA). 328 mg of the target compound (quant., purity ∼45%) was obtained.
LC-MS (Method 4): Rₜ = 0.13 min; MS (ESIpos): m/z = 262 [M-TFA+H]⁺

### Example 141A

### Ethyl 1-[1-{5-chloro-2-[6-(piperazin-1-yl)pyridin-3-yl]phenyl}piperidin-3-yl]-5-(difluoromethyl)-1H-pyrazole-4-carboxylate (Enantiomer 1)

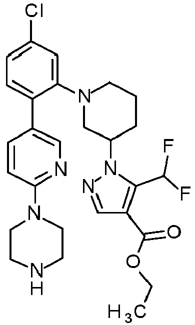

Under argon, a solution of ethyl 1-[1-{5-chloro-2-[(triffuoromethanesulfonyl)oxy]phenyl}piperidin-3-yl]-5-(difluoromethyl)-1H-pyrazole-4-carboxylate (prepared in analogy to Example 11A, Enantiomer 1, 100 mg, 188 µmol) and 1-[5-(4,4,5,5-tetramethyl-1,3,2-dioxaborolan-2-yl)pyridin-2-yl]piperazine (65.2 mg, 226 µmol) in toluene (1.0 ml) and ethanol (1.0 ml) was treated with an aqueous solution of sodium carbonate (280 µl, 2.0 M, 560 µmol) and Pd(PPh₃)₄ (10.9 mg, 9.40 µmol) and stirred 3 hours at 100°C. The reaction mixture was cooled to room temperature, filtered over celite and rinsed with ethyl acetate. The filtrate was evaporated and the residue was purified by preparative HPLC (RP18 column, eluent: Acetonitrile/water gradient) affording 27.0 mg (22 % yield) of the title compound.
LC-MS (Method 3): Rₜ = 1.72 min; MS (ESIpos): m/z = 545 [M+H]⁺

### Example 142A

### 1-[1-{5-Chloro-2-[6-(piperazin-1-yl)pyridin-3-yl]phenyl}piperidin-3-yl]-5-(difluoromethyl)-1H-pyrazole-4-carboxylic acid (Enantiomer 1)

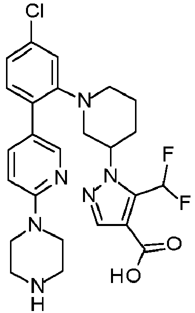

An aqueous solution of lithium hydroxide (500 µl, 1.0 M, 500 µmol) was added to a solution of ethyl 1-[1-{5-chloro-2-[6-(piperazin-1-yl)pyridin-3-yl]phenyl}piperidin-3-yl]-5-(difluoromethyl)-1H-pyrazole-4-carboxylate (Example 141A, Enantiomer 1, 27.0 mg, 49.5 µmol) in a THF/methanol mixture 10:1 (1.1 ml). The resulting mixture was stirred overnight at room temperature. The reaction mixture was acidified with an aqueous solution of hydrogen chloride (4 N) and evaporated. The residue was purified by preparative HPLC (RP18 column, eluent: Acetonitrile/water gradient) affording 14.0 mg (55 % yield) of the title compound.
LC-MS (Method 3): Rₜ = 1.46 min; MS (ESIpos): m/z = 517 [M+H]⁺

### Example 143A

### Ethyl 1-[1-{5-chloro-2-[6-(piperazin-1-yl)pyridin-3-yl]phenyl}piperidin-3-yl]-5-(difluoromethyl)-1H-pyrazole-4-carboxylate (Enantiomer 2)

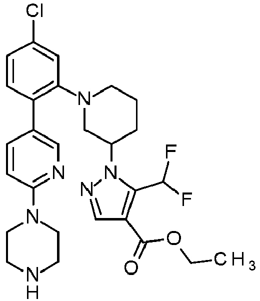

Under argon, a solution of ethyl 1-[1-{5-chloro-2-[(triffuoromethanesulfonyl)oxy]phenyl}piperidin-3-yl]-5-(difluoromethyl)-1H-pyrazole-4-carboxylate (prepared in analogy to Example 16A, Enantiomer 2, 200 mg, 376 µmol) and 1-[5-(4,4,5,5-tetramethyl-1,3,2-dioxaborolan-2-yl)pyridin-2-yl]piperazine (130 mg, 451 µmol) in toluene (2.4 ml) and ethanol (2.4 ml) was treated with an aqueous solution of sodium carbonate (560 µl, 2.0 M, 1.1 mmol) and Pd(PPh₃)₄ (21.7 mg, 18.8 µmol) and stirred 2 hours at 100°C. The reaction mixture was cooled to room temperature, diluted with water, filtered over an EXtrelut NT 3 cartridge and rinsed with ethyl acetate. The filtrate was evaporated and the residue was purified by flash chromatography (silica gel, dichloromethane/methanol gradient) affording 196 mg (96 % yield) of the title compound.
LC-MS (Method 3): Rₜ = 1.81 min; MS (ESIpos): m/z = 545 [M+H]⁺

### Example 144A

### Ethyl 1-[1-(5-chloro-2-{6-[4-(2,2,2-trifluoroethyl)piperazin-1-yl]pyridin-3-yl}phenyl)piperidin-3-yl]-5-(difluoromethyl)-1H-pyrazole-4-carboxylate (Enantiomer 2)

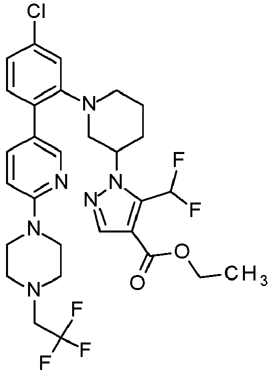

Under argon, a solution of ethyl 1-[1-{5-chloro-2-[6-(piperazin-1-yl)pyridin-3-yl]phenyl}piperidin-3-yl]-5-(difluoromethyl)-1H-pyrazole-4-carboxylate (Example 143A, Enantiomer 2, 196 mg, 360 µmol) in DMF (3.2 ml) was treated with N,N-diisopropylethylamine (190 µl, 1.1 mmol) and 2,2,2-trifluoroethyl trifluoromethanesulfonate (160 µl, 1.1 mmol). The resulting mixture was stirred 2 hours at room temperature and diluted with water. The aqueous solution was extracted three times with ethyl acetate. The combined organic layers were washed with a saturated solution of sodium chloride, dried over sodium sulfate and evaporated affording 189 mg (77 % yield) of the title compound which was used in the next steps without further purification.
LC-MS (Method 3): Rₜ = 2.81 min; MS (ESIpos): m/z = 627 [M+H]⁺

### Example 145A

### {6-[4-(3,3,3-trifluoropropyl)piperazin-1-yl]pyridin-3-yl}boronic acid

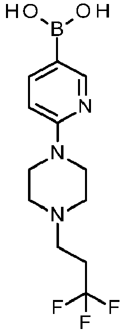

1-[5-(4,4,5,5-tetramethyl-1,3,2-dioxaborolan-2-yl)pyridin-2-yl]piperazine trifluoroacetic acid (prepared in analogy to Example 134A, 398 mg, 47 % purity, 464 µmol) was dissolved in 4.8 ml THF, Potassium carbonate (385 mg, 2.78 mmol) and 1,1,1-trifluoro-3-iodopropane (82 µl, 700 µmol) were added and te mixture was stirred overnight at room temperature. The mixture was transferred to a microwave vial. Caesium carbonate (302 mg, 928 µmol) and 1,1,1-trifluoro-3-iodopropane (82 µl, 700 µmol) were added and the mixture was stirred in the microwave at 100°C for 1 h. Another 1,1,1-trifluoro-3-iodopropane (82 µl, 700 µmol) was added to the reaction mixture and the mixture was stirred in the microwave at 100°C. for 2 h. Another 1,1,1-trifluoro-3-iodopropane (82 µl, 700 µmol) was added and the mixture was stirred again in the microwave at 100°C for 2 h. The mixture was filtered off, washed well with THF and the filtrate was evaporated. It was then purified by means of preparative HPLC (RP18 column, acetonitrile/water gradient). 156 mg of the target compound (92% of theory, purity ∼ 83%) were obtained.
LCMS (Method 7): Rₜ = 1.01 min; MS (ESIpos): m/z = 304 [M+H]⁺
¹H-NMR (600 MHz, DMSO-d6) δ [ppm]: 1.070 (2.21), 1.277 (1.39), 2.387 (0.83), 2.425 (1.05), 2.615 (0.83), 2.654 (0.97), 2.847 (4.85), 2.865 (7.37), 2.875 (8.05), 2.892 (5.81), 4.302 (2.81), 6.771 (0.46), 6.780 (0.52), 6.885 (0.73), 6.899 (0.80), 7.019 (6.80), 7.033 (6.57), 7.152 (0.53), 7.652 (0.55), 7.767 (1.08), 7.772 (1.08), 7.993 (8.97), 8.005 (8.67), 8.162 (1.21), 8.374 (0.61), 8.470 (16.00).

### Example 146A

### Ethyl 1-[1-(5-chloro-2-{6-[4-(3,3,3-trifluoropropyl)piperazin-1-yl]pyridin-3-yl}phenyl)piperidin-3-yl]-5-(difluoromethyl)-1H-pyrazole-4-carboxylate trifluoroacetic acid (Enantiomer 1)

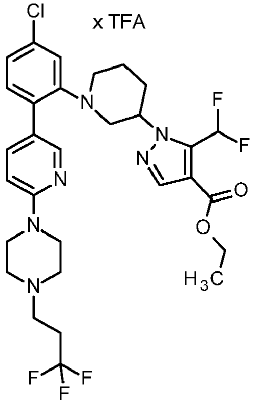

Ethyl 1-[1-{5-chloro-2-[(trifluoromethanesulfonyl)oxy]phenyl}piperidin-3-yl]-5-(difluoromethyl)-1H-pyrazole-4-carboxylate (prepared in analogy to Example 11A, Enantiomer 1, 60.0 mg, 94 % purity, 106 µmol) and {6-[4-(3,3,3-trifluoropropyl)piperazin-1-yl]pyridin-3-yl}boronic acid (Example 145A, 42.6 mg, 83 % purity, 117 µmol) were dissolved under argon in toluene/ethanol (0.59/0.59 ml). Tetrakis(triphenylphosphine)palladium(0) (6.13 mg, 5.30 µmol) and 2 M sodium carbonate solution (160 µl, 320 µmol) were added and stirred at 100°C for 2.5 h. The reaction mixture was filtered through a Millipore filter, washed with ethyl acetate and the filtrate was evaporated. The residue was dissolved in acetonitrile/TFA/water and purified by preparative HPLC (RP18 column, acetonitrile/water gradient with addition of 0.1% TFA). 35 mg of the target compound (44% of theory) were received.
LC-MS (Method 3): Rₜ = 2.29 min; MS (ESIpos): m/z = 641 [M-TFA+H]⁺

### Example 147A

### Ethyl 1-[1-(5-chloro-2-{6-[4-(3,3,3-trifluoropropyl)piperazin-1-yl]pyridin-3-yl}phenyl)piperidin-3-yl]-5-(difluoromethyl)-1H-pyrazole-4-carboxylate trifluoroacetic acid (Enantiomer 2)

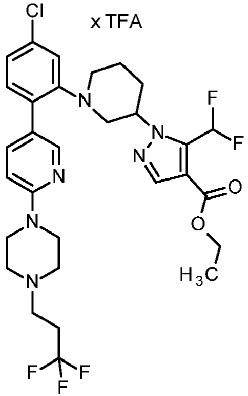

Ethyl 1-[1-{5-chloro-2-[(trifluoromethanesulfonyl)oxy]phenyl}piperidin-3-yl]-5-(difluoromethyl)-1H-pyrazole-4-carboxylate (prepared in analogy to Example 16A, Enantiomer 2, 60.0 mg, 94 % purity, 106 µmol) and {6-[ 4-(3,3,3-trifluoropropyl)piperazin-1-yl]pyridin-3-yl}boronic acid (Example 145A, 44.9 mg, 83 % purity, 123 µmol) were dissolved under argon in toluene/ethanol (0.62/0.62 ml). Tetrakis(triphenylphosphine)palladium(0) (6.45 mg, 5.7 µmol) and 2 M sodium carbonate solution (168 µl, 336 µmol) were added and stirred at 100°C for 2.5 h. The reaction mixture was filtered through a Millipore filter, washed with ethyl acetate and the filtrate was evaporated. The residue was dissolved in acetonitrile/TFA/water and purified by preparative HPLC (RP18 column, acetonitrile/water gradient with addition of 0.1% TFA). 28 mg of the target compound (33% of theory) were received.
LC-MS (Method 3): Rₜ = 2.29 min; MS (ESIpos): m/z = 641 [M-TFA+H]⁺

### Example 148A

### 1-[4-Methyl-5-(4,4,5,5-tetramethyl-1,3,2-dioxaborolan-2-yl)pyridin-2-yl]piperazine trifluoroacetic acid

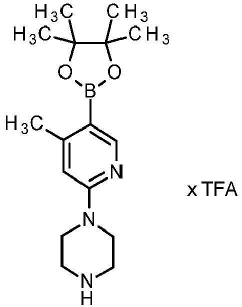

Tert-butyl 4-[4-methyl-5-(4,4,5,5-tetramemyl-1,3,2-dioxaborolan-2-yl)pyridin-2-yl]piperazine-1-carboxylate (100 mg, 248 µmol) was dissolved in 1.9 ml dichloromethane and TFA (480 µl, 6.2 mmol) was added and the mixture was stirred for 1 h at room temperature. The reaction solution was evaporated, dissolved twice in dichloromethane and evaporated again. The residue was then dissolved in acetonitrile/water and lyophilized. 249 mg of the target compound (quant., purity ∼41%) were obtained.
LC-MS (Method 3): Rₜ = 1.11 min; MS (ESIpos): m/z = 304 [M-TFA+H]⁺
¹H-NMR (600 MHz, DMSO-d6) δ [ppm]: 1.071 (1.00), 1.280 (16.00), 2.401 (3.96), 3.188 (1.12), 3.783 (1.15), 3.792 (1.49), 3.800 (1.05), 6.826 (0.87), 8.258 (1.38).

### Example 149A

### {4-methyl-6-[4-(2,2,2-trifluoroethyl)piperazin-1-yl]pyridin-3-yl}boronic acid

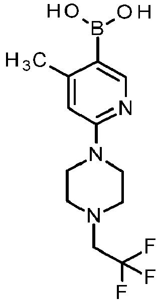

1-[4-Methyl-5-(4,4,5,5-tetramethyl-1,3,2-dioxaborolan-2-yl)pyridin-2-yl]piperazine trifluoroacetic acid (Example 148A, 200 mg, 41 % purity, 201 µmol) was dissolved in 2.1 ml of DMF. N,N-diisopropylethylamine (250 µl, 1.4 mmol and 2,2,2-trifluoroethyl trifluoromethanesulfonate (140 µl, 1.0 mmol) were added and the mixture was stirred at room temperature overnight. The reaction solution was treated with acetonitrile/water/formic acid and purified directly by preparative HPLC (RP18 column, acetonitrile/water gradient with addition of 0.05% formic acid). 28 mg of the target compound (37% of theory, purity ∼80%) were obtained.
LC-MS (Method 3): Rₜ = 0.86 min; MS (ESIpos): m/z = 304 [M+H]⁺
¹H-NMR (600 MHz, DMSO-d6) δ [ppm]: 0.005 (1.49), 1.279 (15.84), 2.386 (0.64), 2.399 (2.10), 2.424 (0.47), 2.465 (1.05), 2.486 (16.00), 2.614 (2.76), 2.653 (0.44), 2.716 (1.22), 2.765 (6.12), 2.774 (7.71), 2.782 (5.71), 3.247 (0.80), 3.268 (2.17), 3.285 (4.83), 3.302 (4.59), 3.319 (2.50), 3.636 (5.96), 3.644 (7.13), 3.652 (5.13), 3.745 (1.51), 7.161 (0.90), 7.986 (3.51), 8.135 (0.50), 8.312 (0.92).

### Example 150A

### 2-(Piperazin-1-yl)-5-(4,4,5,5-tetramethyl-1,3,2-dioxaborolan-2-yl)pyrimidine trifluoroacetic acid

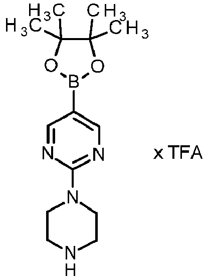

tert-butyl 4-[5-(4,4,5,5-tetramethyl-1,3,2-dioxaborolan-2-yl)pyrimidin-2-yl]piperazine-1-carboxylate (300 mg, 769 µmol) was dissolved in 6.0 ml dichloromethane and trifluoroacetic acid (1.5 ml, 19 mmol) was added. The reaction solution was stirred at room temperature for 1 h. The reaction solution was evaporated, redissolved in dichloromethane and evaporated again (twice). The residue was then dissolved in acetonitrile/water and lyophilized. 600 mg of the target compound (quant., purity ∼51%) were obtained.
LC-MS (Method 7): Rₜ = 0.72 min; MS (ESIpos): m/z = 291 [M+H]⁺
¹H-NMR (600 MHz, DMSO-d6) δ [ppm]: 1.279 (16.00), 3.173 (0.99), 3.984 (1.10), 3.993 (1.40), 4.002 (1.05), 8.547 (3.94).

### Example 151A

### {2-[4-(2-methylpropyl)piperazin-1-yl]pyrimidin-5-yl}boronic acid trifluoroacetic acid

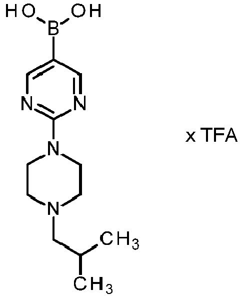

2-(Piperazin-1-yl)-5-(4,4,5,5-tetramethyl-1,3,2-dioxaborolan-2-yl)pyrimidine trifluoroacetic acid (Example 150A, 300 mg, 51 % purity, 379 µmol) was dissolved in 2.3 ml THF and N,N-diisopropylethylamine (99 µl, 570 µmol) was added. Then 2-methylpropanal (140 µl, 1.5 mmol) was added and the mixture was stirred for 10 min. Sodium triacetoxyborohydride (241 mg, 1.14 mmol) was added and the mixture was stirred at 55°C. for 3 h. The reaction solution was mixed with acetonitrile/water/a little trifluoroacetic acid and purified directly by preparative HPLC (RP18 column, acetonitrile/water gradient with addition of 0.1% trifluoroacetic acid). 650 mg of the product (quant., purity ∼22%) were obtained.
LC-MS (Method 7): Rₜ = 0.86 min; MS (ESIpos): m/z = 265 [M-TFA+H]⁺
¹H-NMR(600 MHz, DMSO-d6) δ [ppm]: 0.968 (3.45), 0.979 (3.45), 1.070 (1.17), 1.159 (8.67), 1.236 (3.14), 1.250 (15.25), 1.261 (16.00), 1.266 (12.42), 1.277 (12.04), 1.284 (4.32), 2.985 (0.53), 3.118 (0.55), 3.125 (0.66), 3.130 (1.60), 3.137 (1.65), 3.142 (1.62), 3.149 (1.56), 3.154 (0.60), 3.161 (0.51), 3.384 (0.43), 3.551 (0.49), 3.571 (0.49), 3.598 (0.67), 3.609 (1.25), 3.616 (1.34), 3.620 (1.60), 3.627 (1.61), 3.631 (1.31), 3.638 (1.26), 3.649 (0.70), 3.875 (0.86), 3.885 (0.90), 3.894 (0.83), 8.560 (0.97), 8.686 (2.36).

### Example 152A

### Ethyl 1-[1-{5-chloro-2-[2-(4-methylpiperazin-1-yl)pyrimidin-5-yl]phenyl}piperidin-3-yl]-5-(difluoromethyl)-1H-pyrazole-4-carboxylate (Enantiomer 2)

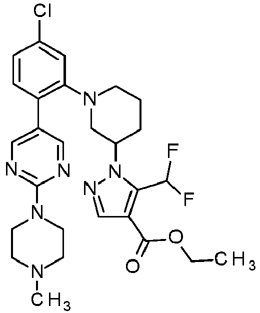

Ethyl 1-[1-{5-chloro-2-[(trifluoromethanesulfonyl)oxy]phenyl}piperidin-3-yl]-5-(difluoromethyl)-1H-pyrazole-4-carboxylate (prepared in analogy to Example 16A, Enantiomer 2, 100 mg, 188 µmol) and 2-(4-methylpiperazin-1-yl)-5-(4,4,5,5-tetramethyl-1,3,2-dioxaborolan-2-yl)pyrimidine (68.6 mg, 226 µmol) were dissolved under argon in toluene/ethanol (2.0/2.0 ml). Tetrakis(triphenylphosphine)palladium(0) (10.9 mg, 9.40 µmol) and 2 M sodium carbonate solution (280 µl, 560 µmol) were added and stirred at 100°C overnight. The reaction mixture was filtered through a Millipore filter, washed with ethyl acetate and the filtrate was evaporated. The residue was dissolved in acetonitrile/formic acid/water and purified by preparative HPLC (RP18 column, acetonitrile/water gradient with addition of 0.1% formic acid). The product fractions were combined and evaporated. The raw material was purified by thick layer chromatography (eluent: dichloromethane/methanol: 10/1). 64 mg of the target compound (61% of theory) were received.
LC-MS (Method 4): Rₜ = 0.95 min; MS (ESIpos): m/z = 560 [M+H]⁺

### Example 153A

### {2-[4-(cyclopropylmethyl)piperazin-1-yl]pyrimidin-5-yl}boronic acid trifluoroacetic acid

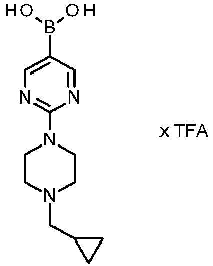

2-(Piperazin-1-yl)-5-(4,4,5,5-tetramethyl-1,3,2-dioxaborolan-2-yl)pyrimidine trifluoroacetic acid (Example 150A, 300 mg, 51 % purity, 379 µmol) was dissolved in 2.3 ml THF and N,N-diisopropylethylamine (99 µl, 570 µmol) was added. Then cyclopropanecarbaldehyde (110 µl, 1.5 mmol) was added and the mixture was stirred for 10 min. Then sodium triacetoxyborohydride (241 mg, 1.14 mmol) was added and the mixture was stirred for 3 h at 55°C. The reaction solution was mixed with acetonitrile/water/a little trifluoroacetic acid and purified directly by preparative HPLC (RP18 column, acetonitrile/water gradient with addition of 0,1% trifluoroacetic acid). 690 mg of the product (97% of theory, purity ∼20%) were obtained.
LC-MS (Method 7): Rₜ = 0.66 min; MS (ESIpos): m/z = 263 [M-TFA+H]⁺
¹H-NMR (600 MHz, DMSO-d6) δ [ppm]: 0.371 (0.79), 0.379 (0.79), 0.649 (0.65), 0.662 (0.66), 1.069 (1.55), 1.158 (11.82), 1.234 (3.13), 1.247 (15.22), 1.259 (16.00), 1.263 (12.76), 1.275 (11.95), 1.284 (2.71), 3.049 (0.81), 3.119 (0.52), 3.131 (1.55), 3.138 (1.59), 3.143 (1.57), 3.150 (1.52), 3.162 (0.50), 3.311 (0.41), 3.597 (0.54), 3.608 (1.40), 3.615 (1.49), 3.619 (1.62), 3.626 (1.66), 3.630 (1.45), 3.637 (1.31), 3.648 (0.53), 4.018 (0.47), 4.782 (0.44), 4.805 (0.42), 8.563 (0.51), 8.691 (2.51).

### Example 154A

### 2-Bromo-1-[(4-methoxyphenyl)methoxy]-4-(trifluoromethyl)benzene

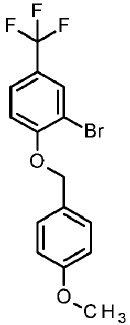

Under argon, a solution of 1-(chloromethyl)-4-methoxybenzene (470 µl, 3.5 mmol) in DMF (7 ml) was treated with potassium carbonate (956 mg, 6.92 mmol), potassium iodide (861 mg, 5.19 mmol) and 2-bromo-4-(trifluoromethyl)phenol (1.00 g, 4.15 mmol) and stirred overnight at 60°C. The reaction mixture was diluted with water and extracted three times with ethyl acetate. The combined organic layers were washed with a saturated solution of sodium hydrogencarbonate and once with a saturated solution of sodium chloride, dried over sodium sulfate and evaporated. The residue was purified by flash chromatography (silica gel, cyclohexane/ethyl acetate gradient) affording 1.20 g (95 % yield) of the title compound.
¹H-NMR (600 MHz, DMSO-d6) δ [ppm]: 3.764 (16.00), 5.229 (7.29), 6.965 (3.58), 6.979 (3.66), 7.380 (1.66), 7.394 (1.84), 7.407 (3.43), 7.421 (3.10), 7.718 (1.10), 7.721 (1.07), 7.732 (0.99), 7.735 (0.96), 7.947 (2.07), 7.951 (1.91).

### Example 155A

### Ethyl 5-(difluoromethyl)-1-[(3R*)-1-{2-[(4-methoxyphenyl)methoxy]-5-(trifluoromethyl)phenyl}piperidin-3-yl]-1H-pyrazole-4-carboxylate (Enantiomer 1)

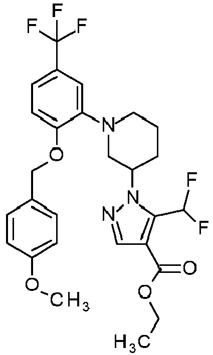

Under argon, a solution of 2-chloro-1-[(4-methoxyphenyl)methoxy]-4-(trifluoromethyl)benzene (Example 154A, 260 mg, 821 µmol) and ethyl 5-(difluoromethyl)-1-[piperidin-3-yl]-1H-pyrazole-4-carboxylate (prepared in analogy to Example 11A, Enantiomer 1, 224 mg, 821 µmol) in 1,4-dioxane (8.0 ml) was treated with caesium carbonate (401 mg, 1.23 mmol), Pd₂dba₃ (75.2 mg, 82.1 µmol) and rac-BINAP (102 mg, 164 µmol) and the resulting mixture was stirred overnight at 100°C. The reaction mixture was diluted with ethyl acetate and water and filtered over an EXtrelut NT 3 cartridge. The cartridge was washed with ethyl acetate and the filtrate was evaporated. The residue was purified by flash chromatography (silica gel, cyclohexane / ethyl acetate gradient) affording 263 mg (82 % purity, 48 % yield) of the title compound.
LC-MS (Method 4): Rₜ = 1.47 min; MS (ESIpos): m/z = 554 [M+H]⁺

### Example 156A

### Ethyl 5-(difluoromethyl)-1-{1-[2-hydroxy-5-(trifluoromethyl)phenyl]piperidin-3-yl}-1H-pyrazole-4-carboxylate (Enantiomer 1)

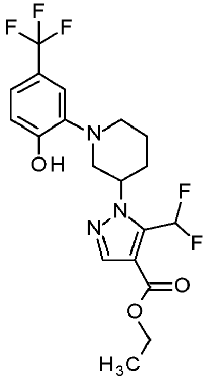

A solution of ethyl 5-(difluoromethyl)-1-[1-{2-[(4-methoxyphenyl)methoxy]-5-(trifluoromethyl)phenyl}piperidin-3-yl]-1H-pyrazole-4-carboxylate (Example 155A, 408 mg, 83 % purity, 610 µmol) in dichloromethane (16 ml) was treated with trifluoroacetic acid (470 µl, 6.1 mmol) and stirred overnight at room temperature. The reaction mixture was diluted with dichloromethane, washed twice with water and once with a saturated solution of sodium chloride. The organic layer was dried over sodium sulfate and evaporated. The residue was purified by flash chromatography (silica gel, cyclohexane / ethyl acetate gradient) affording 350 mg (75 % purity, quant.) of the title compound.
LC-MS (Method 3): Rₜ = 2.45 min; MS (ESIpos): m/z = 434 [M+H]⁺

### Example 157A

### Ethyl 5 -(difluoromethyl)-1-[1-{2-[(trifluoromethanesulfonyl)oxy]-5-(trifluoromethyl)phenyl}piperidin-3-yl]-1H-pyrazole-4-carboxylate (Enantiomer 1)

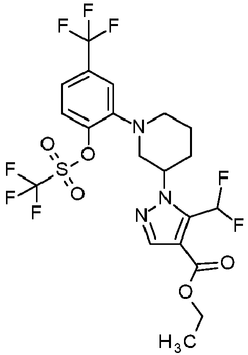

A solution of ethyl 5-(difluoromethyl)-1-{1-[2-hydroxy-5-(trifluoromethyl)phenyl]piperidin-3-yl}-1H-pyrazole-4-carboxylate (Example 156A, 350 mg, 75 % purity, 609 µmol) in dichloromethane (6.1 ml) was treated with triethylamine (210 µl, 1.5 mmol) and cooled to 0°C. Trifluoroacetic acid anhydride (120 µl, 730 µmol) was added dropwise and the resulting mixture was stirred 1 hour at 0°C. The reaction mixture was diluted with dichloromethane (30 ml) and washed three times with water. The organic phase was dried over sodium sulfate and evaporated. The residue was purified by flash chromatography (silica gel, cyclohexane / ethyl acetate) affording 327 mg (91 % yield) of the title compound.
LC-MS (Method 3): Rₜ = 2.72 min; MS (ESIpos): m/z = 566 [M+H]⁺

### Example 158A

### Ethyl 5-(difluoromethyl)-1-{1-[4'-(4-ethylpiperazin-1-yl)-4-(trifluoromethyl)[1,1'-biphenyl]-2-yl]piperidin-3-yl}-1H-pyrazole-4-carboxylate (Enantiomer 1)

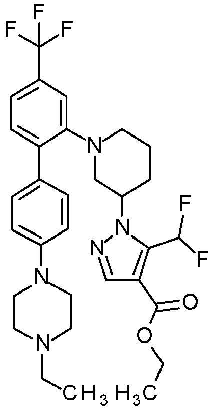

Under argon, a solution of ethyl 5-(difluoromethyl)-1-[1-{2-[(trifluoromethanesulfonyl)oxy]-5-(trifluoromethyl)phenyl}piperidin-3-yl]-1H-pyrazole-4-carboxylate (Example 157A, 80.0 mg, 136 µmol) and 1-ethyl-4-[4-(4,4,5,5-tetramethyl-1,3,2-dioxaborolan-2-yl)phenyl]piperazine (85.9 mg, 272 µmol) in toluene (670 µl) and ethanol (670 µl) was treated with Pd(PPh₃)₄ (7.85 mg, 6.79 µmol) and an aqueous solution of sodium carbonate (200 µl, 2.0 M, 410 µmol). The resulting mixture was stirred overnight at 100°C, cooled to room temperature and acidified with formic acid. The reaction mixture was filtered over an EXtrelut NT 3 cartridge. The cartrige was rinsed with ethyl acetate and the filtrate evaporated. The residue was purified by preparative HPLC (RP18 column, eluent: Acetonitrile/water + 0.1% formic acid gradient) affording 45.0 mg (55 % yield) of the title compound.
LC-MS (Method 3): Rₜ = 2.35 min; MS (ESIpos): m/z = 606 [M+H]⁺

### Example 159A

### Ethyl 5-(difluoromethyl)-1-[1-{2-[(4-methoxyphenyl)methoxy]-5-(trifluoromethyl)phenyl} piperidin-3-yl]-1H-pyrazole-4-carboxylate (Enantiomer 2)

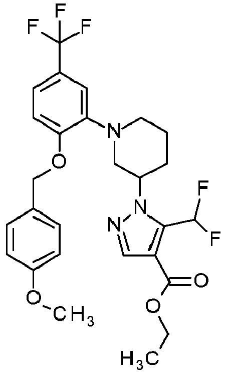

Under argon, a solution of 2-chloro-1-[(4-methoxyphenyl)methoxy]-4-(trifluoromethyl)benzene (Example 154A, 400 mg, 1.26 mmol) and ethyl 5-(difluoromethyl)-1-[piperidin-3-yl]-1H-pyrazole-4-carboxylate (prepared in analogy to Example 16A, Enantiomer 2, 345 mg, 1.26 mmol) in 1,4-dioxane (12 ml) was treated with caesium carbonate (617 mg, 1.89 mmol), Pd₂dba₃ (116 mg, 126 µmol) and rac-BINAP (157 mg, 253 µmol) and the resulting mixture was stirred overnight at 100°C. Additional caesium carbonate (205 mg, 0.63 mmol), Pd₂dba₃ (39 mg, 42 µmol) and rac-BINAP (52 mg, 84 µmol) were added and the resulting mixture was stirred overnight at 100°C. The reaction mixture was filtered over an celite, rinsed with ethyl acetate and the filtrate was evaporated. The residue was purified by flash chromatography (silica gel, cyclohexane / ethyl acetate gradient) affording 403 mg (72 % purity, 42 % yield) of the title compound.
LC-MS (Method 3): Rₜ = 2.76 min; MS (ESIpos): m/z = 554 [M+H]⁺

### Example 160A

### Ethyl 5-(difluoromethyl)-1-{1-[2-hydroxy-5-(trifluoromethyl)phenyl]piperidin-3-yl}-1H-pyrazole-4-carboxylate (Enantiomer 2)

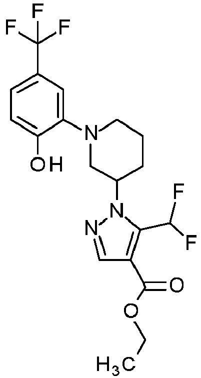

A solution of ethyl 5-(difluoromethyl)-1-[1-{2-[(4-methoxyphenyl)methoxy]-5-(trifluoromethyl)phenyl}piperidin-3-yl]-1H-pyrazole-4-carboxylate (Example 159A, Enantiomer 2, 403 mg, 72 % purity, 527 µmol) in dichloromethane (13 ml) was treated with trifluoroacetic acid (410 µl, 5.3 mmol) and stirred 3 days at room temperature. The reaction mixture was diluted with dichloromethane, washed three times with water and once with a saturated solution of sodium chloride. The organic layer was dried over sodium sulfate and evaporated. The residue was purified by flash chromatography (silica gel, cyclohexane / ethyl acetate gradient) affording 426 mg (quant., ∼66 % purity) of the title compound.
LC-MS (Method 3): Rₜ = 2.44 min; MS (ESIpos): m/z = 434 [M+H]⁺

### Example 161A

### Ethyl 5-(difluoromethyl)-1-[1-{2-[(trifluoromethanesulfonyl)oxy]-5-(trifluoromethyl)phenyl}piperidin-3-yl]-1H-pyrazole-4-carboxylate (Enantiomer 2)

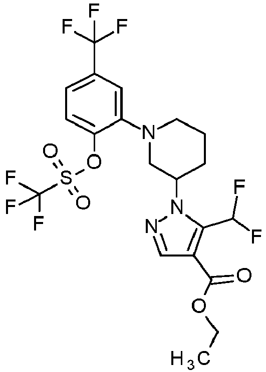

A solution of ethyl 5-(difluoromethyl)-1-{(1-[2-hydroxy-5-(trifluoromethyl)phenyl]piperidin-3-yl}-1H-pyrazole-4-carboxylate (Example 160A, Enantiomer 2, 426 mg, 66 % purity, 644 µmol) in dichloromethane (6.4 ml) was treated with triethylamine (220 µl, 1.6 mmol]) and cooled to 0°C. Trifluoroacetic acid anhydride (130 µl, 770 µmol) was added dropwise and the resulting mixture was stirred 1 hour at 0°C. The reaction mixture was diluted with dichloromethane (30 ml) and washed three times with water. The organic phase was dried over sodium sulfate and evaporated. The residue was purified by flash chromatography (silica gel, cyclohexane / ethyl acetate) affording 241 mg (61 % yield) of the title compound.
LC-MS (Method 3): Rₜ = 2.71 min; MS (ESIpos): m/z = 566 [M+H]⁺

### Example 162A

### Ethyl 5-(difluoromethyl)-1-{1-[4'-(4-ethylpiperazin-1-yl)-4-(trifluoromethyl)[1,1'-biphenyl]-2-yl]piperidin-3-yl}-1H-pyrazole-4-carboxylate (Enantiomer 2)

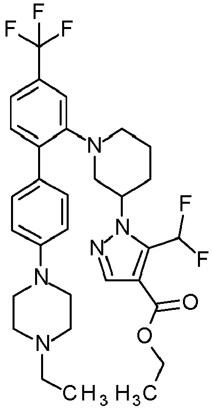

Under argon, a solution of ethyl 5-(difluoromethyl)-1-[1-{2-[(trifluoromethanesulfonyl)oxy]-5-(trifluoromethyl)phenyl}piperidin-3-yl]-1H-pyrazole-4-carboxylate (Example 161A, Enantiomer 2, 80.0 mg, 141 µmol) and 1-ethyl-4-[4-(4,4,5,5-tetramethyl-1,3,2-dioxaborolan-2-yl)phenyl]piperazine (89.5 mg, 283 µmol) in toluene (690 µl) and ethanol (690 µl) was treated with Pd(PPh₃)₄ (8.17 mg, 7.07 µmol) and an aqueous solution of sodium carbonate (210 µl, 2.0 M, 420 µmol). The resulting mixture was stirred overnight at 100°C, cooled to room temperature and acidified with formic acid. The reaction mixture was filtered over an EXtrelut NT 3 cartridge. The cartrige was rinsed with ethyl acetate and the filtrate evaporated. The residue was purified by preparative HPLC (RP18 column, eluent: Acetonitrile/water + 0.1% formic acid gradient) affording 43.0 mg (50 % yield) of the title compound.
LC-MS (Method 3): Rₜ = 1.98 min; MS (ESIpos): m/z = 606 [M+H]⁺

### Example 1

### 1-{1-[4-Chloro-4'-(4-methylpiperazin-1-yl)[biphenyl]-2-yl]piperidin-3-yl}-5-(difluoromethyl)-1H-pyrazole-4-carboxylic acid hydrochloride (Enantiomer 1)

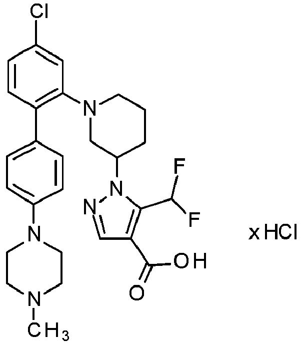

Ethyl 1-[1-{5-chloro-2-[(trifluoromethanesulfonyl)oxy]phenyl}piperidin-3-yl]-5-(difluoromethyl)-1H-pyrazole-4-carboxylate (prepared in analogy to Example 11A, Enantiomer 1, 100 mg, 184 µmol) and [4-(4-methylpiperazin-1-yl)phenyl]boronic acid (48.7 mg, 221 µmol) were dissolved under argon in toluene/ethanol (1.0/1.0 ml). Tetrakis(triphenylphosphine)palladium(0) (10.6 mg, 9.21 µmol) and 2 M sodium carbonate solution (280 µl, 550 µmol) were added and stirred at 100°C overnight. The reaction mixture was filtered through Celite. The filtrate was acidified with 1 M hydrochloric acid. The aqueous reaction solution was extracted with ethyl acetate. The phases were separated and the aqueous phase was extracted three times with ethyl acetate. The organic phase was then dried over sodium sulfate, filtered and evaporated. The residue was dissolved in acetonitrile and a few drops of water and purified by means of prep HPLC (RP18 column, acetonitrile/water gradient with addition of 0.1% formic acid). The product fractions were combined and evaporated. Then the residue was mixed with 0.1 M hydrochloric acid in dioxane, carefully evaporated at 30°C (twice) and then lyophilized. 40 mg of the target compound (37% of theory, purity 97%) were obtained.
LC-MS (Method 3): Rₜ = 1.65 min; MS (ESIpos): m/z = 530 [M-HCl+H]⁺
¹H-NMR (600 MHz, DMSO-d6) δ [ppm]: 1.234(0.52),1.356(0.80),1.470(0.58),1.491(1.51),1.512(1.67), 1.533 (0.69), 1.722 (2.11), 1.743 (1.73), 1.881 (0.53), 1.895 (1.63), 1.902 (1.47), 1.916 (1.72), 1.921 (1.66), 1.936 (0.85), 1.972 (2.20), 1.989 (1.35), 2.426 (0.54), 2.559 (1.65), 2.574 (2.92), 2.594 (1.55), 2.615 (0.44), 2.655 (0.52), 2.832 (16.00), 2.840 (15.51), 3.009 (2.52), 3.027 (5.70), 3.045 (3.93), 3.056 (3.22), 3.078 (3.17), 3.090 (3.19), 3.097 (2.53), 3.109 (2.33), 3.134 (1.84), 3.154 (3.46), 3.170 (3.09), 3.222 (2.44), 3.234 (2.09), 3.390 (0.87), 3.522 (7.04), 3.855 (2.46), 3.879 (3.70), 3.904 (2.30), 4.446 (1.41), 4.464 (2.38), 4.482 (1.28), 7.053 (11.05), 7.067 (11.37), 7.080 (8.26), 7.083 (9.54), 7.107 (4.48), 7.110 (3.38), 7.121 (6.37), 7.124 (5.56), 7.164 (11.21), 7.178 (6.90), 7.438 (2.44), 7.487 (12.67), 7.501 (11.26), 7.525 (4.68), 7.612 (1.92), 7.960 (13.34), 10.566 (1.38).

### Example 2

### 1-{1-[4-Chloro-4'-(4-methylpiperazin-1-yl)[biphenyl]-2-yl]piperidin-3-yl}-5-(difluoromethyl)-1H-pyrazole-4-carboxylic acid hydrochloride (Enantiomer 2)

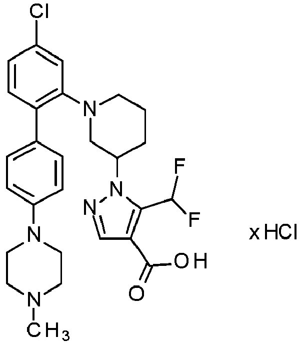

Ethyl 1-[1-{5-chloro-2-[(trifluoromethanesulfonyl)oxy]phenyl}piperidin-3-yl]-5-(difluoromethyl)-1H-pyrazole-4-carboxylate (prepared in analogy to Example 16A, Enantiomer 2, 100 mg, 184 µmol) and [4-(4-methylpiperazin-1-yl)phenyl]boronic acid (48.7 mg, 221 µmol) were dissolved under argon in toluene/ethanol (1.0/1.0 ml). Tetrakis(triphenylphosphine)palladium(0) (10.6 mg, 9.21 µmol) and 2 M sodium carbonate solution (280 µl, 550 µmol) were added and stirred at 100°C overnight. The reaction mixture was filtered through Celite. The filtrate was acidified with 1 M hydrochloric acid. The aqueous reaction solution was extracted with ethyl acetate. The phases were separated and the aqueous phase was extracted three times with ethyl acetate. The organic phase was then dried over sodium sulfate, filtered and evaporated. The residue was dissolved in acetonitrile and a few drops of water and purified by means of prep HPLC (RP18 column, acetonitrile/water gradient with addition of 0.1% formic acid). The product fractions were combined and evaporated. Then the residue was mixed with 0.1 M hydrochloric acid in dioxane, carefully evaporated at 30°C (twice) and then lyophilized. 15 mg of the target compound (14% of theory, purity 94%) were obtained.
LC-MS (Method 8): Rₜ = 0.88 min; MS (ESIpos): m/z = 530 [M-HCl+H]⁺
¹H-NMR(600 MHz, DMSO-d6) δ [ppm]: 1.234 (0.64), 1.356 (1.33), 1.490 (1.65), 1.511 (1.77), 1.720 (2.35), 1.741 (1.92), 1.895 (1.77), 1.915 (1.94), 1.936 (0.94), 1.971 (2.53), 2.386 (0.63), 2.426 (0.59), 2.576 (3.01), 2.593 (1.63), 2.655 (0.52), 2.831 (16.00), 3.009 (2.46), 3.027 (5.97), 3.045 (4.29), 3.096 (3.31), 3.151 (3.54), 3.222 (3.27), 3.234 (3.05), 3.501 (5.28), 3.519 (4.38), 3.853 (2.44), 3.877 (3.65), 3.901 (2.22), 4.447 (1.49), 4.464 (2.45), 4.482 (1.33), 7.052 (9.70), 7.066 (9.98), 7.079 (8.17), 7.082 (8.40), 7.106 (3.76), 7.120 (5.79), 7.123 (5.44), 7.164 (8.57), 7.178 (5.26), 7.210 (0.97), 7.295 (0.85), 7.438 (2.20), 7.486 (10.75), 7.500 (9.57), 7.525 (4.30), 7.612 (1.83), 7.959 (11.21), 10.622 (1.43).

### Example 3

### 1-{1-[4-Chloro-4'-(4-ethylpiperazin-1-yl)[biphenyl]-2-yl]piperidin-3-yl}-5-(difluoromethyl)-1H-pyrazole-4-carboxylic acid hydrochloride (Enantiomer 1)

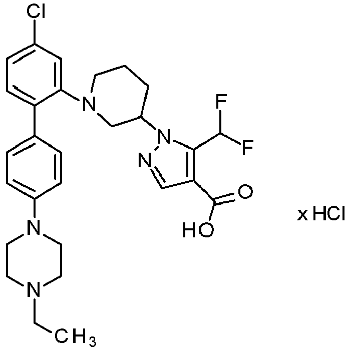

Ethyl 1-{1-[4-chloro-4'-(4-ethylpiperazin-1-yl)[1,1'-biphenyl]-2-yl]piperidin-3-yl}-5-(difluoromethyl)-1H-pyrazole-4-carboxylate (Example 19A, Enantiomer 1, 51.0 mg, 89.1 µmol) was dissolved in THF/ethanol (3.6/0.36 ml). 1 M aqueous lithium hydroxide solution (890 µl, 890 µmol) was added and the mixture was stirred overnight at room temperature. The mixture was evaporatd, then acidified and purified using preparative HPLC (RP18 column, acetonitrile/water gradient with the addition of 0.1% TFA). The product fractions were combined and evaporated. Then the residue was mixed with 1 M hydrochloric acid in dioxane, carefully evaporated at 30°C (thrice) and then lyophilized. 39 mg of the target compound (77% of theory) were obtained.
LC-MS (Method 4): Rₜ = 0.87 min; MS (ESIpos): m/z = 544 [M-HCl+H]⁺
¹H-NMR(400 MHz, DMSO-d6) δ [ppm]: 0.832 (0.51), 0.839 (0.51), 0.851 (0.50), 1.234 (0.56), 1.287 (7.25), 1.305 (16.00), 1.324 (7.61), 1.489 (1.04), 1.520 (1.18), 1.552 (0.52), 1.716 (1.61), 1.749 (1.24), 1.764 (0.99), 1.888 (1.09), 1.898 (1.02), 1.919 (1.26), 1.972 (1.82), 2.075 (2.47), 2.574 (2.16), 2.602 (1.10), 3.002 (1.59), 3.029 (4.63), 3.055 (3.82), 3.084 (2.34), 3.110 (2.80), 3.142 (4.13), 3.156 (4.78), 3.175 (6.43), 3.188 (5.01), 3.215 (2.83), 3.244 (1.53), 3.464 (0.88), 3.475 (1.18), 3.492 (1.25), 3.503 (0.97), 3.550 (3.65), 3.578 (3.72), 3.667 (0.77), 3.681 (1.15), 3.700 (1.15), 3.713 (0.85), 3.843 (2.01), 3.873 (3.81), 3.903 (1.76), 4.445 (0.95), 4.461 (1.16), 4.472 (1.71), 4.483 (1.25), 4.498 (0.90), 5.332 (1.13), 7.055 (7.25), 7.077 (12.72), 7.101 (2.76), 7.122 (4.31), 7.125 (4.17), 7.164 (7.47), 7.184 (3.92), 7.400 (2.01), 7.486 (8.22), 7.508 (7.55), 7.531 (3.92), 7.662 (1.71), 7.957 (8.63), 10.927 (0.76).

### Example 4

### 1-{1-[4-Chloro-4'-(4-ethylpiperazin-1-yl)[biphenyl]-2-yl]piperidin-3-yl}-5-(difluoromethyl)-1H-pyrazole-4-carboxylic acid hydrochloride (Enantiomer 2)

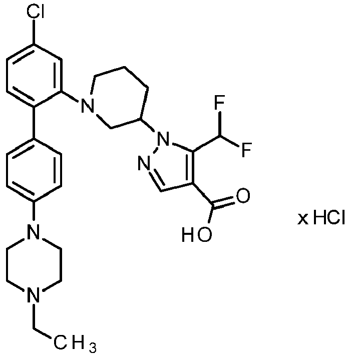

An aqueous solution of lithium hydroxide (3.7 ml, 1.0 M, 3.7 mmol) was added to a solution of ethyl 1-{1-[4-chloro-4'-(4-ethylpiperazin-1-yl)[1,1'-biphenyl]-2-yl]piperidin-3-yl}-5-(difluoromethyl)-1H-pyrazole-4-carboxylate (Example 20A, Enantiomer 2, 236 mg, 90 % purity, 371 µmol) in a THF / methanol mxiture (10/1, 5.5 ml). The resulting mixture was stirred 20 hours at room temperature, treated with an aqueous solution of hydrogen chloride (6 N) and diluted with water (20 ml). The aqueous solution was extracted twice with dichloromethane. The combined organic layers were washed with a saturated solution of sodium chloride, dried over magnesium sulfate and evaporated. The residue was purified by preparative HPLC (RP18 column, eluent: Acetonitrile/water + 0.1% formic acid gradient) affording 135 mg (62 % yield) of the title compound.
LC-MS (Method 3): Rₜ = 1.61 min; MS (ESIpos): m/z = 544 [M-HCl+H]⁺
¹H-NMR (600 MHz, DMSO-d6) δ [ppm]: 1.234 (0.50), 1.306 (7.92), 1.318 (16.00), 1.330 (7.27), 1.478 (0.48), 1.499 (1.26), 1.520 (1.29), 1.542 (0.46), 1.721 (1.75), 1.743 (1.35), 1.880 (0.50), 1.887 (0.53), 1.901 (1.37), 1.908 (1.20), 1.921 (1.44), 1.928 (1.28), 1.942 (0.66), 1.948 (0.54), 1.978 (1.76), 1.995 (0.99), 2.573 (2.13), 2.593 (1.09), 3.018 (2.11), 3.036 (5.46), 3.053 (3.61), 3.074 (1.02), 3.095 (2.56), 3.110 (2.78), 3.131 (1.27), 3.151 (1.28), 3.162 (3.60), 3.171 (4.07), 3.183 (3.41), 3.196 (2.17), 3.208 (1.79), 3.228 (4.31), 3.246 (2.69), 3.549 (4.15), 3.569 (3.70), 3.842 (2.03), 3.867 (3.18), 3.891 (1.79), 4.455 (0.72), 4.462 (1.21), 4.473 (1.34), 4.480 (1.99), 4.486 (1.34), 4.498 (1.09), 4.504 (0.66), 7.059 (7.94), 7.074 (9.43), 7.077 (7.87), 7.080 (7.66), 7.102 (3.30), 7.105 (2.54), 7.116 (4.56), 7.119 (4.06), 7.167 (7.57), 7.180 (5.05), 7.448 (1.88), 7.488 (9.14), 7.503 (8.49), 7.535 (3.78), 7.622 (1.66), 7.954 (10.21), 11.177 (1.03).

### Example 5

### 1-{1-[4-Chloro-4'-(4-propylpiperazin-1-yl)[biphenyl]-2-yl]piperidin-3-yl}-5-(difluoromethyl)-1H-pyrazole-4-carboxylic acid hydrochloride (Enantiomer 2)

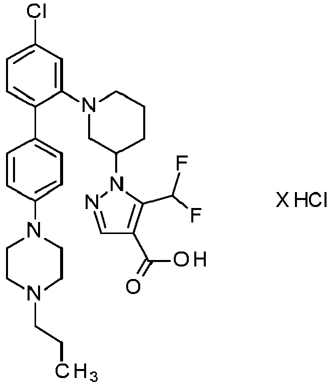

Ethyl 1-{1-[4-chloro-4'-(4-propylpiperazin-1-yl)[1,1'-biphenyl]-2-yl]piperidin-3-yl}-5-(difluoromethyl)-1H-pyrazole-4-carboxylate (prepared in analogy to Example 22A, Enantiomer 2, 97.0 mg, 139 µmol) was dissolved in THF/ethanol (1.9/0.19 ml). 1 M aqueous lithium hydroxide solution (1.4 ml, 1.4 mmol) was added and the mixture was stirred overnight at room temperature. The mixture was evaporated, then acidified and purified using preparative HPLC (RP18 column, acetonitrile/water gradient with the addition of 0.1% TFA). The product fractions were combined and evaporated. Then the residue was mixed with 0.1 M hydrochloric acid in dioxane, carefully evaporated at 30°C (twice) and then lyophilized. 68 mg of the target compound (82% of theory) were obtained.
LC-MS (Method 3): Rₜ = 1.72 min; MS (ESIpos): m/z = 558 [M-HCl+H]⁺
¹H-NMR (600 MHz, DMSO-d6) δ [ppm]: 0.927 (7.59), 0.940 (16.00), 0.952 (7.89), 1.474 (0.44), 1.496 (1.25), 1.517 (1.37), 1.538 (0.56), 1.719 (1.74), 1.741 (1.49), 1.753 (0.97), 1.766 (2.44), 1.779 (3.67), 1.786 (2.49), 1.793 (3.59), 1.806 (2.32), 1.818 (0.65), 1.880 (0.45), 1.886 (0.49), 1.900 (1.35), 1.906 (1.24), 1.921 (1.45), 1.927 (1.37), 1.942 (0.70), 1.977 (1.87), 1.992 (1.13), 2.572 (2.30), 2.592 (1.22), 3.016 (2.00), 3.034 (5.57), 3.052 (5.43), 3.058 (5.00), 3.077 (3.34), 3.086 (2.24), 3.115 (2.60), 3.130 (2.85), 3.150 (1.37), 3.215 (1.95), 3.548 (4.23), 3.569 (5.69), 3.827 (2.12), 3.851 (3.24), 3.876 (1.87), 4.023 (0.52), 4.329 (0.46), 4.459 (1.32), 4.470 (1.47), 4.477 (2.13), 4.484 (1.51), 4.495 (1.26), 4.842 (0.83), 7.053 (8.27), 7.068 (8.76), 7.076 (6.37), 7.079 (6.96), 7.102 (3.18), 7.105 (2.52), 7.115 (4.43), 7.119 (3.97), 7.165 (7.58), 7.179 (4.92), 7.443 (1.92), 7.486 (9.24), 7.500 (8.36), 7.530 (3.79), 7.618 (1.70), 7.952 (9.70), 11.078 (0.84).

### Example 6

### 1-{1-[4-Chloro-4'-(4-isopropylpiperazin-1-yl)[biphenyl]-2-yl]piperidin-3-yl}-5-(difluoromethyl)-1H-pyrazole-4-carboxylic acid (Enantiomer 2)

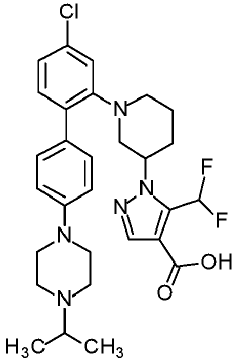

Ethyl 1-[1-{5-chloro-2-[(trifluoromethanesulfonyl)oxy]phenyl}piperidin-3-yl]-5-(difluoromethyl)-1H-pyrazole-4-carboxylate (prepared in analogy to Example 16A, Enantiomer 2, 100 mg, 184 µmol) and 1-isopropyl-4-[4-(4,4,5,5-tetramethyl-1,3,2-dioxaborolan-2-yl)phenyl]piperazine (73.0 mg, 221 µmol) were dissolved under argon in toluene/ethanol (1.0/1.0 ml). Tetrakis(triphenylphosphine)palladium(0) (10.6 mg, 9.21 µmol) and 2 M sodium carbonate solution (280 µl, 556 µmol) were added and stirred at 100°C overnight. The reaction mixture was filtered through a Millipore filter, washed with ethyl acetate and the filtrate was evaporated. The residue was dissolved in acetonitrile/water and purified by preparative HPLC (RP18 column, acetonitrile/water gradient. 12 mg of the target compound (11% of theory, purity 93%) were received.
LC-MS (Method 4): Rₜ = 0.90 min; MS (ESIpos): m/z = 558 [M+H]⁺
¹H-NMR (400 MHz, DMSO-d6) δ [ppm]: -0.008 (2.03), 0.008 (2.30), 1.054 (15.60), 1.070 (16.00), 1.234 (0.61), 1.495 (0.65), 1.527 (0.70), 1.702 (0.95), 1.735 (0.68), 1.876 (0.65), 1.908 (0.72), 1.971 (0.99), 2.327 (0.49), 2.367 (0.68), 2.579 (0.88), 2.701 (4.54), 2.813 (0.86), 2.992 (1.06), 3.017 (2.03), 3.044 (2.29), 3.072 (1.21), 3.208 (5.56), 3.256 (2.43), 3.315 (2.18), 4.472 (0.97), 6.969 (4.43), 6.991 (4.79), 7.054 (3.37), 7.059 (4.12), 7.082 (1.91), 7.087 (1.28), 7.103 (2.99), 7.107 (2.57), 7.150 (5.04), 7.170 (2.70), 7.414 (0.85), 7.439 (5.33), 7.460 (4.72), 7.545 (1.58), 7.676 (0.77), 7.918 (3.74), 8.142 (0.79).

### Example 7

### 1-{1-[4-Chloro-4'-(4-isopropylpiperazin-1-yl)[biphenyl]-2-yl]piperidin-3-yl}-5-(difluoromethyl)-1H-pyrazole-4-carboxylic acid hydrochloride (Enantiomer 2)

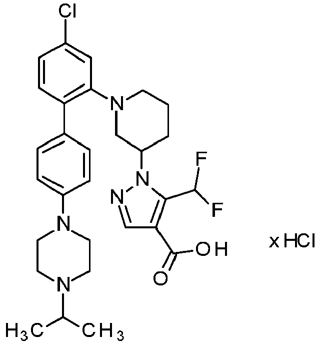

Ethyl 1-[1-{4-chloro-4'-[4-(propan-2-yl)piperazin-1-yl][1,1'-biphenyl]-2-yl}piperidin-3-yl]-5-(difluoromethyl)-1H-pyrazole-4-carboxylate (Example 23A, Enantiomer 2, 20.0 mg, 92 % purity, 31.4 µmol) was dissolved in THF/ethanol (1.3/0.13 ml). 1 M aqueous lithium hydroxide solution (314 µl, 314 µmol) was added and the mixture was stirred overnight at room temperature. The mixture was evaporated, then acidified and purified using preparative HPLC (RP18 column, acetonitrile/water gradient with the addition of 0.1% formic acid). The product fractions were combined and evaporated. Then the residue was mixed with 0.1 M hydrochloric acid in dioxane, carefully evaporated at 30°C (twice) and then lyophilized. 14 mg of the target compound (75% of theory) were obtained.
LC-MS (Method 3): Rₜ = 1.71 min; MS (ESIpos): m/z = 558 [M-HCl+H]⁺
¹H-NMR (400 MHz, DMSO-d6) δ [ppm]: 1.234 (0.57), 1.317 (15.74), 1.334 (16.00), 1.526 (0.72), 1.721 (0.90), 1.756 (0.74), 1.887 (0.63), 1.918 (0.73), 1.971 (1.08), 2.327 (0.45), 2.592 (1.29), 2.618 (0.70), 2.670 (0.52), 2.710 (0.49), 2.990 (0.95), 3.017 (1.98), 3.043 (2.13), 3.070 (1.41), 3.133 (2.31), 3.216 (1.41), 3.236 (1.07), 3.514 (3.07), 3.539 (2.56), 3.874 (0.97), 3.909 (1.50), 3.946 (1.07), 4.459 (0.97), 7.056 (4.54), 7.078 (5.33), 7.083 (4.30), 7.088 (4.66), 7.104 (2.12), 7.124 (3.36), 7.129 (2.86), 7.162 (5.61), 7.182 (2.79), 7.388 (1.18), 7.490 (5.61), 7.512 (5.28), 7.649 (0.93), 7.956 (6.35), 9.966 (0.42).

### Example 8

### 1-{1-[4-Chloro-4'-(4-cyclopropylpiperazin-1-yl)[biphenyl]-2-yl]piperidin-3-yl}-5-(difluoromethyl)-1H-pyrazole-4-carboxylic acid hydrochloride (Enantiomer 1)

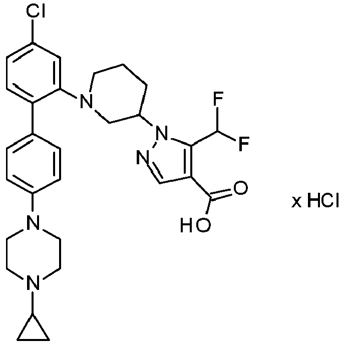

Ethyl 1-{1-[4-chloro-4'-(4-cyclopropylpiperazin-1-yl)[1,1'-biphenyl]-2-yl]piperidin-3-yl}-5-(difluoromethyl)-1H-pyrazole-4-carboxylate trifluoroacetic acid (Example 25A, Enantiomer 1, (21.7 mg, 83 % purity, 25.8 µmol) was dissolved in THF/ethanol (1.0/0.1 ml). 1 M aqueous lithium hydroxide solution (260 µl, 260 µmol) was added and the mixture was stirred overnight at room temperature. The mixture was evaporated, then acidified and purified using preparative HPLC (RP18 column, acetonitrile/water gradient with the addition of 0.1% TFA). The product fractions were combined and evaporated. The raw material was purified by thick layer chromatography (eluent: dichloromethane/methanol/formic acid 10/1/0.1). Then the residue was mixed with 2 M hydrochloric acid in diethyl ether, carefully evaporated at 30°C (twice) and then lyophilized. 9.4 mg of the target compound (57% of theory, purity 92%) were obtained.
LC-MS (Method 4): Rₜ = 0.90 min; MS (ESIpos): m/z = 556 [M-HCl+H]⁺
¹H-NMR(400 MHz, DMSO-d6) δ [ppm]: 0.823 (6.11), 0.840 (6.54), 1.164 (6.23), 1.520 (1.97), 1.718 (2.65), 1.924 (2.25), 1.972 (3.10), 2.060 (1.69), 2.074 (5.21), 2.328 (2.87), 2.343 (3.49), 2.359 (1.75), 2.928 (2.37), 2.999 (2.14), 3.027 (6.06), 3.053 (4.48), 3.222 (3.72), 3.363 (3.13), 3.561 (4.82), 3.589 (5.55), 3.605 (4.76), 3.621 (2.34), 3.873 (4.28), 4.332 (4.76), 4.468 (4.82), 7.048 (10.08), 7.071 (11.58), 7.082 (10.68), 7.101 (5.30), 7.121 (8.17), 7.126 (6.82), 7.163 (12.85), 7.183 (6.93), 7.393 (2.62), 7.487 (12.62), 7.509 (11.24), 7.524 (5.15), 7.654 (2.54), 7.955 (16.00), 10.686 (0.96).

### Example 9

### 1-{1-[4-Chloro-4'-(4-cyclopropylpiperazin-1-yl)[biphenyl]-2-yl]piperidin-3-yl}-5-(difluoromethyl)-1H-pyrazole-4-carboxylic acid hydrochloride (Enantiomer 2)

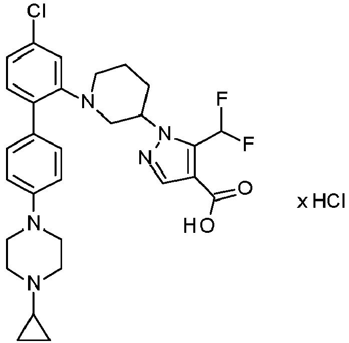

Ethyl 1-[1-[5-chloro-2-[4-(4-cyclopropylpiperazin-1-yl)phenyl]phenyl]-3-piperidyl]-5-(difluoromethyl)pyrazole-4-carboxylate trifluoroacetic acid (Example 26A, Enantiomer 2, 24.4 mg, 71 % purity, 24.8 µmol) was dissolved in THF/ethanol (1.0/0.1 ml). 1 M aqueous lithium hydroxide solution (250 µl, 250 µmol) was added and the mixture was stirred overnight at room temperature. The mixture was evaporated, then acidified and purified using preparative HPLC (RP18 column, acetonitrile/water gradient with the addition of 0.1% TFA). The product fractions were combined and evaporated. The raw material was purified by thick layer chromatography (eluent: dichloromethane/methanol/formic acid 10/1/0.1). Then the residue was mixed with 2 M hydrochloric acid in diethyl ether, carefully evaporated at 30°C (twice) and then lyophilized. 6.8 mg of the target compound (43% of theory, purity 93%) were obtained.
LC-MS (Method 3): Rₜ = 1.68 min; MS (ESIpos): m/z = 556 [M-HCl+H]⁺
¹H-NMR (400 MHz, DMSO-d6) δ [ppm]: -0.149 (1.01), -0.008 (8.90), 0.008 (9.79), 0.146 (1.01), 0.835 (7.44), 0.852 (7.83), 1.110 (7.22), 1.233 (1.34), 1.493 (1.90), 1.524 (2.18), 1.722 (2.91), 1.754 (2.18), 1.886 (2.01), 1.918 (2.35), 1.964 (3.36), 2.054 (1.85), 2.327 (3.47), 2.343 (4.08), 2.359 (2.01), 2.367 (2.13), 2.583 (4.20), 2.613 (2.13), 2.670 (2.07), 2.710 (2.29), 2.951 (2.35), 2.995 (3.02), 3.022 (6.10), 3.048 (5.71), 3.066 (5.09), 3.098 (4.64), 3.127 (3.02), 3.219 (3.92), 3.237 (3.92), 3.353 (4.25), 3.380 (4.70), 3.408 (4.48), 3.575 (10.13), 3.605 (10.74), 3.621 (5.54), 3.857 (2.63), 3.888 (4.70), 3.922 (2.46), 4.435 (1.73), 4.462 (2.91), 7.009 (1.23), 7.034 (1.45), 7.052 (12.87), 7.074 (14.32), 7.081 (12.08), 7.086 (13.59), 7.102 (6.15), 7.108 (3.97), 7.123 (9.62), 7.128 (8.22), 7.161 (15.38), 7.182 (7.72), 7.266 (0.78), 7.385 (3.52), 7.459 (0.90), 7.490 (15.33), 7.512 (16.00), 7.647 (2.91), 7.955 (15.55), 8.890 (0.45), 10.132 (1.51), 13.209 (0.50).

### Example 10

### 1-[1-{4-Chloro-4'-[4-(cyclopropylmethyl)piperazin-1-yl][1,1'-biphenyl]-2-yl}piperidin-3-yl]-5-(difluoromethyl)-1H-pyrazole-4-carboxylic acid hydrochloride (Enantiomer 1)

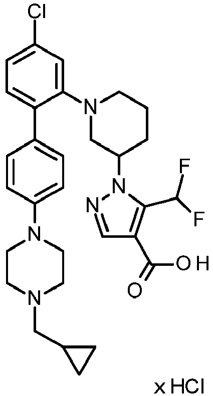

Ethyl 1-[1-{5-chloro-2-[(trifluoromethanesulfonyl)oxy]phenyl}piperidin-3-yl]-5-(difluoromethyl)-1H-pyrazole-4-carboxylate (prepared in analogy to Example 11A, Enantiomer 1, 80.0 mg, 147 µmol) and 1-(cyclopropylmethyl)-4-[4-(4,4,5,5-tetramethyl-1,3,2-dioxaborolan-2-yl)phenyl]piperazine (Example 27A, 71.2 mg, 85 % purity, 177 µmol) were placed under argon in toluene/ethanol (0.82/0.82 ml). 2 M sodium carbonate solution (220 µl, 2.0 M, 440 µmol) and tetrakis(triphenylphosphine)palladium(0) (8.52 mg, 7.37 µmol) were added and the mixture was stirred at 100°C. overnight. The reaction mixture was diluted with ethyl acetate and 1 M hydrochloric acid was added. The aqueous phase was extracted three times with ethyl acetate. The organic phase was dried with sodium sulfate, filtered off and evaporated. The crude mixture was dissolved with THF/ethanol (2/0.2 ml), 1 M lithium hydroxide solution (1.5 ml, 1.5 mmol) was added and the mixture was stirred at room temperature overnight. A 1 M lithium hydroxide solution (1.5 ml, 1.5 mmol) was added again. After about 6 h the reaction mixture was evaporated at 50°C. The residue was dissolved in acetonitrile/water/0.25 ml trifluoroacetic acid and purified by preparative HPLC (RP18 column, acetonitrile/water gradient with the addition of 0.1% trifluoroacetic acid). The crude product was purified by means of thick layer chromatography (dichloromethane/methanol/formic acid: 10/1/0.1). The silica gel mixture was stirred with dichloromethane/1 M hydrochloric acid in dioxane (10/1) in ethanol, filtered off and carefully evaporated at 30°C and lyophilized. 36 mg of the target compound (38% of theory, purity 95%) were obtained.
LC-MS (Method 6): Rₜ = 1.22 min; MS (ESIpos): m/z = 570 [M-HCl+H]⁺
¹H-NMR (600 MHz, DMSO-d6) δ [ppm]: 0.403 (2.47), 0.411 (9.74), 0.420 (10.17), 0.428 (2.68), 0.672 (2.50), 0.681 (8.12), 0.692 (8.38), 0.702 (2.19), 1.138 (2.99), 1.504 (1.97), 1.526 (2.03), 1.732 (2.75), 1.755 (2.30), 1.881 (0.76), 1.895 (2.10), 1.915 (2.23), 1.937 (1.04), 1.975 (2.95), 2.387 (0.74), 2.427 (0.70), 2.576 (2.02), 2.596 (3.59), 2.614 (2.41), 2.656 (0.56), 3.001 (2.80), 3.019 (5.69), 3.037 (3.63), 3.051 (3.32), 3.071 (4.92), 3.084 (10.58), 3.093 (12.72), 3.104 (9.12), 3.170 (4.32), 3.186 (3.97), 3.214 (3.53), 3.233 (2.75), 3.643 (7.18), 3.663 (6.55), 3.900 (11.76), 3.915 (11.29), 3.937 (10.31), 4.440 (1.83), 4.459 (3.12), 4.477 (1.66), 7.039 (1.74), 7.062 (12.26), 7.076 (13.12), 7.089 (11.21), 7.111 (4.85), 7.114 (4.07), 7.125 (8.57), 7.128 (7.37), 7.165 (11.99), 7.178 (7.07), 7.209 (1.67), 7.428 (2.90), 7.497 (14.12), 7.511 (14.84), 7.551 (0.51), 7.602 (2.66), 7.958 (16.00), 9.938 (1.95).

### Example 11

### 1-(1-{4-Chloro-4'-[4-(cyclopropylmethyl)piperazin-1-yl][biphenyl]-2-yl}piperidin-3-yl)-5-(difluoromethyl)-1H-pyrazole-4-carboxylic acid hydrochloride (Enantiomer 2)

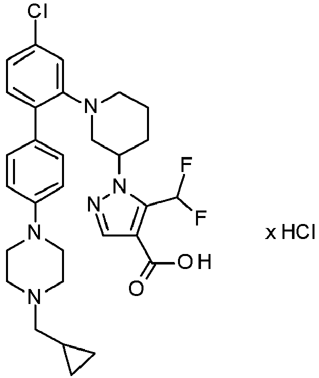

A solution of 1-{1-[4-chloro-4'-(piperazin-1-yl)[1,1'-biphenyl]-2-yl]piperidin-3-yl}-5-(difluoromethyl)-1H-pyrazole-4-carboxylic acid (Example 28A, Enantiomer 2, 175 mg, 339 µmol) in acetonitrile (3.1 ml) was treated with cyclopropanecarboxaldehyde (180 µl, 2.4 mmol) and sodium triacetoxyborohydride (216 mg, 1.02 mmol) and stirred overnight at room temperature. The reaction mixture was diluted with water and purified by preparative HPLC (RP18 column, eluent: Acetonitrile/water gradient) and evaporated. The residue was strirred in an aqeous solution of hydrogen chloride and lyophilized affording 193 mg (94 % yield) of the title compound.
LC-MS (Method 3): Rₜ = 1.71 min; MS (ESIpos): m/z = 570 [M-HCl+H]⁺
¹H-NMR(400 MHz, DMSO-d6) δ [ppm]: 0.008 (3.75), 0.404 (1.78), 0.416 (7.42), 0.428 (7.54), 0.441 (2.23), 0.667 (5.71), 0.687 (6.02), 1.163 (1.94), 1.492 (1.30), 1.524 (1.42), 1.721 (2.07), 1.754 (1.58), 1.889 (1.40), 1.918 (1.60), 1.969 (2.29), 2.329 (0.68), 2.368 (0.70), 2.584 (2.75), 2.613 (1.40), 2.672 (0.72), 2.712 (0.80), 2.999 (2.19), 3.026 (4.93), 3.053 (7.20), 3.069 (7.86), 3.161 (7.98), 3.216 (2.87), 3.242 (2.05), 3.629 (14.26), 3.644 (16.00), 3.864 (4.63), 3.893 (4.81), 3.919 (3.11), 4.440 (1.32), 4.468 (2.19), 4.494 (1.18), 7.054 (9.40), 7.076 (11.45), 7.081 (9.08), 7.086 (9.54), 7.103 (3.93), 7.124 (6.28), 7.128 (5.61), 7.163 (11.39), 7.183 (5.71), 7.394 (2.53), 7.490 (11.05), 7.512 (10.19), 7.525 (5.19), 7.655 (2.09), 7.958 (11.99), 10.563 (0.64).

### Example 12

### 1-[(1-{4-Chloro-4'-[4-(2,2-dimethylpropyl)piperazin-1-yl][biphenyl]-2-yl}piperidin-3-yl]-5-(difluoromethyl)-1H-pyrazole-4-carboxylic acid hydrochloride (Enantiomer 1)

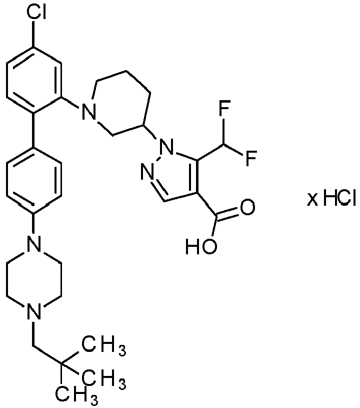

Ethyl 1-[1-{4-chloro-4'-[4-(2,2-dimethylpropyl)piperazin-1-yl][1,1'-biphenyl]-2-yl}piperidin-3-yl]-5-(difluoromethyl)-1H-pyrazole-4-carboxylate trifluoroacetic acid (Example 30A, Enantiomer 1, 26.0 mg, 35.7 µmol) was dissolved in THF/ethanol (930/93 µl). 1 M aqueous lithium hydroxide solution (360 µl, 360 µmol) was added and the mixture was stirred overnight at room temperature. The mixture was evaporated, then acidified and purified using preparative HPLC (RP18 column, acetonitrile/water gradient with the addition of 0.1% TFA). The product fractions were combined and evaporated. The raw material was purified by thick layer chromatography (eluent: dichloromethane/methanol/formic acid 10/1/0.1). Then the residue was mixed with 1 M hydrochloric acid in ethanol, carefully evaporated at 30°C (twice) and then lyophilized. 17 mg of the target compound (74% of theory) were obtained.
LC-MS (Method 3): Rₜ = 1.84 min; MS (ESIpos): m/z = 586 [M-HCl+H]⁺
¹H-NMR (400 MHz, DMSO-d6) δ [ppm]: 1.100 (16.00), 1.965 (0.43), 2.585 (0.50), 3.029 (0.92), 3.056 (0.64), 3.119 (1.44), 3.130 (1.32), 3.232 (0.51), 3.262 (0.60), 3.290 (1.42), 3.313 (1.42), 3.336 (0.49), 3.390 (1.54), 3.706 (0.56), 3.744 (0.51), 6.961 (0.51), 7.046 (1.44), 7.068 (1.56), 7.089 (1.85), 7.105 (0.64), 7.126 (1.01), 7.131 (0.85), 7.162 (1.57), 7.183 (0.76), 7.217 (0.53), 7.496 (1.72), 7.518 (1.91), 7.961 (1.67).

### Example 13

### 1-[(1-{4-Chloro-4'-[4-(2,2-dimethylpropyl)piperazin-1-yl][biphenyl]-2-yl}piperidin-3-yl]-5-(difluoromethyl)-1H-pyrazole-4-carboxylic acid hydrochloride (Enantiomer 2)

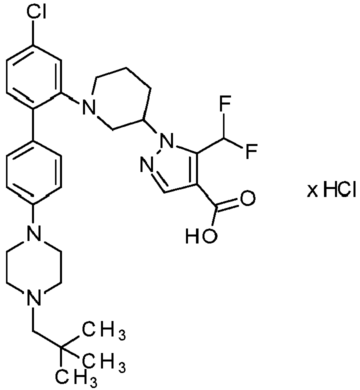

Ethyl 1-[1-{4-chloro-4'-[4-(2,2-dimethylpropyl)piperazin-1-yl][1,1'-biphenyl]-2-yl}piperidin-3-yl]-5-(difluoromethyl)-1H-pyrazole-4-carboxylate trifluoroacetic acid (Example 31A, Enantiomer 2, 39.0 mg, 53.6 µmol) was dissolved in THF/ethanol (1.4/0.14 ml). 1 M aqueous lithium hydroxide solution (540 µl, 540 µmol) was added and the mixture was stirred overnight at room temperature. The mixture was evaporated, then acidified and purified using preparative HPLC (RP18 column, acetonitrile/water gradient with the addition of 0.1% TFA). The product fractions were combined and evaporated. The raw material was purified by thick layer chromatography (eluent: dichloromethane/methanol/formic acid 10/1/0.1). Then the residue was mixed with 1 M hydrochloric acid in ethanol, carefully evaporated at 30°C (twice) and then lyophilized. 21 mg of the target compound (62% of theory, purity 97%) were obtained.
LC-MS (Method 4): Rₜ = 0.93 min; MS (ESIpos): m/z = 586 [M-HCl+H]⁺
¹H-NMR (400 MHz, DMSO-d6) δ [ppm]: -0.008 (1.88), 0.008 (1.94), 1.108 (16.00), 3.032 (0.77), 3.058 (0.54), 3.112 (1.18), 3.123 (1.18), 3.265 (0.44), 3.294 (0.42), 3.327 (0.70), 3.358 (0.64), 3.390 (2.04), 3.588 (0.81), 3.600 (0.71), 3.611 (0.86), 7.044 (1.22), 7.066 (1.33), 7.083 (0.95), 7.088 (1.21), 7.105 (0.57), 7.125 (0.92), 7.130 (0.78), 7.163 (1.54), 7.183 (0.79), 7.495 (1.53), 7.516 (1.41), 7.525 (0.77), 7.961 (1.50).

### Example 14

### 1-[1-{4-Chloro-4'-[4-(cyclobutylmethyl)piperazin-1-yl][biphenyl]-2-yl}piperidin-3-yl]-5-(difluoromethyl)-1H-pyrazole-4-carboxylic acid hydrochloride (Enantiomer 2)

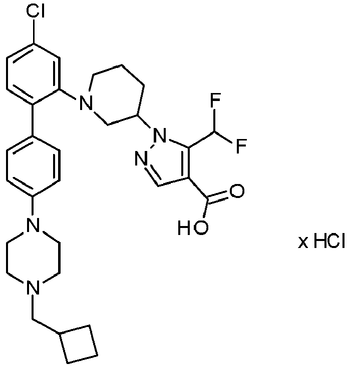

Ethyl 1-[1-{4-chloro-4'-[4-(cyclobutylmethyl)piperazin-1-yl][1,1'-biphenyl]-2-yl}piperidin-3-yl]-5-(difluoromethyl)-1H-pyrazole-4-carboxylat trifluoroacetic acid (Example 33A, Enantiomer 2, (43.0 mg, 59.2 µmol) was dissolved in THF/ethanol (1.5/0.15 ml). 1 M aqueous lithium hydroxide solution (590 µl, 590 µmol) was added and the mixture was stirred overnight at room temperature. The mixture was evaporated, then acidified and purified using preparative HPLC (RP18 column, acetonitrile/water gradient with the addition of 0.1% TFA). The product fractions were combined and evaporated. The raw material was purified by thick layer chromatography (eluent: dichloromethane/methanol/formic acid 10/1/0.1). Then the residue was mixed with 1 M hydrochloric acid in ethanol, carefully evaporated at 30°C (twice) and then lyophilized. 21.6 mg of the target compound (58% of theory) were obtained.
LC-MS (Method 3): Rₜ = 1.77 min; MS (ESIpos): m/z = 584 [M-HCl+H]⁺
¹H-NMR(400 MHz, DMSO-d6) δ [ppm]: 1.523 (0.41), 1.725 (0.48), 1.828 (1.73), 1.843 (1.20), 1.863 (1.00), 1.922 (0.78), 1.963 (0.80), 2.125 (1.11), 2.144 (1.13), 2.597 (0.61), 2.755 (0.44), 2.773 (0.55), 2.988 (0.84), 3.013 (1.68), 3.040 (1.51), 3.124 (0.80), 3.151 (0.75), 3.236 (1.85), 3.390 (1.07), 3.478 (16.00), 3.838 (0.67), 3.871 (0.97), 3.909 (0.52), 4.451 (0.52), 6.968 (0.41), 7.040 (2.09), 7.062 (2.21), 7.090 (2.04), 7.103 (0.97), 7.124 (1.52), 7.156 (2.47), 7.177 (1.15), 7.223 (0.42), 7.375 (0.60), 7.486 (2.58), 7.507 (3.19), 7.636 (0.47), 7.956 (3.05).

### Example 15

### 1-[1-(4-Chloro-4'-{4-[(3,3-difluorocyclobutyl)methyl]piperazin-1-yl}[biphenyl]-2-yl)piperidin-3-yl]-5-(difluoromethyl)-1H-pyrazole-4-carboxylic acid hydrochloride (Enantiomer 1)

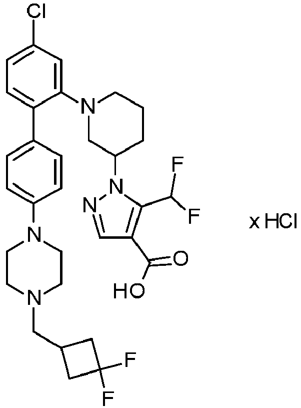

A solution of ethyl 1-[1-[5-chloro-2-[4-[4-[(3,3-difluorocyclobutyl)methyl]piperazin-1-yl]phenyl]phenyl]-3-piperidyl]-5-(difluoromethyl)pyrazole-4-carboxylate (Example 35A, Enantiomer 1, 48.0 mg, 74.1 µmol) in a THF/water mixture (1:1, 2.8 ml) was treated with lithium hydroxide monohydrate (6.22 mg, 148 µmol). The resulting mixture was stirred overnight at room temperature. The reaction mixture was acidified with an aqueous solution of hydrogen chloride (1 N), diluted with methanol and purified by preparative HPLC (RP18 column, eluent: Acetonitrile/water + 0.1% formic acid gradient). The product containing fractions were lyophilized after addition of an aqueous solution of hydrogen chloride (1 N) affording 48.3 mg (quant.) of the title compound.
LC-MS (Method 3): Rₜ = 1.80 min; MS (ESIpos): m/z = 620 [M-HCl+H]⁺
¹H-NMR(600 MHz, DMSO-d6) δ [ppm]: 1.497 (1.88), 1.518 (2.07), 1.724 (2.51), 1.746 (2.15), 1.883 (0.76), 1.898 (1.97), 1.919 (2.08), 1.938 (1.01), 1.973 (2.67), 2.384 (0.87), 2.423 (1.20), 2.521 (2.30), 2.566 (4.30), 2.583 (4.53), 2.606 (2.06), 2.652 (1.25), 2.688 (1.99), 2.819 (3.69), 2.830 (3.54), 2.843 (3.23), 3.008 (3.14), 3.026 (6.08), 3.044 (5.69), 3.059 (2.74), 3.153 (8.68), 3.224 (3.44), 3.237 (3.12), 3.529 (5.54), 3.683 (0.66), 3.835 (2.29), 3.851 (2.98), 3.868 (2.58), 3.884 (2.50), 4.446 (1.65), 4.465 (2.83), 4.482 (1.60), 7.047 (12.84), 7.062 (13.27), 7.080 (9.68), 7.083 (11.53), 7.104 (5.34), 7.107 (3.96), 7.117 (7.57), 7.121 (6.69), 7.160 (13.31), 7.174 (8.15), 7.427 (2.98), 7.486 (14.92), 7.501 (13.54), 7.514 (5.81), 7.601 (2.46), 7.950 (16.00), 10.599 (1.47).

### Example 16

### 1-[1-(4-Chloro-4'-{4-[(1-fluorocyclobutyl)methyl]piperazin-1-yl}[biphenyl]-2-yl)piperidin-3-yl]-5-(difluoromethyl)-1H-pyrazole-4-carboxylic acid hydrochloride (Enantiomer 2)

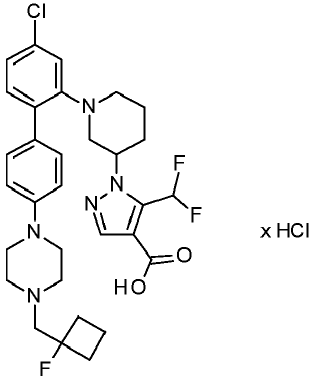

A solution of ethyl 1-[1-[5-chloro-2-[4-[4-[(1-fluorocyclobutyl)methyl]piperazin-1-yl]phenyl]phenyl]-3-piperidyl]-5-(difluoromethyl)pyrazole-4-carboxylate (Example 39A, Enantiomer 2, 73.0 mg, 61% purity, 70.7 µmol) in a THF/methanol mixture (10:1, 3.8 ml) and water (3.8 ml) was treated with lithium hydroxide monohydrate (48.6 mg, 1.16 mmol). The resulting mixture was stirred overnight at room temperature. The reaction mixture was acidified with an aqueous solution of hydrogen chloride (1 N), diluted with acetonitrile and purified by preparative HPLC (RP18 column, eluent: Acetonitrile/water + 0.1% formic acid gradient). The product containing fractions were lyophilized after addition of an aqueous solution of hydrogen chloride (1 N) affording 22.1 mg (47 % yield) of the title compound.
LC-MS (Method 8): Rₜ = 0.94 min; MS (ESIpos): m/z = 602 [M-HCl+H]⁺
¹H-NMR (400 MHz, DMSO-d6) δ [ppm]: -0.149 (0.60), 0.146 (0.48), 1.070 (1.22), 1.234 (0.87), 1.272 (1.37), 1.486 (1.45), 1.518 (1.68), 1.630 (0.95), 1.652 (2.14), 1.674 (2.39), 1.719 (2.46), 1.758 (6.08), 1.865 (2.12), 1.890 (2.28), 1.922 (1.90), 1.966 (2.61), 2.074 (16.00), 2.329 (2.72), 2.359 (2.93), 2.384 (2.73), 2.412 (2.26), 2.436 (1.10), 2.578 (3.18), 2.607 (1.61), 2.671 (0.95), 2.712 (1.29), 2.732 (0.55), 2.891 (0.64), 3.001 (2.26), 3.027 (6.05), 3.054 (4.31), 3.228 (3.49), 3.286 (7.66), 3.529 (6.17), 3.851 (5.29), 3.882 (3.51), 4.256 (0.67), 4.273 (0.55), 4.470 (2.29), 7.037 (10.04), 7.059 (11.01), 7.080 (7.97), 7.085 (9.55), 7.102 (4.41), 7.123 (7.15), 7.128 (6.20), 7.162 (12.27), 7.182 (6.24), 7.396 (2.75), 7.488 (12.36), 7.510 (11.03), 7.526 (5.44), 7.657 (2.28), 7.958 (14.17), 8.122 (0.49), 10.668 (0.85).

### Example 17

### 1-[1-(4-Chloro-4'-{4-[(3-fluorobicyclo[1.1.1]pentan-1-yl)methyl]piperazin-1-yl}[biphenyl]-2-yl)piperidin-3-yl]-5-(difluoromethyl)-1H-pyrazole-4-carboxylic acid hydrochloride (Enantiomer 2)

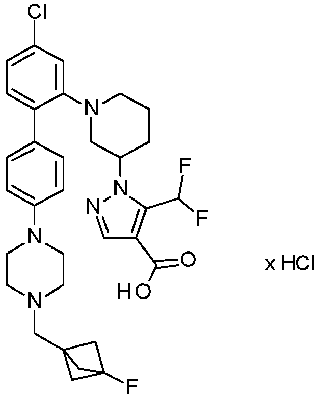

A solution of ethyl 1-[1-[5-chloro-2-[4-[4-[(3-fluoro-1-bicyclo[1.1.1]pentanyl)methyl]piperazin-1-yl]phenyl]phenyl]-3-piperidyl]-5-(difluoromethyl)pyrazole-4-carboxylate (Example 42A, Enantiomer 2, 63.0 mg, 98.1 µmol) in a THF/methanol mixture (10:1, 2.2 ml) and water (2.2 ml) was treated with lithium hydroxide monohydrate (41.2 mg, 981 µmol). The resulting mixture was stirred overnight at room temperature. The reaction mixture was acidified with an aqueous solution of hydrogen chloride (1 N), diluted with acetonitrile and purified by preparative HPLC (RP18 column, eluent: Acetonitrile/water gradient). The product containing fractions were lyophilized after addition of an aqueous solution of hydrogen chloride (1 N) affording 60.0 mg (92 % yield) of the title compound.
LC-MS (Method 3): Rₜ = 1.74 min; MS (ESIpos): m/z = 614 [M-HCl+H]⁺
¹H-NMR(400 MHz, DMSO-d6) δ [ppm]: 1.069 (1.02), 1.487 (0.44), 1.518 (0.49), 1.716 (0.67), 1.757 (0.80), 1.888 (0.47), 1.918 (0.55), 1.972 (0.77), 2.074 (0.81), 2.251 (15.97), 2.257 (16.00), 2.578 (0.94), 2.609 (0.51), 3.000 (0.68), 3.027 (1.82), 3.054 (1.26), 3.193 (1.93), 3.223 (1.81), 3.243 (1.04), 3.811 (1.36), 3.845 (2.64), 3.875 (3.06), 3.961 (2.92), 4.441 (0.44), 4.468 (0.76), 4.494 (0.42), 7.037 (3.16), 7.059 (3.45), 7.081 (2.26), 7.086 (2.93), 7.102 (1.36), 7.107 (0.83), 7.122 (2.15), 7.127 (1.92), 7.160 (3.81), 7.180 (1.91), 7.394 (0.81), 7.488 (3.71), 7.510 (3.40), 7.525 (1.68), 7.655 (0.70), 7.958 (3.47).

### Example 18

### 1-[1-{4-Chloro-4'-[4-(2,2-difluoroethyl)piperazin-1-yl][biphenyl]-2-yl}piperidin-3-yl]-5-(difluoromethyl)-1H-pyrazole-4-carboxylic acid hydrochloride (Enantiomer 1)

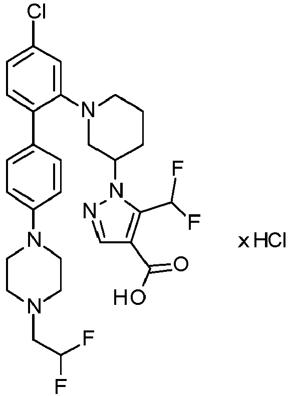

A solution ethyl 1-[1-[5-chloro-2-[4-[4-(2,2-difluoroethyl)piperazin-1-yl]phenyl]phenyl]-3-piperidyl]-5-(difluoromethyl)pyrazole-4-carboxylate (Example 44A, Enantiomer 1, 86.5 mg, 142 µmol) in a THF/Methanol mixture (10:1, 3.0 ml) was treated with lithium hydroxide (1.4 ml, 1 N, 1.4 mmol). The resulting mixutre was stirred for 2 hours at room temperature, diluted with water and acidified with an aqueous solution of hydrogen chloride (1 N). The aqueous solution was extracted three times with ethyl acetate. The combined organic layers were dried over sodium sulfate and evaporated. The residue was purified by preparative HPLC (Method 10). The product containing fractions were lyophilized after addition of an aqueous solution of hydrogen chloride (1 N) affording 50.0 mg (57 % yield) of the title compound.
LC-MS (Method 3): Rₜ = 2.45 min; MS (ESIpos): m/z = 580 [M-HCl+H]⁺
¹H-NMR (400 MHz, DMSO-d6) δ [ppm]: -0.149 (0.67), 0.146 (0.72), 1.460 (0.57), 1.492 (1.74), 1.523 (1.97), 1.555 (0.88), 1.717 (2.71), 1.750 (2.09), 1.866 (0.63), 1.887 (1.87), 1.897 (1.70), 1.917 (2.13), 1.973 (3.03), 2.329 (0.56), 2.368 (0.73), 2.576 (3.67), 2.606 (2.02), 2.672 (0.74), 2.711 (0.89), 2.998 (3.27), 3.024 (6.87), 3.050 (5.72), 3.068 (3.69), 3.224 (5.74), 3.243 (5.37), 4.439 (1.65), 4.466 (2.89), 4.476 (2.03), 4.492 (1.54), 6.563 (0.49), 7.037 (9.39), 7.059 (10.27), 7.077 (9.89), 7.082 (12.35), 7.099 (5.50), 7.105 (3.42), 7.120 (8.86), 7.125 (7.73), 7.159 (16.00), 7.179 (8.08), 7.385 (3.43), 7.480 (13.78), 7.502 (12.50), 7.516 (6.97), 7.647 (2.81), 7.957 (13.97).

### Example 19

### 1-[1-{4-Chloro-4'-[4-(2,2-difluoroethyl)piperazin-1-yl][biphenyl]-2-yl}piperidin-3-yl]-5-(difluoromethyl)-1H-pyrazole-4-carboxylic acid hydrochloride (Enantiomer 2)

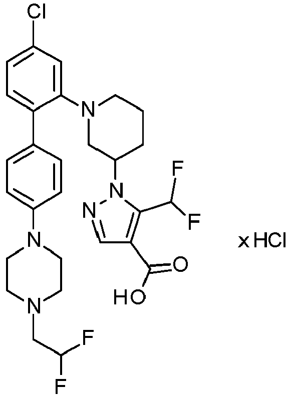

A solution ethyl 1-[1-[5-chloro-2-[4-[4-(2,2-difluoroethyl)piperazin-1-yl]phenyl]phenyl]-3-piperidyl]-5-(difluoromethyl)pyrazole-4-carboxylate (Example 45A, Enantiomer 2, 92.2 mg, 91 % purity, 138 µmol) in a THF/Methanol mixture (10:1, 3.2 ml) was treated with lithium hydroxide (1.5 ml, 1 N, 1.4 mmol). The resulting mixutre was stirred for 2 hours at room temperature, diluted with water and acidified with an aqueous solution of hydrogen chloride (1 N). The aqueous solution was extracted three times with ethyl acetate. The combined organic layers were dried over sodium sulfate and evaporated. The residue was purified by preparative HPLC (Method 10) The product containing fractions were lyophilized after addition of an aqueous solution of hydrogen chloride (1 N) affording 40.0 mg (47 % yield) of the title compound.
LC-MS (Method 3): Rₜ = 2.45 min; MS (ESIpos): m/z = 580 [M-HCl+H]⁺
¹H-NMR (400 MHz, DMSO-d6) δ [ppm]: -0.149 (0.87), 0.068 (0.58), 0.146 (0.85), 1.491 (1.58), 1.523 (1.85), 1.718 (2.53), 1.751 (1.99), 1.888 (1.75), 1.920 (1.99), 1.966 (2.88), 2.368 (0.74), 2.578 (3.36), 2.609 (1.76), 2.712 (0.72), 2.998 (2.88), 3.025 (6.41), 3.051 (4.88), 3.221 (4.82), 3.241 (4.47), 4.438 (1.54), 4.465 (2.71), 4.491 (1.38), 6.476 (0.40), 6.608 (0.73), 6.737 (0.41), 7.045 (9.55), 7.067 (10.56), 7.079 (9.82), 7.084 (11.85), 7.101 (5.30), 7.106 (3.29), 7.122 (8.47), 7.127 (7.54), 7.160 (14.83), 7.180 (7.32), 7.388 (3.19), 7.485 (13.79), 7.506 (12.51), 7.519 (6.66), 7.649 (2.62), 7.957 (16.00).

### Example 20

### 1-[1-[5-Chloro-2-[4-[4-(2,2,2-trifluoroethyl)piperazin-1-yl]phenyl]phenyl]-3-piperidyl]-5-(difluoromethyl)pyrazole-4-carboxylic acid (Enantiomer 1)

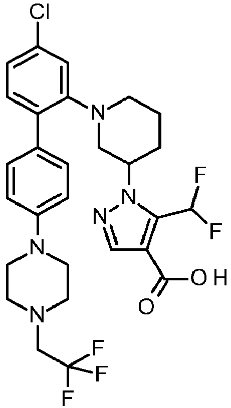

An aqueous solution of lithium hydroxide (1.0 ml, 1.0 M, 1.0 mmol) was added to a solution of ethyl 1-[1-[5-chloro-2-[4-[4-(2,2,2-trifluoroethyl)piperazin-1-yl]phenyl]phenyl]-3-piperidyl]-5-(difluoromethyl)pyrazole-4-carboxylate (Example 49A, Enantiomer 1, 62.7 mg, 100 µmol) in a THF/Methanol mixture (10:1, 2.2 mL). The reaction mixture was stirred overnight at room temperature. An aqeuouse solution of hydrogen chloride (6N) was then added and the resulting mixture was extracted with dichloromethane. The combined organic layers were evaporated affording 57.9 mg (90 % purity, 93 % yield) of the title compound.
LC-MS (Method 3): Rₜ = 2.68 min; MS (ESIpos): m/z = 598 [M+H]⁺
¹H-NMR(400 MHz, DMSO-d6) δ [ppm]: 0.886 (0.89), 1.117 (0.75), 1.128 (1.08), 1.170(2.21), 1.183 (0.78), 1.237 (0.99), 1.271 (0.42), 1.356 (1.93), 1.489 (1.60), 1.520 (1.83), 1.700 (2.49), 1.743 (5.12), 1.752 (5.38), 1.760 (12.17), 1.769 (5.24), 1.776 (4.32), 1.794 (0.56), 1.864 (0.59), 1.885 (1.74), 1.916 (2.02), 1.978 (2.75), 2.329 (0.70), 2.367 (0.92), 2.578 (2.44), 2.671 (0.96), 2.711 (1.13), 2.810 (13.27), 3.005 (2.68), 3.032 (7.52), 3.058 (5.17), 3.262 (4.51), 3.283 (4.75), 3.310 (4.28), 3.585 (8.11), 3.601 (15.55), 3.618 (10.01), 4.459 (1.48), 4.486 (2.61), 4.513 (1.41), 5.754 (12.15), 7.002 (6.93), 7.024 (7.45), 7.059 (8.88), 7.064 (10.57), 7.086 (4.82), 7.091 (3.41), 7.107 (7.47), 7.112 (6.51), 7.155 (12.55), 7.175 (6.93), 7.387 (3.10), 7.452 (12.64), 7.473 (11.21), 7.518 (5.80), 7.649 (2.54), 7.954 (16.00).

### Example 21

### 1-[1-[5-Chloro-2-[4-[4-(2,2,2-trifluoroethyl)piperazin-1-yl]phenyl]phenyl]-3-piperidyl]-5-(difluoromethyl)pyrazole-4-carboxylic acid (Enantiomer 2)

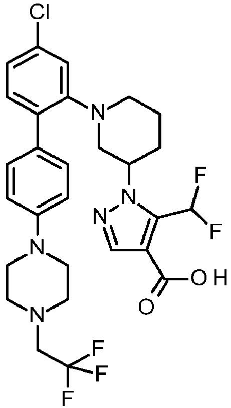

An aqueous solution of lithium hydroxide (1.1 ml, 1.0 M, 1.1 mmol) was added to a solution of ethyl 1-[1-[5-chloro-2-[4-[4-(2,2,2-trifluoroethyl)piperazin-1-yl]phenyl]phenyl]-3-piperidyl]-5-(difluoromethyl)pyrazole-4-carboxylate (Example 50A, Enantiomer 2, 66.6 mg, 106 µmol) in a THF/Methanol mixture (10:1, 2.2 mL). The reaction mixture was stirred overnight at RT. An aqueous solution of hydrogen chloride (6N) was then added and the resulting mixture was extracted with dichloromethane. The combined organic layers were evaporated affording 54.7 mg (90 % purity, 77 % yield) of the title compound.
LC-MS (Method 3): Rₜ = 2.67 min; MS (ESIpos): m/z = 598 [M+H]⁺
¹H-NMR(400 MHz, DMSO-d6) δ [ppm]: 0.886 (0.65), 0.948 (0.41), 1.092 (0.48), 1.107 (0.60), 1.116 (1.02), 1.128 (0.84), 1.169 (2.79), 1.183 (1.08), 1.236 (0.93), 1.271 (0.60), 1.356 (1.02), 1.489 (1.77), 1.521 (2.07), 1.700 (2.77), 1.743 (5.62), 1.751 (5.95), 1.760 (12.78), 1.768 (5.69), 1.776 (4.73), 1.794 (0.61), 1.864 (0.69), 1.885 (1.93), 1.916 (2.20), 1.978 (3.01), 1.988 (2.81), 2.328 (0.80), 2.367 (0.80), 2.580 (2.29), 2.670 (1.00), 2.711 (0.89), 2.823 (13.80), 3.004 (2.83), 3.031 (7.94), 3.057 (5.49), 3.260 (4.30), 3.301 (4.33), 3.325 (4.15), 3.585 (5.71), 3.601 (12.71), 3.618 (6.10), 3.731 (4.95), 4.021 (0.52), 4.038 (0.47), 4.457 (1.62), 4.484 (2.81), 4.511 (1.47), 5.754 (11.96), 7.013 (6.70), 7.034 (7.14), 7.061 (9.75), 7.066 (11.52), 7.088 (5.10), 7.093 (3.57), 7.108 (7.87), 7.113 (6.81), 7.156 (13.10), 7.176 (7.16), 7.387 (3.31), 7.456 (13.47), 7.477 (11.85), 7.518 (6.20), 7.649 (2.72), 7.954 (16.00).

### Example 22

### 1-(1-{4-Chloro-4'-[4-(3,3-difluoropropyl)piperazin-1-yl][biphenyl]-2-yl}piperidin-3-yl)-5-(difluoromethyl)-1H-pyrazole-4-carboxylic acid hydrochloride (Enantiomer 2)

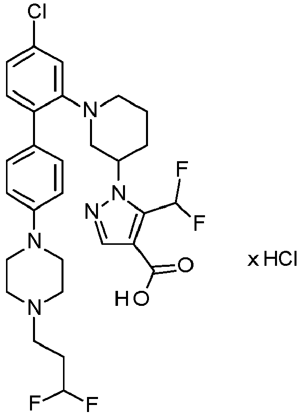

An aqueous solution of lithium hydroxide (1.2 ml, 1.0 M, 1.2 mmol) was added to a solution of ethyl 1-[1-{4-chloro-4'-[4-(3,3-difluoropropyl)piperazin-1-yl][1,1'-biphenyl]-2-yl}piperidin-3-yl]-5-(difluoromethyl)-1H-pyrazole-4-carboxylate (Example 53A, Enantiomer 2, 76.0 mg, 122 µmol) in a THF/methanol mixture (2.7 ml). The resulting mixture was stirred 3 hours at room temperature. The reaction mixture was acidified with an aqueous solution of hydrogen chloride (2 N) and evaporated. The residue was purified by preparative HPLC (RP18 column, eluent: Acetonitrile/water gradient). The product containing fractions were lyophilized after addition of an aqueous solution of hydrogen chloride (1 N) affording 42.0 mg (54 % yield) of the title compound.
LC-MS (Method 3): Rₜ = 1.75 min; MS (ESIpos): m/z = 594 [M-HCl+H]⁺
¹H-NMR(600 MHz, DMSO-d6) δ [ppm]: 1.265 (0.43), 1.276 (0.41), 1.495 (1.85), 1.517 (2.02), 1.538 (0.84), 1.721 (2.47), 1.743 (2.07), 1.887 (0.69), 1.901 (1.93), 1.906 (1.78), 1.921 (2.10), 1.927 (1.96), 1.941 (1.04), 1.976 (2.64), 1.992 (1.60), 2.425 (0.61), 2.451 (1.49), 2.460 (2.36), 2.468 (2.77), 2.481 (2.86), 2.523 (1.48), 2.558 (1.87), 2.578 (3.44), 2.595 (1.82), 2.654 (0.54), 2.692 (1.04), 3.015 (3.10), 3.033 (7.84), 3.051 (5.17), 3.183 (8.55), 3.223 (3.35), 3.241 (2.69), 3.315 (5.23), 3.647 (10.84), 3.860 (4.14), 3.876 (4.60), 3.892 (3.88), 3.906 (3.68), 4.454 (1.75), 4.472 (2.82), 4.479 (1.96), 4.490 (1.58), 6.199 (1.06), 6.205 (1.93), 6.212 (0.89), 6.292 (2.05), 6.299 (4.05), 6.305 (2.01), 6.385 (0.87), 6.392 (1.81), 6.398 (0.96), 7.060 (13.13), 7.075 (15.42), 7.080 (12.58), 7.104 (5.43), 7.107 (4.07), 7.117 (7.41), 7.121 (6.67), 7.165 (13.34), 7.178 (8.45), 7.437 (2.95), 7.486 (15.16), 7.501 (13.58), 7.524 (5.83), 7.611 (2.43), 7.952 (16.00), 11.171 (1.21).

### Example 23

### 1-(1-{4-Chloro-4'-[4-(3,3,3-trifluoropropyl)piperazin-1-yl][biphenyl]-2-yl}piperidin-3-yl)-5-(difluoromethyl)-1H-pyrazole-4-carboxylic acid hydrochloride (Enantiomer 2)

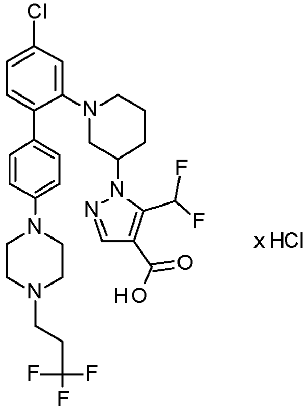

An aqueous solution of lithium hydroxide (1.0 ml, 1.0 M, 1.0 mmol) was added to a solution of ethyl 1-[1-{4-chloro-4'-[4-(3,3,3-trifluoropropyl)piperazin-1-yl][1,1'-biphenyl]-2-yl}pipendin-3-yl]-5-(difluoromethyl)-1H-pyrazole-4-carboxylate (Example 55A, Enantiomer 2, 66.0 mg, 103 µmol) in a THF/methanol mixture (10/1, 2.0 ml). The resulting mixture was stirred 2.5 hours at room temperature. The reaction mixture was acidified with an aqueous solution of hydrogen chloride (2 N) and evaporated. The residue was purified by preparative HPLC (RP18 column, eluent: Acetonitrile/water gradient) affording 61.0 mg (90 % yield) of the title compound.
LC-MS (Method 3): Rₜ = 1.96 min; MS (ESIpos): m/z = 612 [M+H]⁺
¹H-NMR(600 MHz, DMSO-d6) δ [ppm]: 1.234 (0.55), 1.492 (1.90), 1.513 (2.03), 1.534 (0.84), 1.718 (2.57), 1.740 (2.16), 1.883 (0.75), 1.897 (1.97), 1.903 (1.83), 1.918 (2.14), 1.939 (1.02), 1.975 (2.81), 1.990 (1.73), 2.388 (0.46), 2.427 (0.50), 2.568 (3.62), 2.588 (1.88), 2.615 (0.50), 2.656 (0.55), 3.003 (2.57), 3.015 (5.80), 3.033 (11.43), 3.050 (7.57), 3.153 (3.43), 3.173 (3.10), 3.223 (5.38), 3.241 (3.80), 3.301 (0.43), 3.358 (0.46), 3.442 (4.27), 3.454 (5.05), 3.467 (4.02), 3.664 (6.05), 3.682 (6.32), 3.887 (3.38), 3.911 (3.57), 3.942 (2.68), 4.453 (1.74), 4.471 (2.89), 4.489 (1.59), 7.072 (12.93), 7.078 (11.93), 7.082 (14.31), 7.087 (13.77), 7.107 (5.27), 7.111 (4.14), 7.121 (7.31), 7.124 (6.65), 7.166 (12.60), 7.180 (7.84), 7.444 (2.90), 7.490 (14.60), 7.505 (13.14), 7.532 (5.54), 7.619 (2.46), 7.960 (16.00), 11.442 (1.22).

### Example 24

### 1-(1-{4-Chloro-4'-[4-(2,2-difluoropropyl)piperazin-1-yl][biphenyl]-2-yl}piperidin-3-yl)-5-(difluoromethyl)-1H-pyrazole-4-carboxylic acid hydrochloride (Enantiomer 1)

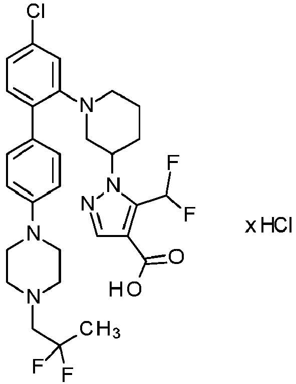

A solution of ethyl 1-[1-[5-chloro-2-[4-[4-(2,2-difluoropropyl)piperazin-1-yl]phenyl]phenyl]-3-piperidyl]-5-(difluoromethyl)pyrazole-4-carboxylate (Example 58A, Enantiomer 1, 68.0 mg, 109 µmol) in a THF/methanol mixture (10:1, 2.3 ml) was treated with lithium hydroxide (1.1 ml, 1 N, 1.1 mmol). The resulting mixture was stirred for 5 days at room temperature. The reaction mixture was acidified with an aqueous solution of hydrogen chloride (1 N) and the aqueous solution was extracted three times with ethyl acetate. The combined organic layers were dried over sodium sulfate and evaporated. The residue was purified by preparative HPLC (RP18 column, eluent: Acetonitrile/water + 0.1% formic acid gradient) The product containing fractions were lyophilized after addition of an aqueous solution of hydrogen chloride (1 N) affording 35.5 mg (51 % yield) of the title compound
LC-MS (Method 3): Rₜ = 2.53 min; MS (ESIpos): m/z = 594 [M-HCl+H]⁺
¹H-NMR(400 MHz, DMSO-d6) δ [ppm]: 1.236 (0.42), 1.339 (0.61), 1.356 (1.34), 1.487 (1.81), 1.519 (2.02), 1.551 (0.92), 1.596 (0.64), 1.720 (2.79), 1.758 (4.94), 1.768 (7.53), 1.817 (13.66), 1.866 (6.73), 1.891 (2.09), 1.900 (1.84), 1.921 (2.23), 1.964 (3.40), 2.074 (1.61), 2.329 (0.74), 2.368 (1.00), 2.581 (3.66), 2.610 (1.96), 2.671 (0.85), 2.711 (1.07), 2.998 (2.83), 3.025 (7.18), 3.052 (5.03), 3.223 (3.99), 3.241 (3.55), 3.365 (2.76), 4.439 (1.80), 4.466 (3.04), 4.476 (2.17), 4.493 (1.62), 7.053 (6.20), 7.074 (7.28), 7.083 (11.84), 7.088 (13.57), 7.104 (5.97), 7.109 (3.57), 7.124 (9.13), 7.130 (8.00), 7.163 (16.00), 7.183 (8.15), 7.393 (3.54), 7.494 (14.70), 7.515 (13.78), 7.524 (8.16), 7.655 (2.90), 7.958 (13.69).

### Example 25

### 1-(1-{4-Chloro-4'-[4-(2,2-difluoropropyl)piperazin-1-yl][biphenyl]-2-yl}piperidin-3-yl)-5-(difluoromethyl)-1H-pyrazole-4-carboxylic acid hydrochloride (Enantiomer 2)

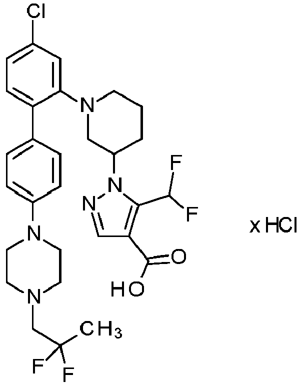

A solution of ethyl 1-[1-[5-chloro-2-[4-[4-(2,2-difluoropropyl)piperazin-1-yl]phenyl]phenyl]-3-piperidyl]-5-(difluoromethyl)pyrazole-4-carboxylate (Example 59A, Enantiomer 2, 73.0 mg, 117 µmol) in a THF/methanol mixture (10:1, 2.5 ml) was treated with lithium hydroxide (1.2 ml, 1 N, 1.2 mmol). The resulting mixture was stirred for 5 days at room temperature. The reaction mixture was acidified with an aqueous solution of hydrogen chloride (1 N) and the aqueous solution was extracted three times with ethyl acetate. The combined organic layers were dried over sodium sulfate and evaporated. The residue was purified by preparative HPLC (RP18 column, eluent: Acetonitrile/water + 0.1% formic acid gradient) The product containing fractions were lyophilized after addition of an aqueous solution of hydrogen chloride (1 N) affording 71.9 mg (97 % yield) of the title compound
LC-MS (Method 3): Rₜ = 2.54 min; MS (ESIpos): m/z = 594 [M-HCl+H]⁺
¹H-NMR (400 MHz, DMSO-d6) δ [ppm]: -0.149 (0.96), 0.146 (0.96), 1.488 (1.21), 1.519 (1.36), 1.718 (1.98), 1.767 (3.58), 1.817 (6.08), 1.865 (3.19), 1.890 (1.44), 1.920 (1.56), 1.965 (2.36), 2.580 (2.37), 2.609 (1.31), 2.998 (1.97), 3.025 (4.94), 3.051 (3.48), 3.222 (3.01), 3.242 (2.75), 3.354 (2.02), 3.531 (16.00), 4.439 (1.18), 4.466 (2.07), 4.492 (1.10), 7.051 (3.97), 7.071 (4.49), 7.082 (7.96), 7.086 (9.06), 7.103 (3.89), 7.108 (2.42), 7.123 (6.08), 7.128 (5.30), 7.162 (10.05), 7.182 (5.00), 7.392 (1.99), 7.492 (8.89), 7.513 (8.42), 7.522 (4.83), 7.653 (1.70), 7.958 (10.43).

### Example 26

### 1-[1-(4-Chloro-4'-{4-[2-(trifluoromethoxy)ethyl]piperazin-1-yl}[biphenyl]-2-yl)piperidin-3-yl]-5-(difluoromethyl)-1H-pyrazole-4-carboxylic acid hydrochloride (Enantiomer 2)

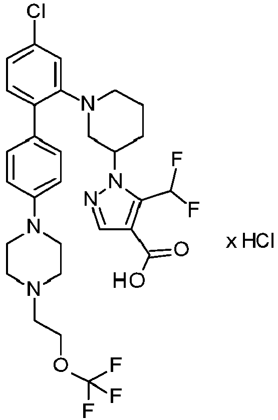

A solution of ethyl 1-[1-[5-chloro-2-[4-[4-[2-(trifluoromethoxy)ethyl]piperazin-1-yl]phenyl]phenyl]-3-piperidyl]-5-(difluoromethyl)pyrazole-4-carboxylate (Example 60A, Enantiomer 2, 30.0 mg, 45.7 µmol) in a THF/methanol mixture (10:1, 960 µl) was treated with lithium hydroxide (460 µl, 1 N, 460µmol). The resulting mixture was stirred overnight at room temperature. The reaction mixture was acidified with an aqueous solution of hydrogen chloride (1 N) and the solution was purified by preparative HPLC (RP18 column, eluent: Acetonitrile/water gradient) The product containing fractions were lyophilized after addition of an aqueous solution of hydrogen chloride (1 N) affording 17.2 mg (57 % yield) of the title compound
LC-MS (Method 3): Rₜ = 1.96 min; MS (ESIpos): m/z = 628 [M-HCl+H]⁺
¹H-NMR (600 MHz, DMSO-d6) δ [ppm]: 1.495 (2.10), 1.515 (2.29), 1.721 (2.95), 1.743 (2.52), 1.901 (2.21), 1.907 (2.13), 1.922 (2.44), 1.942 (1.23), 1.976 (3.09), 1.991 (1.91), 2.425 (0.68), 2.560 (2.51), 2.577 (3.82), 2.597 (1.92), 2.653 (0.47), 3.016 (3.29), 3.034 (8.88), 3.052 (5.98), 3.248 (5.42), 3.606 (12.34), 3.899 (3.51), 4.157 (7.03), 4.454 (2.29), 4.472 (3.54), 4.491 (2.13), 4.628 (7.35), 4.636 (11.46), 4.644 (7.29), 7.054 (12.91), 7.069 (13.76), 7.081 (11.61), 7.104 (5.14), 7.118 (7.28), 7.121 (6.63), 7.164 (12.57), 7.178 (8.08), 7.437 (3.18), 7.487 (14.60), 7.502 (13.61), 7.524 (6.30), 7.611 (2.69), 7.952 (16.00), 11.359 (0.80).

### Example 27

### 1-{1-[4-Chloro-4'-(4-{[2,2-difluorocyclopropyl]methyl}piperazin-1-yl)[biphenyl]-2-yl]piperidin-3-yl}-5-(difluoromethyl)-1H-pyrazole-4-carboxylic acid hydrochloride (Diastereomeric mixture 2)

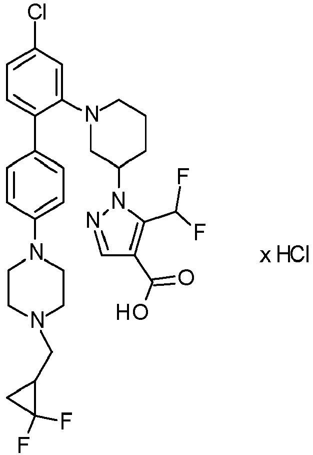

An aqueous solution of lithium hydroxide (630 µl, 1.0 M, 630 µmol) was added to a solution of ethyl 1-{1-[4-chloro-4'-(4-{[2,2-difluorocyclopropyl]methyl}piperazin-1-yl)[1,1'-biphenyl]-2-yl]piperidin-3-yl}-5-(difluoromethyl)-1H-pyrazole-4-carboxylate (Example 69A, diastereomeric mixture 2, 40.0 mg, 63.1 µmol) in a THF/methanol mixture (10/1, 1.4 ml). The resulting mixture was stirred overnight at room temperature. The reaction mixture was acidified with an aqueous solution of hydrogen chloride (2 N) and evaporated. The residue was purified by preparative HPLC (RP18 column, eluent: Acetonitrile/water gradient). The product containing fractions were lyophilized after addition of an aqueous solution of hydrogen chloride (1 N) affording 33.8 mg (82 % yield) of the title compound.
LC-MS (Method 3): Rₜ = 1.74 min; MS (ESIpos): m/z = 606 [M-Cl+H]⁺
¹H-NMR(600 MHz, DMSO-d6) δ [ppm]: 1.237 (0.56), 1.258 (0.90), 1.498 (1.94), 1.520 (2.16), 1.663 (2.03), 1.725 (2.80), 1.746 (2.30), 1.877 (2.75), 1.885 (2.62), 1.899 (3.98), 1.918 (2.80), 1.940 (1.16), 1.976 (2.83), 2.239 (1.22), 2.259 (2.17), 2.271 (2.10), 2.385 (0.87), 2.423 (1.17), 2.566 (2.20), 2.585 (3.39), 2.602 (1.84), 2.652 (0.88), 3.012 (3.12), 3.030 (6.25), 3.047 (5.44), 3.059 (2.85), 3.165 (5.76), 3.228 (4.83), 3.240 (4.67), 3.612 (3.85), 3.880 (1.33), 3.913 (2.47), 4.450 (1.73), 4.469 (2.84), 4.487 (1.52), 7.062 (12.88), 7.077 (14.52), 7.081 (11.91), 7.085 (12.21), 7.106 (5.31), 7.109 (4.03), 7.120 (7.62), 7.123 (6.73), 7.164 (13.35), 7.178 (8.04), 7.248 (0.53), 7.433 (2.86), 7.492 (14.64), 7.507 (13.35), 7.521 (5.56), 7.607 (2.31), 7.953 (16.00), 10.906 (1.49).

### Example 28

### 1-{1-[4-Chloro-4'-(4-{[2,2-difluorocyclopropyl]methyl}piperazin-1-yl)[biphenyl]-2-yl]piperidin-3-yl}-5-(difluoromethyl)-1H-pyrazole-4-carboxylic acid hydrochloride (Diastereomer 1)

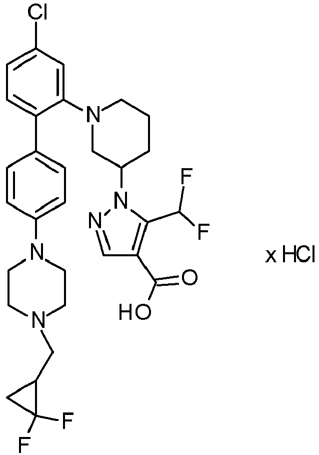

An aqueous solution of lithium hydroxide (300 µl, 1.0 M, 300 µmol) was added to a solution of ethyl 1-{1-[4-chloro-4'-(4-{[2,2-difluorocyclopropyl]methyl}piperazin-1-yl)[1,1'-biphenyl]-2-yl]piperidin-3-yl}-5-(difluoromethyl)-1H-pyrazole-4-carboxylate (Example 64A, diastereomer 1, 19.0 mg, 30.0 µmol) in a THF/methanol mixture (10/1, 660 µl). The resulting mixture was stirred 3 days at room temperature. The reaction mixture was acidified with an aqueous solution of hydrogen chloride (1 N) and evaporated. The residue was purified by preparative HPLC (RP18 column, eluent: Acetonitrile/water gradient). The product containing fractions were lyophilized after addition of an aqueous solution of hydrogen chloride (1 N) affording 12.3 mg (63 % yield) of the title compound.
LC-MS (Method 3): Rₜ = 1.73 min; MS (ESIpos): m/z = 606 [M-HCl+H]⁺
¹H-NMR (400 MHz, DMSO-d6) δ [ppm]: 1.236 (0.51), 1.493 (1.39), 1.524 (1.63), 1.635 (1.51), 1.722 (2.25), 1.755 (1.95), 1.888 (3.01), 1.919 (2.60), 1.967 (2.62), 2.243 (1.42), 2.367 (0.82), 2.585 (3.28), 2.614 (1.69), 2.711 (0.85), 2.999 (1.99), 3.026 (4.74), 3.051 (3.87), 3.149 (4.11), 3.221 (4.36), 3.241 (4.00), 3.615 (3.00), 3.920 (2.99), 4.464 (2.22), 7.016 (0.55), 7.061 (8.79), 7.083 (16.00), 7.104 (3.83), 7.125 (6.00), 7.162 (8.85), 7.182 (4.43), 7.271 (0.56), 7.389 (2.10), 7.491 (10.36), 7.513 (10.19), 7.651 (1.83), 7.958 (9.81), 10.698 (0.57), 13.199 (0.60).

### Example 29

### 1-{1-[4-Chloro-4'-(4-{[2,2-difluorocyclopropyl]methyl}piperazin-1-yl)[biphenyl]-2-yl]piperidin-3-yl}-5-(difluoromethyl)-1H-pyrazole-4-carboxylic acid hydrochloride (Diastereomer 2)

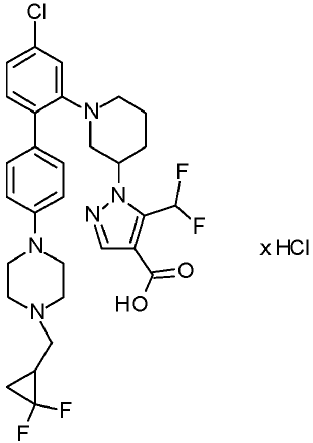

An aqueous solution of lithium hydroxide (330 µl, 1.0 M, 330 µmol) was added to a solution of ethyl 1-{1-[4-chloro-4'-(4-{[2,2-difluorocyclopropyl]methyl}piperazin-1-yl)[1,1'-biphenyl]-2-yl]piperidin-3-yl}-5-(difluoromethyl)-1H-pyrazole-4-carboxylate (Example 65A, diastereoisomer 2, 21.0 mg, 33.1 µmol) in a THF/methanol mixture (10/1, 730 µl). The resulting mixture was stirred 3 days at room temperature. The reaction mixture was acidified with an aqueous solution of hydrogen chloride (1 N) and evaporated. The residue was purified by preparative HPLC (RP18 column, eluent: Acetonitrile/water gradient). The product containing fractions were lyophilized after addition of an aqueous solution of hydrogen chloride (1 N) affording 9.80 mg (46 % yield) of the title compound.
LC-MS (Method 3): Rₜ = 1.72 min; MS (ESIpos): m/z = 606 [M-HCl+H]⁺
¹H-NMR (400 MHz, DMSO-d6) δ [ppm]: 1.235 (0.54), 1.492 (1.37), 1.523 (1.62), 1.643 (1.41), 1.722 (2.21), 1.754 (1.82), 1.887 (2.88), 1.917 (2.53), 1.970 (2.51), 2.233 (1.34), 2.249 (1.32), 2.366 (0.98), 2.586 (3.19), 2.615 (1.67), 2.710 (1.13), 2.998 (2.13), 3.024 (4.79), 3.050 (4.19), 3.069 (3.18), 3.112 (3.24), 3.141 (3.42), 3.221 (4.24), 3.240 (3.79), 3.620 (2.88), 3.888 (1.61), 3.944 (1.77), 4.436 (1.30), 4.463 (2.28), 4.488 (1.20), 7.000 (1.46), 7.061 (8.71), 7.083 (16.00), 7.104 (3.84), 7.125 (7.10), 7.161 (9.98), 7.182 (4.91), 7.255 (1.46), 7.387 (2.53), 7.492 (10.56), 7.514 (10.99), 7.649 (2.19), 7.958 (9.59), 10.529 (1.00), 13.192 (0.64).

### Example 30

### 1-{1-[4-Chloro-4'-(4-{[2,2-difluorocyclopropyl]methyl}piperazin-1-yl)[biphenyl]-2-yl]piperidin-3-yl}-5-(difluoromethyl)-1H-pyrazole-4-carboxylic acid hydrochloride (Diastereomer 3)

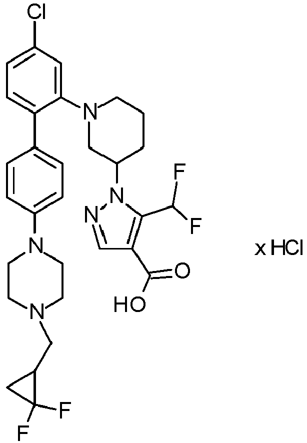

An aqueous solution of lithium hydroxide (550 µl, 1.0 M, 550 µmol) was added to a solution of ethyl 1-{1-[4-chloro-4'-(4-{[2,2-difluorocyclopropyl]methyl}piperazin-1-yl)[1,1'-biphenyl]-2-yl]piperidin-3-yl}-5-(difluoromethyl)-1H-pyrazole-4-carboxylate (Example 70A, Diastereomer 3, 35.0 mg, 55.2 µmol) in a THF/methanol mixture (10/1, 1.2 ml). The resulting mixture was stirred 3 hours at room temperature. The reaction mixture was acidified with an aqueous solution of hydrogen chloride (1 N) and evaporated. The residue was purified by preparative HPLC (RP18 column, eluent: Acetonitrile/water gradient). The product containing fractions were lyophilized after addition of an aqueous solution of hydrogen chloride (1 N) affording 23.5 mg (66 % yield) of the title compound.
LC-MS (Method 8): Rₜ = 0.91 min; MS (ESIpos): m/z = 606 [M-HCl+H]⁺
¹H-NMR(600 MHz, DMSO-d6) δ [ppm]: 1.497 (1.79), 1.518 (1.91), 1.663 (1.88), 1.677 (1.91), 1.723 (2.40), 1.745 (1.95), 1.856 (0.87), 1.877 (2.32), 1.885 (2.31), 1.898 (3.29), 1.919 (2.39), 1.941 (1.02), 1.977 (2.51), 2.240 (0.52), 2.252 (1.25), 2.265 (1.54), 2.273 (2.08), 2.285 (2.05), 2.293 (1.37), 2.305 (1.12), 2.423 (1.03), 2.514 (1.20), 2.562 (1.59), 2.570 (1.19), 2.578 (3.15), 2.599 (1.71), 2.652 (0.89), 3.015 (3.03), 3.033 (6.94), 3.051 (4.52), 3.177 (5.46), 3.190 (5.22), 3.208 (4.38), 3.225 (4.48), 3.242 (3.74), 3.355 (5.69), 3.604 (6.94), 3.874 (2.64), 3.894 (2.67), 3.937 (2.65), 3.954 (2.01), 4.455 (1.63), 4.472 (2.74), 4.491 (1.46), 7.061 (13.16), 7.076 (14.52), 7.079 (11.61), 7.083 (12.16), 7.105 (5.58), 7.108 (4.06), 7.118 (7.70), 7.121 (6.73), 7.165 (14.18), 7.179 (8.73), 7.436 (2.91), 7.491 (15.16), 7.505 (13.84), 7.524 (5.78), 7.611 (2.39), 7.951 (16.00), 11.165 (1.34).

### Example 31

### 1-{1-[4-Chloro-4'-(4-{[2,2-difluorocyclopropyl]methyl}piperazin-1-yl)[biphenyl]-2-yl]piperidin-3-yl}-5-(difluoromethyl)-1H-pyrazole-4-carboxylic acid hydrochloride (Diastereomer 4)

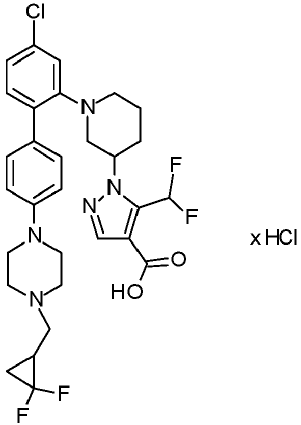

An aqueous solution of lithium hydroxide (930 µl, 1.0 M, 930 µmol) was added to a solution of ethyl 1-{1-[4-chloro-4'-(4-{[2,2-difluorocyclopropyl]methyl}piperazin-1-yl)[1,1'-biphenyl]-2-yl]piperidin-3-yl}-5-(difluoromethyl)-1H-pyrazole-4-carboxylate (Example 71A, diastereomer 4, 58.8 mg, 92.7 µmol) in a THF/methanol mixture (10/1, 2.1 ml). The resulting mixture was stirred 3 hours at room temperature. The reaction mixture was acidified with an aqueous solution of hydrogen chloride (1 N) and evaporated. The residue was purified by preparative HPLC (RP18 column, eluent: Acetonitrile/water gradient). The product containing fractions were lyophilized after addition of an aqueous solution of hydrogen chloride (1 N) affording 30.1 mg (51 % yield) of the title compound.
LC-MS (Method 3): Rₜ = 1.76 min; MS (ESIpos): m/z = 606 [M+H]⁺
¹H-NMR(600 MHz, DMSO-d6) δ [ppm]: 1.497 (2.30), 1.518 (2.74), 1.679 (2.80), 1.723 (3.53), 1.745 (3.08), 1.899 (4.39), 1.919 (3.35), 1.975 (3.77), 2.275 (2.70), 2.286 (2.79), 2.384 (0.99), 2.423 (1.06), 2.579 (7.80), 2.600 (5.68), 2.652 (2.94), 3.014 (3.50), 3.032 (8.26), 3.049 (6.08), 3.173 (7.01), 3.222 (8.18), 3.242 (6.06), 3.353 (8.56), 3.603 (6.99), 3.906 (13.12), 3.924 (11.04), 4.471 (3.66), 7.060 (13.80), 7.075 (16.00), 7.079 (14.43), 7.082 (13.69), 7.104 (6.10), 7.107 (4.92), 7.118 (8.59), 7.121 (7.47), 7.165 (13.38), 7.178 (8.72), 7.435 (3.44), 7.489 (15.79), 7.504 (14.34), 7.522 (6.62), 7.609 (2.84), 7.951 (15.74), 11.205 (2.05).

### Example 32

### 1-[1-[5-chloro-2-[4-[4-[(trans-2-fluorocyclopropyl)methyl]piperazin-1-yl]phenyl]phenyl]-3-piperidyl]-5-(difluoromethyl)pyrazole-4-carboxylic acid hydrochloride (Diastereoisomeric mixture 2)

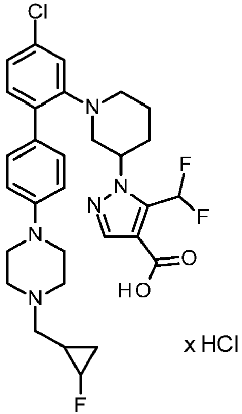

A solution of ethyl 1-[1-[5-chloro-2-[4-[4-[(2-fluorocyclopropyl)methyl]piperazin-1-yl]phenyl]phenyl]-3-piperidyl]-5-(difluoromethyl)pyrazole-4-carboxylate (Example 82A, Enantiomer 2, 40.0 mg, 64.9 µmol) in a THF/methanol mixture (10/1, 1.4 ml) and water (1.4 ml) was treated with lithium hydroxide monohydrate (27.2 mg, 649 µmol). The resulting mixture was stirred overnight at room temperature. The reaction mixture was acidified with an aqueous solution of hydrogen chloride (1 N), diluted with acetonitrile and purified by preparative HPLC (RP18 column, eluent: Acetonitrile/water gradient). The product containing fractions were lyophilized after addition of an aqueous solution of hydrogen chloride (1 N) affording 40.0 mg (93 % purity, 92 % yield) of the title compound.
LC-MS (Method 4): Rₜ = 0.89 min; MS (ESIpos): m/z = 588 [M-HCl+H]⁺
¹H-NMR (400 MHz, DMSO-d6) δ [ppm]: 0.838 (0.96), 0.855 (2.61), 0.872 (2.89), 0.883 (2.88), 0.900 (2.52), 0.917 (0.82), 1.180 (0.67), 1.235 (1.92), 1.252 (1.34), 1.261 (1.87), 1.288 (1.77), 1.316 (1.77), 1.324 (1.32), 1.335 (1.36), 1.493 (1.54), 1.522 (1.72), 1.594 (0.43), 1.662 (0.96), 1.727 (3.40), 1.755 (2.74), 1.887 (1.69), 1.918 (1.89), 1.966 (2.66), 2.365 (1.57), 2.584 (3.36), 2.709 (1.64), 2.996 (2.61), 3.022 (6.15), 3.048 (6.18), 3.069 (4.86), 3.096 (3.74), 3.127 (7.05), 3.150 (8.07), 3.210 (4.74), 3.238 (3.59), 3.603 (5.69), 3.912 (3.34), 3.945 (1.70), 4.435 (1.47), 4.461 (2.56), 4.727 (2.12), 4.741 (1.97), 4.887 (2.07), 4.901 (2.03), 7.056 (12.14), 7.079 (16.00), 7.086 (13.26), 7.103 (5.60), 7.108 (3.24), 7.123 (8.95), 7.128 (7.63), 7.160 (15.08), 7.181 (7.38), 7.387 (3.07), 7.491 (15.07), 7.513 (14.36), 7.518 (8.00), 7.546 (2.17), 7.555 (1.75), 7.564 (2.33), 7.572 (1.97), 7.595 (2.84), 7.613 (2.34), 7.625 (3.25), 7.649 (3.09), 7.956 (14.67), 10.410 (1.40).

### Example 33

### 1-[1-[5-Chloro-2-[4-[4-[(trans-2-fluorocyclopropyl)methyl]piperazin-1-yl]phenyl]phenyl]-3-piperidyl]-5-(difluoromethyl)pyrazole-4-carboxylic acid hydrochloride (Diastereomer 3)

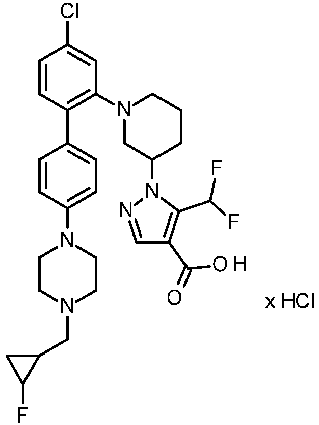

A solution of ethyl 1-[1-[5-chloro-2-[4-[4-[(2-fluorocyclopropyl)methyl]piperazin-1-yl]phenyl]phenyl]-3-piperidyl]-5-(difluoromethyl)pyrazole-4-carboxylate (Example 75A, diastereomer 3, 16.0 mg, 26.0 µmol) in THF/Methanol (10:1, 730 µL) was treated with lithium hydroxide monohydrate (10.9 mg, 260 µmol) and water (730 µL). The reaction mixture was stirred overnight at room temperature, acidifed with an aqueous solution of hydrogen chloride (1N) and evaporated. The crude material obtained was then submitted to preparative RP-HPLC 125x30mm with acetonitrile/water (neutral). The product containing fractions were lyophilized after addition of an aqueous solution of hydrogen chloride (1 N) affording 15.0 mg (92 % yield) of the title compound.
LC-MS (Method 3): Rₜ = 1.69 min; MS (ESIpos): m/z = 588 [M-HCl+H]⁺
¹H-NMR (400 MHz, DMSO-d6) δ [ppm]: -0.149 (0.54), 0.146 (0.55), 0.853 (0.71), 0.869 (1.96), 0.886 (2.25), 0.898 (2.22), 0.914 (1.97), 0.930 (0.72), 1.164 (0.74), 1.232 (1.25), 1.259 (1.52), 1.286 (1.46), 1.314 (1.53), 1.321 (1.15), 1.332 (0.99), 1.339 (0.97), 1.491 (1.47), 1.524 (1.65), 1.678 (0.90), 1.721 (3.62), 1.751 (2.58), 1.867 (0.55), 1.888 (1.52), 1.919 (1.78), 1.973 (2.48), 2.367 (1.05), 2.580 (2.89), 2.612 (1.49), 2.711 (1.08), 2.731 (0.70), 2.891 (0.88), 3.001 (2.40), 3.027 (6.44), 3.054 (5.67), 3.089 (2.16), 3.104 (2.68), 3.124 (3.46), 3.152 (6.34), 3.171 (6.76), 3.188 (6.01), 3.214 (3.88), 3.243 (2.61), 3.862 (2.85), 3.887 (2.58), 3.910 (2.64), 3.936 (2.63), 4.441 (1.32), 4.468 (2.31), 4.494 (1.23), 4.738 (1.78), 4.897 (1.72), 4.912 (1.78), 7.057 (10.65), 7.080 (16.00), 7.086 (11.24), 7.104 (4.57), 7.109 (2.74), 7.124 (7.27), 7.129 (6.11), 7.163 (12.96), 7.183 (6.55), 7.394 (2.75), 7.491 (12.63), 7.513 (11.27), 7.525 (5.61), 7.656 (2.23), 7.958 (12.74), 10.720 (0.67).

### Example 34

### 1-[1-[5-Chloro-2-[4-[4-[(trans-2-fluorocyclopropyl)methyl]piperazin-1-yl]phenyl]phenyl]-3-piperidyl]-5-(difluoromethyl)pyrazole-4-carboxylic acid hydrochloride (Diastereomer 4)

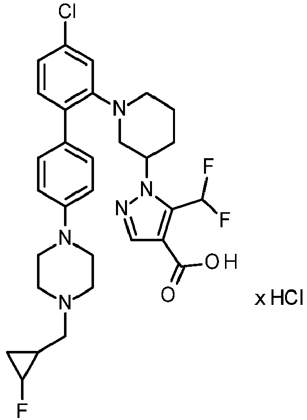

A solution of ethyl 1-[1-[5-chloro-2-[4-[4-[(2-fluorocyclopropyl)methyl]piperazin-1-yl]phenyl]phenyl]-3-piperidyl]-5-(difluoromethyl)pyrazole-4-carboxylate (Example 76A, diastereomer 4, 22.0 mg, 35.7 µmol) in THF/methanol (10:1, 1.0 mL) was treated with lithium hydroxide monohydrate (15.0 mg, 357 µmol) and water (1.0 mL). The reaction mixture was stirred overnight at room temperature, acidifed with an aqueous solution of hydrogen chloride (1N) and evaporated. The crude material obtained was then submitted to preparative RP-HPLC 125x30mm with acetonitrile/water (neutral). The product containing fractions were lyophilized after addition of an aqueous solution of hydrogen chloride (1 N) affording 20.0 mg (90 % yield) of the title compound.
LC-MS (Method 3): Rₜ = 1.70 min; MS (ESIpos): m/z = 588 [M-HCl+H]⁺
¹H-NMR (400 MHz, DMSO-d6) δ [ppm]: -0.149 (0.50), 0.146 (0.63), 0.866 (2.47), 0.882 (2.79), 0.894 (2.74), 0.910 (2.35), 1.259 (1.60), 1.286 (1.61), 1.312 (1.63), 1.522 (1.84), 1.720 (4.19), 1.890 (1.76), 1.919 (1.94), 1.967 (2.83), 2.368 (1.32), 2.583 (3.21), 2.611 (1.72), 2.711 (1.03), 3.000 (2.38), 3.028 (6.82), 3.053 (6.33), 3.121 (3.65), 3.155 (8.18), 3.178 (9.39), 3.213 (3.93), 3.242 (2.80), 3.903 (4.84), 4.467 (2.63), 4.736 (2.21), 4.911 (2.15), 7.057 (10.15), 7.080 (16.00), 7.104 (4.11), 7.125 (6.82), 7.163 (11.32), 7.183 (5.63), 7.394 (3.06), 7.492 (11.76), 7.514 (10.76), 7.525 (6.29), 7.655 (2.51), 7.958 (13.62).

### Example 35

### 1-[1-[5-Chloro-2-[4-[4-[(1R,2R)-2-fluorocyclopropanecarbonyl]piperazin-1-yl]phenyl]phenyl]-3-piperidyl]-5-(difluoromethyl)pyrazole-4-carboxylic acid (Stereoisomer 2)

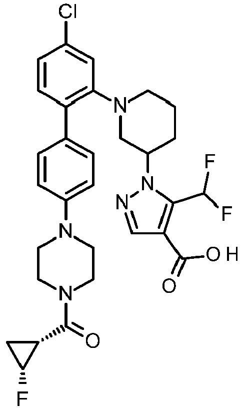

A solution of ethyl 1-[1-[5-chloro-2-[4-[4-[(1R,2R)-2-fluorocyclopropanecarbonyl]piperazin-1-yl]phenyl]phenyl]-3-piperidyl]-5-(difluoromethyl)pyrazole-4-carboxylate (Example 83A, Stereoisomer 2, 104 mg, 165 µmol) in THF/methanol (10:1, 2.0 mL) was treated with an aqueous solution of lithium hydroxide (1.7 ml, 1.0 M, 1.7 mmol). The reaction mixture was stirred overnight at room temperature, acidifed with an aqueous solution of hydrogen chloride (1N) and evaporated. The crude material obtained was then submitted to The crude material obtained was then submitted to preparative RP-HPLC 125x30mm with acetonitrile/water (neutral), to deliver 11.0 mg (11 % yield) of the title compound.
LC-MS (Method 3): Rₜ = 2.32 min; MS (ESIpos): m/z = 602 [M+H]⁺
¹H-NMR (400 MHz, DMSO-d6) δ [ppm]: -0.150 (0.73), 0.146 (0.72), 0.831 (0.72), 0.853 (0.53), 0.991 (1.23), 1.006 (2.35), 1.014 (1.64), 1.022 (2.62), 1.030 (2.47), 1.038 (2.61), 1.045 (2.76), 1.053 (1.62), 1.061 (2.27), 1.076 (1.34), 1.169 (3.92), 1.175 (2.71), 1.234 (2.95), 1.351 (0.53), 1.495 (2.69), 1.503 (2.56), 1.512 (3.77), 1.520 (4.27), 1.530 (3.09), 1.537 (2.47), 1.553 (2.50), 1.561 (2.16), 1.570 (2.94), 1.578 (2.56), 1.588 (1.73), 1.596 (1.40), 1.703 (2.69), 1.735 (2.13), 1.861 (0.73), 1.883 (1.88), 1.913 (2.12), 1.978 (2.85), 1.988 (3.18), 2.183 (1.46), 2.200 (3.36), 2.218 (3.54), 2.223 (3.68), 2.240 (3.07), 2.257 (1.19), 2.328 (1.29), 2.366 (2.15), 2.582 (2.24), 2.670 (1.35), 2.710 (2.06), 3.005 (2.77), 3.032 (7.72), 3.059 (5.96), 3.088 (2.40), 3.160 (2.59), 3.187 (3.37), 3.234 (4.40), 3.269 (3.57), 3.624 (2.30), 3.680 (2.43), 3.816 (4.66), 4.020 (1.05), 4.038 (1.05), 4.458 (1.51), 4.484 (2.68), 4.512 (1.37), 4.839 (1.39), 4.848 (1.55), 4.855 (2.19), 4.864 (2.14), 4.871 (1.40), 4.879 (1.23), 5.006 (1.20), 5.014 (1.36), 5.022 (2.12), 5.030 (2.10), 5.037 (1.60), 5.045 (1.33), 5.754 (4.32), 7.011 (13.35), 7.033 (14.51), 7.061 (9.85), 7.066 (12.37), 7.089 (5.83), 7.094 (3.79), 7.110 (8.76), 7.115 (7.61), 7.160 (15.48), 7.181 (8.80), 7.382 (3.10), 7.458 (16.00), 7.480 (14.22), 7.513 (6.01), 7.644 (2.48), 7.951 (12.38), 13.181 (1.32).

### Example 36

### 1-{1-[4-chloro-4'-(4-{[(1S,2S)-2-fluorocyclopropyl]methyl}piperazin-1-yl)[biphenyl]-2-yl]piperidin-3-yl}-5-(difluoromethyl)-1H-pyrazole-4-carboxylic acid hydrochloride (Stereoisomer 2)

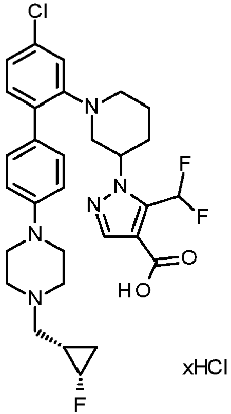

A solution of ethyl 1-[1-[5-chloro-2-[4-[4-[[(1S,2S)-2-fluorocyclopropyl]methyl]piperazin-1-yl]phenyl]phenyl]-3-piperidyl]-5-(difluoromethyl)pyrazole-4-carboxylate (Example 79A, Stereoisomer 2, 78.0 mg, 127 µmol) in a THF/methanol mixture (10:1, 2.8 ml) and water (2.8 ml) was treated with lithium hydroxide monohydrate (53.1 mg, 127 mmol). The resulting mixture was stirred overnight at room temperature. The reaction mixture was acidified with an aqueous solution of hydrogen chloride (1 N), diluted with acetonitrile and purified by preparative HPLC (RP18 column, eluent: Acetonitrile/water gradient). The product containing fractions were lyophilized after addition of an aqueous solution of hydrogen chloride (1 N) affording 41.0 mg (52 % yield) of the title compound.
LC-MS (Method 3): Rₜ = 1.67 min; MS (ESIpos): m/z = 588 [M-HCl+H]⁺
¹H-NMR (400 MHz, DMSO-d6) δ [ppm]: 0.938 (1.40), 0.957 (0.94), 0.997 (1.42), 1.019 (1.49), 1.035 (1.15), 1.051 (1.52), 1.059 (1.53), 1.076 (1.69), 1.090 (1.26), 1.108 (0.62), 1.364 (1.39), 1.494 (1.15), 1.524 (1.35), 1.724 (1.87), 1.757 (1.42), 1.866 (0.45), 1.888 (1.31), 1.918 (1.51), 1.968 (2.08), 2.329 (0.89), 2.367 (1.38), 2.587 (2.45), 2.616 (1.27), 2.671 (0.90), 2.675 (0.69), 2.711 (1.34), 2.997 (1.92), 3.023 (4.09), 3.049 (3.75), 3.069 (1.99), 3.119 (3.44), 3.138 (4.91), 3.221 (3.73), 3.249 (4.19), 3.277 (2.36), 3.910 (3.29), 3.945 (2.22), 4.437 (1.13), 4.463 (1.99), 4.490 (1.00), 4.858 (0.91), 4.865 (1.03), 4.872 (1.87), 4.879 (1.84), 4.887 (1.03), 4.894 (0.84), 5.021 (0.80), 5.028 (0.94), 5.036 (1.82), 5.042 (1.72), 5.050 (1.08), 5.057 (0.93), 7.060 (9.21), 7.082 (16.00), 7.087 (10.54), 7.104 (4.17), 7.109 (2.48), 7.124 (6.41), 7.130 (5.66), 7.163 (11.95), 7.183 (5.99), 7.389 (2.15), 7.491 (11.17), 7.512 (10.65), 7.519 (5.48), 7.650 (1.84), 7.957 (9.88).

### Example 37

### 1-[1-[5-Chloro-2-[4-[4-[[(1R2R)-2-fluorocyclopropyl]methyl]piperazin-1-yl]phenyl]phenyl]-3-piperidyl]-5-(difluoromethyl)pyrazole-4-carboxylic acid hydrochloride (Stereoisomer 2)

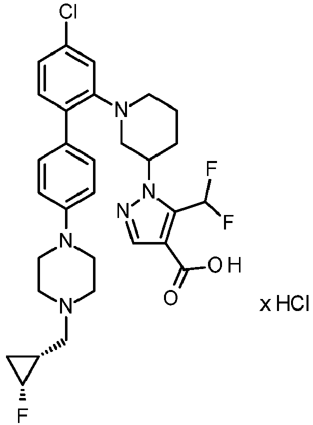

A solution of ethyl 1-[1-[5-chloro-2-[4-[4-[[(1R,2S)-2-fluorocyclopropyl]methyl]piperazin-1-yl]phenyl]phenyl]-3-piperidyl]-5-(difluoromethyl)pyrazole-4-carboxylate (Example 84A, Stereoisomer 2, 16.0 mg, 26.0 µmol) in THF/Methanol (10:1, 730 µL) was treated with lithium hydroxide monohydrate (10.9 mg, 260 µmol) and water (730 µL). The reaction mixture was stirred overnight at room temperature, acidifed with an aqueous solution of hydrogen chloride (1N) and evaporated. The crude material obtained was then submitted to preparative RP-HPLC 125x30mm with acetonitrile/water (neutral). The product containing fractions were lyophilized after addition of an aqueous solution of hydrogen chloride (1 N) affording 13.0 mg (80 % yield) of the title compound.
LC-MS (Method 3): Rₜ = 1.70 min; MS (ESIpos): m/z = 588 [M-HCl+H]⁺
¹H-NMR (400 MHz, DMSO-d6) δ [ppm]: -0.149 (0.90), 0.146 (0.82), 0.935 (1.64), 0.942 (1.68), 0.953 (1.05), 0.994 (1.60), 1.000 (1.55), 1.019 (1.45), 1.036 (1.17), 1.053 (1.53), 1.062 (1.59), 1.077 (1.70), 1.092 (1.31), 1.110 (0.61), 1.353 (1.54), 1.495 (1.32), 1.524 (1.40), 1.724 (1.97), 1.756 (1.84), 1.868 (0.46), 1.887 (1.33), 1.920 (1.51), 1.966 (2.18), 2.328 (1.15), 2.367 (1.83), 2.588 (2.93), 2.618 (1.39), 2.671 (1.33), 2.711 (2.03), 2.996 (1.94), 3.022 (4.17), 3.048 (3.97), 3.073 (2.39), 3.120 (3.94), 3.143 (5.48), 3.220 (3.62), 3.250 (4.58), 3.274 (3.16), 3.630 (2.76), 3.667 (3.96), 3.693 (2.10), 3.880 (2.22), 3.917 (2.35), 3.939 (2.11), 3.964 (2.26), 4.437 (1.13), 4.463 (2.10), 4.874 (1.85), 4.880 (1.77), 5.037 (1.83), 5.043 (1.70), 5.051 (1.14), 7.061 (8.82), 7.083 (16.00), 7.105 (3.92), 7.125 (6.22), 7.130 (5.25), 7.162 (10.98), 7.182 (5.40), 7.387 (2.40), 7.492 (10.57), 7.514 (10.74), 7.649 (2.00), 7.958 (9.49), 10.429 (0.47).

### Example 38

### 1-{1-[4-Chloro-4'-(4-{[cis-2-(trifluoromethyl)cyclopropyl]methyl}piperazin-1-yl)[biphenyl]-2-yl]piperidin-3-yl}-5-(difluoromethyl)-1H-pyrazole-4-carboxylic acid hydrochloride (Diastereoisomeric mixture 1)

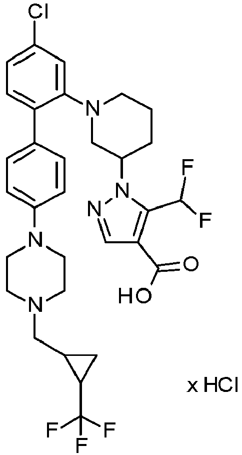

Under argon, a solution of ethyl 1-[1-(5-chloro-2-{[(trifluoromethyl)sulfonyl]oxy}phenyl)piperidin-3-yl]-5-(difluoromethyl)-1H-pyrazole-4-carboxylate (prepared in analogy to Example 11A, Enantiomer 1, 70.0 mg, 132 µmol) and 1-[4-(4,4,5,5-tetramethyl-1,3,2-dioxaborolan-2-yl)phenyl]-4-{[cis-2-(trifluoromethyl)cyclopropyl]methyl}piperazine (Example 87A, 86.4 mg, 75 % purity, 158 µmol) in toluene/ethanol (1:1) (1.9 ml) was treatd with an aqueous solution of sodium carbonate (200 µl, 2 N, 390 µmol) and Pd(PPh₃)₄ (7.60 mg, 6.58 µmol) and the resulting mixture was stirred overnight at 100°C. The reaction mixture was cooled to room temperaute, filtered through Celite, washed with ethyl acetate and concentrated. The residue was purified using preparative HPLC (RP18 column, eluent: Acetonitrile/water + 0.1% formic acid gradient). The product containing fractions were lyophilized after addition of an aqueous solution of hydrogen chloride (1 N) affording 24.8 mg (28 % yield) of the title compound.
LC-MS (Method 3): Rₜ = 1.81 min; MS (ESIpos): m/z = 638 [M-HCl+H]⁺
¹H-NMR (400 MHz, DMSO-d6) δ [ppm]: -0.149 (0.99), -0.008 (8.47), 0.008 (9.19), 0.146 (1.09), 1.073 (1.83), 1.093 (1.93), 1.107 (1.06), 1.147 (1.21), 1.161 (2.22), 1.170 (1.80), 1.183 (2.37), 1.197 (1.04), 1.487 (1.09), 1.519 (1.23), 1.715 (2.69), 1.748 (1.41), 1.890 (1.21), 1.921 (1.38), 1.968 (1.98), 2.145 (1.48), 2.328 (0.72), 2.367 (0.84), 2.578 (2.40), 2.606 (1.23), 2.671 (0.81), 2.711 (0.91), 3.005 (1.80), 3.031 (5.11), 3.058 (3.51), 3.173 (6.10), 3.191 (4.91), 3.219 (3.21), 3.254 (2.67), 3.283 (1.53), 3.301 (1.19), 3.603 (3.93), 4.444 (1.06), 4.470 (1.88), 4.497 (0.96), 7.062 (8.27), 7.085 (16.00), 7.103 (3.60), 7.108 (2.17), 7.123 (5.60), 7.128 (4.79), 7.163 (10.37), 7.183 (5.26), 7.396 (2.17), 7.491 (9.75), 7.512 (8.77), 7.526 (4.37), 7.657 (1.78), 7.959 (9.31), 10.927 (0.49).

### Example 39

### 1-{1-[4-Chloro-4'-(4-{[cis-2-(trifluoromethyl)cyclopropyl]methyl}piperazin-1-yl)[biphenyl]-2-yl]piperidin-3-yl}-5-(difluoromethyl)-1H-pyrazole-4-carboxylic acid hydrochloride (Diastereomeric mixture 2)

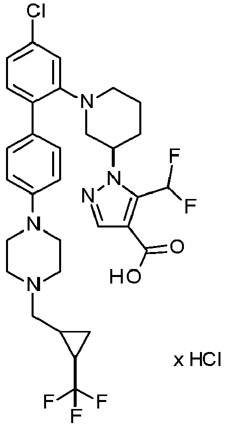

A solution of ethyl 1-[1-[5-chloro-2-[4-[4-[[cis-2-(trifluoromethyl)cyclopropyl]methyl]piperazin-1-yl]phenyl]phenyl]-3-piperidyl]-5-(difluoromethyl)pyrazole-4-carboxylate (Example 88A, Diastereomeric mixture 2, 38.0 mg, 57.0 µmol) in a THF/methanol mixture (10:1, 1.2 ml) was treated with lithium hydroxide (570 µl, 1 N, 570 µmol). The resulting mixture was stirred overnight at room temperature. The reaction mixture was acidified with an aqueous solution of hydrogen chloride (1 N), diluted with water, extracted with ethyl acetate and concentrated. The residue and purified by preparative HPLC (RP18 column, eluent: Acetonitrile/water + 0.1% formic acid gradient). The product containing fractions were lyophilized after addition of an aqueous solution of hydrogen chloride (1 N) affording 35.0 mg (91 % yield) of the title compound.
LC-MS (Method 3): Rₜ = 1.78 min; MS (ESIpos): m/z = 638 [M-HCl+H]⁺
¹H-NMR (600 MHz, DMSO-d6) δ [ppm]: 1.085 (2.27), 1.098 (2.37), 1.108 (1.25), 1.151 (1.63), 1.160 (2.93), 1.166 (2.22), 1.175 (3.02), 1.184 (1.38), 1.494 (1.55), 1.514 (1.69), 1.718 (3.72), 1.740 (2.02), 1.901 (1.55), 1.908 (2.26), 1.921 (1.72), 1.941 (0.85), 1.975 (2.24), 2.148 (1.84), 2.423 (0.94), 2.515 (2.78), 2.574 (2.77), 2.594 (1.46), 2.652 (0.85), 3.017 (2.41), 3.035 (6.68), 3.053 (4.65), 3.159 (2.25), 3.199 (5.54), 3.231 (3.41), 3.250 (3.24), 3.270 (1.92), 3.601 (5.40), 3.868 (2.75), 3.883 (3.26), 3.898 (3.39), 3.912 (2.84), 4.456 (1.33), 4.474 (2.36), 4.492 (1.32), 7.061 (10.74), 7.076 (16.00), 7.081 (11.17), 7.103 (4.70), 7.107 (3.79), 7.117 (6.37), 7.120 (5.80), 7.164 (11.41), 7.178 (7.23), 7.436 (2.42), 7.489 (12.40), 7.503 (11.31), 7.524 (4.82), 7.611 (1.96), 7.951 (12.74), 11.160 (0.93).

### Example 40

### 1-{1-[4-Chloro-4'-(4-{[1-(trifluoromethyl)cyclopropyl]methyl}piperazin-1-yl)[biphenyl]-2-yl]piperidin-3-yl}-5-(difluoromethyl)-1H-pyrazole-4-carboxylic acid hydrochloride (Enantiomer 2)

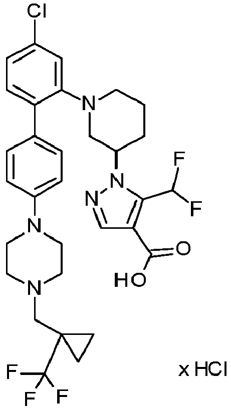

A solution ethyl 1-[1-[5-chloro-2-[4-[4-[[1-(trifluoromethyl)cyclopropyl]methyl]piperazin-1-yl]phenyl]phenyl]-3-piperidyl]-5-(difluoromethyl)pyrazole-4-carboxylate (Example 91A, Enantiomer 2, 10.4 mg, 15.6 µmol) in a THF/Methanol mixture (10:1, 330 µl) was treated with lithium hydroxide (160 µl, 1 N, 160 µ mol). The resulting mixutre was stirred for 4 hours at room temperature, diluted with water and acidified with an aqueous solution of hydrogen chloride (1 N). The aqueous solution was extracted three times with ethyl acetate. The combined organic layers were dried over magnesium sulfate and evaporated. The residue was dissolved in acetonitrile and hydrochloric acid (1 N) and lyophilized affording 10.5 mg (95 % yield) of the title compound.
LC-MS (Method 3): Rₜ = 2.27 min; MS (ESIpos): m/z = 638 [M-HCl+H]⁺
¹H-NMR (400 MHz, DMSO-d6) δ [ppm]: -0.150 (1.39), 0.146 (1.28), 0.853 (2.30), 0.869 (1.79), 1.045 (1.33), 1.110 (1.32), 1.189 (2.25), 1.235 (15.40), 1.268 (16.00), 1.339 (2.50), 1.489 (2.36), 1.521 (2.55), 1.596 (2.72), 1.720 (2.98), 1.752 (2.47), 1.890 (1.98), 1.909 (2.91), 1.921 (2.43), 1.972 (3.73), 2.180 (0.64), 2.328 (2.03), 2.367 (2.62), 2.585 (4.07), 2.612 (2.14), 2.671 (2.35), 2.710 (2.75), 3.001 (3.40), 3.028 (7.67), 3.054 (5.25), 3.222 (9.54), 3.905 (4.74), 4.441 (1.88), 4.467 (3.18), 7.046 (11.14), 7.068 (12.20), 7.088 (11.93), 7.104 (5.58), 7.124 (8.65), 7.128 (7.61), 7.160 (15.26), 7.180 (7.54), 7.225 (2.09), 7.260 (0.92), 7.388 (3.75), 7.493 (14.35), 7.515 (15.32), 7.650 (3.15), 7.862 (0.92), 7.883 (1.06), 7.959 (14.90), 8.104 (1.11), 8.127 (0.90).

### Example 41

### 1-[1-[5-Chloro-2-[4-[4-(spiro[2.2]pentan-2-ylmethyl)piperazin-1-yl]phenyl]phenyl]-3-piperidyl]-5-(difluoromethyl)pyrazole-4-carboxylic acid hydrochloride (Diastereomeric mixture 2)

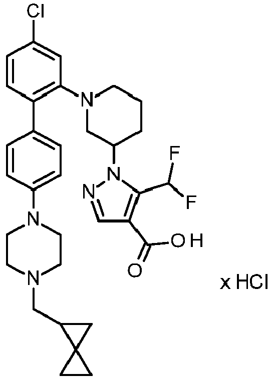

A solution of ethyl 1-[1-[5-chloro-2-[4-[4-(spiro[2.2]pentan-2-ylmethyl)piperazin-1-yl]phenyl]phenyl]-3-piperidyl]-5-(difluoromethyl)pyrazole-4-carboxylate (Example 93A, Enantiomer 2, 75.0 mg, 115 µmol) in THF/Methanol (10:1, 2.4 mL) was treated with lithium hydroxide monohydrate (1.2 ml, 1.0 M, 1.2 mmol). The reaction mixture was stirred overnight at room temperature, acidifed with an aqueous solution of hydrogen chloride (1N) and evaporated. The crude material obtained was submitted to preparative RP-HPLC 125x30mm with acetonitrile/water (neutral). The product containing fractions were lyophilized after addition of an aqueous solution of hydrogen chloride (1 N) affording 35.9 mg (45 % yield) of the title compound.
LC-MS (Method 4): Rₜ = 0.94 min; MS (ESIpos): m/z = 596 [M-HCl+H]⁺
¹H-NMR (400 MHz, DMSO-d6) δ [ppm]: -0.149 (1.12), 0.146 (0.99), 0.735 (2.99), 0.753 (4.02), 0.832 (3.30), 0.855 (5.32), 0.866 (16.00), 0.885 (4.11), 0.896 (6.20), 0.907 (3.41), 1.180 (2.88), 1.192 (3.24), 1.199 (3.58), 1.210 (3.07), 1.522 (3.25), 1.723 (2.23), 1.755 (1.81), 1.888 (1.52), 1.916 (1.80), 1.964 (2.62), 2.366 (1.42), 2.588 (3.06), 2.618 (1.53), 2.710 (1.38), 2.996 (3.21), 3.022 (6.80), 3.048 (5.57), 3.121 (4.32), 3.142 (4.13), 3.174 (3.03), 3.210 (4.15), 3.245 (3.65), 3.519 (2.87), 3.546 (2.02), 3.648 (2.71), 3.674 (2.14), 3.869 (2.46), 3.901 (4.20), 3.933 (2.24), 4.460 (2.20), 7.051 (10.22), 7.073 (11.13), 7.084 (8.60), 7.088 (9.82), 7.104 (4.68), 7.109 (2.75), 7.124 (7.43), 7.129 (6.40), 7.161 (12.08), 7.182 (5.95), 7.269 (0.42), 7.386 (2.45), 7.489 (12.19), 7.511 (11.72), 7.648 (2.00), 7.957 (13.36), 10.194 (0.61), 13.219 (0.53).

### Example 42

### 1-(1-{4-Chloro-4'-[4-(oxetan-3-yl)piperazin-1-yl][biphenyl]-2-yl}piperidin-3-yl)-5-(difluoromethyl)-1H-pyrazole-4-carboxylic acid hydrochloride (Enantiomer 1)

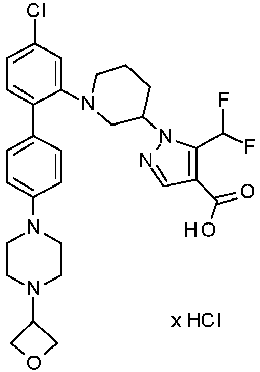

Ethyl 1-[1-{4-chloro-4'-[4-(oxetan-3-yl)piperazin-1-yl] [1,1'-biphenyl]-2-yl}piperidin-3-yl]-5 - (difluoromethyl)-1H-pyrazole-4-carboxylate trifluoroacetic acid (Example 95A, Enantiomer 1, (120 mg, 85 % purity, 143 µmol) was dissolved in THF/ethanol (5.7/0.57 ml). 1 M aqueous lithium hydroxide solution (1.4 ml, 1.4 mmol) was added and the mixture was stirred overnight at room temperature. The mixture was evaporated, then acidified and purified using preparative HPLC (RP18 column, acetonitrile/water gradient with the addition of 0.1% TFA). The product fractions were combined and evaporated. The raw material was purified by thick layer chromatography (eluent: dichloromethane/methanol/formic acid: 10/1/0.1). The raw product fraction was purified again using preparative HPLC (RP18 column, acetonitrile/water gradient with the addition of 0.1% TFA). The suitable fraction was evaporated and the residue was mixed with 0.2 M hydrochloric acid in diethyl ether, carefully evaporated at 30°C (thrice) and then lyophilized. 26 mg of the target compound (30% of theory) were obtained.
LC-MS (Method 3): Rₜ = 1.85 min; MS (ESIpos): m/z = 572 [M-HCl+H]⁺

### Example 43

### 1-(1-{4-Chloro-4'-[4-(oxetan-3-yl)piperazin-1-yl][biphenyl]-2-yl}piperidin-3-yl)-5-(difluoromethyl)-1H-pyrazole-4-carboxylic acid hydrochloride (Enantiomer 2)

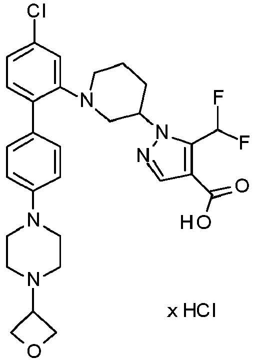

Ethyl 1-[1-{4-chloro-4'-[4-(oxetan-3-yl)piperazin-1-yl][1,1'-biphenyl]-2-yl}piperidin-3-yl]-5-(difluoromethyl)-1H-pyrazole-4-carboxylate trifluoroacetic acid (Example 96A, Enantiomer 2, (108 mg, 96 % purity, 145 µmol) was dissolved in THF/ethanol (5.8/0.58 ml). 1 M aqueous lithium hydroxide solution (1.5 ml, 1.5 mmol) was added and the mixture was stirred overnight at room temperature. The mixture was evaporated, then acidified and purified using preparative HPLC (RP18 column, acetonitrile/water gradient with the addition of 0.1% TFA). The product fractions were combined and evaporated. The raw material was purified by thick layer chromatography (eluent: dichloromethane/methanol/formic acid: 10/1/0.1). The raw product fraction was purified again using preparative HPLC (RP18 column, acetonitrile/water gradient with the addition of 0.1% TFA). The suitable fraction was evaporated and the residue was mixed with 0.2 M hydrochloric acid in diethyl ether, carefully evaporated at 30°C (thrice) and then lyophilized. 20 mg of the target compound (23% of theory) were obtained.
LC-MS (Method 3): Rₜ = 1.87 min; MS (ESIpos): m/z = 572 [M-HCl+H]⁺

### Example 44

### 1-{1-[4-Chloro-4'-(pyrrolidin-1-yl)[biphenyl]-2-yl]piperidin-3-yl}-5-(difluoromethyl)-1H-pyrazole-4-carboxylic acid (Enantiomer 1)

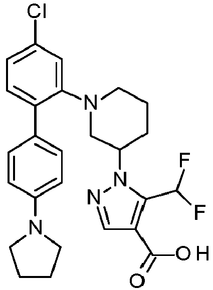

A solution of lithium hydroxide (3.43 mg, 143 µmol) in water (140 µl) was added to a solution of ethyl 1-{1-[4-chloro-4'-(pyrrolidin-1-yl)[1,1'-biphenyl]-2-yl]piperidin-3-yl}-5-(difluoromethyl)-1H-pyrazole-4-carboxylate (Example 97A, Enantiomer 1, 9.00 mg, 84 % purity, 14.3 µmol) in THF (1.0 ml) and methanol (100 µl). The resulting mixutre was stirred 17 hours at room temperature, diluted with water (10 ml) and brought to pH = 2 with an aqueous solution of hydrogen chloride (1 N). The aqueous solution was extracted twice with ethyl acetate. The combined organic layers were dried over sodium sulfate and evaporated. The residue was purified by preparative HPLC (RP18 column, eluent: Acetonitrile/water+ 0.1 % formic acid gradient) affording 5.80 mg (81 % yield) of the title compound.
LC-MS (Method 3): Rₜ = 2.76 min; MS (ESIpos): m/z = 501 [M+H]⁺
¹H-NMR (500 MHz, DMSO-d6) δ [ppm]: 1.232 (1.12), 1.513 (1.35), 1.539 (1.51), 1.685 (2.04), 1.712 (1.52), 1.870 (0.64), 1.887 (1.47), 1.911 (1.76), 1.964 (14.31), 3.022 (1.89), 3.043 (3.74), 3.064 (4.17), 3.087 (2.24), 3.251 (16.00), 3.263 (10.66), 4.506 (1.14), 4.527 (1.93), 6.575 (7.36), 6.592 (7.39), 7.016 (6.16), 7.061 (2.88), 7.077 (4.15), 7.133 (6.08), 7.149 (3.92), 7.412 (9.37), 7.429 (7.53), 7.518 (2.91), 7.623 (1.33), 7.950 (7.97).

### Example 45

### 1-{1-[4-Chloro-4'-(pyrrolidin-1-yl)[biphenyl]-2-yl]piperidin-3-yl}-5-(difluoromethyl)-1H-pyrazole-4-carboxylic acid (Enantiomer 2)

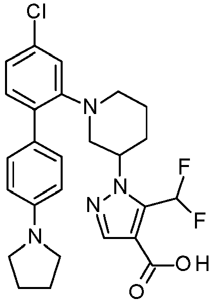

A solution of lithium hydroxide (29.4 mg, 1.23 mmol) in water (1.2 ml) was added to a solution of 2thyl 1-{1-[4-chloro-4'-(pyrrolidin-1-yl)[1,1'-biphenyl]-2-yl]pipendin-3-yl}-5-(difluoromethyl)-1H-pyrazole-4-carboxylate (Example 98A, Enantiomer 2, 65.0 mg, 123 µmol) in THF (7.4 ml) and methanol (740 µl). The resulting mixutre was stirred 17 hours at room temperature, diluted with water (10 ml) and brought to pH = 2 with an aqueous solution of hydrogen chloride (1 N). The aqueous solution was extracted twice with ethyl acetate. The combined organic layers were dried over sodium sulfate and evaporated. The residue was purified by preparative HPLC (RP18 column, eluent: Acetonitrile/water gradient) affording 38.0 mg (62 % yield) of the title compound.
LC-MS (Method 3): Rₜ = 2.76 min; MS (ESIneg): m/z = 499 [M-H]⁻
¹H-NMR (400 MHz, DMSO-d6) δ [ppm]: -0.149 (0.44), -0.008 (4.06), 0.008 (4.97), 0.146 (0.46), 1.234 (1.10), 1.511 (1.16), 1.542 (1.39), 1.684 (1.89), 1.717 (1.39), 1.863 (0.46), 1.885 (1.27), 1.894 (1.27), 1.924 (1.65), 1.951 (6.06), 1.967 (16.00), 1.982 (8.06), 2.477 (1.46), 2.671 (0.49), 2.710 (0.41), 3.020 (1.93), 3.047 (4.09), 3.073 (3.62), 3.092 (1.93), 3.239 (5.88), 3.254 (14.25), 3.270 (8.04), 4.505 (1.09), 4.531 (1.93), 4.558 (1.03), 6.575 (8.96), 6.597 (9.41), 7.015 (6.72), 7.020 (7.87), 7.060 (3.46), 7.065 (2.77), 7.081 (5.57), 7.086 (5.04), 7.134 (9.72), 7.155 (5.62), 7.392 (2.30), 7.413 (10.47), 7.435 (9.80), 7.522 (4.18), 7.653 (1.96), 7.953 (11.34), 13.189 (0.75).

### Example 46

### 1-(1-{4-Chloro-4'-[4-(2-cyanoethyl)piperazin-1-yl][biphenyl]-2-yl}piperidin-3-yl)-5-(difluoromethyl)-1H-pyrazole-4-carboxylic acid (Enantiomer 2)

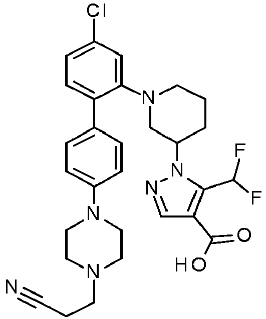

A solution of ethyl 1-[1-[5-chloro-2-[4-[4-(2-cyanoethyl)piperazin-1-yl]phenyl]phenyl]-3-piperidyl]-5-(difluoromethyl)pyrazole-4-carboxylate (Example 101A, Enantiomer 2, 100.0 mg, 167 µmol) in a THF/methanol mixture (10:1, 3.7 ml) and water (3.7 ml) was treated with lithium hydroxide monohydrate (70.3 mg, 1.67 mmol). The resulting mixture was stirred overnight at room temperature. The reaction mixture was acidified with an aqueous solution of hydrogen chloride (1 N), and the aqueous mixture was extracted with dichloromethane, dried and cvoncentrated. The residue was purified by preparative HPLC (RP18 column, eluent: Acetonitrile/water gradient). The product containing fractions were lyophilized after addition of an aqueous solution of hydrogen chloride (1 N) affording 14.0 mg (14 % yield) of the title compound and 21.0 mg (21 % yield) of Example 47.
LC-MS (Method 3): Rₜ = 1.88 min; MS (ESIpos): m/z = 569 [M+H]⁺
¹H-NMR (400 MHz, DMSO-d6) δ [ppm]: 0.831 (0.44), 1.169 (2.83), 1.233 (1.50), 1.268 (0.79), 1.492 (1.67), 1.524 (1.90), 1.697 (2.49), 1.731 (1.88), 1.880 (1.70), 1.912 (1.90), 1.988 (2.81), 2.328 (1.12), 2.366 (1.30), 2.575 (12.44), 2.587 (16.00), 2.599 (11.80), 2.616 (4.71), 2.633 (11.09), 2.649 (8.23), 2.715 (8.87), 2.731 (12.42), 2.748 (4.23), 3.004 (2.46), 3.030 (6.49), 3.057 (4.44), 3.188 (14.37), 3.248 (5.03), 4.037 (0.64), 4.490 (2.56), 5.753 (7.52), 6.978 (10.80), 7.000 (11.47), 7.051 (8.57), 7.056 (9.88), 7.082 (4.38), 7.102 (7.05), 7.107 (5.96), 7.153 (11.43), 7.173 (6.23), 7.384 (2.49), 7.438 (12.92), 7.460 (11.61), 7.515 (5.04), 7.647 (2.08), 7.949 (14.27).

### Example 47

### 1-(1-{4'-[4-(3-Amino-3-oxopropyl)piperazin-1-yl]-4-chloro[biphenyl]-2-yl}piperidin-3-yl)-5-(difluoromethyl)-1H-pyrazole-4-carboxylic acid (Enantiomer 2)

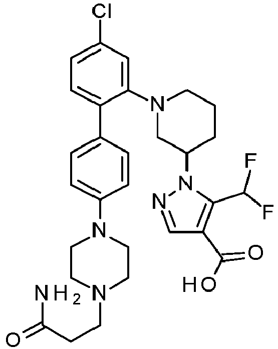

For procedure see Example 46.
LC-MS (Method 3): Rₜ = 1.52 min; MS (ESIpos): m/z = 587 [M+H]⁺
¹H-NMR (500 MHz, DMSO-d6) δ [ppm]: 1.251 (1.41), 1.267 (5.69), 1.281 (5.44), 1.469 (0.62), 1.494 (1.61), 1.519 (1.70), 1.545 (0.69), 1.703 (2.33), 1.729 (1.79), 1.863 (0.65), 1.871 (0.71), 1.888 (1.76), 1.895 (1.58), 1.912 (1.88), 1.919 (1.68), 1.937 (0.92), 1.975 (2.40), 1.995 (1.42), 2.376 (4.53), 2.391 (8.29), 2.405 (4.58), 2.571 (1.55), 2.780 (10.25), 2.802 (4.97), 2.818 (5.74), 3.009 (2.55), 3.030 (5.83), 3.052 (3.94), 3.103 (0.49), 3.118 (1.06), 3.132 (1.04), 3.147 (0.48), 3.581 (0.41), 3.594 (0.70), 3.607 (0.86), 3.620 (0.69), 4.452 (0.93), 4.460 (1.53), 4.474 (1.76), 4.482 (2.48), 4.490 (1.71), 4.504 (1.30), 6.876 (3.58), 6.992 (10.36), 7.010 (10.60), 7.058 (8.35), 7.062 (9.43), 7.088 (4.71), 7.092 (3.50), 7.104 (6.61), 7.108 (5.76), 7.154 (10.98), 7.171 (6.68), 7.432 (3.32), 7.450 (16.00), 7.468 (11.47), 7.537 (4.84), 7.642 (2.11), 7.943 (13.76).

### Example 48

### 1-{1-[4-Chloro-4'-(4-isobutyrylpiperazin-1-yl)[biphenyl]-2-yl]piperidin-3-yl}-5-(difluoromethyl)-1H-pyrazole-4-carboxylic acid (Enantiomer 2)

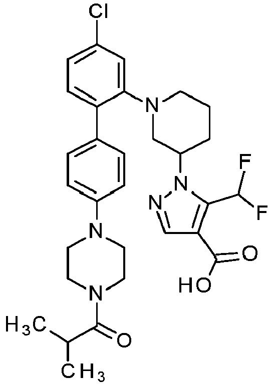

An aqueous solution of lithium hydroxide (1.2 ml, 1.0 M, 1.2 mmol) was added to a solution of ethyl 1-[1-{4-chloro-4'-[4-(2-methylpropanoyl)piperazin-1-yl][1,1'-biphenyl]-2-yl}piperidin-3-yl]-5-(difluoromethyl)-1H-pyrazole-4-carboxylate (Example 102A, Enantiomer 2, 71.0 mg, 116 µmol) in a THF/methanol mixture (10/1, 2.6 ml). The resulting mixture was stirred 3 hours at room temperature. The reaction mixture was acidified with an aqueous solution of hydrogen chloride (1 N) and evaporated. The residue was purified by preparative HPLC (RP18 column, eluent: Acetonitrile/water gradient) affording 61.0 mg (85 % yield) of the title compound.
LC-MS (Method 3): Rₜ = 2.44 min; MS (ESIpos): m/z = 586 [M+H]⁺
¹H-NMR (600 MHz, DMSO-d6) δ [ppm]: 1.028 (16.00), 1.039 (15.90), 1.500 (0.45), 1.521 (0.50), 1.711 (0.60), 1.732 (0.50), 1.895 (0.47), 1.902 (0.43), 1.916 (0.51), 1.922 (0.49), 1.978 (0.61), 2.567 (0.82), 2.584 (0.42), 2.907 (0.43), 2.918 (1.09), 2.929 (1.46), 2.940 (1.06), 2.952 (0.40), 3.012 (0.74), 3.030 (1.63), 3.047 (1.11), 3.057 (0.65), 3.179 (0.84), 3.231 (1.69), 3.249 (0.85), 4.479 (0.66), 7.056 (1.36), 7.068 (3.56), 7.071 (3.91), 7.094 (1.47), 7.098 (1.11), 7.108 (1.91), 7.111 (1.70), 7.163 (3.42), 7.176 (2.30), 7.419 (0.72), 7.474 (3.17), 7.488 (2.94), 7.506 (1.47), 7.593 (0.59), 7.945 (3.95).

### Example 49

### 1-(1-{4-Chloro-4'-[4-(2-fluoro-2-methylpropanoyl)piperazin-1-yl][biphenyl]-2-yl}piperidin-3-yl)-5-(difluoromethyl)-1H-pyrazole-4-carboxylic acid (Enantiomer 2)

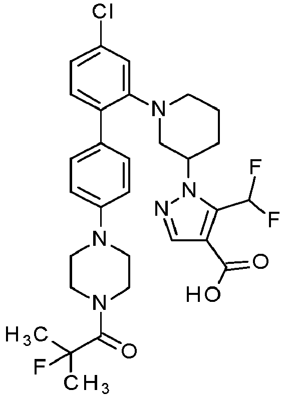

An aqueous solution of lithium hydroxide (1.1 ml, 1.0 M, 1.1 mmol) was added to a solution of ethyl 1-[1-{4-chloro-4'-[4-(2-fluoro-2-methylpropanoyl)piperazin-1-yl][1,1'-biphenyl]-2-yl}piperidin-3-yl]-5-(difluoromethyl)-1H-pyrazole-4-carboxylate (10/1, Example 103A, Enantiomer 2, 68.0 mg, 108 µmol) in a THF/methanol mixture (2.4 ml). The resulting mixture was stirred 3 hours at room temperature. The reaction mixture was acidified with an aqueous solution of hydrogen chloride (1 N) and evaporated. The residue was purified by preparative HPLC (RP18 column, eluent: Acetonitrile/water gradient) affording 43.0 mg (62 % yield) of the title compound.
LC-MS (Method 3): Rₜ = 2.54 min; MS (ESIpos): m/z = 604 [M+H]⁺
¹H-NMR (500 MHz, DMSO-d6) δ [ppm]: 1.492 (0.47), 1.513 (0.65), 1.563 (16.00), 1.607 (15.97), 1.706 (0.64), 1.733 (0.57), 1.893 (0.51), 1.900 (0.43), 1.918 (0.53), 1.925 (0.50), 1.976 (0.68), 2.560 (0.80), 2.584 (0.42), 3.009 (0.73), 3.030 (2.00), 3.051 (1.33), 3.720 (0.51), 3.970 (1.83), 4.456 (0.45), 4.478 (0.72), 7.072 (2.50), 7.076 (3.11), 7.085 (1.47), 7.099 (2.64), 7.102 (2.42), 7.115 (2.30), 7.119 (1.90), 7.166 (3.58), 7.182 (2.19), 7.412 (0.81), 7.485 (3.28), 7.503 (2.99), 7.517 (1.62), 7.621 (0.64), 7.952 (4.23).

### Example 50

### 1-(1-{4-Chloro-4'-[4-(3-cyclopropylcarbonyl)piperazin-1-yl][biphenyl]-2-yl}piperidin-3-yl)-5-(difluoromethyl)-1H-pyrazole-4-carboxylic acid (Enantiomer 2)

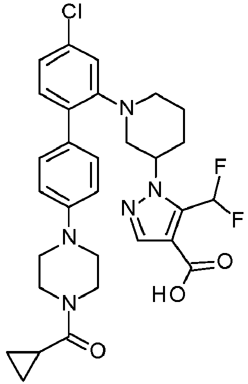

An aqueous solution of lithium hydroxide (1.2 ml, 1.0 M, 1.2 mmol) was added to a solution of ethyl 1-[1-{4-chloro-4'-[4-(cyclopropanecarbonyl)piperazin-1-yl][1,1'-biphenyl]-2-yl}piperidin-3-yl]-5-(difluoromethyl)-1H-pyrazole-4-carboxylate (Example 104A, Enantiomer 2, 74.0 mg, 121 µmol) in a THF/methanol mixture (10/1, 2.7 ml). The resulting mixture was stirred 3 hours at room temperature. The reaction mixture was acidified with an aqueous solution of hydrogen chloride (1 N) and evaporated. The residue was purified by preparative HPLC (RP18 column, eluent: Acetonitrile/water gradient) affording 62.0 mg (83 % yield) of the title compound.
LC-MS (Method 3): Rₜ = 2.39 min; MS (ESIpos): m/z = 584 [M+H]⁺
¹H-NMR (600 MHz, DMSO-d6) δ [ppm]: 0.724 (2.51), 0.731 (4.88), 0.735 (9.38), 0.740 (5.59), 0.744 (5.63), 0.748 (8.77), 0.752 (5.98), 0.764 (4.58), 0.768 (9.05), 0.772 (10.11), 0.776 (9.61), 0.780 (6.24), 0.788 (1.94), 1.483 (0.70), 1.504 (1.91), 1.526 (2.05), 1.547 (0.84), 1.715 (2.58), 1.737 (2.11), 1.760 (0.66), 1.886 (0.77), 1.900 (2.01), 1.906 (1.89), 1.920 (2.14), 1.926 (2.03), 1.941 (1.06), 1.979 (2.66), 1.995 (1.67), 2.012 (1.58), 2.020 (2.71), 2.025 (2.94), 2.033 (4.42), 2.041 (2.63), 2.046 (2.44), 2.054 (1.18), 2.424 (0.57), 2.570 (3.33), 2.590 (1.70), 2.653 (0.45), 3.012 (3.04), 3.030 (6.21), 3.047 (5.08), 3.060 (2.61), 3.204 (2.93), 3.237 (4.61), 3.250 (4.11), 3.280 (3.12), 3.665 (4.20), 3.776 (5.84), 3.867 (4.65), 4.462 (1.71), 4.480 (2.83), 4.498 (1.51), 7.073 (9.77), 7.076 (11.68), 7.090 (5.20), 7.098 (9.89), 7.101 (9.31), 7.111 (9.27), 7.115 (7.73), 7.167 (13.70), 7.180 (9.24), 7.425 (3.01), 7.485 (12.50), 7.500 (11.47), 7.512 (6.17), 7.599 (2.47), 7.946 (16.00).

### Example 51

### 1-(1-{4-Chloro-4'-[4-(3-methylbutanoyl)piperazin-1-yl][biphenyl]-2-yl}piperidin-3-yl)-5-(difluoromethyl)-1H-pyrazole-4-carboxylic acid (Enantiomer 2)

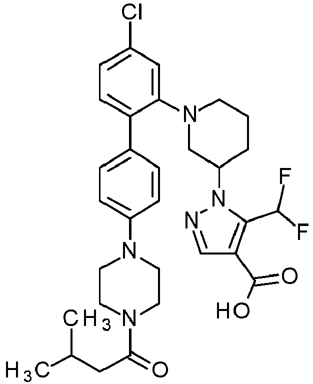

An aqueous solution of lithium hydroxide (1.0 ml, 1.0 M, 1.0 mmol) was added to a solution of ethyl 1-[1-{4-chloro-4'-[4-(3-methylbutanoyl)piperazin-1-yl][1,1'-biphenyl]-2-yl}piperidin-3-yl]-5-(difluoromethyl)-1H-pyrazole-4-carboxylate (Example 105A, Enantiomer 2, 64.0 mg, 102 µmol) in a THF/methanol mixture (10/1, 2.3 ml). The resulting mixture was stirred 3 hours at room temperature. The reaction mixture was acidified with an aqueous solution of hydrogen chloride (1 N) and evaporated. The residue was purified by preparative HPLC (RP18 column, eluent: Acetonitrile/water gradient) affording 53.0 mg (82 % yield) of the title compound.
LC-MS (Method 3): Rₜ = 2.53 min; MS (ESIpos): m/z = 600 [M+H]⁺
¹H-NMR (600 MHz, DMSO-d6) δ [ppm]: 0.922 (16.00), 0.932 (15.89), 1.357 (0.76), 1.502 (0.78), 1.522 (0.88), 1.712 (1.15), 1.733 (0.92), 1.894 (0.84), 1.915 (0.92), 1.935 (0.47), 1.977 (1.27), 1.997 (1.26), 2.008 (1.30), 2.020 (1.39), 2.031 (1.11), 2.042 (0.60), 2.246 (5.14), 2.258 (4.60), 2.567 (1.48), 2.587 (0.79), 3.008 (1.04), 3.026 (2.11), 3.043 (2.20), 3.058 (1.19), 3.165 (2.33), 3.209 (3.03), 3.246 (1.24), 3.637 (6.85), 4.475 (1.15), 7.034 (2.67), 7.048 (2.91), 7.070 (3.69), 7.094 (1.68), 7.107 (2.31), 7.161 (3.19), 7.174 (2.19), 7.414 (0.91), 7.467 (4.09), 7.482 (3.94), 7.501 (1.97), 7.588 (0.84), 7.943 (4.44).

### Example 52

### 1-{1-[4-chloro-4'-(4-ethylpiperazin-1-yl)[biphenyl]-2-yl]-5,5-difluoropiperidin-3-yl}-5-(difluoromethyl)-1H-pyrazole-4-carboxylic (Enantiomer 2)

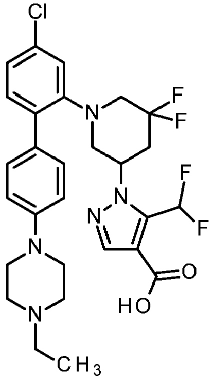

Under argon, a solution of ethyl 1-[1-{5-chloro-2-[(trifluoromethanesulfonyl)oxy]phenyl}-5,5-difluoropiperidin-3-yl]-5-(difluoromethyl)-1H-pyrazole-3-carboxylate (prepared in analogy to Example 123A, Enantiomer 2, 85.0 mg, 150 µmol) and 1-ethyl-4-[4-(4,4,5,5-tetramethyl-1,3,2-dioxaborolan-2-yl)phenyl]piperazine (52.1 mg, 165 µmol) in toluene/ethanol mixture (1:1) (1.6 ml) was treated with an aqueous solution of sodium carbonate (220 µl, 2.0 M, 450 µmol) and Pd(PPh₃)₄ (8.65 mg, 7.48 µmol) and stirred overnight at 100°C. The reaction mixture was cooled to room temperature, diluted with water and filtered over EXtrelut NT 3 cartridge and rinsed with ethyl acetate. The filtrate was evaporated and the residue purified by preparative HPLC (RP18 column, eluent: Acetonitrile/water + 0.5% formic acid gradient) affording 26 mg
LC-MS (Method 3): Rₜ = 1.69 min; MS (ESIpos): m/z = 580 [M+H]⁺
¹H-NMR (600 MHz, DMSO-d6) δ [ppm]: 1.072 (7.43), 1.084 (16.00), 1.097 (7.73), 2.074 (6.85), 2.386 (0.42), 2.425 (0.80), 2.447 (0.82), 2.519 (5.42), 2.572 (0.48), 2.614 (0.63), 2.663 (6.94), 3.104 (1.60), 3.122 (3.14), 3.141 (2.44), 3.175 (2.56), 3.194 (1.95), 3.334 (2.76), 3.354 (3.23), 3.383 (2.63), 3.403 (3.11), 3.428 (2.40), 3.449 (2.99), 3.467 (1.74), 4.559 (1.15), 4.568 (1.42), 4.576 (1.15), 4.586 (0.76), 6.955 (7.60), 6.970 (7.87), 7.166 (1.36), 7.169 (1.35), 7.180 (4.89), 7.183 (6.70), 7.189 (9.83), 7.202 (8.64), 7.385 (8.81), 7.399 (7.87), 7.430 (1.41), 7.517 (2.46), 7.605 (1.17), 7.885 (7.18), 8.158 (0.64).

### Example 53

### 1-{1-[4-chloro-4'-(4-ethylpiperazin-1-yl)[biphenyl]-2-yl]-5,5-difluoropiperidin-3-yl}-5-(difluoromethyl)-1H-pyrazole-4-carboxylic acid hydrochloride (Enantiomer 2)

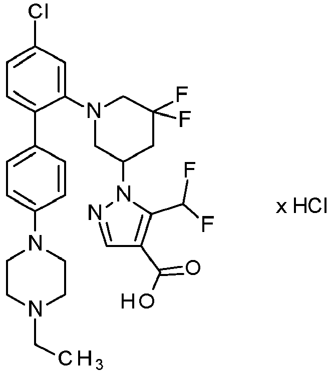

A solution of 1-{1-[4-chloro-4'-(4-ethylpiperazin-1-yl)[biphenyl]-2-yl]-5,5-difluoropiperidin-3-yl}-5-(difluoromethyl)-1H-pyrazole-4-carboxylic (Example 52, Enantiomer 2,26.4 mg, 45.5 µmol) in 1,4-dioxane (2.0 ml) was treated with a solution of hydrogen chloride in dioxane (14 µl, 4.0 M, 55 µmol) and stirred 30 minutes at room temperature. The reaction mixture was diluted with water and lyophilized affording 18.3 mg (65 % yield) of the title compound.
LC-MS (Method 3): Rₜ = 1.64 min; MS (ESIpos): m/z = 580 [M-HCl+H]⁺
¹H-NMR (600 MHz, DMSO-d6) δ [ppm]: 0.005 (1.06), 1.235 (0.40), 1.292 (7.50), 1.305 (16.00), 1.317 (7.46), 1.356 (2.26), 2.466 (0.95), 2.474 (0.99), 2.519 (1.86), 3.123 (4.68), 3.144 (5.19), 3.163 (7.67), 3.170 (6.33), 3.182 (5.17), 3.191 (3.68), 3.203 (0.99), 3.355 (1.02), 3.375 (1.60), 3.391 (0.60), 3.404 (0.83), 3.424 (2.51), 3.448 (2.05), 3.465 (0.72), 3.569 (8.92), 3.583 (3.90), 3.841 (2.16), 3.859 (1.78), 3.885 (2.07), 3.904 (1.82), 4.265 (1.99), 4.563 (0.97), 4.571 (1.29), 4.581 (1.54), 4.589 (1.24), 4.598 (0.85), 7.038 (7.88), 7.053 (8.07), 7.189 (1.04), 7.192 (0.98), 7.203 (6.29), 7.208 (11.24), 7.221 (1.73), 7.229 (6.78), 7.377 (1.80), 7.435 (9.03), 7.450 (8.06), 7.464 (3.20), 7.551 (1.47), 7.978 (9.63), 10.715 (0.75).

### Example 54

### 1-{1-[4-Chloro-4'-(4-isobutylpiperazin-1-yl)[biphenyl]-2-yl]-5,5-difluoropiperidin-3-yl}-5-(difluoromethyl)-1H-pyrazole-4-carboxylic acid hydrochloride (Enantiomer 1)

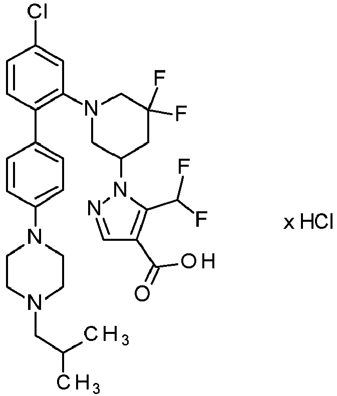

A solution of 1-{1-[4-chloro-4'-(piperazin-1-yl)[1,1'-biphenyl]-2-yl]-5,5-difluoropiperidin-3-yl}-5-(difluoromethyl)-1H-pyrazole-4-carboxylic acid-hydrochloride (Example 118A, Enantiomer 1, 34.0 mg, 57.8 µmol) in THF (520 µl) was treated with isobutyraldehyde (37 µl, 400 µmol) and sodium triacetoxyborohydride (36.7 mg, 173 µmol) and stirred 2.5 hours at room temperature. The reaction mixture was diluted with water and purified by preparative HPLC (RP18 column, eluent: Acetonitrile/water gradient). The product containing fractions were lyophilized after addition of an aqueous solution of hydrogen chloride (1 N) affording 24.0 mg (64 % yield) of the title compound.
LC-MS (Method 3): Rₜ = 1.74 min; MS (ESIpos): m/z = 608 [M-HCl+H]⁺
¹H-NMR (600 MHz, DMSO-d6) δ [ppm]: 1.018 (15.78), 1.030 (16.00), 2.134 (0.47), 2.146 (0.92), 2.157 (1.15), 2.168 (0.90), 2.179 (0.46), 2.474 (0.47), 3.006 (1.90), 3.016 (2.16), 3.136 (1.48), 3.154 (2.36), 3.172 (2.02), 3.197 (0.60), 3.244 (0.69), 3.262 (1.33), 3.280 (1.39), 3.393 (0.79), 3.414 (1.40), 3.438 (1.21), 3.457 (0.41), 3.572 (1.50), 3.590 (1.34), 3.790 (0.75), 3.811 (0.71), 3.837 (0.76), 3.859 (0.68), 4.594 (0.72), 7.027 (3.81), 7.041 (3.99), 7.186 (0.42), 7.204 (5.98), 7.217 (0.80), 7.228 (3.51), 7.372 (0.93), 7.434 (4.35), 7.449 (4.04), 7.458 (1.80), 7.546 (0.79), 7.973 (4.45), 10.057 (0.43).

### Example 55

### 1-{1-[4-Chloro-4'-(4-isobutylpiperazin-1-yl)[biphenyl]-2-yl]-5,5-difluoropiperidin-3-yl}-5-(difluoromethyl)-1H-pyrazole-4-carboxylic acid hydrochloride (Enantiomer 2)

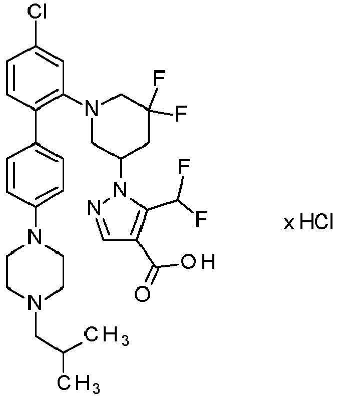

A solution of 1-{1-[4-chloro-4'-(piperazin-1-yl)[1,1'-biphenyl]-2-yl]-5,5-difluoropiperidin-3-yl}-5-(difluoromethyl)-1H-pyrazole-4-carboxylic acid hydrochloride (Example 126A, Enantiomer 2, 50.0 mg, 85.0 µmol) in THF (770 µl) was treated with isobutyraldehyde (54 µl, 590 µmol) and sodium triacetoxyborohydride (54.0 mg, 255 µmol) and stirred overnight at room temperature. The reaction mixture was diluted with water and purified by preparative HPLC (RP18 column, eluent: Acetonitrile/water gradient). The product containing fractions were lyophilized after addition of an aqueous solution of hydrogen chloride (1 N) affording 39.0 mg (75 % yield) of the title compound.
LC-MS (Method 3): Rₜ = 1.74 min; MS (ESIpos): m/z = 608 [M-HCl+H]⁺
¹H-NMR (600 MHz, DMSO-d6) δ [ppm]: 1.016 (15.85), 1.026 (16.00), 2.131 (0.48), 2.142 (0.91), 2.154 (1.12), 2.165 (0.88), 2.176 (0.44), 2.474 (0.53), 3.010 (1.76), 3.020 (2.06), 3.135 (1.32), 3.154 (2.24), 3.172 (2.07), 3.196 (0.60), 3.217 (0.67), 3.236 (1.17), 3.252 (1.18), 3.272 (0.74), 3.365 (1.24), 3.394 (0.57), 3.415 (1.25), 3.439 (1.06), 3.575 (1.38), 3.594 (1.23), 3.792 (0.67), 3.815 (0.66), 3.842 (0.71), 3.864 (0.64), 4.591 (0.67), 7.028 (3.72), 7.042 (3.86), 7.185 (0.44), 7.203 (6.70), 7.217 (0.56), 7.228 (3.31), 7.367 (0.89), 7.435 (4.25), 7.449 (4.18), 7.454 (2.12), 7.541 (0.73), 7.974 (4.42), 9.904 (0.41).

### Example 56

### 1-{1-[4-Chloro-4'-(1-propanoylpiperidin-4-yl)[biphenyl]-2-yl]piperidin-3-yl}-5-(difluoromethyl)-1H-pyrazole-4-carboxylic acid hydrochloride (Enantiomer 2)

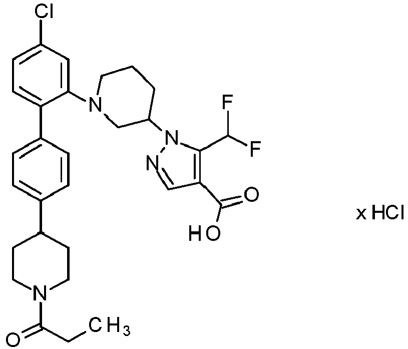

Ethyl 1-{1-[4-chloro-4'-(1-propanoylpiperidin-4-yl)[1,1'-biphenyl]-2-yl]piperidin-3-yl}-5-(difluoromethyl)-1H-pyrazole-4-carboxylate trifluoroacetic acid (Example 128A, Enantiomer 2, (37.0 mg, 51.9 µmol) was dissolved in THF/ethanol (1.3/0.13 ml). 1 M aqueous lithium hydroxide solution (520 µl, 520 µmol) was added and the mixture was stirred overnight at room temperature. The mixture was evaporated, then acidified and purified using preparative HPLC (RP18 column, acetonitrile/water gradient with the addition of 0.1% TFA). The product fractions were combined and evaporated. The raw material was purified by thick layer chromatography (eluent: dichloromethane/methanol/formic acid: 10/1/0.1). The suitable fraction was evaporated and the residue was mixed with 0.1 M hydrochloric acid in dioxane, carefully evaporated at 30°C (twice) and then lyophilized. 26 mg of the target compound (78% of theory, purity 95%) were obtained.
LC-MS (Method 3): Rₜ = 2.46 min; MS (ESIpos): m/z = 571 [M-HCl+H]⁺
¹H-NMR (500 MHz, DMSO-d6) δ [ppm]: 1.000 (7.64), 1.015 (16.00), 1.030 (7.83), 1.430 (0.98), 1.455 (1.75), 1.472 (1.65), 1.481 (1.55), 1.579 (0.99), 1.704 (1.58), 1.730 (1.28), 1.809 (1.89), 1.867 (1.21), 1.891 (1.20), 1.918 (0.57), 1.962 (1.60), 1.981 (1.00), 2.332 (1.45), 2.337 (1.50), 2.347 (3.90), 2.352 (4.05), 2.362 (4.28), 2.367 (4.24), 2.376 (1.35), 2.382 (1.34), 2.401 (0.81), 2.578 (1.92), 2.602 (3.77), 2.627 (2.01), 2.676 (0.60), 2.761 (1.01), 2.785 (1.95), 2.809 (0.89), 2.978 (1.46), 2.999 (3.10), 3.020 (2.96), 3.036 (1.52), 3.077 (0.98), 3.102 (1.81), 3.129 (1.05), 3.188 (1.74), 3.205 (1.47), 3.500 (0.41), 3.568 (0.94), 3.603 (1.00), 3.680 (0.52), 3.962 (1.37), 3.988 (1.28), 4.226 (0.48), 4.236 (0.62), 4.245 (0.54), 4.392 (0.93), 4.414 (1.61), 4.435 (0.85), 4.556 (1.35), 4.578 (1.29), 4.635 (0.44), 4.652 (0.96), 7.006 (0.43), 7.108 (5.59), 7.112 (8.21), 7.116 (5.39), 7.120 (1.90), 7.132 (5.56), 7.136 (4.20), 7.183 (8.50), 7.199 (5.20), 7.209 (0.50), 7.283 (7.89), 7.299 (9.01), 7.381 (1.91), 7.482 (9.86), 7.498 (7.34), 7.590 (1.55), 7.934 (9.72), 8.239 (0.75), 13.171 (0.62).

### Example 57

### 1-(1-{4-Chloro-4'-[1-(cyclopropylcarbonyl)piperidin-4-yl][biphenyl]-2-yl}piperidin-3-yl)-5-(difluoromethyl)-1H-pyrazole-4-carboxylic acid hydrochloride (Enantiomer 2)

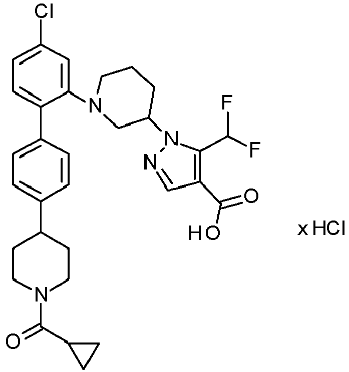

Ethyl 1-[1-{4-chloro-4'-[1-(cyclopropanecarbonyl)piperidin-4-yl][1,1'-biphenyl]-2-yl}piperidin-3-yl]-5-(difluoromethyl)-1H-pyrazole-4-carboxylate trifluoroacetic acid (Example 130A, Enantiomer 2, 32.0 mg, 44.1 µmol) was dissolved in THF/ethanol (1.1/0.11 ml). 1 M aqueous lithium hydroxide solution (440 µl, 440 µmol) was added and the mixture was stirred overnight at room temperature. The mixture was evaporated, then acidified and purified using preparative HPLC (RP18 column, acetonitrile/water gradient with the addition of 0.1% TFA). The product fractions were combined and evaporated. The raw material was purified by thick layer chromatography (eluent: dichloromethane/methanol/formic acid: 10/1/0.1). The suitable fraction was evaporated and the residue was mixed with 0.1 M hydrochloric acid in dioxane, carefully evaporated at 30°C (twice) and then lyophilized. 24 mg of the target compound (83% of theory, purity 95%) were obtained.
LC-MS (Method 3): Rₜ = 2.50 min; MS (ESIpos): m/z = 583 [M-HCl+H]⁺
¹H-NMR (500 MHz, DMSO-d6) δ [ppm]: 0.701 (4.94), 0.717 (5.78), 0.766 (1.84), 1.234 (0.84), 1.471 (2.16), 1.496 (2.08), 1.522 (0.96), 1.598 (0.90), 1.707 (1.88), 1.734 (1.55), 1.805 (1.16), 1.844 (1.26), 1.869 (2.42), 1.877 (2.34), 1.893 (2.48), 1.900 (2.21), 1.918 (1.09), 1.963 (1.93), 1.989 (2.01), 1.999 (1.97), 2.004 (1.93), 2.015 (3.24), 2.025 (1.57), 2.039 (0.88), 2.363 (0.45), 2.402 (0.50), 2.587 (1.40), 2.610 (2.64), 2.630 (2.15), 2.677 (1.15), 2.797 (1.31), 2.814 (1.44), 2.822 (2.39), 2.828 (1.35), 2.846 (1.13), 2.976 (2.02), 2.997 (4.04), 3.019 (4.26), 3.041 (1.82), 3.191 (3.18), 3.205 (2.42), 3.391 (0.53), 3.499 (0.64), 3.568 (2.87), 3.591 (0.81), 3.601 (1.65), 3.604 (1.45), 3.674 (0.42), 3.680 (0.52), 4.227 (0.43), 4.236 (0.50), 4.394 (2.07), 4.407 (2.32), 4.416 (2.79), 4.438 (1.32), 4.527 (1.10), 4.636 (0.88), 4.653 (0.92), 7.022 (0.71), 7.110 (6.19), 7.114 (10.45), 7.117 (7.33), 7.122 (2.48), 7.134 (6.85), 7.137 (5.17), 7.185 (10.20), 7.201 (6.27), 7.226 (0.68), 7.294 (9.48), 7.310 (10.89), 7.380 (2.30), 7.485 (16.00), 7.502 (9.98), 7.589 (1.81), 7.934 (12.55), 8.239 (0.56), 13.175 (0.63).

### Example 58

### 1-(1-{4-Chloro-4'-[1-(2,2,2-trifluoroethyl)azetidin-3-yl][biphenyl]-2-yl}pipendin-3-yl)-5-(difluoromethyl)-1H-pyrazole-4-carboxylic acid hydrochloride (Enantiomer 2)

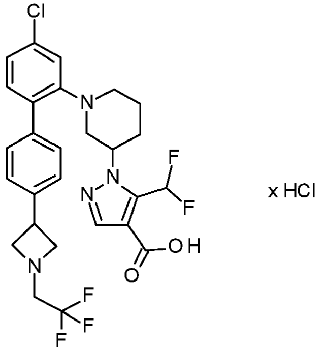

Ethyl 1-[1-{5-chloro-2-[(trifluoromethanesulfonyl)oxy]phenyl}piperidin-3-yl]-5-(difluoromethyl)-1H-pyrazole-4-carboxylate (prepared in analogy to Example 16A, Enantiomer 2, 39.0 mg, 73.3 µmol) and 3-[4-(4,4,5,5-tetramethyl-1,3,2-dioxaborolan-2-yl)phenyl]-1-(2,2,2-trifluoroethyl)azetidine (Example 132A, 60.0 mg, 50 % purity, 87.9 µmol) were dissolved under argon in toluene/ethanol (0.41/0.41 ml). Tetrakis(triphenylphosphine)palladium(0) (4.23 mg, 3.66 µmol) and 2 M sodium carbonate solution (110 µl, 220 µmol) were added and stirred at 100°C for 2 h. The reaction mixture was diluted with ethyl acetate and water. The aqueous phase was acidified with 1 M hydrochloric acid. The phases were separated and the aqueous phase was extracted twice with ethyl acetate. The combined organic phases were dried over sodium sulfate, filtered and evaporated. The crude product was dissolved in THF/ethanol (1.9/0.19 ml), 1 M aqueous lithium hydroxide solution (730 µl, 730 µmol) was added and the mixture was stirred overnight at room temperature. The mixture was then acidified and purified using preparative HPLC (RP18 column, acetonitrile/water gradient with the addition of 0.1% TFA). The product fractions were combined and evaporated. Then the residue was mixed with 0.1 M hydrochloric acid in dioxane, carefully evaporated at 30°C (twice) and then lyophilized. 20 mg of the target compound (44% of theory) were obtained.
LC-MS (Method 4): Rₜ = 1.30 min; MS (ESIpos): m/z = 569 [M-HCl+H]⁺
¹H-NMR(600 MHz, DMSO-d6) δ [ppm]: 1.234 (1.49), 1.356 (7.45), 1.482 (2.35), 1.503 (2.39), 1.729 (3.16), 1.751 (2.64), 1.878 (2.43), 1.898 (2.65), 1.918 (1.33), 1.957 (3.45), 2.183 (1.26), 2.386 (0.88), 2.425 (0.91), 2.617 (2.53), 2.641 (4.04), 2.656 (2.42), 2.991 (3.27), 3.009 (6.50), 3.027 (4.50), 3.037 (3.69), 3.058 (3.41), 3.159 (3.88), 3.172 (3.20), 3.568 (7.63), 4.020 (1.68), 4.330 (1.68), 4.354 (2.89), 4.372 (4.28), 4.839 (0.66), 6.871 (0.75), 7.140 (12.59), 7.145 (6.80), 7.159 (8.31), 7.189 (12.33), 7.203 (6.56), 7.402 (3.02), 7.490 (6.43), 7.516 (7.36), 7.564 (12.04), 7.577 (10.54), 7.939 (16.00), 13.207 (0.44).

### Example 59

### 1-(1-{4-Chloro-4'-[1-(3,3,3-trifluoropropyl)azetidin-3-yl][biphenyl]-2-yl}piperidin-3-yl)-5-(difluoromethyl)-1H-pyrazole-4-carboxylic acid hydrochloride (Enantiomer 2)

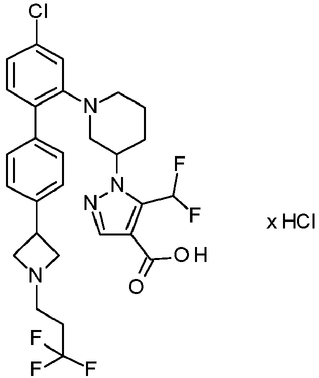

Ethyl 1-[1-{5-chloro-2-[(trifluoromethanesulfonyl)oxy]phenyl}piperidin-3-yl]-5-(difluoromethyl)-1H-pyrazole-4-carboxylate (prepared in analogy to Example 16A, Enantiomer 2, 60.0 mg, 113 µmol) and 3-[4-(4,4,5,5-tetramethyl-1,3,2-dioxaborolan-2-yl)phenyl]-1-(3,3,3-trifluoropropyl)azetidine (Example 133A, 58 mg, purity 83%, 135 µmol) were dissolved under argon in toluene/ethanol (0.63/0.63 ml). Tetrakis(triphenylphosphine)palladium(0) (6.52 mg, 5.64 µmol) and 2 M sodium carbonate solution (170 µl, 340 µmol) were added and the mixture was stirred at 100°C overnight. The reaction mixture was diluted with ethyl acetate and water. The aqueous phase was acidified with 1 M hydrochloric acid. The phases were separated and the aqueous phase was extracted twice with ethyl acetate. The combined organic phases were dried over sodium sulfate, filtered and evaporated. The crude product was dissolved in THF/ethanol (1.9/0.19 ml), 1 M aqueous lithium hydroxide solution (1.1 ml, 1.1 mmol) was added and the mixture was stirred overnight at room temperature. The mixture was then acidified and purified using preparative HPLC (RP18 column, acetonitrile/water gradient with the addition of 0.1% TFA). The product fractions were combined and evaporated. The raw material was purified by thick layer chromatography (eluent: dichloromethane/methanol/formic acid 10/1/0.1). Then the residue was mixed with 0.1 M hydrochloric acid in dioxane, carefully evaporated at 30°C (twice) and then lyophilized. 28 mg of the target compound (37% of theory, purity 92%) were obtained.
LC-MS (Method 3): Rₜ = 1.76 min; MS (ESIpos): m/z = 583 [M-HCl+H]⁺
¹H-NMR (600 MHz, DMSO-d6) δ [ppm]: 1.234 (0.55), 1.262 (0.72), 1.273 (0.68), 1.293 (0.65), 1.304 (0.61), 1.494 (1.61), 1.514 (1.73), 1.741 (2.31), 1.762 (2.00), 1.863 (0.65), 1.877 (1.79), 1.883 (1.66), 1.898 (2.01), 1.918 (1.02), 1.953 (2.53), 1.970 (1.59), 2.616 (0.54), 2.637 (1.70), 2.656 (3.39), 2.674 (1.74), 2.751 (2.75), 2.983 (2.32), 3.001 (4.74), 3.019 (2.85), 3.047 (2.66), 3.066 (2.43), 3.145 (2.87), 3.158 (2.42), 3.532 (2.91), 3.569 (8.88), 4.075 (1.16), 4.160 (1.90), 4.340 (1.71), 4.359 (3.41), 4.367 (3.23), 4.382 (3.97), 4.396 (3.08), 5.341 (0.44), 6.521 (0.61), 7.147 (11.66), 7.163 (7.53), 7.166 (6.00), 7.192 (10.76), 7.205 (5.56), 7.408 (2.24), 7.495 (4.10), 7.529 (7.97), 7.543 (13.71), 7.576 (16.00), 7.589 (8.78), 7.617 (0.52), 7.938 (13.67).

### Example 60

### 1-(1-{5-Chloro-2-[6-(4-ethylpiperazin-1-yl)pyridin-3-yl]phenyl}piperidin-3-yl)-5-(difluoromethyl)-1H-pyrazole-4-carboxylic acid hydrochloride (Enantiomer 2)

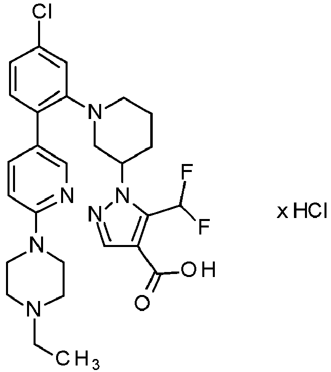

Ethyl 1-[1-{5-chloro-2-[6-(4-ethylpiperazin-1-yl)pyridin-3-yl]phenyl}piperidin-3-yl]-5-(difluoromethyl)-1H-pyrazole-4-carboxylate (Example 136A, Enantiomer 2, 10.0 mg, 95 % purity, 16.6 µmol) was dissolved in THF/ethanol (660/0.66 µl). 1 M aqueous lithium hydroxide solution (170 µl, 170 µmol) was added and the mixture was stirred overnight at room temperature. The mixture was evaporated, then acidified and purified using preparative HPLC (RP18 column, acetonitrile/water gradient with the addition of 0.1% TFA). The suitable fraction was evaporated and the residue was mixed with 0.1 M hydrochloric acid in dioxane, carefully evaporated at 30°C (twice) and then lyophilized. 7 mg of the target compound (73% of theory) were obtained.
LC-MS (Method 3): Rₜ = 1.59 min; MS (ESIpos): m/z = 545 [M-HCl+H]⁺
¹H-NMR (500 MHz, DMSO-d6) δ [ppm]: 1.235 (0.44), 1.290 (7.52), 1.304 (16.00), 1.319 (7.56), 1.535 (1.17), 1.560 (1.27), 1.784 (1.81), 1.811 (1.49), 1.916 (0.45), 1.933 (1.20), 1.981 (2.80), 2.363 (0.42), 2.403 (0.53), 2.655 (1.15), 2.677 (2.55), 2.699 (1.15), 2.984 (1.84), 3.005 (3.69), 3.026 (3.02), 3.052 (4.27), 3.072 (4.02), 3.156 (3.29), 3.170 (5.07), 3.181 (5.22), 3.195 (3.49), 3.209 (1.25), 3.361 (2.25), 3.379 (2.27), 3.591 (5.02), 3.615 (4.75), 3.758 (3.72), 4.399 (1.21), 4.410 (1.57), 4.420 (2.12), 4.441 (3.56), 4.471 (2.64), 7.134 (1.87), 7.151 (2.07), 7.170 (13.18), 7.184 (5.64), 7.187 (3.92), 7.235 (5.81), 7.252 (3.38), 7.439 (1.92), 7.544 (3.66), 7.648 (1.60), 7.960 (10.54), 7.980 (1.96), 7.998 (1.85), 8.330 (6.39), 8.334 (6.20), 10.816 (0.89).

### Example 61

### 1-(1-{5-Chloro-2-[6-(4-propylpiperazin-1-yl)pyridin-3-yl]phenyl}piperidin-3-yl)-5-(difluoromethyl)-1H-pyrazole-4-carboxylic acid hydrochloride (Enantiomer 2)

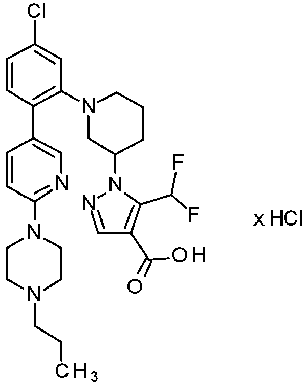

Ethyl 1-[1-{5-chloro-2-[6-(4-propylpiperazin-1-yl)pyridin-3-yl]phenyl}piperidin-3-yl]-5-(difluoromethyl)-1H-pyrazole-4-carboxylate (Example 138A, Enantiomer 2, 50.0 mg, 78 % purity, 66.4 µmol) was dissolved in THF/ethanol (1.7/0.17 ml). 1 M aqueous lithium hydroxide solution (660 µl, 660 µmol) was added and the mixture was stirred overnight at room temperature. The mixture was evaporated, then acidified and purified using preparative HPLC (RP18 column, acetonitrile/water gradient with the addition of 0.1% TFA). The suitable fraction was evaporated and the residue was mixed with 0.1 M hydrochloric acid in dioxane, carefully evaporated at 30°C (twice) and then lyophilized. 37 mg of the target compound (93% of theory) were obtained.
LC-MS (Method 3): Rₜ = 1.60 min; MS (ESIneg): m/z = 557 [M-HCl-H]⁻
¹H-NMR (400 MHz, DMSO-d6) δ [ppm]: 0.146 (0.43), 0.922 (7.33), 0.941 (16.00), 0.959 (8.08), 1.516 (1.14), 1.548 (1.33), 1.728 (1.85), 1.749 (3.28), 1.769 (4.68), 1.806 (2.00), 1.901 (0.42), 1.921 (1.27), 1.980 (3.05), 2.329 (0.56), 2.367 (0.62), 2.634 (1.31), 2.664 (2.69), 2.689 (1.36), 2.711 (0.79), 2.982 (1.71), 3.008 (3.73), 3.036 (4.48), 3.083 (7.21), 3.160 (2.45), 3.180 (1.83), 3.313 (2.11), 3.492 (0.85), 3.580 (5.23), 3.610 (5.21), 3.710 (5.38), 4.020 (1.39), 4.418 (5.12), 4.447 (4.41), 7.061 (1.30), 7.082 (2.40), 7.101 (1.49), 7.158 (14.18), 7.176 (6.54), 7.219 (5.66), 7.239 (3.01), 7.404 (2.06), 7.535 (3.93), 7.666 (1.77), 7.936 (2.22), 7.960 (10.20), 8.331 (6.28), 10.585 (0.40).

### Example 62

### 1-(1-{5-Chloro-2-[6-(4-isobutylpiperazin-1-yl)pyridin-3-yl]phenyl}piperidin-3-yl)-5-(difluoromethyl)-1H-pyrazole-4-carboxylic acid hydrochloride (Enantiomer 2)

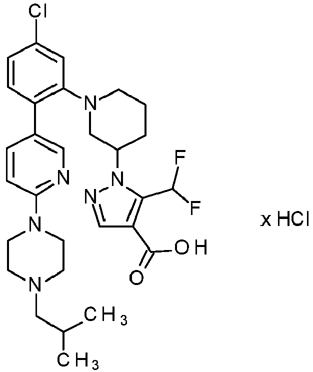

Ethyl 1-[1-{5-chloro-2-[(trifluoromethanesulfonyl)oxy]phenyl}piperidin-3-yl]-5-(difluoromethyl)-1H-pyrazole-4-carboxylate (prepared in analogy to Example 16A, Enantiomer 2, 60.0 mg, 113 µmol) and [6-(4-isobutylpiperazin-1-yl)pyridin-3-yl]boronic acid trifluoroacetic acid (Example 139A, 382 mg, 39 % purity, 395 µmol) were dissolved under argon in toluene/ethanol (0.63/0.63 ml). Tetrakis(triphenylphosphine)palladium(0) (6.52 mg, 5.64 µmol) and 2 M sodium carbonate solution (200 µl, 390 µmol) were added and the mixture was stirred at 100°C for 2 h. The reaction mixture was diluted with ethyl acetate and water. The aqueous phase was acidified with 1 M hydrochloric acid. The phases were separated and the aqueous phase was extracted twice with ethyl acetate. The combined organic phases were dried over sodium sulfate, filtered and evaporated. The crude product was dissolved in THF/ethanol (2.9/0.29 ml), 1 M aqueous lithium hydroxide solution (1.1 ml, 1.1 mmol) was added and the mixture was stirred overnight at room temperature. The same amout of 1 M aqueous lithium hydroxide solution (1.1 ml, 1.1 mmol) was added and the mixture was stirred for 4 h at room temperature. The mixture was then acidified and purified using preparative HPLC (RP18 column, acetonitrile/water gradient with the addition of 0.1% TFA). The product fractions were combined and evaporated. Then the residue was mixed with 0.1 M hydrochloric acid in dioxane, carefully evaporated at 30°C (twice) and then lyophilized. 23 mg of the target compound (37% of theory, purity 97%) were obtained.
LC-MS (Method 8): Rₜ = 0.85 min; MS (ESIpos): m/z = 573 [M-HCl+H]⁺
¹H-NMR (600 MHz, DMSO-d6) δ [ppm]: 1.023 (16.00), 1.034 (15.81), 1.543 (0.81), 1.563 (0.86), 1.788 (1.21), 1.810 (1.02), 1.940 (0.84), 1.965 (1.15), 1.981 (1.92), 2.141 (0.61), 2.151 (1.12), 2.162 (1.36), 2.174 (1.06), 2.185 (0.57), 2.657 (0.82), 2.678 (1.44), 2.697 (0.77), 3.006 (5.62), 3.057 (1.55), 3.075 (1.99), 3.098 (1.73), 3.162 (1.53), 3.176 (1.20), 3.498 (0.58), 3.554 (1.59), 3.573 (1.94), 3.619 (2.43), 3.833 (0.62), 4.022 (1.49), 4.106 (1.33), 4.331 (0.75), 4.419 (3.03), 7.143 (1.14), 7.155 (1.27), 7.173 (6.92), 7.186 (2.89), 7.240 (2.82), 7.253 (1.97), 7.463 (1.01), 7.550 (1.92), 7.637 (0.90), 7.963 (4.92), 7.999 (1.22), 8.011 (1.18), 8.239 (0.47), 8.328 (3.73), 10.472 (0.61).

### Example 63

### 1-[1-(5-Chloro-2-{6-[4-(cyclopropylmethyl)piperazin-1-yl]pyridin-3-yl}phenyl)piperidin-3-yl]-5-(difluoromethyl)-1H-pyrazole-4-carboxylic acid hydrochloride (Enantiomer 2)

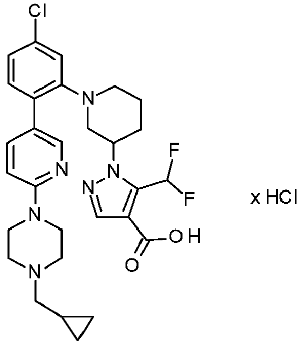

Ethyl 1-[1-{5-chloro-2-[(trifluoromethanesulfonyl)oxy]phenyl}piperidin-3-yl]-5-(difluoromethyl)-1H-pyrazole-4-carboxylate (prepared in analogy to Example 16A, Enantiomer 2, 30.0 mg, 56.4 µmol) and {6-[4-(cyclopropylmethyl)piperazin-1-yl]pyridin-3-yl}boronic acid trifluoroacetatic acid (Example 140A, 165 mg, 45 % purity, 197 µmol) were dissolved under argon in toluene/ethanol (0.31/0.31 ml). Tetrakis(triphenylphosphine)palladium(0) (3.26 mg, 2.82 µmol) and 2 M sodium carbonate solution (200 µl, 396 µmol) were added and the mixture was stirred at 100°C for 5.5 h. The reaction mixture was diluted with ethyl acetate and water. The aqueous phase was acidified with 1 M hydrochloric acid. The phases were separated and the aqueous phase was extracted twice with ethyl acetate. The combined organic phases were dried over sodium sulfate, filtered and evaporated. The crude product was dissolved in THF/ethanol (1.5/0.15 ml), 1 M aqueous lithium hydroxide solution (560 µl, 560 µmol) was added and the mixture was stirred overnight at room temperature. 1 M aqueous lithium hydroxide solution (280 µl, 280 µmol) was added and the mixture was stirred for 4 h at room temperature.The mixture was then acidified and purified using preparative HPLC (RP18 column, acetonitrile/water gradient with the addition of 0.1% TFA). The product fractions were combined and evaporated. The raw material was purified by thick layer chromatography (eluent: dichloromethane/methanol/formic acid 10/1/0.1). The resulting product fraction was mixed with 0.1 M hydrochloric acid in dioxane, carefully evaporated at 30°C (twice) and then lyophilized. 16 mg of the target compound (45% of theory, purity 97%) were obtained.
LC-MS (Method 5): Rₜ = 2.81 min; MS (ESIpos): m/z = 571 [M-HCl+H]⁺
¹H-NMR (600 MHz, DMSO-d6) δ [ppm]: 0.406 (3.68), 0.414 (3.79), 0.687 (2.96), 0.700 (3.08), 1.121 (1.29), 1.545 (0.78), 1.783 (1.02), 1.804 (0.97), 1.930 (0.76), 1.953 (0.95), 1.978 (1.46), 2.384 (0.85), 2.423 (1.70), 2.460 (0.84), 2.612 (0.63), 2.652 (2.23), 2.672 (1.38), 2.689 (0.77), 2.993 (1.16), 3.011 (2.21), 3.029 (1.34), 3.054 (1.24), 3.072 (1.18), 3.095 (2.84), 3.106 (3.97), 3.115 (3.40), 3.164 (1.52), 3.178 (1.91), 3.199 (1.99), 3.219 (1.75), 3.609 (16.00), 3.684 (4.84), 4.417 (1.33), 4.449 (1.99), 4.473 (1.87), 6.983 (0.67), 7.057 (1.72), 7.070 (1.94), 7.159 (7.94), 7.172 (2.95), 7.214 (3.39), 7.227 (2.09), 7.429 (1.12), 7.516 (2.07), 7.604 (0.95), 7.920 (1.50), 7.932 (1.42), 7.955 (6.02), 8.336 (3.70), 8.340 (3.60), 9.577 (0.71).

### Example 64

### 1-[1-(5-Chloro-2-{6-[4-(2,2,2-trifluoroethyl)piperazin-1-yl]pyridin-3-yl}phenyl)piperidin-3-yl]-5-(difluoromethyl)-1H-pyrazole-4-carboxylic (Enantiomer 1)

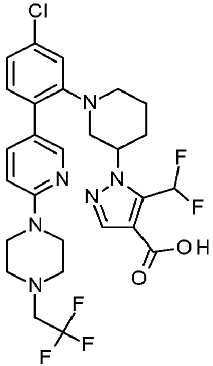

A solution of 1-[1-{5-Chloro-2-[6-(piperazin-1-yl)pyridin-3-yl]phenyl}piperidin-3-yl]-5-(difluoromethyl)-1H-pyrazole-4-carboxylic acid (Example 142A, Enantiomer 1, 14.0 mg, 27.1 µmol) in DMF (240 µl) was treated with N,N-diisopropylethylamine (15 µl, 84 µmol) and 2,2,2-trifluoroethyl trifluoromethanesulfonate (12 µl, 81 µmol). The resulting mixture was stirred 2 hours at room temperature. Lithium hydroxide was then added and the reaction mixture was stirred 1 hour at room temperature. The reaction mixture was acidified with an aqueous solution of hydrogen chloride (1N) and purified by preparative HPLC (RP18 column, eluent: Acetonitrile/water gradient) affording 6.50 mg (39 % yield) of the title compound.
LC-MS (Method 3): Rₜ = 2.24 min; MS (ESIpos): m/z = 599 [M+H]⁺
¹H-NMR (400 MHz, DMSO-d6) δ [ppm]: 0.123 (3.72), 0.147 (0.53), 1.233 (0.82), 1.505 (1.69), 1.537 (1.95), 1.748 (2.63), 1.780 (2.04), 1.883 (0.63), 1.904 (1.78), 1.933 (2.10), 1.975 (3.26), 2.328 (0.92), 2.366 (1.47), 2.583 (2.10), 2.611 (3.46), 2.637 (1.98), 2.670 (1.24), 2.710 (12.47), 2.723 (16.00), 2.734 (11.96), 2.989 (2.61), 3.015 (5.52), 3.041 (5.99), 3.069 (2.73), 3.197 (3.80), 3.209 (5.22), 3.234 (10.96), 3.260 (11.35), 3.527 (11.82), 3.539 (15.75), 3.550 (11.66), 4.424 (1.52), 4.451 (2.76), 4.478 (1.37), 6.878 (7.06), 6.900 (7.41), 7.112 (7.89), 7.117 (13.02), 7.121 (8.50), 7.142 (8.41), 7.147 (6.62), 7.192 (12.67), 7.212 (7.23), 7.406 (2.75), 7.537 (5.11), 7.668 (2.24), 7.798 (5.27), 7.804 (5.34), 7.820 (4.98), 7.826 (5.12), 7.939 (11.11), 8.262 (9.50), 8.267 (9.52).

### Example 65

### 1-[1-(5-Chloro-2-{6-[4-(2,2,2-trifluoroethyl)piperazin-1-yl]pyridin-3-yl}phenyl)piperidin-3-yl]-5-(difluoromethyl)-1H-pyrazole-4-carboxylic acid hydrochloride (Enantiomer 2)

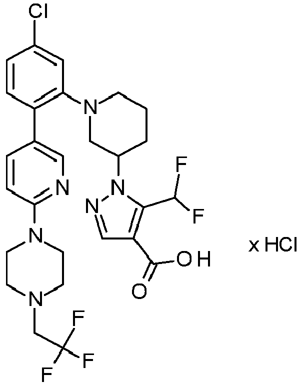

An aqueous solution of lithium hydroxide (2.8 ml, 1.0 M, 2.8 mmol) was added to a solution of ethyl 1-[1-(5-chloro-2-{6-[4-(2,2,2-trifluoroethyl)piperazin-1-yl]pyridin-3-yl}phenyl)piperidin-3-yl]-5-(difluoromethyl)-1H-pyrazole-4-carboxylate (10/1, Example 144A, Enantiomer 2, 189 mg, 276 µmol) in a THF/methanol mixture (6.1 ml). The resulting mixture was stirred 3 hours at room temperature. The reaction mixture was acidified with an aqueous solution of hydrogen chloride (2 N) and evaporated. The residue was purified by preparative HPLC (RP18 column, eluent: Acetonitrile/water gradient). The product containing fractions were lyophilized after addition of an aqueous solution of hydrogen chloride (1 N) affording 118 mg (68 % yield) of the title compound.
LC-MS (Method 3): Rₜ = 2.27 min; MS (ESIpos): m/z = 599 [M-HCl+H]⁺
¹H-NMR (400 MHz, DMSO-d6) δ [ppm]: 0.146 (0.42), 1.623 (1.11), 1.756 (0.40), 1.843 (1.74), 1.876 (1.39), 1.990 (3.78), 2.329 (0.71), 2.367 (0.94), 2.524 (2.40), 2.671 (0.82), 2.711 (1.07), 2.753 (1.10), 2.781 (2.10), 2.833 (10.93), 2.906 (1.72), 2.933 (3.33), 2.960 (2.15), 3.096 (4.08), 3.123 (3.61), 3.324 (2.17), 3.350 (4.97), 3.375 (5.15), 3.400 (2.88), 3.736 (10.06), 4.369 (1.53), 7.204 (2.50), 7.209 (3.58), 7.229 (16.00), 7.289 (5.87), 7.309 (3.86), 7.357 (1.19), 7.411 (2.49), 7.542 (4.07), 7.673 (1.83), 7.952 (10.26), 8.166 (1.50), 8.188 (1.50), 8.214 (7.50), 8.219 (6.18).

### Example 66

### 1-[1-(5-Chloro-2-{6-[4-(3,3,3-trifluoropropyl)piperazin-1-yl]pyridin-3-yl}phenyl)piperidin-3-yl]-5-(difluoromethyl)-1H-pyrazole-4-carboxylic acid hydrochloride (Enantiomer 1)

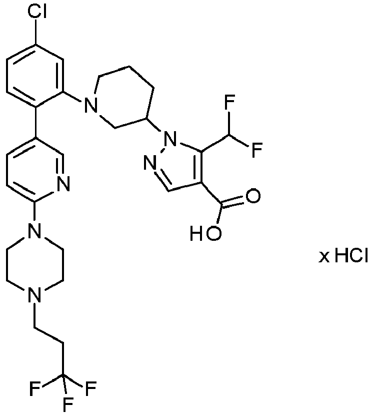

Ethyl 1-[1-(5-chloro-2-{6-[4-(3,3,3 -trifluoropropyl)piperazin-1-yl]pyridin-3-yl}phenyl)piperidin-3-yl]-5-(difluoromethyl)-1H-pyrazole-4-carboxylate trifluoroacetic acid (Example 146A, Enantiomer 1, 35.0 mg, 46.4 µmol) was dissolved in THF/ethanol (1.2/0.12 ml). 1 M aqueous lithium hydroxide solution (460 µl, 460 µmol) was added and the mixture was stirred overnight at room temperature. The mixture was evaporated, then acidified and purified using preparative HPLC (RP18 column, acetonitrile/water gradient with the addition of 0.1% TFA). The product fractions were combined and evaporated. Then the residue was mixed with 0.1 M hydrochloric acid in dioxane, carefully evaporated at 30°C (twice) and then lyophilized. 23 mg of the target compound (75% of theory) were obtained.
LC-MS (Method 4): Rₜ = 0.90 min; MS (ESIpos): m/z = 613 [M-HCl+H]⁺
¹H-NMR (400 MHz, DMSO-d6) δ [ppm]: -0.149 (0.56), 0.146 (0.59), 1.186 (0.46), 1.235 (0.65), 1.506 (1.23), 1.538 (1.34), 1.769 (1.91), 1.800 (1.54), 1.918 (1.25), 1.977 (3.04), 2.329 (0.57), 2.367 (0.80), 2.623 (1.39), 2.651 (2.45), 2.676 (1.74), 2.711 (0.94), 2.991 (3.96), 3.017 (5.18), 3.043 (4.81), 3.067 (2.36), 3.167 (4.68), 3.185 (3.80), 3.492 (3.45), 3.604 (1.66), 3.684 (2.53), 4.020 (0.87), 4.224 (0.51), 4.235 (0.54), 4.248 (0.50), 4.329 (0.51), 4.400 (1.54), 4.414 (2.16), 4.426 (3.09), 4.437 (3.07), 4.451 (2.88), 4.633 (0.58), 4.652 (0.83), 7.062 (3.50), 7.084 (3.64), 7.153 (16.00), 7.172 (6.89), 7.176 (5.02), 7.212 (7.55), 7.233 (3.81), 7.403 (2.39), 7.534 (4.49), 7.665 (2.02), 7.896 (2.85), 7.901 (2.98), 7.918 (2.80), 7.923 (2.81), 7.961 (9.81), 8.337 (6.89), 8.343 (6.79), 10.945 (0.41).

### Example 67

### 1-[1-(5-Chloro-2-{6-[4-(3,3,3-trifluoropropyl)piperazin-1-yl]pyridin-3-yl}phenyl)piperidin-3-yl]-5-(difluoromethyl)-1H-pyrazole-4-carboxylic acid hydrochloride (Enantiomer 2)

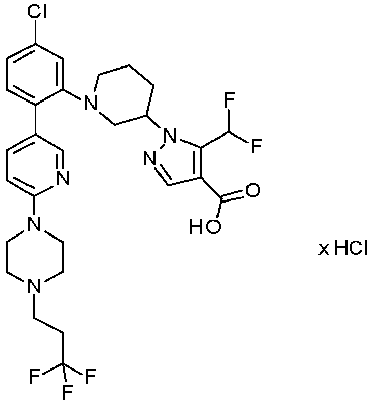

Ethyl 1-[1-(5-chloro-2-{6-[4-(3,3,3-trifluoropropyl)piperazin-1-yl]pyridin-3-yl}phenyl)piperidin-3-yl]-5-(difluoromethyl)-1H-pyrazole-4-carboxylate trifluoroacetic acid (Example 147A, Enantiomer 2, 28.0 mg, 37 µmol) was dissolved in THF/ethanol (1.0/0.10 ml). 1 M aqueous lithium hydroxide solution (380 µl, 380 µmol) was added and the mixture was stirred overnight at room temperature. The mixture was evaporated, then acidified and purified using preparative HPLC (RP18 column, acetonitrile/water gradient with the addition of 0.1% TFA). The product fractions were combined and evaporated. Then the residue was mixed with 0.1 M hydrochloric acid in dioxane, carefully evaporated at 30°C (twice) and then lyophilized. 23 mg of the target compound (95% of theory) were obtained.
LC-MS (Method 4): Rₜ = 0.90 min; MS (ESIpos): m/z = 613 [M-HCl+H]⁺
¹H-NMR (400 MHz, DMSO-d6) δ [ppm]: -0.149 (0.46), 0.146 (0.52), 1.234 (0.68), 1.504 (1.25), 1.535 (1.35), 1.768 (1.92), 1.802 (1.52), 1.916 (1.24), 1.975 (2.96), 2.329 (0.68), 2.367 (1.11), 2.624 (1.40), 2.652 (2.46), 2.676 (1.73), 2.711 (1.34), 2.954 (2.40), 2.989 (2.79), 3.016 (4.30), 3.042 (4.73), 3.069 (2.39), 3.167 (5.00), 3.184 (4.40), 3.568 (2.51), 3.603 (2.05), 3.684 (1.87), 4.020 (0.45), 4.236 (0.54), 4.329 (0.73), 4.396 (1.64), 4.422 (2.99), 4.434 (2.63), 4.448 (2.35), 4.638 (0.52), 4.652 (0.94), 7.050 (4.26), 7.072 (4.44), 7.152 (16.00), 7.170 (6.95), 7.175 (5.13), 7.208 (7.65), 7.229 (3.76), 7.396 (2.41), 7.527 (4.52), 7.658 (1.99), 7.886 (3.33), 7.892 (3.44), 7.908 (3.20), 7.914 (3.29), 7.962 (9.31), 8.337 (6.62), 8.342 (6.74).

### Example 68

### 1-(1-{2-[6-(4-Acetylpiperazin-1-yl)pyridin-3-yl]-5-chlorophenyl}piperidin-3-yl)-5-(difluoromethyl)-1H-pyrazole-4-carboxylic acid hydrochloride (Enantiomer 2)

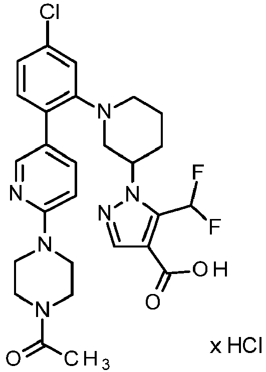

Ethyl 1-[1-{5-chloro-2-[(trifluoromethanesulfonyl)oxy]phenyl}piperidin-3-yl]-5-(difluoromethyl)-1H-pyrazole-4-carboxylate (prepared in analogy to Example 16A, Enantiomer 2, 80.0 mg, 150 µmol) and 1-{4-[5-(4,4,5,5-tetramethyl-1,3,2-dioxaborolan-2-yl)pyridin-2-yl]piperazin-1-yl}ethan-1-one (59.8 mg, 180 µmol) were dissolved under argon in toluene/ethanol (0.83/0.83 ml). Tetrakis(triphenylphosphine)palladium(0) (8.69 mg, 7.52 µmol) and 2 M sodium carbonate solution (228 µl, 456 µmol) were added and the mixture was stirred at 100°C overnight. The reaction mixture was diluted with ethyl acetate and water. The aqueous phase was acidified with 1 M hydrochloric acid. The phases were separated and the aqueous phase was extracted twice with ethyl acetate. The combined organic phases were dried over sodium sulfate, filtered and evaporated. The crude product was dissolved in THF/ethanol (2.0/0.2 ml), 1 M aqueous lithium hydroxide solution (1.5 ml, 1.5 mmol) was added and the mixture was stirred overnight at room temperature. The mixture was then acidified and purified using preparative HPLC (RP18 column, acetonitrile/water gradient with the addition of 0.1% formic acid). The product fractions were combined and evaporated. Then the residue was mixed with 0.1 M hydrochloric acid in dioxane, carefully evaporated at 30°C (thrice) and then lyophilized. 55 mg of the target compound (61% of theory) were obtained.
LC-MS (Method 3): Rₜ = 1.79 min; MS (ESIpos): m/z = 559 [M-HCl+H]⁺
¹H-NMR(500 MHz, DMSO-d6) δ [ppm]: 1.623 (0.41), 1.835 (0.66), 1.861 (0.52), 1.964 (0.48), 1.985 (1.47), 2.084 (16.00), 2.762 (0.66), 2.928 (0.62), 2.950 (1.24), 2.971 (0.76), 3.094 (0.80), 3.118 (1.44), 3.132 (0.70), 3.568 (3.32), 3.606 (1.20), 3.653 (3.93), 3.689 (1.92), 3.773 (1.66), 4.385 (0.56), 4.395 (0.56), 4.403 (0.56), 7.201 (0.85), 7.205 (1.37), 7.219 (5.29), 7.285 (1.96), 7.302 (1.66), 7.435 (0.84), 7.540 (1.49), 7.644 (0.66), 7.952 (4.60), 8.153 (0.43), 8.241 (2.56), 8.245 (2.50).

### Example 69

### 1-[1-(5-Chloro-2-{4-methyl-6-[4-(2,2,2-trifluoroethyl)piperazin-1-yl]pyridin-3-yl}phenyl)piperidin-3-yl]-5-(difluoromethyl)-1H-pyrazole-4-carboxylic acid hydrochloride (Enantiomer 2)

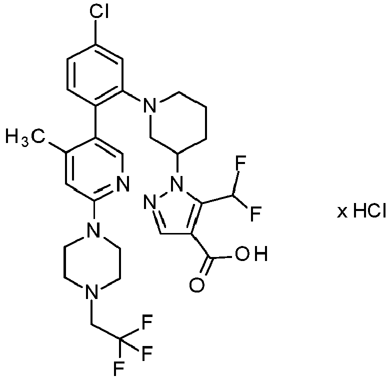

Ethyl 1-[1-{5-chloro-2-[(trifluoromethanesulfonyl)oxy]phenyl}piperidin-3-yl]-5-(difluoromethyl)-1H-pyrazole-4-carboxylate (prepared in analogy to Example 16A, Enantiomer 2, 40.9 mg, 77.0 µmol) and {4-methyl-6-[4-(2,2,2-trifluoroethyl)piperazin-1-yl]pyridin-3-yl}boronic acid (Example 149A, 35.0 mg, 80 % purity, 92.4 µmol) were dissolved under argon in toluene/ethanol (0.43/0.43 ml). Tetrakis(triphenylphosphine)palladium(0) (4.45 mg, 3.85 µmol) and 2 M sodium carbonate solution (116 µl, 232 µmol) were added and the mixture was stirred at 100°C overnight. The reaction mixture was diluted with ethyl acetate and water. The aqueous phase was acidified with 1 M hydrochloric acid. The phases were separated and the aqueous phase was extracted twice with ethyl acetate. The combined organic phases were dried over sodium sulfate, filtered and evaporated. The crude product was dissolved in THF/ethanol (2.0/0.20 ml), 1 M aqueous lithium hydroxide solution (770 µl, 770 µmol) was added and the mixture was stirred overnight at room temperature. The mixture was then acidified and purified using preparative HPLC (RP18 column, acetonitrile/water gradient with the addition of 0.1% formic acid). The product fractions were combined and evaporated. Then the residue was mixed with 0.1 M hydrochloric acid in dioxane, carefully evaporated at 30°C (twice) and then lyophilized. 25 mg of the target compound (50% of theory) were obtained.
LC-MS (Method 3): Rₜ = 2.09 min; MS (ESIpos): m/z = 613 [M-HCl+H]⁺
¹H-NMR (500 MHz, DMSO-d6) δ [ppm]: -0.007 (0.71), 0.007 (0.49), 1.235 (0.49), 1.948 (1.68), 2.160 (16.00), 2.677 (0.43), 2.820 (6.51), 3.082 (0.89), 3.340 (2.17), 3.359 (2.19), 3.568 (12.08), 3.729 (5.44), 7.165 (1.96), 7.181 (3.09), 7.233 (1.71), 7.249 (1.37), 7.299 (0.74), 7.549 (0.57), 7.871 (0.71), 7.952 (0.94).

### Example 70

### 1-(1-{5-Chloro-2-[2-(4-isobutylpiperazin-1-yl)pyrimidin-5-yl]phenyl}piperidin-3-yl)-5-(difluoromethyl)-1H-pyrazole-4-carboxylic acid hydrochloride (Enantiomer 2)

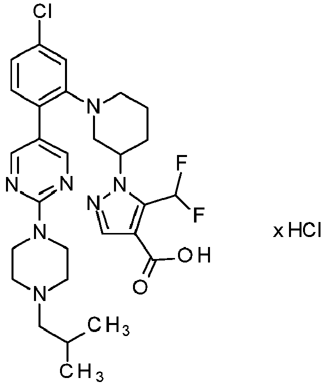

Ethyl 1-[1-{5-chloro-2-[(trifluoromethanesulfonyl)oxy]phenyl}piperidin-3-yl]-5-(difluoromethyl)-1H-pyrazole-4-carboxylate (prepared in analogy to Example 16A, Enantiomer 2, 57.5 mg, 108 µmol) and {2-[4-(2-methylpropyl)piperazin-1-yl]pyrimidin-5-yl}boronic acid trifluoroacetic acid (1/1) (Example 151A, 650 mg, ∼22 % purity, 378 µmol) were dissolved under argon in toluene/ethanol (0.60/0.60 ml). Tetrakis(triphenylphosphine)palladium(0) (6.24 mg, 5.40 µmol) and 2 M sodium carbonate solution (380 µl, 760 µmol) were added and the mixture was stirred at 100°C for 2 h. The reaction mixture was diluted with ethyl acetate and water. The aqueous phase was acidified with 1 M hydrochloric acid. The phases were separated and the aqueous phase was extracted twice with ethyl acetate. The combined organic phases were dried over sodium sulfate, filtered and evaporated. The crude product was dissolved in THF/ethanol (2.8/0.28 ml), 1 M aqueous lithium hydroxide solution (1.1 ml, 1.1 mmol) was added and the mixture was stirred overnight at room temperature. The same amout of 1 M aqueous lithium hydroxide solution (1.1 ml, 1.1 mmol) was added and the mixture was stirred overnight at room temperature. The mixture was then acidified and purified using preparative HPLC (RP18 column, acetonitrile/water gradient with the addition of 0.1% TFA). The product fractions were combined and evaporated. The raw material was purified by thick layer chromatography (eluent: dichloromethane/methanol/formic acid 10/1/0.1). Then the residue was mixed with dichloromethane and 1 M hydrochloric acid in ethanol, carefully evaporated at 30°C (twice) and then lyophilized. 20 mg of the target compound (30% of theory) were obtained.
LC-MS (Method 3): Rₜ = 1.63 min; MS (ESIpos): m/z = 574 [M-HCl+H]⁺
¹H-NMR (400 MHz, DMSO-d6) δ [ppm]: 1.010 (15.63), 1.026 (16.00), 1.569 (0.46), 1.820 (0.73), 1.853 (0.58), 1.948 (0.45), 1.987 (1.31), 2.130 (0.76), 2.147 (0.96), 2.164 (0.74), 2.367 (0.49), 2.711 (0.82), 2.734 (0.90), 2.762 (0.49), 2.946 (0.71), 2.973 (1.50), 3.000 (1.17), 3.011 (1.72), 3.026 (2.69), 3.043 (2.46), 3.079 (1.69), 3.107 (1.37), 3.126 (0.73), 3.478 (1.15), 4.366 (0.61), 4.379 (0.73), 4.390 (0.64), 4.699 (1.01), 7.195 (0.99), 7.200 (1.44), 7.219 (7.26), 7.263 (3.10), 7.282 (1.72), 7.399 (0.92), 7.530 (1.74), 7.661 (0.75), 7.955 (4.31), 8.666 (12.02).

### Example 71

### 1-(1-{5-Chloro-2-[2-(4-methylpiperazin-1-yl)pyrimidin-5-yl]phenyl}piperidin-3-yl)-5-(difluoromethyl)-1H-pyrazole-4-carboxylic acid hydrochloride (Enantiomer 2)

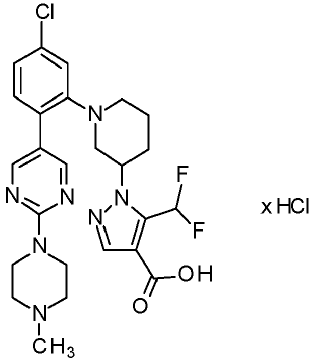

Ethyl 1-[1-{5-chloro-2-[2-(4-methylpiperazin-1-yl)pyrimidin-5-yl]phenyl}piperidin-3-yl]-5-(difluoromethyl)-1H-pyrazole-4-carboxylate (Example 152A, Enantiomer 2, 64.0 mg, 114 µmol) was dissolved in THF/ethanol (4.6/0.46 ml). 1 M aqueous lithium hydroxide solution (1.1 ml, 1.1 mmol) was added and the mixture was stirred overnight at room temperature. The mixture was evaporated, then acidified and purified using preparative HPLC (RP18 column, acetonitrile/water gradient with the addition of 0.1% TFA). The product fractions were combined and evaporated. Then the residue was mixed with 0.1 M hydrochloric acid in dioxane, carefully evaporated at 30°C (twice) and then lyophilized. 54 mg of the target compound (82% of theory) were obtained.
LC-MS (Method 4): Rₜ = 0.80 min; MS (ESIpos): m/z = 532 [M-HCl+H]⁺
¹H-NMR(400 MHz, DMSO-d6) δ [ppm]: 1.563 (0.54), 1.818 (0.87), 1.850 (0.69), 1.947 (0.51), 1.986 (1.59), 2.710 (0.66), 2.734 (1.10), 2.760 (0.63), 2.848 (5.55), 2.859 (5.44), 2.944 (0.85), 2.971 (1.80), 2.997 (1.16), 3.054 (1.93), 3.089 (2.47), 3.118 (1.27), 3.329 (1.27), 3.389 (0.47), 3.523 (3.45), 4.360 (0.72), 4.373 (0.82), 4.385 (0.75), 4.724 (1.12), 4.762 (1.09), 7.194 (1.25), 7.199 (1.83), 7.219 (8.73), 7.265 (3.83), 7.285 (2.26), 7.402 (1.06), 7.533 (2.01), 7.664 (0.88), 7.953 (4.25), 8.665 (16.00).

### Example 72

### 1-[1-(5-Chloro-2-{2-[4-(cyclopropylmethyl)piperazin-1-yl]pyrimidin-5-yl}phenyl)piperidin-3-yl]-5-(difluoromethyl)-1H-pyrazole-4-carboxylic acid hydrochloride (Enantiomer 2)

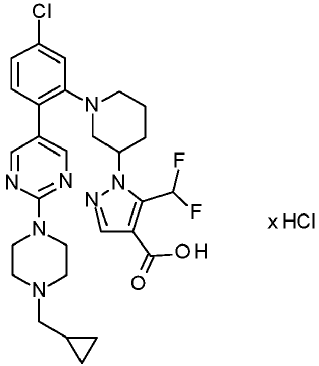

Ethyl 1-[1-{5-chloro-2-[(trifluoromethanesulfonyl)oxy]phenyl}piperidin-3-yl]-5-(difluoromethyl)-1H-pyrazole-4-carboxylate (prepared in analogy to Example 16A, Enantiomer 2, 55.8 mg, 105 µmol) and {2-[4-(cyclopropylmethyl)piperazin-1-yl]pyrimidin-5-yl}boronic acid trifluoroacetic acid (Example 153A, 690 mg, 20 % purity, 367 µmol) were dissolved under argon in toluene/ethanol (0.58/0.58 ml). Tetrakis(triphenylphosphine)palladium(0) (6.06 mg, 5.24 µmol) and 2 M sodium carbonate solution (368 µl, 736 µmol) were added and the mixture was stirred at 100°C for 2 h. The reaction mixture was diluted with ethyl acetate and water. The aqueous phase was acidified with 1 M hydrochloric acid. The phases were separated and the aqueous phase was extracted twice with ethyl acetate. The combined organic phases were dried over sodium sulfate, filtered and evaporated. The crude product was dissolved in THF/ethanol (2.7/0.27 ml), 1 M aqueous lithium hydroxide solution (1.0 ml, 1.0 mmol) was added and the mixture was stirred overnight at room temperature. Thee same amounts of 1 M aqueous lithium hydroxide solution (1.0 ml, 1.0 mmol) was added and the mixture was stirred 5 h at room temperature. The mixture was then acidified and purified using preparative HPLC (RP18 column, acetonitrile/water gradient with the addition of 0.1% TFA). The product fractions were combined and evaporated. The raw material was purified by thick layer chromatography (eluent: dichloromethane/methanol/formic acid 10/1/0.1). The product fraction was mixed with 1 M hydrochloric acid in ethanol, carefully evaporated at 30°C (twice) and then lyophilized. 31 mg of the target compound (48% of theory) were obtained.
LC-MS (Method 3): Rₜ = 1.61 min; MS (ESIpos): m/z = 572 [M-HCl+H]⁺
¹H-NMR(400 MHz, DMSO-d6) δ [ppm]: 0.405 (0.76), 0.416 (3.10), 0.430 (3.25), 0.442 (0.96), 0.663 (0.85), 0.674 (2.51), 0.677 (2.49), 0.693 (2.72), 0.709 (0.80), 1.132 (0.70), 1.143 (0.94), 1.162 (0.63), 1.570 (0.57), 1.821 (0.91), 1.854 (0.72), 1.948 (0.56), 1.987 (1.60), 2.711 (0.80), 2.738 (1.11), 2.765 (0.62), 2.944 (0.84), 2.971 (1.79), 2.998 (1.17), 3.072 (3.49), 3.090 (4.82), 3.102 (3.90), 3.122 (1.50), 3.345 (0.79), 3.359 (0.89), 3.374 (1.32), 3.388 (1.33), 3.657 (2.16), 3.686 (2.00), 3.958 (6.86), 4.363 (0.86), 4.376 (1.05), 4.388 (0.86), 4.750 (1.23), 4.786 (1.18), 7.195 (1.28), 7.200 (1.86), 7.220 (9.18), 7.265 (3.77), 7.285 (2.13), 7.398 (1.07), 7.529 (2.08), 7.660 (0.91), 7.954 (5.46), 8.668 (16.00), 10.213 (0.62).

### Example 73

### 5-(Difluoromethyl)-1-{1-[4'-(4-ethylpiperazin-1-yl)-4-(trifluoromethyl)[biphenyl]-2-yl]piperidin-3-yl}-1H-pyrazole-4-carboxylic acid hydrochloride (Enantiomer 1)

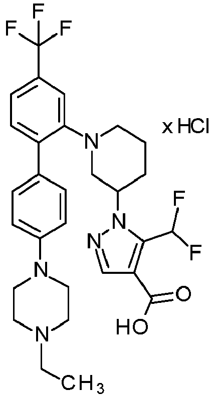

An aqueous solution of lithium hydroxide (740 µl, 1.0 M, 740 µmol) was added to a solution of ethyl 5-(difluoromethyl)-1- {1-[4'-(4-ethylpiperazin-1-yl)-4-(trifluoromethyl) [1,1'-biphenyl]-2-yl]piperidin-3-yl}-1H-pyrazole-4-carboxylate (Example 158A, Enantiomer 1, 45.0 mg, 74.3 µmol) in THF/methanol (10:1, 2.6 ml). The resulting mixutre was stirred overnight at room temperature. The reaction mixture was acidified with an aqueous solution of hydrogen chloride (1 N), diluted with water. The aqueous solution was extracted ethyl acetate. The combined organic layers were dried over sodium sulfate and evaporated. The residue was lyophilized from acetonitrile/water affording 40.0 mg (83 % yield) of the title compound.
LC-MS (Method 3): Rₜ = 1.81 min; MS (ESIpos): m/z = 578 [M-HCl+H]⁺
¹H-NMR (600 MHz, DMSO-d6) δ [ppm]: -0.100 (0.55), 0.005 (3.72), 0.097 (0.60), 0.886 (0.72), 1.069 (0.60), 1.128 (0.74), 1.159 (1.15), 1.169 (6.77), 1.236 (1.38), 1.266 (7.85), 1.278 (16.00), 1.290 (7.81), 1.312 (4.36), 1.356 (2.85), 1.512 (1.51), 1.532 (1.66), 1.736 (2.17), 1.755 (2.06), 1.914 (1.55), 1.934 (1.70), 1.954 (0.81), 1.987 (2.21), 2.004 (1.32), 2.183 (0.45), 2.326 (1.62), 2.386 (0.77), 2.422 (1.51), 2.445 (1.02), 2.614 (2.15), 2.633 (2.81), 2.654 (2.13), 3.067 (3.66), 3.085 (6.40), 3.102 (4.40), 3.156 (3.72), 3.274 (4.83), 4.471 (1.34), 4.489 (2.34), 4.508 (1.30), 6.518 (0.47), 6.580 (0.83), 7.045 (0.53), 7.087 (10.06), 7.102 (10.36), 7.315 (7.87), 7.366 (3.60), 7.379 (7.83), 7.398 (5.83), 7.411 (2.62), 7.441 (2.47), 7.528 (4.51), 7.556 (11.96), 7.570 (10.85), 7.615 (2.02), 7.963 (13.19), 8.426 (0.91), 10.253 (0.45).

### Example 74

### 5-(Difluoromethyl)-1-{1-[4'-(4-ethylpiperazin-1-yl)-4-(trifluoromethyl)[biphenyl]-2-yl]piperidin-3-yl}-1H-pyrazole-4-carboxylic acid hydrochloride (Enantiomer 2)

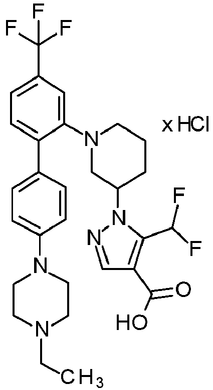

An aqueous solution of lithium hydroxide (710 µl, 1.0 M, 710 µmol) was added to a solution of ethyl 5-(difluoromethyl)-1-{1-[4'-(4-ethylpiperazin-1-yl)-4-(trifluoromethyl)[1,1'-biphenyl]-2-yl]piperidin-3-yl}-1H-pyrazole-4-carboxylate (Example 162A, Enantiomer 2, 43.0 mg, 71.0 µmol)) in THF/methanol (10:1, 2.6 ml). The resulting mixutre was stirred overnight at room temperature. The reaction mixture was acidified with an aqueous solution of hydrogen chloride (1 N), diluted with water. The aqueous solution was extracted ethyl acetate. The combined organic layers were dried over sodium sulfate and evaporated. The residue was lyophilized from acetonitrile/water affording 38.0 mg (83 % yield) of the title compound.
LC-MS (Method 3): Rₜ = 1.72 min; MS (ESIpos): m/z = 578 [M-HCl+H]⁺
¹H-NMR (400 MHz, DMSO-d6) δ [ppm]: -0.150 (0.47), -0.008 (3.61), 0.008 (3.85), 0.146 (0.47), 0.886 (0.59), 1.128 (0.66), 1.169 (1.00), 1.235 (0.88), 1.263 (7.29), 1.281 (16.00), 1.300 (7.60), 1.356 (5.46), 1.506 (1.14), 1.537 (1.23), 1.733 (1.66), 1.766 (1.31), 1.906 (1.12), 1.938 (1.38), 1.978 (2.21), 2.183 (0.64), 2.328 (0.78), 2.366 (0.85), 2.609 (1.21), 2.638 (2.14), 2.665 (1.76), 2.710 (0.95), 3.056 (2.94), 3.082 (5.79), 3.109 (4.56), 3.175 (4.32), 3.193 (4.20), 3.269 (3.61), 3.535 (0.85), 3.900 (0.71), 4.461 (1.00), 4.487 (1.76), 4.513 (0.93), 6.511 (0.40), 6.870 (0.47), 7.086 (8.09), 7.108 (8.64), 7.316 (6.39), 7.360 (2.47), 7.380 (7.31), 7.395 (6.96), 7.416 (1.88), 7.524 (4.08), 7.553 (9.73), 7.575 (8.66), 7.655 (1.71), 7.962 (9.95).

### Comparative Example 174 (WO2012/058132)

### 1-{1-[4-Chloro-4'-(4-cyclopropylmethylpiperazin-1-yl)[biphenyl]-2-yl]piperidin-3-yl}-5-(trifluoromethyl)-1H-pyrazole-4-carboxylic acid

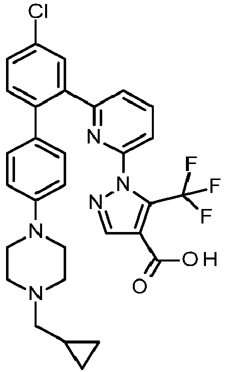

The compound was synthesized according to the procedures disclosed in WO 2012/058132 (experimental part, pages 58 to 84).

### BIOLOGICAL ASSAYS

### B. Assessment of pharmacological efficacy and pharmacokinetic profile

The following abbreviations are used:
- ATP: adenosine triphosphate
- Brij35: polyoxyethylene(23) lauryl ether
- BSA: bovine serum albumin:
- DTT: dithiothreitol
- TEA: triethanolamine

### Biological investigations

The example testing experiments described herein serve to illustrate the present invention and the invention is not limited to the examples given.

The following assays can be used to illustrate the commercial utility of the compounds according to the present invention.

Examples were tested in selected biological assays one or more times. When tested more than once, data are reported as either average values or as median values, wherein
- the average value, also referred to as the arithmetic mean value, represents the sum of the values obtained divided by the number of times tested, and
- the median value represents the middle number of the group of values when ranked in ascending or descending order. If the number of values in the data set is odd, the median is the middle value. If the number of values in the data set is even, the median is the arithmetic mean of the two middle values.

Examples were synthesized one or more times. When synthesized more than once, data from biological assays represent average values calculated utilizing data sets obtained from testing of one or more synthetic batch.

The *in vitro* activity of the compounds of the present invention can be demonstrated in the following assays.

### Biological assays

### B-1. Effect on a recombinant guanylate cyclase reporter cell line

The cellular activity of the compounds according to the invention was determined using a recombinant guanylate cyclase reporter cell line, as described in F. Wunder et al., Anal. Biochem. 339, 104-112 (2005).

Representative MEC values (MEC = minimum effective concentration) and EC₅₀ values (half maximal effective concentration) for the compounds of the invention are shown in the table below (in some cases as mean values from individual determinations):

**Table 2:**

| Example | MEC [nM] | EC₅₀ [nM] |
|---|---|---|
| 1 | 10.0 | 96.5 |
| 2 | 7.0 | 25.2 |
| 3 | 3.0 | 24.6 |
| 4 | 1.3 | 13.0 |
| 5 | 0.3 | 3.2 |
| 6 | 3.0 | 33.5 |
| 7 | 10.0 | 43.3 |
| 8 | 3.0 | 24.5 |
| 9 | 3.0 | 36.5 |
| 10 | 0.8 | 9.2 |
| 11 | < 0.3 | 3.6 |
| 12 | 10.0 | 61.5 |
| 13 | 3.0 | 89.0 |
| 14 | 1.0 | 13.2 |
| 15 | 0.6 | 6.7 |
| 16 | 0.3 | 2.6 |
| 17 | 1.0 | 10.2 |
| 18 | 0.3 | < 1.0 |
| 19 | 0.3 | 1.9 |
| 20 | 1.6 | 19.3 |
| 21 | 1.6 | 13.7 |
| 22 | < 0.3 | 1.0 |
| 23 | < 0.3 | 1.0 |
| 24 | 0.3 | 1.8 |
| 25 | 0.3 | 2.4 |
| 26 | 1.0 | 6.5 |
| 27 | 0.3 | 2.8 |
| 28 | 0.3 | 0.8 |
| 29 | 0.3 | 2.0 |
| 30 | 0.3 | 2.0 |
| 31 | 1.0 | 5.1 |
| 32 | 0.3 | 2.7 |
| 33 | 0.3 | 4.1 |
| 34 | 0.3 | 3.4 |
| 35 | 2.0 | 11.3 |
| 36 | 1.0 | 4.2 |
| 37 | 1.0 | 2.1 |
| 38 | 1.0 | 7.1 |
| 39 | 1.0 | 14.5 |
| 40 | 1.0 | 11.8 |
| 41 | 1.0 | 8.3 |
| 42 | 30.0 | 71.0 |
| 43 | 10.0 | 42.5 |
| 44 | 10.0 | 45.0 |
| 45 | 3.0 | 42.5 |
| 46 | 0.3 | 1.7 |
| 47 | 3.0 | 18.0 |
| 48 | 3.0 | 7.5 |
| 49 | 3.0 | 13.9 |
| 50 | 0.3 | 1.9 |
| 51 | 3.0 | 28.4 |
| 52 | | |
| 53 | 10.0 | 34.0 |
| 54 | 3.0 | 8.4 |
| 55 | 10.0 | 56.5 |
| 56 | 1.0 | 1.5 |
| 57 | 0.3 | < 1.0 |
| 58 | 3.0 | 31.5 |
| 59 | 3.0 | 8.7 |
| 60 | 30.0 | 70.0 |
| 61 | 10.0 | 28.5 |
| 62 | 3.0 | 7.5 |
| 63 | 10.0 | 68.0 |
| 64 | 1.0 | 1.9 |
| 65 | 1.0 | 6.9 |
| 66 | < 0.3 | < 1.0 |
| 67 | < 0.3 | < 1.0 |
| 68 | 30.0 | 68.0 |
| 69 | 30.0 | 45.5 |
| 70 | 3.0 | 21.5 |
| 71 | 30.0 | 92.0 |
| 72 | 3.0 | 18.0 |
| 73 | 10.0 | 66.0 |
| 74 | 10.0 | 79.5 |

### B-2. Vasorelaxant effect in vitro

Rabbits were killed in deep anaesthesia and exsanguinated. The aorta was removed, freed from adhering tissue and divided into rings of width 1.5 mm, which were placed individually under prestress into 10 ml organ baths with carbogen-sparged Krebs-Henseleit solution at 37°C having the following composition (each in mM): sodium chloride: 119; potassium chloride: 4.8; calcium chloride dihydrate: 1; magnesium sulfate heptahydrate: 1.4; potassium dihydrogenphosphate: 1.2; sodium bicarbonate: 25; glucose: 10. To generate a contraction, phenylephrine was added to the bath cumulatively in increasing concentration. After several control cycles, the substance to be studied was added in increasing dosage each time in every further run, and the magnitude of the contraction was compared with the magnitude of the contraction attained in the last preceding run. This was used to calculate the concentration needed to reduce the magnitude of the control value by 50% (IC₅₀ value). The standard administration volume was 5 µl; the DMSO content in the bath solution corresponds to 0.1%.

### B-3. Blood pressure measurement on anaesthetized rats

Male Wistar rats having a body weight of 300-350 g were anaesthetized with thiopental (100 mg/kg i.p.). After tracheotomy, a catheter was introduced into the femoral artery to measure the blood pressure. The substances to be tested were administered as solutions, either orally by means of a gavage or intravenously via the femoral vein (Stasch et al. Br. J. Pharmacol. 2002; 135: 344-355).

### B-4. Radiotelemetry measurement of blood pressure in conscious, spontaneously hypertensive rats

A commercially available telemetry system from DATA SCIENCES INTERNATIONAL DSI, USA, was employed for the blood pressure measurement on conscious rats described below.

The system consists of 3 main components:
implantable transmitters (Physiotel^{®} telemetry transmitter)
receivers (Physiotel^{®} receiver) which are linked via a multiplexer (DSI Data Exchange Matrix 2.0) to a data acquisition computer.

The telemetry system makes it possible to continuously record blood pressure, heart rate and body motion of conscious animals in their usual habitat.

### Animal material

The studies were conducted on adult female spontaneously hypertensive rats (SHR Okamoto) with a body weight of > 200 g. SHR/NCrl from the Okamoto Kyoto School of Medicine, 1963, were a cross of male Wistar Kyoto rats having greatly elevated blood pressure and female rats having slightly elevated blood pressure, and were handed over at F13 to the U.S. National Institutes of Health.

After transmitter implantation, the experimental animals were housed singly in type 3 Makrolon cages. They had free access to standard feed and water.

The day/night rhythm in the experimental laboratory was changed by the room lighting at 6:00 am and at 7:00 pm.

### Transmitter implantation

The HD S 10 telemetry transmitters used were surgically implanted under aseptic conditions in the experimental animals at least 14 days before the first experimental use. The animals instrumented in this way can be used repeatedly after the wound has healed and the implant has settled.

For the implantation, the fasted animals were anesthetized with isoflurane (Rimadyl analgesia) and shaved and disinfected over a large area of their abdomens. After the abdominal cavity has been opened along the linea alba, the liquid-filled measuring catheter of the system was inserted into the descending aorta in the cranial direction above the bifurcation and fixed with tissue glue (VetBonD TM, 3M). The transmitter housing was fixed intraperitoneally to the abdominal wall muscle, and the wound was closed layer by layer.

An antibiotic (Ursocyclin 10% pro inj., Serumwerk, s.c.) was administered postoperatively for prophylaxis of infection.

### Substances and solutions

Unless stated otherwise, the substances to be studied were administered orally by gavage to a group of animals in each case (n = 6). In accordance with an administration volume of 2ml/kg of body weight, the test substances were dissolved in suitable solvent mixtures or suspended in 0.5% tylose.

A solvent-treated group of animals was used as control.

### Experimental procedure

The telemetry measuring unit present was configured for 24 animals. Each experiment was recorded under an experiment number (Vyear month day).

Each of the instrumented rats living in the system was assigned a separate receiving antenna (RPC-1 Receiver, DSI).

The implanted transmitters can be activated externally by means of an incorporated magnetic switch. They were switched to transmission in the run-up to the experiment. The signals emitted can be detected online by a data acquisition system (Physio Tel HD, DSI) and processed accordingly. The data were stored in each case in a file created for this purpose and bearing the experiment number.

In the standard procedure, the following were measured for 10-second periods in each case:
systolic blood pressure (SBP)
diastolic blood pressure (DBP)
mean arterial pressure (MAP)
heart rate (HR)
activity (TEMP).

The acquisition of measurements was repeated under computer control at 5-minute intervals. The source data obtained as absolute values were corrected in the diagram with the currently measured barometric pressure (Ambient Pressure Reference Monitor; APR-1) and stored as individual data. Further technical details were given in the extensive documentation from the manufacturer company (DSI).

Unless indicated otherwise, the test substances were administered at 9:00 am on the day of the experiment. Following the administration, the parameters described above were measured over 24 hours.

### Evaluation

After the end of the experiment, the acquired individual data were sorted using the analysis software (Ponemah V 6.x). The blank value was assumed here to be the time 2 hours before administration, and so the selected data set encompasses the period from 7:00 am on the day of the experiment to 9:00 am on the following day.

The data were smoothed over a predefinable period by determination of the average (30-minute average) and transferred as an excel file to a storage medium. The measured values presorted and compressed in this way were transferred to Excel templates and tabulated. For each day of the experiment, the data obtained were stored in a dedicated file bearing the number of the experiment. Results and test protocols were stored in files in paper form sorted by numbers.

### Literature:

Klaus Witte, Kai Hu, Johanna Swiatek, Claudia Müssig, Georg Ertl and Björn Lemmer: Experimental heart failure in rats: effects on cardiovascular circadian rhythms and on myocardial β-adrenergic signaling. Cardiovasc Res 47 (2): 203-405, 2000; Kozo Okamoto: Spontaneous hypertension in rats. Int Rev Exp Pathol 7: 227- 270, 1969; Maarten van den Buuse: Circadian Rhythms of Blood Pressure, Heart Rate, and Locomotor Activity in Spontaneously Hypertensive Rats as Measured With Radio-Telemetry. Physiology & Behavior 55(4): 783-787, 1994.

### B-5. Determination of pharmacokinetic parameters following intravenous and oral administration

The pharmacokinetic parameters of the compounds according to the invention were determined in male Wistar rats and and/or in female beagles and/or in cynomolgus monkeys and/or in male CD-1 mice. Intravenous administration in the case of mice and rats was carried out by means of a species-specific plasma/DMSO formulation, and in the case of dogs and monkeys by means of a water/PEG400/ethanol formulation. In all species, oral administration of the dissolved substance was performed via gavage, based on a water/PEG400/ethanol formulation.

An internal standard (which may also be a chemically unrelated substance) was added to the samples of the compounds of the invention, calibration samples and qualifiers, and there followed protein precipitation by means of acetonitrile in excess. Addition of a buffer solution matched to the LC conditions, and subsequent vortexing, was followed by centrifugation at 1000 g. The supernatant was analysed by LC-MS/MS using C18 reversed-phase columns and variable mobile phase mixtures. The substances were quantified via the peak heights or areas from extracted ion chromatograms of specific selected ion monitoring experiments.

The plasma concentration/time plots determined were used to calculate the pharmacokinetic parameters such as AUC, Cₘₐₓ, t_{1/2} (terminal half-life), F (bioavailability), MRT (mean residence time) and CL (clearance), by means of a validated pharmacokinetic calculation program

Since the substance quantification was performed in plasma, it was necessary to determine the blood/plasma distribution of the substance in order to be able to adjust the pharmacokinetic parameters correspondingly. For this purpose, a defined amount of substance was incubated in K3 EDTA whole blood of the species in question in a rocking roller mixer for 20 min. After centrifugation at 1000 g, the plasma concentration was measured (by means of LC-MS/MS; see above) and determined by calculating the ratio of the C_{blood}/Cₚₗₐₛₘₐ value.

Table 3 shows data of representative compounds of the present invention following intravenous administration in rats:

**Table 3:**

| Example | AUCnorm [kg·h/L] | CLplasma [L/h/kg] | t1/2 [h] | MRT [h] |
|---|---|---|---|---|
| 20 | 2.29 | 0.44 | 5.13 | 4.16 |
| 21 | 7.23 | 0.14 | 3.83 | 4.41 |
| 11 | 1.23 | 0.81 | 2.43 | 2.88 |
| 19 | 3.09 | 0.32 | 4.24 | 5.66 |
| 5 | 1.40 | 0.72 | 2.09 | 2.62 |
| 174 (WO20 12/ 058132) | 0.77 | 1.30 | 2.33 | 2.78 |

Table 4 shows data of representative compounds of the present invention following oral administration in rats:

**Table 4:**

| Example | AUCnorm [kg·h/L] | t1/2 [h] | MRT [h] | F [%] |
|---|---|---|---|---|
| 20 | 2.11 | 7.37 | 14.2 | 91.7 |
| 21 | 4.04 | 3.63 | 7.32 | 56.1 |
| 11 | 0.70 | 3.76 | 7.53 | 58.2 |
| 19 | 0.25 | 4.65 | 7.46 | 8.01 |
| 5 | 0.84 | 3.02 | 5.91 | 59.9 |
| 174 (WO2012/ 058132) | 0.63 | 3.60 | 8.41 | 81.8 |

The compounds according to the present invention show similar to superior pharmacokinetic (PK) properties in comparison to compounds disclosed in the prior art (WO 2012/058132, e.g. ex. 174) (see experimental part, tables 3 to 4). The shown examples show a lower plasma clearance (CL_{Plasma}) and therefore a higher exposure (AUCₙₒᵣₘ) in comparison to the prior art compound disclosed as example 174 in WO 2012/058132 in rats. Half live and mean residence time (MRT) of most examples are in a comparable range after i.v. application. However example 20 shows a longer half live and mean residence time (MRT). After p.o. application in rats (see experimental part, table 4) most examples (except example 19) do show comparable to higher exposures (AUCₙₒᵣₘ) in comparison to the prior art compound disclosed as example 174 in WO 2012/058132.

### B-6. Metabolic study

To determine the metabolic profile of the inventive compounds, they were incubated with recombinant human cytochrome P450 (CYP) enzymes, liver microsomes or primary fresh hepatocytes from various animal species (e.g. rats, dogs), and also of human origin, in order to obtain and to compare information about a very substantially complete hepatic phase I and phase II metabolism, and about the enzymes involved in the metabolism.

The compounds of the invention were incubated with a concentration of about 0.1-10 µM. To this end, stock solutions of the compounds of the invention having a concentration of 0.01-1 mM in acetonitrile were prepared, and then pipetted with a 1:100 dilution into the incubation mixture. The liver microsomes and recombinant enzymes were incubated at 37°C in 50 mM potassium phosphate buffer pH 7.4 with and without NADPH-generating system consisting of 1 mM NADP⁺, 10 mM glucose-6-phosphate and 1 unit glucose-6-phosphate dehydrogenase. Primary hepatocytes were incubated in suspension in Williams E medium, likewise at 37°C. After an incubation time of 0-4 h, the incubation mixtures were stopped with acetonitrile (final concentration about 30%) and the protein was centrifuged off at about 15 000 x g. The samples thus stopped were either analyzed directly or stored at -20°C until analysis.

The analysis was carried out by high-performance liquid chromatography with ultraviolet and mass spectrometry detection (HPLC-UV-MS/MS). To this end, the supernatants of the incubation samples were chromatographed with suitable C18 reversed-phase columns and variable mobile phase mixtures of acetonitrile and 10 mM aqueous ammonium formate solution or 0.05% formic acid. The UV chromatograms in conjunction with mass spectrometry data serve for identification, structural elucidation and quantitative estimation of the metabolites, and for quantitative metabolic reduction of the compound of the invention in the incubation mixtures.

### B-7. Caco-2 permeability test

The permeability of a test substance was determined with the aid of the Caco-2 cell line, an established *in vitro* model for permeability prediction at the gastrointestinal barrier (Artursson, P. and Karlsson, J. (1991). Correlation between oral drug absorption in humans and apparent drug permeability coefficients in human intestinal epithelial (Caco-2) cells. Biochem. Biophys.175 (3), 880-885). The Caco-2 cells (ACC No. 169, DSMZ, Deutsche Sammlung von Mikroorganismen und Zellkulturen, Braunschweig, Germany) were sown in 24-well plates having an insert and cultivated for 14 to 16 days. For the permeability studies, the test substance was dissolved in DMSO and diluted to the final test concentration with transport buffer (Hanks Buffered Salt Solution, Gibco/Invitrogen, with 19.9 mM glucose and 9.8 mM HEPES). In order to determine the apical to basolateral permeability (PₐₚₚA-B) of the test substance, the solution comprising the test substance was applied to the apical side of the Caco-2 cell monolayer, and transport buffer to the basolateral side. In order to determine the basolateral to apical permeability (PₐₚₚB-A) of the test substance, the solution comprising the test substance was applied to the basolateral side of the Caco-2 cell monolayer, and transport buffer to the apical side. At the start of the experiment, samples were taken from the respective donor compartment in order to ensure the mass balance. After an incubation time of two hours at 37°C, samples were taken from the two compartments. The samples were analyzed by means of LC-MS/MS and the apparent permeability coefficients (Pₐₚₚ) were calculated. For each cell monolayer, the permeability of Lucifer Yellow was determined to ensure cell layer integrity. In each test run, the permeability of atenolol (marker for low permeability) and sulfasalazine (marker for active excretion) was also determined as quality control.

### B-8. Solubility determination of substances in buffer pH 6.5

2 - 4 mg of the test compound were dissolved in DMSO to reach a concentration of 50 g/L (solution A, 515 µg/l). To 10 µl of this solution 960 µl PBS buffer pH 6.5 were added; the mixture was shaken for 24h at rt in a 96 well plate. An aliquot was centrifuged at 42000 rpm for 30 min. The supernatant was diluted with ACN/water (8:2) 1:10 and 1:1000 resp. This diluted samples were analyzed by LC-MSMS.

*Calibration:* 10 µl of solution A were diluted with 823 µl DMSO (final concentration: 600 µg/ml), which was further diluted with ACN/water 8:2 by a factor of 100 (solution B).

The calibration curve was obtained from solution B by further diluting with ACN/water 8:2 with target concentrations of 1.2 -12 - 60 - 600 ng/ml and injecting these four solutions for MS measurement.

### MS method optimization:

Solution B was utilized for MS method optimization.

*PBS-Puffer:* 6.18 g sodium chloride and 3.96 g sodium dihydrogen phosphate were dissolved in 1L aqua dist., the pH was adjusted to 6.5 with IN sodium hydroxide.

### LC-MSMS optimization:

The following configurations were used for optimization

AB Sciex TRIPLE QUAD 4500, Agilent 1260 Infinity (G1312B), degasser (G4225A), column oven (G1316C or G1316A), CTC Analytics PAL injection system HTS-xt or HTC-xt.

Eluent A: 0.5 ml formic acid (50%ig)/ L water, Eluent B: 0.5 ml formic acid (50%ig) / L acetonitrile

| time [min] | flow [µl/min] | %B |
|---|---|---|
| 0.00 | 200 | 70 |
| 0.08 | 200 | 70 |
| 0.09 | 25 | 70 |
| 0.60 | 25 | 70 |
| 0.65 | 200 | 70 |
| 1.10 | 200 | 70 |

| | |
|---|---|
| Autosampler: | without auto inject ahead setting |
| column: | stainless steel capillary |
| oven temperature: | 22°C |
| flow rate: | flow gradient |
| injected volume: | 2 µl |

Water Quattro Micro MS, Agilent 1100 (G1312A), degasser (G1322A), column oven (G1316A), CTC Analytics PAL injection system HTS, eluents as above

| time [min] | flow [µl/min] | % B |
|---|---|---|
| 0.00 | 250 | 70 |
| 1.50 | 250 | 70 |

| | |
|---|---|
| Autosampler: | with auto inject ahead setting |
| column: | stainless steel capillary |
| oven temperature: | 22°C |
| flow rate: | flow gradient |
| injected volume: | 5 µl |

MS method: Flow Injection Analysis (FIA) for optimization ("MS-OPTI"); Ionization mode ABSciex-MS: ESI-pos/neg, Waters-MS: ESI-pos

### HPLC method for MSMS quantification:

### Eluent A, B as above

### ABSciex-MS

| time [min] | % A | % B |
|---|---|---|
| 0 | 90 | 10 |
| 0.5 | 5 | 95 |
| 0.84 | 5 | 95 |
| 0.85 | 90 | 10 |
| 1.22 | 90 | 10 |

| | |
|---|---|
| Autosampler: | without auto inject ahead setting |
| column: | Waters OASIS HLB, 2,1 x 20 mm, 25 µ |
| column temperature: | 30°C |
| flow rate: | 2.5 ml |
| injected volume: | 2 µl |
| Splitter (before MS) | 1:20 |

### Waters-MS

| time [min] | % A | % B |
|---|---|---|
| 0 | 90 | 10 |
| 0.5 | 5 | 95 |
| 0.84 | 5 | 95 |
| 0.85 | 90 | 10 |
| 1.5 | 90 | 10 |

| | |
|---|---|
| Autosampler: | with auto inject ahead setting |
| column: | Waters OASIS HLB, 2,1 x 20 mm, 25 µ |
| column temperature: | 30°C |
| flow rate: | 2.5 ml |
| injected volume: | 5 µl |
| Splitter (before MS) | 1:20 |
| MS method: | Multiple Reaction Monitoring (MRM) |

### B-9. Determination of solubility from solid

For each solvent, an Eppendorf plastic vial was charged with 0.5 - 1 mg of the test compound (exact weight), 2-3 glass pearls (diameter 3 mm) and 1.0 ml of the respective solvent. The vial was closed and shaken at RT for 24 h (1400 rpm; Thermomixer, Eppendorf). Thereafter, 230 µl each of the solution/suspension was transferred into one or more centrifuge vials (Beckman Coulter) and were centrifuged at 42000 rpm for 30 min (Beckman Coulter Optima L90). At least 100 µl of the supernatant were withdrawn and further diluted with DMSO in two dilution strength: 1:5 and 1:50 (the latter obtained from the 1:5 dilution step by subsequent DMSO addition). This liquid handling was done either manually or with the help of a pipetting robot (Lissy, Zinsser Analytic).

For HPLC quantification, calibration solutions of the test compound in DMSO were prepared. Starting from an initial concentration of 600 µg/ml, three calibration solutions were prepared: 100 µg/ml, 20 µg/ml and 2.5 µg/ml (manually or via Lissy).

Both calibration solutions and the supernatant were analyzed by HPLC/UV-detection at an appropriate wave length. The solubility was determined using the linear calibration curve.

### HPLC systems:

Hewlett Packard / Agilent HPLC systems, G1311A+G1316A+G1315B as well as G1312A+G1316A+G1315A
injector system: CTC-Analytik HTC PAL
or with a Agilent UPLC System (G7117C, G7116B, G7167B and G7120)
oven temperature: 30°C, detection: 210 and/or 254 nm, injected volume: 20µl
eluent A: 0.1% TFA in water, eluent B: 0.1% TFA in acetonitrile
column: ZORBAX Extend-C18, 3.0 x 50mm, 3.5µm
Gradient:

| time [min] | A [%] | B [%] | Flow rate: [ml/min] |
|---|---|---|---|
| 0.0 | 98 | 2 | 1.5 |
| 0.2 | 98 | 2 | 1.5 |
| 3.3 | 10 | 90 | 1.5 |
| 4.0 | 10 | 90 | 1.5 |
| 4.1 | 98 | 2 | 2.5 |
| 4.7 | 98 | 2 | 2.5 |
| 5.0 | 98 | 2 | 1.5 |

## Claims

1. Compound of the formula (I) 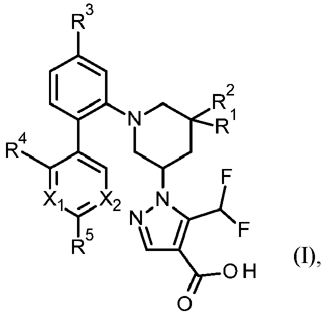 in which
R¹ represents hydrogen or halogen,
R² represents hydrogen or halogen,
R³ represents chloro or trifluoromethyl,
R⁴ represents hydrogen or C₁-C₄-alkyl
R⁵ represents a group of the formula 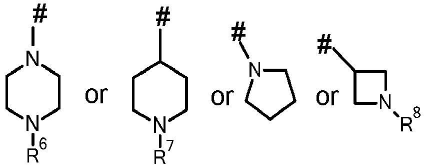
where # is the point of attachment to the aromatic or heteroaromatic 6 ring system
R⁶ represents
C₁-C₆-alkyl, substituted by one or more substituent independently selected from the group consisting of trifluoromethoxy, nitril, amido,
C₂-C₆-halogenoalkyl, substituted by 1 to 5 fluoro substituents,
C₃-C₆-cycloalkyl,
C₃-C₆-cycloalkyl-methyl, optionally substituted by 1 to 5 fluoro substituents or a trifluoromethyl group,
C₁-C₆-alkylcarbonyl, optionally substituted by 1 to 3 fluoro substituents,
C₃-C₆-cycloalkyl-carbonyl, optionally substituted by 1 to 3 fluoro substituents,
oxetanyl,
spiro[2.2]pentan-2-ylmethyl or [(3-fluoro-1-bicyclo[1.1.1]pentanyl)methyl,
R⁷ represents C₁-C₄-alkylcarbonyl, optionally substituted by a C₃-C₆-cycloalkyl group,
R⁸ represents C₂-C₄- halogenoalkyl substituted by 1 to 6 fluoro substituents,
X₁ represents nitrogen or carbon,
X₂ represents nitrogen or carbon
or one of the salts thereof, solvates thereof or solvates of the salts thereof.

2. Compound according to Claim 1, **characterized in that**
R¹ represents hydrogen, fluorine
R² represents hydrogen, fluorine
R³ represents chloro or trifluoromethyl,
R⁴ represents hydrogen or methyl
R⁵ represents a group of the formula 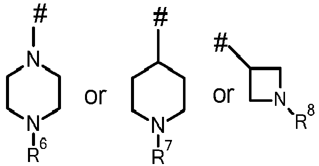
where # is the point of attachment to the aromatic or heteroaromatic 6 ring system
R⁶ represents
C₁-C₄-alkyl, substituted by one or more substituent independently selected from the group consisting of trifluoromethoxy, nitril,
C₂-C₆-halogenoalkyl, substituted by 1 to 5 fluoro substituents,
C₃-C₆-cycloalkyl-methyl, optionally substituted by 1 to 2 fluoro substituents or a trifluoromethyl group,
C₁-C₃-alkylcarbonyl, optionally substituted by 1 to 3 fluoro substituents, C₃ -C₆-cycloalkyl-carbonyl,
R⁷ represents C₁-C₃-alkylcarbonyl, optionally substituted by cyclopropyl
R⁸ represents C₂-C₄-halogenoalkyl, optionally substituted by 1 to 3 fluoro substituents,
X₁ represents nitrogen or carbon
X₂ represents nitrogen or carbon
or one of the salts thereof, solvates thereof or solvates of the salts thereof.

3. Compound according to Claim 1 or 2, **characterized in that**
R¹ represents hydrogen
R² represents hydrogen
R³ represents chloro
R⁴ represents hydrogen
R⁵ represents a group of the formula 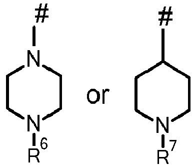
where # is the point of attachment to the aromatic or heteroaromatic 6 ring system
R⁶ represents C₁-C₄-alkyl, substituted by trifluoromethoxy or nitril, C₂-C₃-halogenoalkyl, substituted by 1 to 5 fluoro substituents, C₃-C₄-cycloalkyl-methyl, optionally substituted by 1 to 2 fluoro substituents or a trifluoromethyl group, C₁-C₃-alkylcarbonyl, optionally substituted by 1 to 3 fluoro substituents, cyclopropyl-carbonyl,
R⁷ represents C₁-C₃-alkylcarbonyl, optionally substituted by cyclopropyl
X₁ represents carbon
X₂ represents carbon
or one of the salts thereof, solvates thereof or solvates of the salts thereof.

4. A process for preparing a compound of the formula (I) or one of the salts thereof, solvates thereof or solvates of the salts thereof according to one of Claims 1 to 3, **characterized in that**
in a first step [B] the compounds of the formula (III) 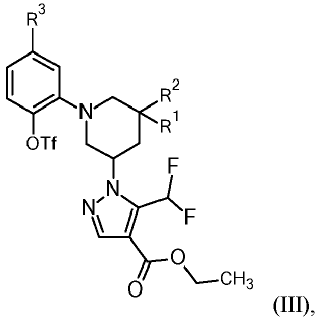 in which R¹, R² and R³ are defined as above,
are reacted with compounds of the formula (IV) 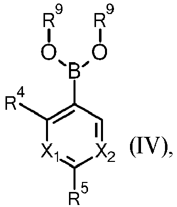 in which R⁴, R⁵, and X₁ and X₂ are defined as above,
and
in which R⁹ represents hydrogen, methyl, or both R⁹ form via the adjacent oxygen atoms a 4,4,5,5-tetramethyl-1,3,2-dioxaborolane
in the presence of a palladium source, a suitable ligand and a base to provide compounds of the formula (II) 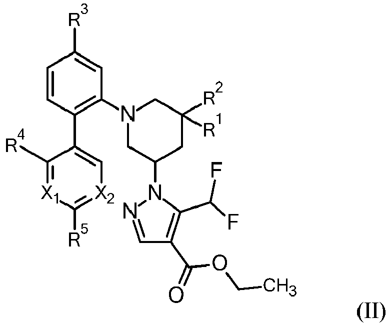 in which R¹, R² , R³, R⁴, R⁵ and X₁ and X₂ are defined as above
and
in a second step [A]
compounds of formula (II) are reacted with a base to provide compounds of the formula (I), 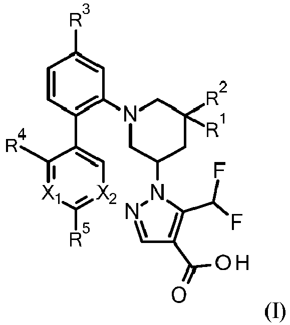 in which R¹, R², R³, R⁴, R⁵ and X₁ and X₂ are defined as above
optionally compounds of formula (I) are transferred into the corresponding salts of formula (Ia) 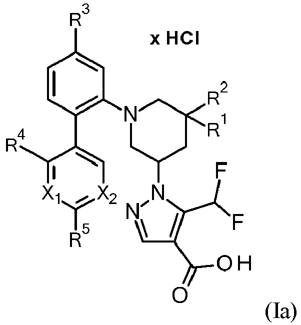 in the presence of a suitable acid in a suitable solvent.

5. A compound according to any of Claims 1 to 3 for the treatment and/or prophylaxis of diseases.

6. A compound according to any of Claims 1 to 3 for the treatment and/or prophylaxis of heart failure (HFrEF and HFpEF), hypertension (HTN), chronic and diabetic kidney disease (CKD, DKD), pulmonary hypertension (PH), systemic sclerosis (SSc), sickle cell disease (SCD), neurodegenerative diseases and dementias, and diabetic foot ulcer (DFU).

7. Use of a compound according to any of Claims 1 to 3 for producing a medicament for the treatment and/or prophylaxis of diseases.

8. Use of a compound according to any of Claims 1 to 3 for producing a medicament for the treatment and/or prophylaxis of heart failure (HFrEF and HFpEF), hypertension (HTN), chronic and diabetic kidney disease (CKD, DKD), pulmonary hypertension (PH), systemic sclerosis (SSc), sickle cell disease (SCD), neurodegenerative diseases and dementias, and diabetic foot ulcer (DFU).

9. Medicament comprising a compound according to any of Claims 1 to 3 in combination with an inert, nontoxic, pharmaceutically suitable excipient.

10. Medicament according to Claim 9 for the treatment and/or prophylaxis of heart failure (HFrEF and HFpEF), hypertension (HTN), chronic and diabetic kidney disease (CKD, DKD), pulmonary hypertension (PH), systemic sclerosis (SSc), sickle cell disease (SCD), neurodegenerative diseaseses and dementias, and diabetic foot ulcer (DFU).

11. Method for the treatment and/or prophylaxis of heart failure (HFrEF and HFpEF), hypertension (HTN), chronic and diabetic kidney disease (CKD, DKD), pulmonary hypertension (PH), systemic sclerosis (SSc), sickle cell disease (SCD), and diabetic foot ulcer (DFU) in humans and animals by administration of a therapeutically effective amount of at least one compound according to any of Claims 1 to 3, of a medicament according to Claim 9 or 10 or of a medicament obtained according to Claim 7 or 8.
